(19) Europäisches Patentamt
European Patent Office
Office européen des brevets

(11) EP 2 561 867 A1

(12) **EUROPEAN PATENT APPLICATION**

(43) Date of publication:
**27.02.2013 Bulletin 2013/09**

(21) Application number: **11178328.8**

(22) Date of filing: **22.08.2011**

(51) Int Cl.:
***A61K 31/00*** *(2006.01)* ***A61K 31/4418*** *(2006.01)*
***A61K 31/53*** *(2006.01)* *A61P 35/00 (2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Lead Discovery Center GmbH**
  **44227 Dortmund (DE)**
- **Max-Planck-Gesellschaft zur Förderung der Wissenschaften e.V.**
  **80539 München (DE)**

(72) Inventors:
- **Choidas, Axel**
  **58313 Herdecke (DE)**
- **Klebl, Bert**
  **44229 Dortmund (DE)**
- **Habenberger, Peter**
  **44139 Dortmund (DE)**
- **Eickhoff, Jan**
  **58131 Herdecke (DE)**
- **Thomas, Roman**
  **53332 Bornheim (DE)**
- **Heuckmann, Johannes**
  **50937 Köln (DE)**

(74) Representative: **Vossius & Partner**
**Siebertstrasse 4**
**81675 München (DE)**

(54) **CDK9 inhibitors in the treatment of midline carcinoma**

(57) The present invention relates to a CDK9 inhibitor, especially a selective CDK9 inhibitor, for use in treating, ameliorating and/or preventing midline carcinoma. Also corresponding methods for treating, preventing or ameliorating midline carcinoma are subject of the present invention. Preferably, NUT midline carcinoma is treated with the CDK9 inhibitors in accordance with the present invention.



Printed by Jouve, 75001 PARIS (FR)

SEQUENCE LISTING

<110> Lead Discovery Center GmbH
Max-Planck-Gesellschaft zur FÃ¶rderung der Wissenschaften e.V
.

<120> CDK9 inhibitors in the treatment of midline carcinoma

<130> T2092 EP S3

<160> 12

<170> BiSSAP 1.0

<210> 1
<211> 3795
<212> DNA
<213> Homo sapiens

<220>
<221> source
<222> 1..3795
<223> /mol_type="DNA"
/organism="Homo sapiens"

<400> 1
gagttccgta ttctagttct gtgtgatctg atctttacct tcccttcctt ggatccctgt 60

gcacctactg gagccaggtt actctgggtc ctggacctga ctgcctcatt ctggaggctt 120

ccagacagcc acagttagtg cccaaacctg agaggatggc ttcagatgga gcatctgcat 180

tgccgggacc ggatatgagc atgaaaccta gtgccgccct gtctccatcc cctgcacttc 240

cctttctccc accaacttct gacccaccag accacccacc cagggagcca cctccacagc 300

ccatcatgcc ttcagtattc tctccagaca accctctgat gctctctgct ttccccagct 360

cactgttggt gacaggggac gggggccctt gcctcagtgg ggctggggct ggcaaggtca 420

ttgtcaaagt caagacagaa gggggggtcag ctgagccctc tcaaactcag aactttatcc 480

ttactcagac tgccctcaat tcgactgccc cgggcactcc ctgtggaggc cttgagggtc 540

ctgcacctcc atttgtgaca gcatctaatg tgaagaccat tctgccctct aaggctgttg 600

gtgtcagcca ggagggtcct ccaggccttc cgcctcagcc tccaccacca gttgctcaac 660

tggtccccat tgtgcccctg gaaaaagctt ggccagggcc acatgggaca accggggaag 720

gaggtcctgt ggccactcta tccaagcctt ccctaggtga ccgctccaaa atttccaagg 780

acgtttatga gaacttccgt cagtggcagc gttacaaagc cttggcccgg aggcacctat 840

cccagagtcc tgacacagaa gctctttcct gttttcttat cccagtgctt cgttccctgg 900

cccggctgaa gcccactatg accctggagg agggactgcc attggctgtg caggagtggg 960

agcacaccag caactttgac cggatgatct tttatgagat ggcagaaagg ttcatggagt 1020

ttgaggctga ggagatgcag attcagaaca cacagctgat gaatgggtct cagggcctgt 1080

ctcctgcaac ccctttgaaa cttgatcctc tagggcccct ggcctctgag gtttgccagc 1140

agccagtgta cattccgaag aaggcagcct ccaagacacg ggcccccgc cggcgtcagc 1200

```
gtaaagccca gagacctcct gctcctgagg cacccaagga gatcccacca gaagctgtga     1260
aggagtatgt tgacatcatg gaatggctgg tggggactca cttggccact ggggagtcag     1320
atggaaaaca agaggaagaa gggcagcagc aggaggagga agggatgtat ccagatccag     1380
gtctcctgag ctacatcaat gagctgtgtt ctcagaaggt ctttgtctcc aaggtggagg     1440
ctgtcattca ccctcaattt ctggcagatc tgctgtcccc agaaaaacag agagatccct     1500
tggccttaat tgaggagcta gagcaagaag aaggactcac tcttgcccag ctggtccaga     1560
agcgactcat ggccttggaa gaggaggaag atgcagaggc gcctccaagt ttcagtggcg     1620
ctcagttgga ctcaagtcct tctggttctg ttgaggatga agatggggat gggcggcttc     1680
ggccctcacc tgggcttcag gggctgggg gcgccgcttg ccttggaaag gtttcttctt      1740
caggaaaacg ggcaagagaa gtgcatggtg ggcaggagca agccctagat agccccagag     1800
ggatgcacag ggatgggaac actctgccat cccccagcag ctgggacctg cagccagaac     1860
ttgcagctcc acagggaact ccgggaccct tgggtgtgga gaggagaggg tctgggaagg     1920
ttataaacca ggtatctcta catcaggatg gccatctagg aggcgctggg cctcctgggc     1980
actgcctggt ggctgatagg acttcagagg ctctgcccct ttgttggcag ggaggcttcc     2040
agcctgagag cactcccagt ttggatgctg gacttgcaga gctggctcct ctgcaaggac     2100
aagggttaga aaagcaagtc ctgggattgc agaaaggaca acaaacaggg ggtcgtggag     2160
tgcttcctca agggaaggag cctttagcag tgccctggga aggctcttca ggagccatgt     2220
ggggagatga cagaggtacc cccatggctc agagttatga tcagaatcct tcccctagag     2280
cagctgggga gagggacgat gtctgtctca gcccaggagt ttggctgagc agtgagatgg     2340
atgctgtagg cttggagctg cctgtacaaa tagaggaggt catagagagc ttccaagttg     2400
agaagtgtgt aactgagtat caggaaggct gccagggact gggctccagg ggcaacattt     2460
ccctgggtcc tggagaaacc ctagtacctg gggatacgga gagcagtgtg attccctgtg     2520
gaggcacagt tgcggcagct gccctagaaa agagaaacta ttgcagcttg ccaggacctt     2580
tgagggccaa cagcccaccc ttgaggtcca aagaaaatca agaacagagc tgtgaaaccg     2640
tagggcatcc cagtgatctg tgggcagaag gttgcttccc attgctagaa agtggtgatt     2700
ccacactggg gtcttccaaa gaaacccttc cacccacatg ccaaggcaat ctccttatca     2760
tggggactga ggatgcctcc tccttgcctg aagccagtca agaggcaggg agcagaggca     2820
attccttttc tcctctgttg gaaaccatag aacctgtcaa catactagat gttaaagatg     2880
actgtggcct ccaactaagg gtcagcgagg acacctgccc actgaatgtt cattcttatg     2940
acccccaagg agaaggcagg gtggatcctg atctgtccaa gcctaaaaac cttgctcctt     3000
tacaagagag tcaggagtct tacacaactg ggactcccaa agcaacatct tctcaccagg     3060
gccttggaag cactttgcct agaaggggaa ccaggaatgc catagttccg agagaaactt     3120
```

ctgttagtaa aacacacagg tcagcagaca gggccaaagg aaaggagaaa aagaaaaagg 3180

aagcagagga agaggatgag gaactctcca actttgctta cctcttggcc tctaaactta 3240

gcctctcacc aagggagcat cccctcagtc ctcaccatgc ctcaggaggt cagggcagcc 3300

agagagcatc ccacctgctc cctgctggag caaaaggccc cagcaaactt ccatatcctg 3360

ttgccaagtc tgggaagcga gctctagctg gaggtccagc ccctactgaa aagacacccc 3420

actcaggagc tcaacttggg gtccccaggg agaaacccct agctctggga gtagttcgac 3480

cctcacagcc tcgtaaaagg cggtgtgaca gttttgtcac gggcagaagg aagaaacgac 3540

gtcgtagcca gtagggagca gcgggaccat ctgaccccac ttgccagtcc ctaaaggtgg 3600

gtgccccaga gtagattcca cccctgctgc ccaccaatgg agaatcccaa tgttgaatct 3660

catcccaatg ttgttttgtt gttctgcaaa agtggcaagc atggagagag aggtcagact 3720

ggctaggctg caggggggaat tacctttgga aggagctata tagaaaaaaa atgaataaag 3780

tgttttgttg gaaaa 3795

<210> 2
<211> 1132
<212> PRT
<213> Homo sapiens

<220>
<221> SOURCE
<222> 1..1132
<223> /mol_type="protein"
/organism="Homo sapiens"

<400> 2
```
Met Ala Ser Asp Gly Ala Ser Ala Leu Pro Gly Pro Asp Met Ser Met
1               5                   10                  15
Lys Pro Ser Ala Ala Leu Ser Pro Ser Pro Ala Leu Pro Phe Leu Pro
            20                  25                  30
Pro Thr Ser Asp Pro Pro Asp His Pro Pro Arg Glu Pro Pro Pro Gln
        35                  40                  45
Pro Ile Met Pro Ser Val Phe Ser Pro Asp Asn Pro Leu Met Leu Ser
    50                  55                  60
Ala Phe Pro Ser Ser Leu Leu Val Thr Gly Asp Gly Gly Pro Cys Leu
65                  70                  75                  80
Ser Gly Ala Gly Ala Gly Lys Val Ile Val Lys Val Lys Thr Glu Gly
                85                  90                  95
Gly Ser Ala Glu Pro Ser Gln Thr Gln Asn Phe Ile Leu Thr Gln Thr
            100                 105                 110
Ala Leu Asn Ser Thr Ala Pro Gly Thr Pro Cys Gly Gly Leu Glu Gly
        115                 120                 125
Pro Ala Pro Pro Phe Val Thr Ala Ser Asn Val Lys Thr Ile Leu Pro
    130                 135                 140
Ser Lys Ala Val Gly Val Ser Gln Glu Gly Pro Pro Gly Leu Pro Pro
145                 150                 155                 160
Gln Pro Pro Pro Pro Val Ala Gln Leu Val Pro Ile Val Pro Leu Glu
                165                 170                 175
Lys Ala Trp Pro Gly Pro His Gly Thr Thr Gly Glu Gly Gly Pro Val
            180                 185                 190
Ala Thr Leu Ser Lys Pro Ser Leu Gly Asp Arg Ser Lys Ile Ser Lys
        195                 200                 205
Asp Val Tyr Glu Asn Phe Arg Gln Trp Gln Arg Tyr Lys Ala Leu Ala
    210                 215                 220
```

Arg Arg His Leu Ser Gln Ser Pro Asp Thr Glu Ala Leu Ser Cys Phe
225 230 235 240
Leu Ile Pro Val Leu Arg Ser Leu Ala Arg Leu Lys Pro Thr Met Thr
245 250 255
Leu Glu Glu Gly Leu Pro Leu Ala Val Gln Glu Trp Glu His Thr Ser
260 265 270
Asn Phe Asp Arg Met Ile Phe Tyr Glu Met Ala Glu Arg Phe Met Glu
275 280 285
Phe Glu Ala Glu Glu Met Gln Ile Gln Asn Thr Gln Leu Met Asn Gly
290 295 300
Ser Gln Gly Leu Ser Pro Ala Thr Pro Leu Lys Leu Asp Pro Leu Gly
305 310 315 320
Pro Leu Ala Ser Glu Val Cys Gln Gln Pro Val Tyr Ile Pro Lys Lys
325 330 335
Ala Ala Ser Lys Thr Arg Ala Pro Arg Arg Arg Gln Arg Lys Ala Gln
340 345 350
Arg Pro Pro Ala Pro Glu Ala Pro Lys Glu Ile Pro Pro Glu Ala Val
355 360 365
Lys Glu Tyr Val Asp Ile Met Glu Trp Leu Val Gly Thr His Leu Ala
370 375 380
Thr Gly Glu Ser Asp Gly Lys Gln Glu Glu Glu Gly Gln Gln Gln Glu
385 390 395 400
Glu Glu Gly Met Tyr Pro Asp Pro Gly Leu Leu Ser Tyr Ile Asn Glu
405 410 415
Leu Cys Ser Gln Lys Val Phe Val Ser Lys Val Glu Ala Val Ile His
420 425 430
Pro Gln Phe Leu Ala Asp Leu Leu Ser Pro Glu Lys Gln Arg Asp Pro
435 440 445
Leu Ala Leu Ile Glu Glu Leu Glu Gln Glu Glu Gly Leu Thr Leu Ala
450 455 460
Gln Leu Val Gln Lys Arg Leu Met Ala Leu Glu Glu Glu Glu Asp Ala
465 470 475 480
Glu Ala Pro Pro Ser Phe Ser Gly Ala Gln Leu Asp Ser Ser Pro Ser
485 490 495
Gly Ser Val Glu Asp Glu Asp Gly Asp Gly Arg Leu Arg Pro Ser Pro
500 505 510
Gly Leu Gln Gly Ala Gly Gly Ala Ala Cys Leu Gly Lys Val Ser Ser
515 520 525
Ser Gly Lys Arg Ala Arg Glu Val His Gly Gly Gln Glu Gln Ala Leu
530 535 540
Asp Ser Pro Arg Gly Met His Arg Asp Gly Asn Thr Leu Pro Ser Pro
545 550 555 560
Ser Ser Trp Asp Leu Gln Pro Glu Leu Ala Ala Pro Gln Gly Thr Pro
565 570 575
Gly Pro Leu Gly Val Glu Arg Arg Gly Ser Gly Lys Val Ile Asn Gln
580 585 590
Val Ser Leu His Gln Asp Gly His Leu Gly Gly Ala Gly Pro Pro Gly
595 600 605
His Cys Leu Val Ala Asp Arg Thr Ser Glu Ala Leu Pro Leu Cys Trp
610 615 620
Gln Gly Gly Phe Gln Pro Glu Ser Thr Pro Ser Leu Asp Ala Gly Leu
625 630 635 640
Ala Glu Leu Ala Pro Leu Gln Gly Gln Gly Leu Glu Lys Gln Val Leu
645 650 655
Gly Leu Gln Lys Gly Gln Gln Thr Gly Gly Arg Gly Val Leu Pro Gln
660 665 670
Gly Lys Glu Pro Leu Ala Val Pro Trp Glu Gly Ser Ser Gly Ala Met
675 680 685
Trp Gly Asp Asp Arg Gly Thr Pro Met Ala Gln Ser Tyr Asp Gln Asn
690 695 700
Pro Ser Pro Arg Ala Ala Gly Glu Arg Asp Asp Val Cys Leu Ser Pro
705 710 715 720
Gly Val Trp Leu Ser Ser Glu Met Asp Ala Val Gly Leu Glu Leu Pro
725 730 735
Val Gln Ile Glu Glu Val Ile Glu Ser Phe Gln Val Glu Lys Cys Val

```
            740                 745                 750
Thr Glu Tyr Gln Glu Gly Cys Gln Gly Leu Gly Ser Arg Gly Asn Ile
        755                 760                 765
Ser Leu Gly Pro Gly Glu Thr Leu Val Pro Gly Asp Thr Glu Ser Ser
    770                 775                 780
Val Ile Pro Cys Gly Gly Thr Val Ala Ala Ala Ala Leu Glu Lys Arg
785                 790                 795                 800
Asn Tyr Cys Ser Leu Pro Gly Pro Leu Arg Ala Asn Ser Pro Pro Leu
                805                 810                 815
Arg Ser Lys Glu Asn Gln Glu Gln Ser Cys Glu Thr Val Gly His Pro
            820                 825                 830
Ser Asp Leu Trp Ala Glu Gly Cys Phe Pro Leu Leu Glu Ser Gly Asp
        835                 840                 845
Ser Thr Leu Gly Ser Ser Lys Glu Thr Leu Pro Pro Thr Cys Gln Gly
    850                 855                 860
Asn Leu Leu Ile Met Gly Thr Glu Asp Ala Ser Ser Leu Pro Glu Ala
865                 870                 875                 880
Ser Gln Glu Ala Gly Ser Arg Gly Asn Ser Phe Ser Pro Leu Leu Glu
                885                 890                 895
Thr Ile Glu Pro Val Asn Ile Leu Asp Val Lys Asp Asp Cys Gly Leu
            900                 905                 910
Gln Leu Arg Val Ser Glu Asp Thr Cys Pro Leu Asn Val His Ser Tyr
        915                 920                 925
Asp Pro Gln Gly Glu Gly Arg Val Asp Pro Asp Leu Ser Lys Pro Lys
    930                 935                 940
Asn Leu Ala Pro Leu Gln Glu Ser Gln Glu Ser Tyr Thr Thr Gly Thr
945                 950                 955                 960
Pro Lys Ala Thr Ser Ser His Gln Gly Leu Gly Ser Thr Leu Pro Arg
                965                 970                 975
Arg Gly Thr Arg Asn Ala Ile Val Pro Arg Glu Thr Ser Val Ser Lys
            980                 985                 990
Thr His Arg Ser Ala Asp Arg Ala Lys Gly Lys Glu Lys Lys Lys Lys
        995                 1000                1005
Glu Ala Glu Glu Glu Asp Glu Glu Leu Ser Asn Phe Ala Tyr Leu Leu
    1010                1015                1020
Ala Ser Lys Leu Ser Leu Ser Pro Arg Glu His Pro Leu Ser Pro His
1025                1030                1035                1040
His Ala Ser Gly Gly Gln Gly Ser Gln Arg Ala Ser His Leu Leu Pro
                1045                1050                1055
Ala Gly Ala Lys Gly Pro Ser Lys Leu Pro Tyr Pro Val Ala Lys Ser
            1060                1065                1070
Gly Lys Arg Ala Leu Ala Gly Gly Pro Ala Pro Thr Glu Lys Thr Pro
        1075                1080                1085
His Ser Gly Ala Gln Leu Gly Val Pro Arg Glu Lys Pro Leu Ala Leu
    1090                1095                1100
Gly Val Val Arg Pro Ser Gln Pro Arg Lys Arg Arg Cys Asp Ser Phe
1105                1110                1115                1120
Val Thr Gly Arg Arg Lys Lys Arg Arg Arg Ser Gln
                1125                1130
```

<210> 3
<211> 5198
<212> DNA
<213> Homo sapiens

<220>
<221> source
<222> 1..5198
<223> /mol_type="DNA"
/organism="Homo sapiens"

<400> 3

```
attctttgga atactactgc tagaagtctg acttaagacc cagcttatgg gccacatggc      60

acccagctgc ttctgcagag aaggcaggcc actgatgggt acagcaaagt gtggtgctgc     120
```

tggccaagcc aaagacccgt gtaggatgac tgggcctctg ccccttgtgg gtgttgccac 180

tgtgcttgag tgcctggtga agaatgtgat gggatcacta gcatgtctgc ggagagcggc 240

cctgggacga gattgagaaa tctgccagta atgggggatg gactagaaac ttcccaaatg 300

tctacaacac aggcccaggc ccaaccccag ccagccaacg cagccagcac caaccccccg 360

cccccagaga cctccaaccc taacaagccc aagaggcaga ccaaccaact gcaatacctg 420

ctcagagtgg tgctcaagac actatggaaa caccagtttg catggccttt ccagcagcct 480

gtggatgccg tcaagctgaa cctccctgat tactataaga tcattaaaac gcctatggat 540

atgggaacaa taaagaagcg cttggaaaac aactattact ggaatgctca ggaatgtatc 600

caggacttca acactatgtt tacaaattgt tacatctaca acaagcctgg agatgacata 660

gtcttaatgg cagaagctct ggaaaagctc ttcttgcaaa aaataaatga gctacccaca 720

gaagaaaccg agatcatgat agtccaggca aaaggaagag gacgtgggag gaaagaaaca 780

gggacagcaa aacctggcgt ttccacggta ccaaacacaa ctcaagcatc gactcctccg 840

cagacccaga cccctcagcc gaatcctcct cctgtgcagg ccacgcctca ccccttccct 900

gccgtcaccc cggacctcat cgtccagacc cctgtcatga cagtggtgcc tccccagcca 960

ctgcagacgc cccgccagt gccccccag ccacaacccc cacccgctcc agctccccag 1020

cccgtacaga gccacccacc catcatcgcg gccaccccac agcctgtgaa gacaaagaag 1080

ggagtgaaga ggaaagcaga caccaccacc cccaccacca ttgaccccat tcacgagcca 1140

ccctcgctgc ccccggagcc caagaccacc aagctgggcc agcggcggga gagcagccgg 1200

cctgtgaaac ctccaaagaa ggacgtgccc gactctcagc agcacccagc accagagaag 1260

agcagcaagg tctcggagca gctcaagtgc tgcagcggca tcctcaagga gatgtttgcc 1320

aagaagcacg ccgcctacgc ctggcccttc tacaagcctg tggacgtgga ggcactgggc 1380

ctacacgact actgtgacat catcaagcac cccatggaca tgagcacaat caagtctaaa 1440

ctggaggccc gtgagtaccg tgatgctcag gagtttggtg ctgacgtccg attgatgttc 1500

tccaactgct ataagtacaa ccctcctgac catgaggtgg tggccatggc ccgcaagctc 1560

caggatgtgt tcgaaatgcg ctttgccaag atgccggacg agcctgagga gccagtggtg 1620

gccgtgtcct ccccggcagt gccccctccc accaaggttg tggccccgcc ctcatccagc 1680

gacagcagca gcgatagctc ctcggacagt gacagttcga ctgatgactc tgaggaggag 1740

cgagcccagc ggctggctga gctccaggag cagctcaaag ccgtgcacga gcagcttgca 1800

gccctctctc agccccagca gaacaaacca aagaaaaagg agaaagacaa gaaggaaaag 1860

aaaaaagaaa agcacaaaag gaaagaggaa gtggaagaga ataaaaaaag caaagccaag 1920

gaacctcctc ctaaaaagac gaagaaaaat aatagcagca acagcaatgt gagcaagaag 1980

gagccagcgc ccatgaagag caagcccccct cccacgtatg agtcggagga agaggacaag 2040

tgcaagccta tgtcctatga ggagaagcgg cagctcagct tggacatcaa caagctcccc 2100
ggcgagaagc tgggccgcgt ggtgcacatc atccagtcac gggagccctc cctgaagaat 2160
tccaaccccg acgagattga aatcgacttt gagaccctga agccgtccac actgcgtgag 2220
ctggagcgct atgtcacctc ctgtttgcgg aagaaaagga aacctcaagc tgagaaagtt 2280
gatgtgattg ccggctcctc caagatgaag ggcttctcgt cctcagagtc ggagagctcc 2340
agtgagtcca gctcctctga cagcgaagac tccgaaacag agatggctcc gaagtcaaaa 2400
aagaaggggc accccgggag ggagcagaag aagcaccatc atcaccacca tcagcagatg 2460
cagcaggccc cggctcctgt gccccagcag ccgcccccgc ctccccagca gcccccaccg 2520
cctccacctc cgcagcagca acagcagccg ccacccccgc ctcccccacc ctccatgccg 2580
cagcaggcag ccccggcgat gaagtcctcg cccccaccct tcattgccac ccaggtgccc 2640
gtcctggagc cccagctccc aggcagcgtc tttgacccca tcggccactt cacccagccc 2700
atcctgcacc tgccgcagcc tgagctgccc cctcacctgc cccagccgcc tgagcacagc 2760
actccacccc atctcaacca gcacgcagtg gtctctcctc cagctttgca caacgcacta 2820
ccccagcagc catcacggcc cagcaaccga gccgctgccc tgcctcccaa gcccgcccgg 2880
cccccagccg tgtcaccagc cttgacccaa acacccctgc tcccacagcc ccccatggcc 2940
caaccccccc aagtgctgct ggaggatgaa gagccacctg ccccacccct cacctccatg 3000
cagatgcagc tgtacctgca gcagctgcag aaggtgcagc cccctacgcc gctactccct 3060
tccgtgaagg tgcagtccca gcccccaccc cccctgccgc ccccacccca cccctctgtg 3120
cagcagcagc tgcagcagca gccgccacca cccccaccac cccagcccca gcctccaccc 3180
cagcagcagc atcagccccc tccacggccc gtgcacttgc agcccatgca gttttccacc 3240
cacatccaac agcccccgcc accccagggc cagcagcccc cccatccgcc cccaggccag 3300
cagccacccc cgccgcagcc tgccaagcct cagcaagtca tccagcacca ccattcaccc 3360
cggcaccaca agtcggaccc ctactcaacc ggtcacctcc gcgaagcccc ctccccgctt 3420
atgatacatt ccccccagat gtcacagttc cagagcctga cccaccagtc tccaccccag 3480
caaaacgtcc agcctaagaa acaggagctg cgtgctgcct ccgtggtcca gccccagccc 3540
ctcgtggtgg tgaaggagga gaagatccac tcacccatca tccgcagcga gcccttcagc 3600
ccctcgctgc ggccggagcc ccccaagcac ccggagagca tcaaggcccc cgtccacctg 3660
ccccagcggc cggaaatgaa gcctgtggat gtcgggaggc ctgtgatccg gcccccagag 3720
cagaacgcac cgccaccagg ggcccctgac aaggacaaac agaaacagga gccgaagact 3780
ccagttgcgc ccaaaaagga cctgaaaatc aagaacatgg gctcctgggc cagcctagtg 3840
cagaagcatc cgaccacccc ctcctccaca gccaagtcat ccagcgacag cttcgagcag 3900
ttccgccgcg ccgctcggga gaaagaggag cgtgagaagg ccctgaaggc tcaggccgag 3960
cacgctgaga aggagaagga gcggctgcgg caggagcgca tgaggagccg agaggacgag 4020

gatgcgctgg agcaggcccg gcgggcccat gaggaggcac gtcggcgcca ggagcagcag 4080

cagcagcagc gccaggagca acagcagcag cagcaacagc aagcagctgc ggtggctgcc 4140

gccgccaccc cacaggccca gagctcccag ccccagtcca tgctggacca gcagagggag 4200

ttggcccgga agcgggagca ggagcgaaga cgccgggaag ccatggcagc taccattgac 4260

atgaatttcc agagtgatct attgtcaata tttgaagaaa atcttttctg agcgcaccta 4320

ggtggcttct gactttgatt ttctggcaaa acattgactt tccatagtgt taggggcggt 4380

ggtggaggtg ggatcagcgg ccaggggatg cctcagggcc tggccctcct gcatgctatg 4440

cccggggcag gcctgacggg cagctgagga ttgcagagcc tgtctgcctt acggccagtc 4500

ggacagacgt cccgccaccc accacccctc acaggacgtc cgctcagcac acgccttgtt 4560

acgagcaagt gccggctgga cccaagccct gcatccccac atgcggggca gaggcccttc 4620

tctccgccaa atgtctacac agtatacaca ggacatcgtt gctgccgccg tgactggttt 4680

tctgtcccca agaacgtgac gttcgtgatg tcctgcccgc cgggagtctt tccccacacc 4740

ccagccatcg ccgcccgctc ccaggaggcc agggcaggcc tgcgtgggct ggaggcgggc 4800

gaggccggcc cacccccctcg ctggcactga ctttgccttg aacagacccc ccgaccctcc 4860

cccacaagcc tttaattgag agccgctctc tgtaagtgtt tgcttgtgca aaagggaata 4920

gtgccgtgga ggtgtgtgtg tccatggcat ccggagcgag gcgactgtcc tgcgtgggta 4980

gccctcggcc ggggagtgag gccaccaacc aaagtcagtt ccttccccacc tgtgtttctg 5040

tttcgttttt ttttttcttt tttttctata tatatttttt gttgaattct attttatttt 5100

taattctctc ttctcctcca gacacaatgg cactgcttat ctccgaaatg gtgtgatcgt 5160

ctcctcattg agcagcggct gccaccgcgc tgtgggta 5198

<210> 4
<211> 1362
<212> PRT
<213> Homo sapiens

<220>
<221> SOURCE
<222> 1..1362
<223> /mol_type="protein"
/organism="Homo sapiens"

<400> 4
Met Ser Ala Glu Ser Gly Pro Gly Thr Arg Leu Arg Asn Leu Pro Val
1 5 10 15
Met Gly Asp Gly Leu Glu Thr Ser Gln Met Ser Thr Thr Gln Ala Gln
20 25 30
Ala Gln Pro Gln Pro Ala Asn Ala Ala Ser Thr Asn Pro Pro Pro Pro
35 40 45
Glu Thr Ser Asn Pro Asn Lys Pro Lys Arg Gln Thr Asn Gln Leu Gln
50 55 60
Tyr Leu Leu Arg Val Val Leu Lys Thr Leu Trp Lys His Gln Phe Ala
65 70 75 80
Trp Pro Phe Gln Gln Pro Val Asp Ala Val Lys Leu Asn Leu Pro Asp

```
                85                  90                  95
Tyr Tyr Lys Ile Ile Lys Thr Pro Met Asp Met Gly Thr Ile Lys Lys
            100                 105                 110
Arg Leu Glu Asn Asn Tyr Tyr Trp Asn Ala Gln Glu Cys Ile Gln Asp
        115                 120                 125
Phe Asn Thr Met Phe Thr Asn Cys Tyr Ile Tyr Asn Lys Pro Gly Asp
    130                 135                 140
Asp Ile Val Leu Met Ala Glu Ala Leu Glu Lys Leu Phe Leu Gln Lys
145                 150                 155                 160
Ile Asn Glu Leu Pro Thr Glu Glu Thr Glu Ile Met Ile Val Gln Ala
                165                 170                 175
Lys Gly Arg Gly Arg Gly Arg Lys Glu Thr Gly Thr Ala Lys Pro Gly
            180                 185                 190
Val Ser Thr Val Pro Asn Thr Thr Gln Ala Ser Thr Pro Pro Gln Thr
        195                 200                 205
Gln Thr Pro Gln Pro Asn Pro Pro Pro Val Gln Ala Thr Pro His Pro
    210                 215                 220
Phe Pro Ala Val Thr Pro Asp Leu Ile Val Gln Thr Pro Val Met Thr
225                 230                 235                 240
Val Val Pro Pro Gln Pro Leu Gln Thr Pro Pro Pro Val Pro Pro Gln
                245                 250                 255
Pro Gln Pro Pro Pro Ala Pro Ala Pro Gln Pro Val Gln Ser His Pro
            260                 265                 270
Pro Ile Ile Ala Ala Thr Pro Gln Pro Val Lys Thr Lys Lys Gly Val
        275                 280                 285
Lys Arg Lys Ala Asp Thr Thr Thr Pro Thr Thr Ile Asp Pro Ile His
    290                 295                 300
Glu Pro Pro Ser Leu Pro Pro Glu Pro Lys Thr Thr Lys Leu Gly Gln
305                 310                 315                 320
Arg Arg Glu Ser Ser Arg Pro Val Lys Pro Pro Lys Lys Asp Val Pro
                325                 330                 335
Asp Ser Gln Gln His Pro Ala Pro Glu Lys Ser Ser Lys Val Ser Glu
            340                 345                 350
Gln Leu Lys Cys Cys Ser Gly Ile Leu Lys Glu Met Phe Ala Lys Lys
        355                 360                 365
His Ala Ala Tyr Ala Trp Pro Phe Tyr Lys Pro Val Asp Val Glu Ala
    370                 375                 380
Leu Gly Leu His Asp Tyr Cys Asp Ile Ile Lys His Pro Met Asp Met
385                 390                 395                 400
Ser Thr Ile Lys Ser Lys Leu Glu Ala Arg Glu Tyr Arg Asp Ala Gln
                405                 410                 415
Glu Phe Gly Ala Asp Val Arg Leu Met Phe Ser Asn Cys Tyr Lys Tyr
            420                 425                 430
Asn Pro Pro Asp His Glu Val Val Ala Met Ala Arg Lys Leu Gln Asp
        435                 440                 445
Val Phe Glu Met Arg Phe Ala Lys Met Pro Asp Glu Pro Glu Glu Pro
    450                 455                 460
Val Val Ala Val Ser Ser Pro Ala Val Pro Pro Pro Thr Lys Val Val
465                 470                 475                 480
Ala Pro Pro Ser Ser Ser Asp Ser Ser Ser Asp Ser Ser Ser Asp Ser
                485                 490                 495
Asp Ser Ser Thr Asp Asp Ser Glu Glu Glu Arg Ala Gln Arg Leu Ala
            500                 505                 510
Glu Leu Gln Glu Gln Leu Lys Ala Val His Glu Gln Leu Ala Ala Leu
        515                 520                 525
Ser Gln Pro Gln Gln Asn Lys Pro Lys Lys Lys Glu Lys Asp Lys Lys
    530                 535                 540
Glu Lys Lys Lys Glu Lys His Lys Arg Lys Glu Glu Val Glu Glu Asn
545                 550                 555                 560
Lys Lys Ser Lys Ala Lys Glu Pro Pro Pro Lys Lys Thr Lys Lys Asn
                565                 570                 575
Asn Ser Ser Asn Ser Asn Val Ser Lys Lys Glu Pro Ala Pro Met Lys
            580                 585                 590
Ser Lys Pro Pro Pro Thr Tyr Glu Ser Glu Glu Glu Asp Lys Cys Lys
        595                 600                 605
```

```
Pro Met Ser Tyr Glu Glu Lys Arg Gln Leu Ser Leu Asp Ile Asn Lys
    610                 615                 620
Leu Pro Gly Glu Lys Leu Gly Arg Val Val His Ile Ile Gln Ser Arg
625                 630                 635                 640
Glu Pro Ser Leu Lys Asn Ser Asn Pro Asp Glu Ile Glu Ile Asp Phe
                645                 650                 655
Glu Thr Leu Lys Pro Ser Thr Leu Arg Glu Leu Glu Arg Tyr Val Thr
            660                 665                 670
Ser Cys Leu Arg Lys Lys Arg Lys Pro Gln Ala Glu Lys Val Asp Val
        675                 680                 685
Ile Ala Gly Ser Ser Lys Met Lys Gly Phe Ser Ser Ser Glu Ser Glu
    690                 695                 700
Ser Ser Ser Glu Ser Ser Ser Ser Asp Ser Glu Asp Ser Glu Thr Glu
705                 710                 715                 720
Met Ala Pro Lys Ser Lys Lys Lys Gly His Pro Gly Arg Glu Gln Lys
                725                 730                 735
Lys His His His His His His Gln Gln Met Gln Gln Ala Pro Ala Pro
            740                 745                 750
Val Pro Gln Gln Pro Pro Pro Pro Pro Gln Gln Pro Pro Pro Pro Pro
        755                 760                 765
Pro Pro Gln Gln Gln Gln Gln Pro Pro Pro Pro Pro Pro Pro Pro Ser
    770                 775                 780
Met Pro Gln Gln Ala Ala Pro Ala Met Lys Ser Ser Pro Pro Pro Phe
785                 790                 795                 800
Ile Ala Thr Gln Val Pro Val Leu Glu Pro Gln Leu Pro Gly Ser Val
                805                 810                 815
Phe Asp Pro Ile Gly His Phe Thr Gln Pro Ile Leu His Leu Pro Gln
            820                 825                 830
Pro Glu Leu Pro Pro His Leu Pro Gln Pro Pro Glu His Ser Thr Pro
        835                 840                 845
Pro His Leu Asn Gln His Ala Val Val Ser Pro Pro Ala Leu His Asn
    850                 855                 860
Ala Leu Pro Gln Gln Pro Ser Arg Pro Ser Asn Arg Ala Ala Ala Leu
865                 870                 875                 880
Pro Pro Lys Pro Ala Arg Pro Pro Ala Val Ser Pro Ala Leu Thr Gln
                885                 890                 895
Thr Pro Leu Leu Pro Gln Pro Pro Met Ala Gln Pro Pro Gln Val Leu
            900                 905                 910
Leu Glu Asp Glu Glu Pro Pro Ala Pro Pro Leu Thr Ser Met Gln Met
        915                 920                 925
Gln Leu Tyr Leu Gln Gln Leu Gln Lys Val Gln Pro Pro Thr Pro Leu
    930                 935                 940
Leu Pro Ser Val Lys Val Gln Ser Gln Pro Pro Pro Pro Leu Pro Pro
945                 950                 955                 960
Pro Pro His Pro Ser Val Gln Gln Gln Leu Gln Gln Gln Pro Pro Pro
                965                 970                 975
Pro Pro Pro Pro Gln Pro Gln Pro Pro Pro Gln Gln Gln His Gln Pro
            980                 985                 990
Pro Pro Arg Pro Val His Leu Gln Pro Met Gln Phe Ser Thr His Ile
        995                 1000                1005
Gln Gln Pro Pro Pro Pro Gln Gly Gln Gln Pro Pro His Pro Pro Pro
    1010                1015                1020
Gly Gln Gln Pro Pro Pro Pro Gln Pro Ala Lys Pro Gln Gln Val Ile
1025                1030                1035                1040
Gln His His His Ser Pro Arg His His Lys Ser Asp Pro Tyr Ser Thr
                1045                1050                1055
Gly His Leu Arg Glu Ala Pro Ser Pro Leu Met Ile His Ser Pro Gln
            1060                1065                1070
Met Ser Gln Phe Gln Ser Leu Thr His Gln Ser Pro Pro Gln Gln Asn
        1075                1080                1085
Val Gln Pro Lys Lys Gln Glu Leu Arg Ala Ala Ser Val Val Gln Pro
    1090                1095                1100
Gln Pro Leu Val Val Val Lys Glu Glu Lys Ile His Ser Pro Ile Ile
1105                1110                1115                1120
Arg Ser Glu Pro Phe Ser Pro Ser Leu Arg Pro Glu Pro Pro Lys His
```

```
                1125                1130                1135
Pro Glu Ser Ile Lys Ala Pro Val His Leu Pro Gln Arg Pro Glu Met
            1140                1145                1150
Lys Pro Val Asp Val Gly Arg Pro Val Ile Arg Pro Pro Glu Gln Asn
        1155                1160                1165
Ala Pro Pro Pro Gly Ala Pro Asp Lys Asp Lys Gln Lys Gln Glu Pro
    1170                1175                1180
Lys Thr Pro Val Ala Pro Lys Lys Asp Leu Lys Ile Lys Asn Met Gly
1185                1190                1195                1200
Ser Trp Ala Ser Leu Val Gln Lys His Pro Thr Thr Pro Ser Ser Thr
                1205                1210                1215
Ala Lys Ser Ser Ser Asp Ser Phe Glu Gln Phe Arg Arg Ala Ala Arg
            1220                1225                1230
Glu Lys Glu Glu Arg Glu Lys Ala Leu Lys Ala Gln Ala Glu His Ala
        1235                1240                1245
Glu Lys Glu Lys Glu Arg Leu Arg Gln Glu Arg Met Arg Ser Arg Glu
    1250                1255                1260
Asp Glu Asp Ala Leu Glu Gln Ala Arg Arg Ala His Glu Glu Ala Arg
1265                1270                1275                1280
Arg Arg Gln Glu Gln Gln Gln Gln Gln Arg Gln Glu Gln Gln Gln Gln
                1285                1290                1295
Gln Gln Gln Gln Ala Ala Ala Val Ala Ala Ala Ala Thr Pro Gln Ala
            1300                1305                1310
Gln Ser Ser Gln Pro Gln Ser Met Leu Asp Gln Gln Arg Glu Leu Ala
        1315                1320                1325
Arg Lys Arg Glu Gln Glu Arg Arg Arg Glu Ala Met Ala Ala Thr
    1330                1335                1340
Ile Asp Met Asn Phe Gln Ser Asp Leu Leu Ser Ile Phe Glu Glu Asn
1345                1350                1355                1360
Leu Phe
```

<210> 5
<211> 5673
<212> DNA
<213> Homo sapiens

<220>
<221> source
<222> 1..5673
<223> /mol_type="DNA"
/organism="Homo sapiens"

<400> 5

```
tgccggggcc ggcgagccaa agaggagccg gccgcgcggg ccgggagggg acggccgccg      60

gagccgcgag gccaactgtc gcctggttgg gcccggaaat gggacgtcgc gctttctcag     120

ggagcgtaga agcagccagg gcctctccaa gccgctgctg tgacagaaag tgagtgagct     180

gccggaggat gtccaccgcc acgacagtcg cccccgcggg gatcccggcg accccgggcc     240

ctgtgaaccc accccccccg gaggtctcca accccagcaa gcccggccgc aagaccaacc     300

agctgcagta catgcagaat gtggtggtga agacgctctg gaaacaccag ttcgcctggc     360

ccttctacca gcccgtggac gcaatcaaat tgaacctgcc ggattatcat aaaataatta     420

aaaacccaat ggatatgggg actattaaga agagactaga aaataattat tattggagtg     480

caagcgaatg tatgcaggac ttcaacacca tgtttacaaa ttgttacatt tataacaagc     540

ccacagatga catagtgcta atggcccaag ctttagagaa aatttttcta caaaaagtgg     600

cccagatgcc ccaagaggaa gttgaattat tacccccctgc tccaaagggc aaaggtcgga     660
```

agccggctgc gggagcccag agcgcaggta cacagcaagt ggcggccgtg tcctctgtct 720
ccccagcgac cccctttcag agcgtgcccc ccaccgtctc ccagacgccc gtcatcgctg 780
ccacccctgt accaaccatc actgcaaacg tcacgtcggt cccagtcccc ccagctgccg 840
ccccacctcc tcctgccaca cccatcgtcc ccgtggtccc tcctacgccg cctgtcgtca 900
agaaaaaggg cgtgaagcgg aaagcagaca caaccactcc cacgacgtcg gccatcactg 960
ccagccggag tgagtcgccc ccgccgttgt cagaccccaa gcaggccaaa gtggtggccc 1020
ggcgggagag tggtggccgc cccatcaagc ctcccaagaa ggacctggag gacggcgagg 1080
tgccccagca cgcaggcaag aagggcaagc tgtcggagca cctacgctac tgcgacagca 1140
tcctcaggga gatgctatcc aagaagcacg cggcctacgc ctggcccttc tacaagccag 1200
tggatgccga ggccctggag ctgcacgact accacgacat catcaagcac ccgatggacc 1260
tcagcaccgt gaaaaggaag atggatggcc gagagtaccc agacgcacag ggctttgctg 1320
ctgatgtccg gctgatgttc tcgaattgct acaaatacaa tcccccagac cacgaggttg 1380
tggccatggc ccggaagctc caggacgtgt ttgagatgag gtttgccaag atgccagatg 1440
agcccgtgga ggcaccggcg ctgcctgccc ccgcggcccc catggtgagc aagggcgctg 1500
agagcagccg tagcagtgag gagagctctt cggactcagg cagctcggac tcggaggagg 1560
agcgggccac caggctggcg gagctgcagg agcagctgaa ggccgtgcac gagcagctgg 1620
ccgccctgtc tcaggcccca gtaaacaaac caaagaagaa gaaggagaag aaggagaagg 1680
agaagaagaa gaaggacaag gagaaggaga aggagaagca caaagtgaag gccgaggaag 1740
agaagaaggc caaggtggct ccgcctgcca agcaggctca gcagaagaag gctcctgcca 1800
agaaggccaa cagcacgacc acggccggca gacagctgaa gaaaggcggc aagcaggcat 1860
ctgcctccta cgactcagag gaagaggagg agggcctgcc catgagctac gatgaaaagc 1920
gccagcttag cctggacatc aaccggctgc ccggggagaa gctgggccgg gtagtgcaca 1980
tcatccaatc tcgggagccc tcgctcaggg actccaaccc cgacgagata gaaattgact 2040
ttgagactct gaaacccacc actttgcggg aactggagag atatgtcaag tcttgtttac 2100
agaaaaagca aaggaaaccg ttctcagcaa gcgggaagaa acaggcagcc aagtcgaaag 2160
aggagctagc tcaggaaaag aagaaggagc tggaaaagcg tctgcaggat gtcagcgggc 2220
agctgagcag cagcaagaag cccgcccgga aagagaagcc cggctcagca ccctcagggg 2280
gcccgtccag gctcagcagc agcagctcct ccgagtctgg gagcagcagc tccagcgggt 2340
ccagctctga cagcagtgac tcagaatgaa ctggcttcgg acagaacagg acagatggat 2400
gtcgcacacg ccgagactct gccgtacccc tctgtggttc atattactac ttctgttcca 2460
tggtgtgcag gtctgcttct taattcagtg ttatgatatc ttccagtttt tgctttcata 2520
ggtcagagat ctatcttgtg tgtgcgttag acttgatgag aaggtgtgaa ctctgcagaa 2580

agtctcttct tcatcactga attcagtcac ttggagatga caacttcaaa tgctaacccg 2640

atgaccccag aaaaccgtgt gagattcgta ccgaagaacc ttgtggaatc cctttgctta 2700

ggcccaacct ggtcgatagc tcgagaaaga attttttcca aggaaatgtc tcggatatgg 2760

gtactgtatt tgaaagctgt tagctttgtc aacacgcatt gtccttgtca tttgggcccc 2820

gagctctgac cctcgtgtct gacgcggcca cctctttctg gaggggctga ggacagatgt 2880

gcctgcttgt ggagaccagg ctgggcctaa gcgaagggtc atcgcagccc cagcccggag 2940

cgtggagccc ttgggggtg gtcgggtggg atgtgcgttc tccgctcgtg gtgatgtcag 3000

gagctcctcg gagggaacag agcggctgtg tatgcagcct gcaggtttcc atacactgaa 3060

gcttttacct caactttaaa aaaaaaaaaa gaagaaaaga tttaaaaaaa aaaaaaaagg 3120

agactttttt tgtaaggttc ggcaattttc tacggaagtc cagcccgctg tgagtccccg 3180

cctggctagc gctgcccctg ctgctgctgc cgcgccaccc ttgggaacca cttcccgagc 3240

ttatgtgtat ataaatagtt atttattaac atcattggtc atttttaaaa aaaagaaaat 3300

aagaaaaaac cgcagaagaa atgcattcac acagtcgcag agatgcaggc cttgccagtg 3360

gtgtgccggg cgcgggtcct gtctggcggc ggcctgtcgt ctccaggtgc taactcctgc 3420

caccgcgcgg tgctcaccca cgtctgttcg cgcgctcgcc cctgggtttg ttgggttttt 3480

tggttttttt ctttgtggtt tttttttttt tttttttttg tatgaaactt ggaggcttac 3540

aggtatagac agctttcagc tacagcacat tctaattttt tattttgttt agttcttttg 3600

tattcacttc tggtctcttt aagactgttt taaaagaaat caatttaggg aaccccagtt 3660

atataatata aactttgtaa tctgagagaa aaaatgtata gtaaatctaa gtcttgattt 3720

ttaactttct attgtaaaaa ataataatat acagagttta atagaaggtg atgttttggt 3780

tttgttttcc cagaggctgc catatggtct ttgagtacgg ggatgtccca aactggccca 3840

ccaatgagca tggcggctcc ggccaggaat gccagagtta gcctcccagg cttgcgggtg 3900

gacatgcctg ctccctgcca gcctccagtg gcctggccag gccctcccga gcctgtctgc 3960

cctccccagg ggtggaggag tctctgggcc ccaggaggat tccctcccgg agactcgcac 4020

ggtgctccct gctcacgcgt tgtcacagtt agtccggaaa tgactgaaac caggcattct 4080

cccggacctc agcgtggggg agcctccagg cagacgctgg gtatggagct gtggtgtggt 4140

ctgtcctgta tggtggccag tgctttctgc cagcatttct ggatggatat agggactatc 4200

attagtatcc taatacacgg tgattttaaa acaaccataa aattgattca gagtccactg 4260

acccttacag atgtaggtat acccttactg gagagggaac tctgatgagg agatgctggt 4320

aaattatcat tttttaaatt gctggtgagt ctgacacttg gtgagttttc agccagtttg 4380

ttaaactttt aattaagttt tgtttataat aaaaatataa atggatttga aagtttccat 4440

tttttaaagt taccctcgtt ttcaaaggta ttttctaaac agatctttaa tggactattt 4500

aaaccgaatt taaggaattc acacacgaca gttgacaggt cttcacgcag gctggttggt 4560

aacgtgctgc cagcacaggg ctgggtgata cgtacaccct aagccggggg tgcctggggc 4620

tggggggcgc tccttgcaat gcccctccag ccacagggca gtgaggtgct gcctgtgtga 4680

gccgtcgggg gagcggccgg ctgtgggggc agcgcagcag gagcatcgtg gggcctttcc 4740

ttctcggctg gttctctgtg acggtggcgt cggctcgcct ctgctccttt catctagaaa 4800

gaagccactg accctgacag cccacggcgg gtacactgag cagctgcatt ggtgctgtca 4860

ctttttaag gctttctgtc cagacttcaa cactggtttc ttttcagagt ttcgaaggat 4920

taatgacttc ctcagcgccc ttgctggcgg gctgagggtg acagtcacgt ccgtttcttc 4980

tgtattagaa ggctgcggtg attcaattag attgtcccac tgctgagacc tgtagggcag 5040

cttctaacat gctttttca aggggagagg agtagtgaca agtcgtgtgt cggaattgga 5100

tttgagaaca ctctgaatga cccctggagg ccgagggggc aggcttcggg cgtgaactga 5160

actccagacc cctctttgtg ttgggcagtg tcatcttgct tacaaactgt aagacacatt 5220

tttttgtgtg tttgtttttg ttgttgttct tttgcagcac tcacgcctct gacagtcttt 5280

tgggaaagag taacacccac atacagaatt tgtcacatcc agagtagcac tgttccttaa 5340

tactggcata atgcttccag gaagtttttc ttttttatat ttaaaatgtt actttctgt 5400

atgatgtgca tgcaagttta ccgtaacttt tcttaaactt tttagtgccg tttctagtat 5460

attcctgtaa atgtcagtta ctgaaaatga gtccaatgta agtagtttag cttgtttatt 5520

gcaatgctgg cctcaacaca acagaataaa aatggtagaa agtactcttt gatgtttctg 5580

gtaatcatgg acccttctcc tggggcattt gttttgtttt cataataaaa agcaaaaaaa 5640

aaaaaaaaaa aaaaaaaaaa aaaaaaaaaa aaa 5673

<210> 6
<211> 726
<212> PRT
<213> Homo sapiens

<220>
<221> SOURCE
<222> 1..726
<223> /mol_type="protein"
/organism="Homo sapiens"

<400> 6
```
Met Ser Thr Ala Thr Thr Val Ala Pro Ala Gly Ile Pro Ala Thr Pro
1               5                   10                  15
Gly Pro Val Asn Pro Pro Pro Pro Glu Val Ser Asn Pro Ser Lys Pro
            20                  25                  30
Gly Arg Lys Thr Asn Gln Leu Gln Tyr Met Gln Asn Val Val Val Lys
        35                  40                  45
Thr Leu Trp Lys His Gln Phe Ala Trp Pro Phe Tyr Gln Pro Val Asp
    50                  55                  60
Ala Ile Lys Leu Asn Leu Pro Asp Tyr His Lys Ile Ile Lys Asn Pro
65                  70                  75                  80
Met Asp Met Gly Thr Ile Lys Lys Arg Leu Glu Asn Asn Tyr Tyr Trp
                85                  90                  95
Ser Ala Ser Glu Cys Met Gln Asp Phe Asn Thr Met Phe Thr Asn Cys
```

```
                100                 105                 110
Tyr Ile Tyr Asn Lys Pro Thr Asp Asp Ile Val Leu Met Ala Gln Ala
            115                 120                 125
Leu Glu Lys Ile Phe Leu Gln Lys Val Ala Gln Met Pro Gln Glu Glu
        130                 135                 140
Val Glu Leu Leu Pro Pro Ala Pro Lys Gly Lys Gly Arg Lys Pro Ala
145                 150                 155                 160
Ala Gly Ala Gln Ser Ala Gly Thr Gln Gln Val Ala Ala Val Ser Ser
                165                 170                 175
Val Ser Pro Ala Thr Pro Phe Gln Ser Val Pro Pro Thr Val Ser Gln
            180                 185                 190
Thr Pro Val Ile Ala Ala Thr Pro Val Pro Thr Ile Thr Ala Asn Val
        195                 200                 205
Thr Ser Val Pro Val Pro Pro Ala Ala Ala Pro Pro Pro Pro Ala Thr
    210                 215                 220
Pro Ile Val Pro Val Val Pro Pro Thr Pro Pro Val Val Lys Lys Lys
225                 230                 235                 240
Gly Val Lys Arg Lys Ala Asp Thr Thr Thr Pro Thr Thr Ser Ala Ile
                245                 250                 255
Thr Ala Ser Arg Ser Glu Ser Pro Pro Pro Leu Ser Asp Pro Lys Gln
            260                 265                 270
Ala Lys Val Val Ala Arg Arg Glu Ser Gly Gly Arg Pro Ile Lys Pro
        275                 280                 285
Pro Lys Lys Asp Leu Glu Asp Gly Glu Val Pro Gln His Ala Gly Lys
    290                 295                 300
Lys Gly Lys Leu Ser Glu His Leu Arg Tyr Cys Asp Ser Ile Leu Arg
305                 310                 315                 320
Glu Met Leu Ser Lys Lys His Ala Ala Tyr Ala Trp Pro Phe Tyr Lys
                325                 330                 335
Pro Val Asp Ala Glu Ala Leu Glu Leu His Asp Tyr His Asp Ile Ile
            340                 345                 350
Lys His Pro Met Asp Leu Ser Thr Val Lys Arg Lys Met Asp Gly Arg
        355                 360                 365
Glu Tyr Pro Asp Ala Gln Gly Phe Ala Ala Asp Val Arg Leu Met Phe
    370                 375                 380
Ser Asn Cys Tyr Lys Tyr Asn Pro Pro Asp His Glu Val Val Ala Met
385                 390                 395                 400
Ala Arg Lys Leu Gln Asp Val Phe Glu Met Arg Phe Ala Lys Met Pro
                405                 410                 415
Asp Glu Pro Val Glu Ala Pro Ala Leu Pro Ala Pro Ala Ala Pro Met
            420                 425                 430
Val Ser Lys Gly Ala Glu Ser Ser Arg Ser Ser Glu Glu Ser Ser Ser
        435                 440                 445
Asp Ser Gly Ser Ser Asp Ser Glu Glu Glu Arg Ala Thr Arg Leu Ala
    450                 455                 460
Glu Leu Gln Glu Gln Leu Lys Ala Val His Glu Gln Leu Ala Ala Leu
465                 470                 475                 480
Ser Gln Ala Pro Val Asn Lys Pro Lys Lys Lys Lys Glu Lys Lys Glu
                485                 490                 495
Lys Glu Lys Lys Lys Lys Asp Lys Glu Lys Glu Lys Glu Lys His Lys
            500                 505                 510
Val Lys Ala Glu Glu Glu Lys Lys Ala Lys Val Ala Pro Pro Ala Lys
        515                 520                 525
Gln Ala Gln Gln Lys Lys Ala Pro Ala Lys Lys Ala Asn Ser Thr Thr
    530                 535                 540
Thr Ala Gly Arg Gln Leu Lys Lys Gly Gly Lys Gln Ala Ser Ala Ser
545                 550                 555                 560
Tyr Asp Ser Glu Glu Glu Glu Glu Gly Leu Pro Met Ser Tyr Asp Glu
                565                 570                 575
Lys Arg Gln Leu Ser Leu Asp Ile Asn Arg Leu Pro Gly Glu Lys Leu
            580                 585                 590
Gly Arg Val Val His Ile Ile Gln Ser Arg Glu Pro Ser Leu Arg Asp
        595                 600                 605
Ser Asn Pro Asp Glu Ile Glu Ile Asp Phe Glu Thr Leu Lys Pro Thr
    610                 615                 620
```

```
Thr Leu Arg Glu Leu Glu Arg Tyr Val Lys Ser Cys Leu Gln Lys Lys
625                 630                 635                 640
Gln Arg Lys Pro Phe Ser Ala Ser Gly Lys Lys Gln Ala Ala Lys Ser
                645                 650                 655
Lys Glu Glu Leu Ala Gln Glu Lys Lys Lys Glu Leu Glu Lys Arg Leu
            660                 665                 670
Gln Asp Val Ser Gly Gln Leu Ser Ser Ser Lys Lys Pro Ala Arg Lys
        675                 680                 685
Glu Lys Pro Gly Ser Ala Pro Ser Gly Gly Pro Ser Arg Leu Ser Ser
    690                 695                 700
Ser Ser Ser Ser Glu Ser Gly Ser Ser Ser Ser Ser Gly Ser Ser Ser
705                 710                 715                 720
Asp Ser Ser Asp Ser Glu
                725

<210> 7
<211> 2472
<212> DNA
<213> Homo sapiens

<220>
<221> source
<222> 1..2472
<223> /mol_type="DNA"
      /organism="Homo sapiens"

<400> 7
aagtggccgt ggaggcggaa gtggcgcggc cgcggagggg cctggagtgc ggcggcggcg      60

ggacccggag caggagcggc ggcagcagcg actgggggcg gcggcggcgc gttggaggcg     120

gccatggcaa agcagtacga ctcggtggag tgcccttttt gtgatgaagt ttccaaatac     180

gagaagctcg ccaagatcgg ccaaggcacc ttcggggagg tgttcaaggc caggcaccgc     240

aagaccggcc agaaggtggc tctgaagaag gtgctgatgg aaaacgagaa ggaggggttc     300

cccattacag ccttgcggga gatcaagatc cttcagcttc taaaacacga gaatgtggtc     360

aacttgattg agatttgtcg aaccaaagct tccccctata accgctgcaa gggtagtata     420

tacctggtgt tcgacttctg cgagcatgac cttgctgggc tgttgagcaa tgttttggtc     480

aagttcacgc tgtctgagat caagagggtg atgcagatgc tgcttaacgg cctctactac     540

atccacagaa acaagatcct gcatagggac atgaaggctg ctaatgtgct tatcactcgt     600

gatggggtcc tgaagctggc agactttggg ctggcccggg ccttcagcct ggccaagaac     660

agccagccca accgctacac caaccgtgtg gtgacactct ggtaccggcc cccggagctg     720

ttgctcgggg agcgggacta cggccccccc attgacctgt ggggtgctgg gtgcatcatg     780

gcagagatgt ggacccgcag ccccatcatg cagggcaaca cggagcagca ccaactcgcc     840

ctcatcagtc agctctgcgg ctccatcacc cctgaggtgt ggccaaacgt ggacaactat     900

gagctgtacg aaaagctgga gctggtcaag ggccagaagc ggaaggtgaa ggacaggctg     960

aaggcctatg tgcgtgaccc atacgcactg gacctcatcg acaagctgct ggtgctggac    1020

cctgcccagc gcatcgacag cgatgacgcc ctcaaccacg acttcttctg gtccgacccc    1080

atgccctccg acctcaaggg catgctctcc acccacctga cgtccatgtt cgagtacttg    1140
```

gcaccaccgc gccggaaggg cagccagatc acccagcagt ccaccaacca gagtcgcaat 1200

cccgccacca ccaaccagac ggagtttgag cgcgtcttct gagggccggc gcttgccact 1260

agggctcttg tgtttttttt cttctgctat gtgacttgca tcgtggagac agggcatttg 1320

agtttatatc tctcatgcat attttattta atccccaccc tgggctctgg gagcagcccg 1380

ctgagtggac tggagtggag cattggctga gagaccagga gggcactgga gctgtcttgt 1440

ccttgctggt ttctggatg gttcccagag ggtttccatg gggtaggagg atgggctcgc 1500

ccaccagtga ctttttctaa gagctcccgg cgtggtggaa gaggggacag gtccctcacc 1560

cacccacaat cctattctcg ggctgagaac cctgcgtggg gacagggctc gcctcaggaa 1620

tgggctgttt ttggcctaac cctcagaaac actggggctg gcacaaactc ttggtttctt 1680

caacaggaga attttactgt gtttcttttg gttccattgt ttggagacat tcctgggcac 1740

agtttggtcc gttagaatta aaagttgaat tttttttttt tttaaatttt ttttttcct 1800

ccaggacttg tgtgttttgt tctgcgcaca caccgccaac tgttccccca cagtcagcag 1860

caggttgggc ctgaccattg ggacttgatt gtcaagtcac tggaggtctt gactttttta 1920

tctcagttca tgttctcttc cataattgga aaggaccttt gtctgttttt cctcttgggt 1980

gccttccaga acgcatctca tgtccctggt gagggaattg gtgagggcct gctgtgagct 2040

gctgtggctg cgatggtcac ccagctgggc aaatcactgg agtgacaatt tgacctgtca 2100

cctgagaagg atggtccctc agactgctgg gtagagggcc tggggcaggc tggagagaga 2160

aagtgggcag agggtgaagg gatcacaggg gtcttggaag gtggcagtag tttggacggg 2220

ggtggggagt atgtgggaga aaaaaacaga ctgaaggtag aatccttggg aacctttgag 2280

gagcggcaca ttctggcagg cacgttttct gtgagcctga agttaggaag agacattctg 2340

gaggtcaatt tcctacatcc tcttacaggc ggagaccttg aagtggggcc aggaaggaag 2400

gttggcaaaa cctttgacca gaactgtcct tcatttacag aaactgaccc agaccacaac 2460

acaaaaggcc ag 2472

<210> 8
<211> 372
<212> PRT
<213> Homo sapiens

<220>
<221> SOURCE
<222> 1..372
<223> /mol_type="protein"
/organism="Homo sapiens"

<400> 8
Met Ala Lys Gln Tyr Asp Ser Val Glu Cys Pro Phe Cys Asp Glu Val
1 5 10 15
Ser Lys Tyr Glu Lys Leu Ala Lys Ile Gly Gln Gly Thr Phe Gly Glu
20 25 30
Val Phe Lys Ala Arg His Arg Lys Thr Gly Gln Lys Val Ala Leu Lys
35 40 45

```
Lys Val Leu Met Glu Asn Glu Lys Glu Gly Phe Pro Ile Thr Ala Leu
    50                  55                  60
Arg Glu Ile Lys Ile Leu Gln Leu Leu Lys His Glu Asn Val Val Asn
65                  70                  75                  80
Leu Ile Glu Ile Cys Arg Thr Lys Ala Ser Pro Tyr Asn Arg Cys Lys
                85                  90                  95
Gly Ser Ile Tyr Leu Val Phe Asp Phe Cys Glu His Asp Leu Ala Gly
            100                 105                 110
Leu Leu Ser Asn Val Leu Val Lys Phe Thr Leu Ser Glu Ile Lys Arg
        115                 120                 125
Val Met Gln Met Leu Leu Asn Gly Leu Tyr Tyr Ile His Arg Asn Lys
    130                 135                 140
Ile Leu His Arg Asp Met Lys Ala Ala Asn Val Leu Ile Thr Arg Asp
145                 150                 155                 160
Gly Val Leu Lys Leu Ala Asp Phe Gly Leu Ala Arg Ala Phe Ser Leu
                165                 170                 175
Ala Lys Asn Ser Gln Pro Asn Arg Tyr Thr Asn Arg Val Val Thr Leu
            180                 185                 190
Trp Tyr Arg Pro Pro Glu Leu Leu Leu Gly Glu Arg Asp Tyr Gly Pro
        195                 200                 205
Pro Ile Asp Leu Trp Gly Ala Gly Cys Ile Met Ala Glu Met Trp Thr
    210                 215                 220
Arg Ser Pro Ile Met Gln Gly Asn Thr Glu Gln His Gln Leu Ala Leu
225                 230                 235                 240
Ile Ser Gln Leu Cys Gly Ser Ile Thr Pro Glu Val Trp Pro Asn Val
                245                 250                 255
Asp Asn Tyr Glu Leu Tyr Glu Lys Leu Glu Leu Val Lys Gly Gln Lys
            260                 265                 270
Arg Lys Val Lys Asp Arg Leu Lys Ala Tyr Val Arg Asp Pro Tyr Ala
        275                 280                 285
Leu Asp Leu Ile Asp Lys Leu Leu Val Leu Asp Pro Ala Gln Arg Ile
    290                 295                 300
Asp Ser Asp Asp Ala Leu Asn His Asp Phe Phe Trp Ser Asp Pro Met
305                 310                 315                 320
Pro Ser Asp Leu Lys Gly Met Leu Ser Thr His Leu Thr Ser Met Phe
                325                 330                 335
Glu Tyr Leu Ala Pro Pro Arg Arg Lys Gly Ser Gln Ile Thr Gln Gln
            340                 345                 350
Ser Thr Asn Gln Ser Arg Asn Pro Ala Thr Thr Asn Gln Thr Glu Phe
        355                 360                 365
Glu Arg Val Phe
    370

<210> 9
<211> 2568
<212> DNA
<213> Homo sapiens

<220>
<221> source
<222> 1..2568
<223> /mol_type="DNA"
      /organism="Homo sapiens"

<400> 9
gggaagatac acggttttta agaaagaact tctaacccca ctccaccgcc caacggtcac      60

gtgacgcgcc tgcgtcctcg cctcaaggtt ttttctggtc gcatattggc tgttaactcg     120

gctacggggt gtagcgaggt gcattccgga agtaggtcct ttgacttttg cttcctctac     180

ggaggcaagt aacaacccgg cggtcgacgc ttagcggaag ttcgtcaagt cgcagttgcc     240

cccgcacagc ggatgtgggt cgcctcttag gtgacgctgg gaagtgcctg caaccttcgc     300
```

cgctgccttc tggttgaagc actatggagg gagagaggaa gaacaacaac aaacggtggt 360
atttcactcg agaacagctg gaaaatagcc catcccgtcg ttttggcgtg gacccagata 420
aagaactttc ttatcgccag caggcggcca atctgcttca ggacatgggg cagcgtctta 480
acgtctcaca attgactatc aacactgcta tagtatacat gcatcgattc tacatgattc 540
agtccttcac acagttccct ggaaattctg tggctccagc agccttgttt ctagcagcta 600
aagtggagga gcagcccaaa aaattggaac atgtcatcaa ggtagcacat acttgtctcc 660
atcctcagga atcccttcct gatactagaa gtgaggctta tttgcaacaa gttcaagatc 720
tggtcatttt agaaagcata attttgcaga ctttaggctt tgaactaaca attgatcacc 780
cacatactca tgtagtaaag tgcactcaac ttgttcgagc aagcaaggac ttagcacaga 840
cttcttactt catggcaacc aacagcctgc atttgaccac atttagcctg cagtacacac 900
ctcctgtggt ggcctgtgtc tgcattcacc tggcttgcaa gtggtccaat tgggagatcc 960
cagtctcaac tgacgggaag cactggtggg agtatgttga cgccactgtg accttggaac 1020
ttttagatga actgacacat gagtttctac agattttgga gaaaactccc aacaggctca 1080
aacgcatttg gaattggagg gcatgcgagg ctgccaagaa aacaaaagca gatgaccgag 1140
gaacagatga aaagacttca gagcagacaa tcctcaatat gatttcccag agctcttcag 1200
acacaaccat tgcaggttta atgagcatgt caacttctac cacaagtgca gtgccttccc 1260
tgccagtctc cgaagagtca tccagcaact taaccagtgt ggagatgttg ccgggcaagc 1320
gttggctgtc ctcccaacct tctttcaaac tagaacctac tcagggtcat cggactagtg 1380
agaatttagc acttacagga gttgatcatt ccttaccaca ggatggttca aatgcattta 1440
tttcccagaa gcagaatagt aagagtgtgc catcagctaa agtgtcactg aaagaatacc 1500
gcgcgaagca tgcagaagaa ttggctgccc agaagaggca actggagaac atggaagcca 1560
atgtgaagtc acaatatgca tatgctgccc agaatctcct ttctcatcat gatagccatt 1620
cttcagtcat tctaaaaatg cccatagagg gttcagaaaa ccccgagcgg ccttttctgg 1680
aaaaggctga caaaacagct ctcaaaatga gaatcccagt ggcaggtgga gataaagctg 1740
cgtcttcaaa accagaggag ataaaaatgc gcataaaagt ccatgctgca gctgataagc 1800
acaattctgt agaggacagt gttacaaaga gccgagagca caaagaaaag cacaagactc 1860
acccatctaa tcatcatcat catcataatc accactcaca caagcactct cattcccaac 1920
ttccagttgg tactgggaac aaacgtcctg gtgatccaaa acatagtagc cagacaagca 1980
acttagcaca taaaacctat agcttgtcta gttctttttc ctcttccagt tctactcgta 2040
aaaggggacc ctctgaagag actggagggg ctgtgtttga tcatccagcc aagattgcca 2100
agagtactaa atcctcttcc ctaaatttct ccttcccttc acttcctaca atgggtcaga 2160
tgcctgggca tagctcagac acaagtggcc tttccttttc acagcccagc tgtaaaactc 2220
gtgtccctca ttcgaaactg gataaagggc ccactggggc caatggtcac aacacgaccc 2280

```
agacaataga ctatcaagac actgtgaata tgcttcactc cctgctcagt gcccagggtg    2340

ttcagcccac tcagcccact gcatttgaat ttgttcgtcc ttatagtgac tatctgaatc    2400

ctcggtctgg tggaatctcc tcgagatctg gcaatacaga caaaccccgg ccaccacctc    2460

tgccatcaga acctcctcca ccacttccac cccttcctaa gtaaaaaaag aaaaagaaga    2520

ggagaaaaaa acttctttaa aaaaacacat aatttttctt tttttttt                 2568


<210> 10
<211> 726
<212> PRT
<213> Homo sapiens

<220>
<221> SOURCE
<222> 1..726
<223> /mol_type="protein"
      /organism="Homo sapiens"

<400> 10
Met Glu Gly Glu Arg Lys Asn Asn Asn Lys Arg Trp Tyr Phe Thr Arg
1               5                   10                  15
Glu Gln Leu Glu Asn Ser Pro Ser Arg Arg Phe Gly Val Asp Pro Asp
            20                  25                  30
Lys Glu Leu Ser Tyr Arg Gln Gln Ala Ala Asn Leu Leu Gln Asp Met
        35                  40                  45
Gly Gln Arg Leu Asn Val Ser Gln Leu Thr Ile Asn Thr Ala Ile Val
    50                  55                  60
Tyr Met His Arg Phe Tyr Met Ile Gln Ser Phe Thr Gln Phe Pro Gly
65                  70                  75                  80
Asn Ser Val Ala Pro Ala Ala Leu Phe Leu Ala Ala Lys Val Glu Glu
                85                  90                  95
Gln Pro Lys Lys Leu Glu His Val Ile Lys Val Ala His Thr Cys Leu
            100                 105                 110
His Pro Gln Glu Ser Leu Pro Asp Thr Arg Ser Glu Ala Tyr Leu Gln
        115                 120                 125
Gln Val Gln Asp Leu Val Ile Leu Glu Ser Ile Ile Leu Gln Thr Leu
    130                 135                 140
Gly Phe Glu Leu Thr Ile Asp His Pro His Thr His Val Val Lys Cys
145                 150                 155                 160
Thr Gln Leu Val Arg Ala Ser Lys Asp Leu Ala Gln Thr Ser Tyr Phe
                165                 170                 175
Met Ala Thr Asn Ser Leu His Leu Thr Thr Phe Ser Leu Gln Tyr Thr
            180                 185                 190
Pro Pro Val Val Ala Cys Val Cys Ile His Leu Ala Cys Lys Trp Ser
        195                 200                 205
Asn Trp Glu Ile Pro Val Ser Thr Asp Gly Lys His Trp Trp Glu Tyr
    210                 215                 220
Val Asp Ala Thr Val Thr Leu Glu Leu Leu Asp Glu Leu Thr His Glu
225                 230                 235                 240
Phe Leu Gln Ile Leu Glu Lys Thr Pro Asn Arg Leu Lys Arg Ile Trp
                245                 250                 255
Asn Trp Arg Ala Cys Glu Ala Ala Lys Lys Thr Lys Ala Asp Asp Arg
            260                 265                 270
Gly Thr Asp Glu Lys Thr Ser Glu Gln Thr Ile Leu Asn Met Ile Ser
        275                 280                 285
Gln Ser Ser Ser Asp Thr Thr Ile Ala Gly Leu Met Ser Met Ser Thr
    290                 295                 300
Ser Thr Thr Ser Ala Val Pro Ser Leu Pro Val Ser Glu Glu Ser Ser
305                 310                 315                 320
Ser Asn Leu Thr Ser Val Glu Met Leu Pro Gly Lys Arg Trp Leu Ser
```

```
                325                 330                 335
Ser Gln Pro Ser Phe Lys Leu Glu Pro Thr Gln Gly His Arg Thr Ser
            340                 345                 350
Glu Asn Leu Ala Leu Thr Gly Val Asp His Ser Leu Pro Gln Asp Gly
        355                 360                 365
Ser Asn Ala Phe Ile Ser Gln Lys Gln Asn Ser Lys Ser Val Pro Ser
    370                 375                 380
Ala Lys Val Ser Leu Lys Glu Tyr Arg Ala Lys His Ala Glu Glu Leu
385                 390                 395                 400
Ala Ala Gln Lys Arg Gln Leu Glu Asn Met Glu Ala Asn Val Lys Ser
                405                 410                 415
Gln Tyr Ala Tyr Ala Ala Gln Asn Leu Leu Ser His His Asp Ser His
            420                 425                 430
Ser Ser Val Ile Leu Lys Met Pro Ile Glu Gly Ser Glu Asn Pro Glu
        435                 440                 445
Arg Pro Phe Leu Glu Lys Ala Asp Lys Thr Ala Leu Lys Met Arg Ile
    450                 455                 460
Pro Val Ala Gly Gly Asp Lys Ala Ala Ser Ser Lys Pro Glu Glu Ile
465                 470                 475                 480
Lys Met Arg Ile Lys Val His Ala Ala Ala Asp Lys His Asn Ser Val
                485                 490                 495
Glu Asp Ser Val Thr Lys Ser Arg Glu His Lys Glu Lys His Lys Thr
            500                 505                 510
His Pro Ser Asn His His His His His Asn His His Ser His Lys His
        515                 520                 525
Ser His Ser Gln Leu Pro Val Gly Thr Gly Asn Lys Arg Pro Gly Asp
    530                 535                 540
Pro Lys His Ser Ser Gln Thr Ser Asn Leu Ala His Lys Thr Tyr Ser
545                 550                 555                 560
Leu Ser Ser Ser Phe Ser Ser Ser Ser Ser Thr Arg Lys Arg Gly Pro
                565                 570                 575
Ser Glu Glu Thr Gly Gly Ala Val Phe Asp His Pro Ala Lys Ile Ala
            580                 585                 590
Lys Ser Thr Lys Ser Ser Ser Leu Asn Phe Ser Phe Pro Ser Leu Pro
        595                 600                 605
Thr Met Gly Gln Met Pro Gly His Ser Ser Asp Thr Ser Gly Leu Ser
    610                 615                 620
Phe Ser Gln Pro Ser Cys Lys Thr Arg Val Pro His Ser Lys Leu Asp
625                 630                 635                 640
Lys Gly Pro Thr Gly Ala Asn Gly His Asn Thr Thr Gln Thr Ile Asp
                645                 650                 655
Tyr Gln Asp Thr Val Asn Met Leu His Ser Leu Leu Ser Ala Gln Gly
            660                 665                 670
Val Gln Pro Thr Gln Pro Thr Ala Phe Glu Phe Val Arg Pro Tyr Ser
        675                 680                 685
Asp Tyr Leu Asn Pro Arg Ser Gly Gly Ile Ser Ser Arg Ser Gly Asn
    690                 695                 700
Thr Asp Lys Pro Arg Pro Pro Pro Leu Pro Ser Glu Pro Pro Pro Pro
705                 710                 715                 720
Leu Pro Pro Leu Pro Lys
                725
```

<210> 11
<211> 8089
<212> DNA
<213> Homo sapiens

<220>
<221> source
<222> 1..8089
<223> /mol_type="DNA"
/organism="Homo sapiens"

<400> 11
gcggcgcgga ggctggcccg ggacgcgccc ggagcccagg gaaggaggga ggaggggagg 60

gtcgcggccg gccgccatgg ggccgggggc ccgtggccgc cgccgccgcc gtcgcccgat 120

gtcgccgcca ccgccaccgc cacccgtgcg ggcgctgccc ctgctgctgc tgctagcggg 180

gccgggggct gcagcccccc cttgcctgga cggaagcccg tgtgcaaatg gaggtcgttg 240

cacccagctg ccctcccggg aggctgcctg cctgtgcccg cctggctggg tgggtgagcg 300

gtgtcagctg gaggacccct gtcactcagg cccctgtgct ggccgtggtg tctgccagag 360

ttcagtggtg gctggcaccg cccgattctc atgccggtgc cccgtggct tccgaggccc 420

tgactgctcc ctgccagatc cctgcctcag cagcccttgt gcccacggtg cccgctgctc 480

agtggggccc gatggacgct tcctctgctc ctgcccacct ggctaccagg gccgcagctg 540

ccgaagcgac gtggatgagt gccgggtggg tgagccctgc cgccatggtg gcacctgcct 600

caacacacct ggctccttcc gctgccagtg tccagctggc tacacagggc cactatgtga 660

gaaccccgcg gtgccctgtg caccctcacc atgccgtaac gggggcacct gcaggcagag 720

tggcgacctc acttacgact gtgcctgtct tcctgggttt gagggtcaga attgtgaagt 780

gaacgtggac gactgtccag gacaccgatg tctcaatggg gggacatgcg tggatggcgt 840

caacacctat aactgccagt gccctcctga gtggacaggc cagttctgca cggaggacgt 900

ggatgagtgt cagctgcagc ccaacgcctg ccacaatggg ggtacctgct tcaacacgct 960

gggtggccac agctgcgtgt gtgtcaatgg ctggacaggc gagagctgca gtcagaatat 1020

cgatgactgt gccacagccg tgtgcttcca tggggccacc tgccatgacc gcgtggcttc 1080

tttctactgt gcctgcccca tgggcaagac tggcctcctg tgtcacctgg atgacgcctg 1140

tgtcagcaac ccctgccacg aggatgctat ctgtgacaca aatccggtga acggccgggc 1200

catttgcacc tgtcctcccg gcttcacggg tggggcatgt gaccaggatg tggacgagtg 1260

ctctatcggc gccaacccct gcgagcactt gggcaggtgc gtgaacacgc agggctcctt 1320

cctgtgccag tgcggtcgtg gctacactgg acctcgctgt gagaccgatg tcaacgagtg 1380

tctgtcgggg ccctgccgaa accaggccac gtgcctcgac cgcataggcc agttcacctg 1440

tatctgtatg gcaggcttca caggaaccta ttgcgaggtg gacattgacg agtgtcagag 1500

tagcccctgt gtcaacggtg ggtctgcaa ggaccgagtc aatggcttca gctgcacctg 1560

cccctcgggc ttcagcggct ccacgtgtca gctggacgtg gacgaatgcg ccagcacgcc 1620

ctgcaggaat ggcgccaaat gcgtggacca gcccgatggc tacgagtgcc gctgtgccga 1680

gggctttgag ggcacgctgt gtgatcgcaa cgtggacgac tgctcccctg acccatgcca 1740

ccatggtcgc tgcgtggatg gcatcgccag cttctcatgt gcctgtgctc ctggctacac 1800

gggcacacgc tgcgagagcc aggtggacga atgccgcagc cagccctgcc gccatggcgg 1860

caaatgccta gacctggtgg acaagtacct ctgccgctgc ccttctggga ccacaggtgt 1920

gaactgcgaa gtgaacattg acgactgtgc cagcaacccc tgcacctttg gagtctgccg 1980

tgatggcatc aaccgctacg actgtgtctg ccaacctggc ttcacagggc ccctttgtaa 2040
cgtggagatc aatgagtgtg cttccagccc atgcggcgag ggaggttcct gtgtggatgg 2100
ggaaaatggc ttccgctgcc tctgcccgcc tggctccttg cccccactct gcctcccccc 2160
gagccatccc tgtgcccatg agccctgcag tcacggcatc tgctatgatg cacctggcgg 2220
gttccgctgt gtgtgtgagc ctggctggag tggcccccgc tgcagccaga gcctggcccg 2280
agacgcctgt gagtcccagc cgtgcagggc cggtgggaca tgcagcagcg atggaatggg 2340
tttccactgc acctgcccgc ctggtgtcca gggacgtcag tgtgaactcc tctccccctg 2400
caccccgaac ccctgtgagc atgggggccg ctgcgagtct gcccctggcc agctgcctgt 2460
ctgctcctgc ccccagggct ggcaaggccc acgatgccag caggatgtgg acgagtgtgc 2520
tggccccgca ccctgtggcc ctcatggtat ctgcaccaac ctggcaggga gtttcagctg 2580
cacctgccat ggagggtaca ctggcccttc ctgcgatcag gacatcaatg actgtgaccc 2640
caacccatgc ctgaacggtg gctcgtgcca agacggcgtg ggctcctttt cctgctcctg 2700
cctccctggt ttcgccggcc cacgatgcgc ccgcgatgtg gatgagtgcc tgagcaaccc 2760
ctgcggcccg ggcacctgta ccgaccacgt ggcctccttc acctgcacct gcccgccagg 2820
ctacggaggc ttccactgcg aacaggacct gcccgactgc agccccagct cctgcttcaa 2880
tggcgggacc tgtgtggacg gcgtgaactc gttcagctgc ctgtgccgtc ccggctacac 2940
aggagcccac tgccaacatg aggcagaccc ctgcctctcg cggccctgcc tacacggggg 3000
cgtctgcagc gccgcccacc ctggcttccg ctgcacctgc ctcgagagct tcacgggccc 3060
gcagtgccag acgctggtgg attggtgcag ccgccagcct tgtcaaaacg gggtcgctg 3120
cgtccagact ggggcctatt gcctttgtcc ccctggatgg agcggacgcc tctgtgacat 3180
ccgaagcttg ccctgcaggg aggccgcagc ccagatcggg gtgcggctgg agcagctgtg 3240
tcaggcgggt gggcagtgtg tggatgaaga cagctcccac tactgcgtgt gcccagaggg 3300
ccgtactggt agccactgtg agcaggaggt ggacccctgc ttggcccagc cctgccagca 3360
tgggggggacc tgccgtggct atatgggggg ctacatgtgt gagtgtcttc ctggctacaa 3420
tggtgataac tgtgaggacg acgtggacga gtgtgcctcc cagccctgcc agcacggggg 3480
ttcatgcatt gacctcgtgg cccgctatct ctgctcctgt cccccaggaa cgctgggggt 3540
gctctgcgag attaatgagg atgactgcgg cccaggccca ccgctggact cagggccccg 3600
gtgcctacac aatggcacct gcgtggacct ggtgggtggt ttccgctgca cctgtccccc 3660
aggatacact ggtttgcgct gcgaggcaga catcaatgag tgtcgctcag gtgcctgcca 3720
cgcggcacac acccgggact gcctgcagga cccaggcgga ggtttccgtt gcctttgtca 3780
tgctggcttc tcaggtcctc gctgtcagac tgtcctgtct ccctgcgagt cccagccatg 3840
ccagcatgga ggccagtgcc gtcctagccc gggtcctggg ggtgggctga ccttcacctg 3900
tcactgtgcc cagccgttct ggggtccgcg ttgcgagcgg gtggcgcgct cctgccggga 3960

gctgcagtgc ccggtgggcg tcccatgcca gcagacgccc cgcgggccgc gctgcgcctg 4020

ccccccaggg ttgtcgggac cctcctgccg cagcttcccg gggtcgccgc cgggggccag 4080

caacgccagc tgcgcggccg ccccctgtct ccacgggggc tcctgccgcc ccgcgccgct 4140

cgcgcccttc ttccgctgcg cttgcgcgca gggctggacc gggccgcgct gcgaggcgcc 4200

cgccgcggca cccgaggtct cggaggagcc gcggtgcccg cgcgccgcct gccaggccaa 4260

gcgcggggac cagcgctgcg accgcgagtg caacagccca ggctgcggct gggacggcgg 4320

cgactgctcg ctgagcgtgg gcgacccctg gcggcaatgc gaggcgctgc agtgctggcg 4380

cctcttcaac aacagccgct gcgaccccgc ctgcagctcg cccgcctgcc tctacgacaa 4440

cttcgactgc cacgccggtg gccgcgagcg cacttgcaac ccggtgtacg agaagtactg 4500

cgccgaccac tttgccgacg gccgctgcga ccagggctgc aacacggagg agtgcggctg 4560

ggatgggctg gattgtgcca gcgaggtgcc ggccctgctg gcccgcggcg tgctggtgct 4620

cacagtgctg ctgccgccag aggagctact gcgttccagc gccgactttc tgcagcggct 4680

cagcgccatc ctgcgcacct cgctgcgctt ccgcctggac gcgcacggcc aggccatggt 4740

cttcccttac caccggccta gtcctggctc cgaaccccgg gcccgtcggg agctggcccc 4800

cgaggtgatc ggctcggtag taatgctgga gattgacaac cggctctgcc tgcagtcgcc 4860

tgagaatgat cactgcttcc ccgatgccca gagcgccgct gactacctgg gagcgttgtc 4920

agcggtggag cgcctggact tcccgtaccc actgcgggac gtgcgggggg agccgctgga 4980

gcctccagaa cccagcgtcc cgctgctgcc actgctagtg gcgggcgctg tcttgctgct 5040

ggtcattctc gtcctgggtg tcatggtggc ccggcgcaag cgcgagcaca gcaccctctg 5100

gttccctgag ggcttctcac tgcacaagga cgtggcctct ggtcacaagg gccggcggga 5160

acccgtgggc caggacgcgc tgggcatgaa gaacatggcc aagggtgaga gcctgatggg 5220

ggaggtggcc acagactgga tggacacaga gtgcccagag gccaagcggc taaaggtaga 5280

ggagccaggc atgggggctg aggaggctgt ggattgccgt cagtggactc aacaccatct 5340

ggttgctgct gacatccgcg tggcaccagc catggcactg acaccaccac agggcgacgc 5400

agatgctgat ggcatggatg tcaatgtgcg tggcccagat ggcttcaccc cgctaatgct 5460

ggcttccttc tgtggggggg ctctggagcc aatgccaact gaagaggatg aggcagatga 5520

cacatcagct agcatcatct ccgacctgat ctgccagggg gctcagcttg ggcacggac 5580

tgaccgtact ggcgagactg ctttgcacct ggctgcccgt tatgcccgtg ctgatgcagc 5640

caagcggctg ctggatgctg ggcagacac caatgcccag gaccactcag gccgcactcc 5700

cctgcacaca gctgtcacag ccgatgccca gggtgtcttc cagattctca tccgaaaccg 5760

ctctacagac ttggatgccc gcatggcaga tggctcaacg gcactgatcc tggcggcccg 5820

cctggcagta gagggcatgg tggaagagct catcgccagc catgctgatg tcaatgctgt 5880

ggatgagctt gggaaatcag ccttacactg ggctgcggct gtgaacaacg tggaagccac 5940
tttggccctg ctcaaaaatg gagccaataa ggacatgcag gatagcaagg aggagacccc 6000
cctattcctg gccgcccgcg agggcagcta tgaggctgcc aagctgctgt tggaccactt 6060
tgccaaccgt gagatcaccg accacctgga caggctgccg cgggacgtag cccaggagag 6120
actgcaccag gacatcgtgc gcttgctgga tcaacccagt gggccccgca gcccccccgg 6180
tccccacggc ctggggcctc tgctctgtcc tccaggggcc ttcctccctg gcctcaaagc 6240
ggcacagtcg gggtccaaga agagcaggag gcccccccggg aaggcggggc tggggccgca 6300
ggggccccgg gggcggggca agaagctgac gctggcctgc ccgggccccc tggctgacag 6360
ctcggtcacg ctgtcgcccg tggactcgct ggactccccg cggcctttcg gtgggccccc 6420
tgcttcccct ggtggcttcc cccttgaggg gccctatgca gctgccactg ccactgcagt 6480
gtctctggca cagcttggtg gcccaggccg ggcgggtcta gggcgccagc cccctggagg 6540
atgtgtactc agcctgggcc tgctgaaccc tgtggctgtg cccctcgatt gggcccggct 6600
gcccccacct gcccctccag gcccctcgtt cctgctgcca ctggcgccgg gaccccagct 6660
gctcaaccca gggacccccg tctccccgca ggagcggccc ccgccttacc tggcagtccc 6720
aggacatggc gaggagtacc cggcggctgg ggcacacagc agccccccaa aggcccgctt 6780
cctgcgggtt cccagtgagc acccttacct gaccccatcc cccgaatccc ctgagcactg 6840
ggccagcccc tcacctccct ccctctcaga ctggtccgaa tccacgccta gcccagccac 6900
tgccactggg gccatggcca ccaccactgg ggcactgcct gcccagccac ttcccttgtc 6960
tgttcccagc tcccttgctc aggcccagac ccagctgggg ccccagccgg aagttacccc 7020
caagaggcaa gtgttggcct gagacgctcg tcagttctta gatcttgggg gcctaaagag 7080
acccccgtcc tgcctccttt ctttctctgt ctcttccttc cttttagtct tttcatcct 7140
cttctctttc caccaaccct cctgcatcct tgccttgcag cgtgaccgag ataggtcatc 7200
agcccagggc ttcagtcttc ctttatttat aatgggtggg ggctaccacc caccctctca 7260
gtcttgtgaa gagtctggga cctccttctt ccccacttct ctcttccctc attcctttct 7320
ctctccttct ggcctctcat ttccttacac tctgacatga atgaattatt attattttta 7380
tttttctttt tttttttaca ttttgtatag aaacaaattc atttaaacaa acttattatt 7440
attatttttt acaaaatata tatatggaga tgctccctcc ccctgtgaac cccccagtgc 7500
ccccgtgggg ctgagtctgt gggcccattc ggccaagctg gattctgtgt acctagtaca 7560
caggcatgac tgggatcccg tgtaccgagt acacgaccca ggtatgtacc aagtaggcac 7620
ccttgggcgc acccactggg gccaggggtc gggggagtgt tgggagcctc ctccccaccc 7680
cacctccctc acttcactgc attccagatg ggacatgttc catagccttg ctggggaagg 7740
gcccactgcc aactccctct gccccagccc caccccttggc catctccctt tgggaactag 7800
ggggctgctg gtgggaaatg ggagccaggg cagatgtatg cattcctttg tgtccctgta 7860

aatgtgggac tacaagaaga ggagctgcct gagtggtact ttctcttcct ggtaatcctc 7920

tggcccagcc tcatggcaga atagaggtat ttttaggcta tttttgtaat atggcttctg 7980

gtcaaaatcc ctgtgtagct gaattcccaa gccctgcatt gtacagcccc ccactcccct 8040

caccacctaa taaaggaata gttaacactc aaaaaaaaaa aaaaaaaaa 8089

<210> 12
<211> 2321
<212> PRT
<213> Homo sapiens

<220>
<221> SOURCE
<222> 1..2321
<223> /mol_type="protein"
/organism="Homo sapiens"

<400> 12
Met Gly Pro Gly Ala Arg Gly Arg Arg Arg Arg Arg Arg Pro Met Ser
1 5 10 15
Pro Pro Pro Pro Pro Pro Pro Val Arg Ala Leu Pro Leu Leu Leu Leu
20 25 30
Leu Ala Gly Pro Gly Ala Ala Ala Pro Pro Cys Leu Asp Gly Ser Pro
35 40 45
Cys Ala Asn Gly Gly Arg Cys Thr Gln Leu Pro Ser Arg Glu Ala Ala
50 55 60
Cys Leu Cys Pro Pro Gly Trp Val Gly Glu Arg Cys Gln Leu Glu Asp
65 70 75 80
Pro Cys His Ser Gly Pro Cys Ala Gly Arg Gly Val Cys Gln Ser Ser
85 90 95
Val Val Ala Gly Thr Ala Arg Phe Ser Cys Arg Cys Pro Arg Gly Phe
100 105 110
Arg Gly Pro Asp Cys Ser Leu Pro Asp Pro Cys Leu Ser Ser Pro Cys
115 120 125
Ala His Gly Ala Arg Cys Ser Val Gly Pro Asp Gly Arg Phe Leu Cys
130 135 140
Ser Cys Pro Pro Gly Tyr Gln Gly Arg Ser Cys Arg Ser Asp Val Asp
145 150 155 160
Glu Cys Arg Val Gly Glu Pro Cys Arg His Gly Gly Thr Cys Leu Asn
165 170 175
Thr Pro Gly Ser Phe Arg Cys Gln Cys Pro Ala Gly Tyr Thr Gly Pro
180 185 190
Leu Cys Glu Asn Pro Ala Val Pro Cys Ala Pro Ser Pro Cys Arg Asn
195 200 205
Gly Gly Thr Cys Arg Gln Ser Gly Asp Leu Thr Tyr Asp Cys Ala Cys
210 215 220
Leu Pro Gly Phe Glu Gly Gln Asn Cys Glu Val Asn Val Asp Asp Cys
225 230 235 240
Pro Gly His Arg Cys Leu Asn Gly Gly Thr Cys Val Asp Gly Val Asn
245 250 255
Thr Tyr Asn Cys Gln Cys Pro Pro Glu Trp Thr Gly Gln Phe Cys Thr
260 265 270
Glu Asp Val Asp Glu Cys Gln Leu Gln Pro Asn Ala Cys His Asn Gly
275 280 285
Gly Thr Cys Phe Asn Thr Leu Gly Gly His Ser Cys Val Cys Val Asn
290 295 300
Gly Trp Thr Gly Glu Ser Cys Ser Gln Asn Ile Asp Asp Cys Ala Thr
305 310 315 320
Ala Val Cys Phe His Gly Ala Thr Cys His Asp Arg Val Ala Ser Phe
325 330 335
Tyr Cys Ala Cys Pro Met Gly Lys Thr Gly Leu Leu Cys His Leu Asp

```
                340                 345                 350
Asp Ala Cys Val Ser Asn Pro Cys His Glu Asp Ala Ile Cys Asp Thr
            355                 360                 365
Asn Pro Val Asn Gly Arg Ala Ile Cys Thr Cys Pro Pro Gly Phe Thr
        370                 375                 380
Gly Gly Ala Cys Asp Gln Asp Val Asp Glu Cys Ser Ile Gly Ala Asn
385                 390                 395                 400
Pro Cys Glu His Leu Gly Arg Cys Val Asn Thr Gln Gly Ser Phe Leu
                405                 410                 415
Cys Gln Cys Gly Arg Gly Tyr Thr Gly Pro Arg Cys Glu Thr Asp Val
            420                 425                 430
Asn Glu Cys Leu Ser Gly Pro Cys Arg Asn Gln Ala Thr Cys Leu Asp
        435                 440                 445
Arg Ile Gly Gln Phe Thr Cys Ile Cys Met Ala Gly Phe Thr Gly Thr
    450                 455                 460
Tyr Cys Glu Val Asp Ile Asp Glu Cys Gln Ser Ser Pro Cys Val Asn
465                 470                 475                 480
Gly Gly Val Cys Lys Asp Arg Val Asn Gly Phe Ser Cys Thr Cys Pro
                485                 490                 495
Ser Gly Phe Ser Gly Ser Thr Cys Gln Leu Asp Val Asp Glu Cys Ala
            500                 505                 510
Ser Thr Pro Cys Arg Asn Gly Ala Lys Cys Val Asp Gln Pro Asp Gly
        515                 520                 525
Tyr Glu Cys Arg Cys Ala Glu Gly Phe Glu Gly Thr Leu Cys Asp Arg
    530                 535                 540
Asn Val Asp Asp Cys Ser Pro Asp Pro Cys His His Gly Arg Cys Val
545                 550                 555                 560
Asp Gly Ile Ala Ser Phe Ser Cys Ala Cys Ala Pro Gly Tyr Thr Gly
                565                 570                 575
Thr Arg Cys Glu Ser Gln Val Asp Glu Cys Arg Ser Gln Pro Cys Arg
            580                 585                 590
His Gly Gly Lys Cys Leu Asp Leu Val Asp Lys Tyr Leu Cys Arg Cys
        595                 600                 605
Pro Ser Gly Thr Thr Gly Val Asn Cys Glu Val Asn Ile Asp Asp Cys
    610                 615                 620
Ala Ser Asn Pro Cys Thr Phe Gly Val Cys Arg Asp Gly Ile Asn Arg
625                 630                 635                 640
Tyr Asp Cys Val Cys Gln Pro Gly Phe Thr Gly Pro Leu Cys Asn Val
                645                 650                 655
Glu Ile Asn Glu Cys Ala Ser Ser Pro Cys Gly Glu Gly Gly Ser Cys
            660                 665                 670
Val Asp Gly Glu Asn Gly Phe Arg Cys Leu Cys Pro Pro Gly Ser Leu
        675                 680                 685
Pro Pro Leu Cys Leu Pro Pro Ser His Pro Cys Ala His Glu Pro Cys
    690                 695                 700
Ser His Gly Ile Cys Tyr Asp Ala Pro Gly Gly Phe Arg Cys Val Cys
705                 710                 715                 720
Glu Pro Gly Trp Ser Gly Pro Arg Cys Ser Gln Ser Leu Ala Arg Asp
                725                 730                 735
Ala Cys Glu Ser Gln Pro Cys Arg Ala Gly Gly Thr Cys Ser Ser Asp
            740                 745                 750
Gly Met Gly Phe His Cys Thr Cys Pro Pro Gly Val Gln Gly Arg Gln
        755                 760                 765
Cys Glu Leu Leu Ser Pro Cys Thr Pro Asn Pro Cys Glu His Gly Gly
    770                 775                 780
Arg Cys Glu Ser Ala Pro Gly Gln Leu Pro Val Cys Ser Cys Pro Gln
785                 790                 795                 800
Gly Trp Gln Gly Pro Arg Cys Gln Gln Asp Val Asp Glu Cys Ala Gly
                805                 810                 815
Pro Ala Pro Cys Gly Pro His Gly Ile Cys Thr Asn Leu Ala Gly Ser
            820                 825                 830
Phe Ser Cys Thr Cys His Gly Gly Tyr Thr Gly Pro Ser Cys Asp Gln
        835                 840                 845
Asp Ile Asn Asp Cys Asp Pro Asn Pro Cys Leu Asn Gly Gly Ser Cys
    850                 855                 860
```

Gln Asp Gly Val Gly Ser Phe Ser Cys Ser Cys Leu Pro Gly Phe Ala
865 870 875 880
Gly Pro Arg Cys Ala Arg Asp Val Asp Glu Cys Leu Ser Asn Pro Cys
885 890 895
Gly Pro Gly Thr Cys Thr Asp His Val Ala Ser Phe Thr Cys Thr Cys
900 905 910
Pro Pro Gly Tyr Gly Gly Phe His Cys Glu Gln Asp Leu Pro Asp Cys
915 920 925
Ser Pro Ser Ser Cys Phe Asn Gly Gly Thr Cys Val Asp Gly Val Asn
930 935 940
Ser Phe Ser Cys Leu Cys Arg Pro Gly Tyr Thr Gly Ala His Cys Gln
945 950 955 960
His Glu Ala Asp Pro Cys Leu Ser Arg Pro Cys Leu His Gly Gly Val
965 970 975
Cys Ser Ala Ala His Pro Gly Phe Arg Cys Thr Cys Leu Glu Ser Phe
980 985 990
Thr Gly Pro Gln Cys Gln Thr Leu Val Asp Trp Cys Ser Arg Gln Pro
995 1000 1005
Cys Gln Asn Gly Gly Arg Cys Val Gln Thr Gly Ala Tyr Cys Leu Cys
1010 1015 1020
Pro Pro Gly Trp Ser Gly Arg Leu Cys Asp Ile Arg Ser Leu Pro Cys
1025 1030 1035 1040
Arg Glu Ala Ala Ala Gln Ile Gly Val Arg Leu Glu Gln Leu Cys Gln
1045 1050 1055
Ala Gly Gly Gln Cys Val Asp Glu Asp Ser Ser His Tyr Cys Val Cys
1060 1065 1070
Pro Glu Gly Arg Thr Gly Ser His Cys Glu Gln Glu Val Asp Pro Cys
1075 1080 1085
Leu Ala Gln Pro Cys Gln His Gly Gly Thr Cys Arg Gly Tyr Met Gly
1090 1095 1100
Gly Tyr Met Cys Glu Cys Leu Pro Gly Tyr Asn Gly Asp Asn Cys Glu
1105 1110 1115 1120
Asp Asp Val Asp Glu Cys Ala Ser Gln Pro Cys Gln His Gly Gly Ser
1125 1130 1135
Cys Ile Asp Leu Val Ala Arg Tyr Leu Cys Ser Cys Pro Pro Gly Thr
1140 1145 1150
Leu Gly Val Leu Cys Glu Ile Asn Glu Asp Asp Cys Gly Pro Gly Pro
1155 1160 1165
Pro Leu Asp Ser Gly Pro Arg Cys Leu His Asn Gly Thr Cys Val Asp
1170 1175 1180
Leu Val Gly Gly Phe Arg Cys Thr Cys Pro Pro Gly Tyr Thr Gly Leu
1185 1190 1195 1200
Arg Cys Glu Ala Asp Ile Asn Glu Cys Arg Ser Gly Ala Cys His Ala
1205 1210 1215
Ala His Thr Arg Asp Cys Leu Gln Asp Pro Gly Gly Gly Phe Arg Cys
1220 1225 1230
Leu Cys His Ala Gly Phe Ser Gly Pro Arg Cys Gln Thr Val Leu Ser
1235 1240 1245
Pro Cys Glu Ser Gln Pro Cys Gln His Gly Gly Gln Cys Arg Pro Ser
1250 1255 1260
Pro Gly Pro Gly Gly Gly Leu Thr Phe Thr Cys His Cys Ala Gln Pro
1265 1270 1275 1280
Phe Trp Gly Pro Arg Cys Glu Arg Val Ala Arg Ser Cys Arg Glu Leu
1285 1290 1295
Gln Cys Pro Val Gly Val Pro Cys Gln Gln Thr Pro Arg Gly Pro Arg
1300 1305 1310
Cys Ala Cys Pro Pro Gly Leu Ser Gly Pro Ser Cys Arg Ser Phe Pro
1315 1320 1325
Gly Ser Pro Pro Gly Ala Ser Asn Ala Ser Cys Ala Ala Ala Pro Cys
1330 1335 1340
Leu His Gly Gly Ser Cys Arg Pro Ala Pro Leu Ala Pro Phe Phe Arg
1345 1350 1355 1360
Cys Ala Cys Ala Gln Gly Trp Thr Gly Pro Arg Cys Glu Ala Pro Ala
1365 1370 1375
Ala Ala Pro Glu Val Ser Glu Glu Pro Arg Cys Pro Arg Ala Ala Cys

1380 1385 1390
Gln Ala Lys Arg Gly Asp Gln Arg Cys Asp Arg Glu Cys Asn Ser Pro
1395 1400 1405
Gly Cys Gly Trp Asp Gly Gly Asp Cys Ser Leu Ser Val Gly Asp Pro
1410 1415 1420
Trp Arg Gln Cys Glu Ala Leu Gln Cys Trp Arg Leu Phe Asn Asn Ser
1425 1430 1435 1440
Arg Cys Asp Pro Ala Cys Ser Ser Pro Ala Cys Leu Tyr Asp Asn Phe
1445 1450 1455
Asp Cys His Ala Gly Gly Arg Glu Arg Thr Cys Asn Pro Val Tyr Glu
1460 1465 1470
Lys Tyr Cys Ala Asp His Phe Ala Asp Gly Arg Cys Asp Gln Gly Cys
1475 1480 1485
Asn Thr Glu Glu Cys Gly Trp Asp Gly Leu Asp Cys Ala Ser Glu Val
1490 1495 1500
Pro Ala Leu Leu Ala Arg Gly Val Leu Val Leu Thr Val Leu Leu Pro
1505 1510 1515 1520
Pro Glu Glu Leu Leu Arg Ser Ser Ala Asp Phe Leu Gln Arg Leu Ser
1525 1530 1535
Ala Ile Leu Arg Thr Ser Leu Arg Phe Arg Leu Asp Ala His Gly Gln
1540 1545 1550
Ala Met Val Phe Pro Tyr His Arg Pro Ser Pro Gly Ser Glu Pro Arg
1555 1560 1565
Ala Arg Arg Glu Leu Ala Pro Glu Val Ile Gly Ser Val Val Met Leu
1570 1575 1580
Glu Ile Asp Asn Arg Leu Cys Leu Gln Ser Pro Glu Asn Asp His Cys
1585 1590 1595 1600
Phe Pro Asp Ala Gln Ser Ala Ala Asp Tyr Leu Gly Ala Leu Ser Ala
1605 1610 1615
Val Glu Arg Leu Asp Phe Pro Tyr Pro Leu Arg Asp Val Arg Gly Glu
1620 1625 1630
Pro Leu Glu Pro Pro Glu Pro Ser Val Pro Leu Leu Pro Leu Leu Val
1635 1640 1645
Ala Gly Ala Val Leu Leu Leu Val Ile Leu Val Leu Gly Val Met Val
1650 1655 1660
Ala Arg Arg Lys Arg Glu His Ser Thr Leu Trp Phe Pro Glu Gly Phe
1665 1670 1675 1680
Ser Leu His Lys Asp Val Ala Ser Gly His Lys Gly Arg Arg Glu Pro
1685 1690 1695
Val Gly Gln Asp Ala Leu Gly Met Lys Asn Met Ala Lys Gly Glu Ser
1700 1705 1710
Leu Met Gly Glu Val Ala Thr Asp Trp Met Asp Thr Glu Cys Pro Glu
1715 1720 1725
Ala Lys Arg Leu Lys Val Glu Glu Pro Gly Met Gly Ala Glu Glu Ala
1730 1735 1740
Val Asp Cys Arg Gln Trp Thr Gln His His Leu Val Ala Ala Asp Ile
1745 1750 1755 1760
Arg Val Ala Pro Ala Met Ala Leu Thr Pro Pro Gln Gly Asp Ala Asp
1765 1770 1775
Ala Asp Gly Met Asp Val Asn Val Arg Gly Pro Asp Gly Phe Thr Pro
1780 1785 1790
Leu Met Leu Ala Ser Phe Cys Gly Gly Ala Leu Glu Pro Met Pro Thr
1795 1800 1805
Glu Glu Asp Glu Ala Asp Asp Thr Ser Ala Ser Ile Ile Ser Asp Leu
1810 1815 1820
Ile Cys Gln Gly Ala Gln Leu Gly Ala Arg Thr Asp Arg Thr Gly Glu
1825 1830 1835 1840
Thr Ala Leu His Leu Ala Ala Arg Tyr Ala Arg Ala Asp Ala Ala Lys
1845 1850 1855
Arg Leu Leu Asp Ala Gly Ala Asp Thr Asn Ala Gln Asp His Ser Gly
1860 1865 1870
Arg Thr Pro Leu His Thr Ala Val Thr Ala Asp Ala Gln Gly Val Phe
1875 1880 1885
Gln Ile Leu Ile Arg Asn Arg Ser Thr Asp Leu Asp Ala Arg Met Ala
1890 1895 1900

```
Asp Gly Ser Thr Ala Leu Ile Leu Ala Ala Arg Leu Ala Val Glu Gly
1905                1910                1915                1920
Met Val Glu Glu Leu Ile Ala Ser His Ala Asp Val Asn Ala Val Asp
                1925                1930                1935
Glu Leu Gly Lys Ser Ala Leu His Trp Ala Ala Ala Val Asn Asn Val
            1940                1945                1950
Glu Ala Thr Leu Ala Leu Leu Lys Asn Gly Ala Asn Lys Asp Met Gln
        1955                1960                1965
Asp Ser Lys Glu Glu Thr Pro Leu Phe Leu Ala Ala Arg Glu Gly Ser
    1970                1975                1980
Tyr Glu Ala Ala Lys Leu Leu Leu Asp His Phe Ala Asn Arg Glu Ile
1985                1990                1995                2000
Thr Asp His Leu Asp Arg Leu Pro Arg Asp Val Ala Gln Glu Arg Leu
                2005                2010                2015
His Gln Asp Ile Val Arg Leu Leu Asp Gln Pro Ser Gly Pro Arg Ser
            2020                2025                2030
Pro Pro Gly Pro His Gly Leu Gly Pro Leu Leu Cys Pro Pro Gly Ala
        2035                2040                2045
Phe Leu Pro Gly Leu Lys Ala Ala Gln Ser Gly Ser Lys Lys Ser Arg
    2050                2055                2060
Arg Pro Pro Gly Lys Ala Gly Leu Gly Pro Gln Gly Pro Arg Gly Arg
2065                2070                2075                2080
Gly Lys Lys Leu Thr Leu Ala Cys Pro Gly Pro Leu Ala Asp Ser Ser
                2085                2090                2095
Val Thr Leu Ser Pro Val Asp Ser Leu Asp Ser Pro Arg Pro Phe Gly
            2100                2105                2110
Gly Pro Pro Ala Ser Pro Gly Gly Phe Pro Leu Glu Gly Pro Tyr Ala
        2115                2120                2125
Ala Ala Thr Ala Thr Ala Val Ser Leu Ala Gln Leu Gly Gly Pro Gly
    2130                2135                2140
Arg Ala Gly Leu Gly Arg Gln Pro Pro Gly Gly Cys Val Leu Ser Leu
2145                2150                2155                2160
Gly Leu Leu Asn Pro Val Ala Val Pro Leu Asp Trp Ala Arg Leu Pro
                2165                2170                2175
Pro Pro Ala Pro Pro Gly Pro Ser Phe Leu Leu Pro Leu Ala Pro Gly
            2180                2185                2190
Pro Gln Leu Leu Asn Pro Gly Thr Pro Val Ser Pro Gln Glu Arg Pro
        2195                2200                2205
Pro Pro Tyr Leu Ala Val Pro Gly His Gly Glu Glu Tyr Pro Ala Ala
    2210                2215                2220
Gly Ala His Ser Ser Pro Pro Lys Ala Arg Phe Leu Arg Val Pro Ser
2225                2230                2235                2240
Glu His Pro Tyr Leu Thr Pro Ser Pro Glu Ser Pro Glu His Trp Ala
                2245                2250                2255
Ser Pro Ser Pro Pro Ser Leu Ser Asp Trp Ser Glu Ser Thr Pro Ser
            2260                2265                2270
Pro Ala Thr Ala Thr Gly Ala Met Ala Thr Thr Thr Gly Ala Leu Pro
        2275                2280                2285
Ala Gln Pro Leu Pro Leu Ser Val Pro Ser Ser Leu Ala Gln Ala Gln
    2290                2295                2300
Thr Gln Leu Gly Pro Gln Pro Glu Val Thr Pro Lys Arg Gln Val Leu
2305                2310                2315                2320
Ala
```

## Description

The present invention relates to a CDK9 inhibitor, especially a selective CDK9 inhibitor, for use in treating, ameliorating and/or preventing midline carcinoma. Also corresponding methods for treating, preventing or ameliorating midline carcinoma are subject of the present invention. Preferably, NUT midline carcinoma is treated with the CDK9 inhibitors in accordance with the present invention.

Midline carcinomas are carcinomas arising in midline organs of subjects/patients, such as in the head, neck or mediastinum. One type of midline carcinomas are NUT midline carcinomas (subsequently referred to as "NMC"). NMC is a highly lethal cancer that has previously been described to occur in young adults and children; see French (2004) J Clin Oncology 22(20), 4135-4139. However, recent publications indicate that NMC occurs in children and adults of all ages; see French (2010) J Clin Pathol 63: 492-496.

NMC is a disease which is genetically defined by rearrangements in the nuclear protein in testis (NUT) gene on chromosome 15q14 most commonly in a balanced translocation with the BRD4 gene or the BRD3 gene. A corresponding rearrangement has first been disclosed in a cell line termed Ty-82 which had been derived from a 22-year old woman with undifferentiated thymic carcinoma; see Kuzume (1992) Int J Cancer 50, 259-264. Later, it has been found that this translocation involving rearrangement in the NUT gene is characteristic for a particularly aggressive form of a midline carcinoma and the term NUT midline carcinoma has been coined; see French (2001) Am J Pathol 159(6), 1987-1992.

NMC as a genetically defined disease does not arise from a specific organ. Most cases occur in the mediastinum and upper aerodigestive tract, but in some cases tumors have arisen in bone, bladder, abdominal retroperitoneum, pancrease and salivary glands; see French (2010), Cancer Genetics and Cytogenetics 203, 16-20 and Ziai (2010) Head and Neck Pathol 4, 163-168.

In about two thirds of NMC cases NUT is fused to BRD4 on chromosome 19; see French (2003) Cancer Res 63, 304-307 and French (2008) Oncogene 27, 2237-2242. French (2008) found that in certain cases NUT may also be fused to BRD3. Further, the authors of this document investigated the functional role of BRD-NUT fusion proteins using an siRNA assay for silencing expression. It was found that the suppression of expression of such fusion genes results in squameous differentiation and cell cycle arrest and it was concluded that BRD-NUT fusion proteins contribute to carcinogenesis. It has been suggested in the art that NUT rearrangement is a very early, possible tumour-initiating event; see French (2010) J Clin Pathol (loc. cit.).

NUT rearrangements are restricted to NMC and, therefore, the diagnosis of NMC is not in question once NUT rearrangement has been detected by immunuohistochemical testing (e.g. FISH) or by molecular testing like detection of the expression of NUT fusion genes, in particular BRD4-NUT fusion genes, BRD3-NUT fusion genes or fusions of NUT with other uncharacterised genes (termed NUT-variant fusion genes). The expression of such fusion genes goes along with corresponding NUT rearrangements. Also NMC diagnosis via detection of NUT expression with a NUT specific monoclonal antibody has been disclosed in the art; see Haack (2009) Am J Surg Pathol 33(7), 984-991. Thus, the challenge is not the diagnosis of NMC but rather the decision to perform the diagnosis on subject suspected of suffering from NMC.

Generally, it is believed that midline carcinoma, especially NMC, is a rare type of cancer; however, most cases of NMC currently go unrecognized due to its lack of characteristic histological features; see French (2010) J Clin Pathol loc. cit. NMCs are often mistaken for other cancer types such as thymic carcinoma, squamous cell carcinoma of the head and neck, lung carcinoma, Ewing sarcoma, and acute leukemia; see Schwartz (2011) Cancer Res 71(7), 2686-2696. French (2010) J Clin Pathol loc. cit. has proposed to consider any poorly differentiated, monomorphic, midline neoplasm that does not stain for lineage-specific markers for NUT rearrangement testing. Many patients with presently undiagnosed NMC would profit enormously from diagnosis and subsequent effective treatment of NMC.

Unfortunately, an effective therapy of midline carcinoma, such as NMC, is presently not available resulting in a low survival rate (1 survival out of 22 reported cases) and a mean survival of less than 1 year (9.5 months) despite aggressive chemotherapy and radiation treatment, as summarized in Table 1 of French (2010) J Clin Pathol, loc.cit. and French (2010), Cancer Genetics and Cytogenetics 203, 16-20. Further, numerous NMC tumors might not be treated at all or treatment might commence late due to a late or absent NMC diagnosis. Though reliable diagnosis of NMC is, in principle, available, there is, thus, a need in the art for the efficient treatment of midline carcinoma, especially of NMC.

Potential therapies of midline carcinoma, such as NMC, have been proposed in the art. Schwartz (loc.cit.) has investigated the mechanism underlying an NMC subtype that is characterized by the expression of the BRD4-NUT fusion gene. Schwartz found that expression of BRD4-NUT is associated with globally decreased histone deacetylation and transcriptional repression. Therefore, the authors of this document suggest the use of histone deacetylase inhibitors (HDACi) such as vorinostat and romidepsin in order to revert this effect and to thereby treat NMC. Schwartz also suggests the use of small molecule bromodomain inhibitors (Brdi) to target BRD4-NUT; yet, the authors emphasize that the most specific targeting of BRD4-NUT would be directed at NUT and that the potential difficulties in identifying deliverable NUT-directed inhibitors may be facilitated by the recent development of stapled peptides. In line with Schwartz (loc. cit.)

the international patent application WO 2010/011700 describes the use of compounds, in particular histone deacetylase inhibitors, that promote increased acetylation of histones for the treatment of a cancer characterized by NUT or BRD chromosomal rearrangments. Also Filippakopoulos (2010) propose the BRD4-NUT fusion as therapeutic target in NMC using a BRD4-directed inhibitor termed JQ1 (a thieno-triazolo-1,4-diazepine).

Alsarraj (2011) Cancer Res (author manuscript accepted for publication, doi:10.1158/0008-5472.CAN-10-4417) explores the role of the bromodomain-containing chromatin modifying factor BRD4 in development of cancer. Alsarraj describes that ectopic Brd4 expression represses primary tumor growth and that Brd4 activation is predictive for good outcome in human breast cancer; the authors of this document speculate that the tumor- and the metastasis-suppressive properties of Brd4 may be associated with the presence of a proline-rich region in the C-terminal part of one isoform while the extreme C-terminal domain containing a P-TEFb binding region is found to act as a metastasis enhancer. However, Alsarraj does not suggest a potential treatment of cancer and is not concerned at all with midline carcinoma, such as NMC.

Thus, the technical problem underlying the present invention is the provision of means and methods allowing the therapeutic intervention in midline carcinoma.

The technical problem is solved by provision of the embodiments characterized in the claims.

Accordingly, the present invention relates to a CDK9 inhibitor for use in treating, ameliorating and/or preventing midline carcinoma.

In a further embodiment, the present invention relates to a method for treating, preventing or ameliorating midline carcinoma comprising the administration of a CDK9 inhibitor to a subject in need of such a treatment, prevention or amelioration. Preferably, the subject is a human.

As shown in the appended examples, it was surprisingly found that cells that comprise a rearrangement in the NUT gene are in particular susceptible to a CDK9 inhibitors. The CDK9 inhibitors are also useful in the treatment of midline carcinomas in general. The examples provided herein show that CDK9 inhibitors, such as selective CDK9 inhibitors, can successfully be employed in the treatment of NUT midline carcinoma (NMC) which is, by definition, characterized by rearrangements in the NUT gene. Nothing in the art suggested the use of CDK9 inhibitors in this context. Preferably, CDK9 inhibitors Cpd B2 and Cpd B1 are used. These and further CDK9 inhibitor that may be used are described herein below in more detail. In accordance with the above, the treatment, prevention or amelioration of NUT midline carcinoma (NMC) is preferred herein. Further it is expected that the use of CDK9 inhibitors, especially of the selective CDK9 inhibitors is associated with less side effects.

The tumor cell or cancer cells of the NMC to be treated in accordance with the present invention may comprise at least one rearrangement in the NUT gene, i.e. the NMC is characterized by the presence of at least one rearrangment in the NUT gene in said tumor cell or cancer cell. The term "rearrangement in the NUT gene" refers to any rearrangement in the NUT gene that is characteristic for NUT midline carcinoma (NMC) or a rearrangement resulting in the expression of a Brd/Nut fusion protein. Exemplary "rearrangments in the NUT gene" as well as methods for their detection are known in the art (see, for example, French (2010) J Clin Pathol, loc. cit.) and also described herein. Whether a tumor or cancer cell has such a rearrangement, may be determined in an individual, isolated tumor cell or biological/medical/pathological samples, like body fluids, isolated body tissue samples and the like, wherein said samples preferably comprise cells or cell debris to be analyzed.

As mentioned above, rearrangements in the NUT gene have never been disclosed in context with susceptibility to CDK9 inhibitors, such as selective CDK9 inhibitors. At most, HDAC inhibitors, BRD inhbitors or NUT inhbitors have been proposed in context of the development of potential therapies of midline carcinomas; see Schwartz, loc. cit. In particular, Patients suffering from cancer with (a) rearrangement(s) in the NUT gene (like patients suffering from NMC) have a particularly low survival rate and a bad prognosis and known therapies are not effective. These patients and also patients suffering from midline carcinoma in general will, therefore, profit enormously from the herein provided therapy with CDK9 inhibitors.

It is also envisaged herein that one, two or more different CDK9 inhibitors (i.e. CDK9 inhibitors having different chemical formulae, optionally non-structurally related CDK9 inhibitors) may be used simultaneously. Preferred CDK9 inhibitors to be used in the present invention are described herein below.

As used herein, a kinase "inhibitor" refers to any compound capable of downregulating, decreasing, suppressing or otherwise regulating the amount and/or activity of a kinase. Inhibition of these kinases can be achieved by any of a variety of mechanisms known in the art, including, but not limited to binding directly to the kinase polypeptide, denaturing or otherwise inactivating the kinase, or inhibiting the expression of the gene (e.g., transcription to mRNA, translation to a nascent polypeptide, and/or final polypeptide modifications to a mature protein), which encodes the kinase. Generally, kinase inhibitors may be proteins, polypeptides, nucleic acids, small molecules, or other chemical moieties.

As used herein the term "inhibiting" or "inhibition" refers to the ability of a compound to downregulate, decrease, reduce, suppress, inactivate, or inhibit at least partially the activity of an enzyme, or the expression of an enzyme or protein and/or the virus replication.

The term "CDK9 inhibitor" means accordingly in this context a compound capable of inhibiting the expression

and/or activity of "CDK9" defined herein. An CDK9 inhibitor may, for example, interfere with transcription of a CDK9 gene, processing (e.g. splicing, export from the nucleus and the like) of the gene product (e.g. unspliced or partially spliced mRNA) and/or translation of the gene product (e.g. mature mRNA). The CDK9 inhibitor may also interfere with further modification (like phosphorylation) of the polypeptide/protein encoded by the CDK9 gene and thus completely or partially inhibit the activity of the CDK9 protein as described herein above. Furthermore, the CDK9 inhibitor may interfere with interactions of the CDK9 protein with other proteins.

In accordance with the above, the compounds according to the general formula (I) disclosed herein below as well as pharmaceutically acceptable salts thereof are used as an inhibitor for a protein kinase, preferably as an inhibitor for a cellular protein kinase.

In a preferred embodiment of this aspect said cellular protein kinase consists of Cyclin-dependent protein kinases (CDKs). The cyclin-dependent protein kinase can be selected from the group comprising: CDK1, CDK2, CDK2, CDK4, CDK5, CDK6, CDK7, CDK8, CDK9, CDK10, CDK11, CrkRS (Crk7, CDC2-related protein kinase 7), CDKL1 (cyclin-dependent kinase-like 1); KKIALRE, CDKL2 (cyclin-dependent kinase-like 2), KKIAMRE, CDKL3 (cyclin-dependent kinase-like 3), NKIAMRE, CDKL4, similar to cyclin-dependent kinase-like 1, CDC2L1 (cell division cycle 2-like 1), PITSLRE B, CDC2L1 (cell division cycle 2-like 1), PITSLRE A, CDC2L5 (cell division cycle 2-like 5), PCTK1 (PCTAIRE protein kinase 1), PCTK2 (PCTAIRE protein kinase 2), PCTK3 (PCTAIRE protein kinase 3) or PFTK1 (PFTAIRE protein kinase 1).

In a particularly preferred embodiment said cyclin-dependent protein kinase is CDK9. Thus, the compounds according to the general formula (I) as well as pharmaceutically acceptable salts thereof are, in a very preferred embodiment, used as an inhibitor for CDK9, in particular as a selective CDK9 inhibitor.

Furthermore, in another particularly preferred embodiment the compounds according to the invention show a high potency (demonstrated by a low $IC_{50}$ value) for inhibiting CDK9 activity. In context of the present invention, the $IC_{50}$ value with respect to CDK9 can be determined by the methods described in the method section of PCT patent application PCT/EP2011/001445 which is incorporated herein by reference in its entirety. Preferably, it is determined according to the method described in section 3.6 of said PCT patent application PCT/EP2011/001445.

Surprisingly it turned out that the compounds according to the general formula (I) as well as pharmaceutically acceptable salts thereof selectively inhibit CDK9 in comparison to other protein kinases and in comparison to other cyclin-dependent protein kinases. Thus, the compounds according to the general formula (I) as well as pharmaceutically acceptable salts thereof are used as selective inhibitors for CDK9.

Particularly preferred compounds of the present invention according to formula (I) show a stronger CDK9 than CDK2 inhibition. In context of the present invention, the $IC_{50}$ value with respect to CDK2 can be determined by the methods described in the method section of PCT patent application PCT/EP2011/001445. Preferably, it is determined according to the method described in section 3.5 of PCT/EP2011/001445.

Selectivity expresses the biologic fact that at a given compound concentration enzymes (or proteins) are affected to different degrees. In the case of enzymes selective inhibition can be defined as preferred inhibition by a compound at a given concentration. Or in other words, an enzyme is selectively inhibited over another enzyme when there is a concentration which results in inhibition of the first enzyme whereas the second enzyme is not affected. To compare compound effects on different enzymes it is crucial to employ similar assay formats, such as the LANCE assay as described in more detail below.

The inhibitors to be used herein are preferably specific for CDK9, i.e. the compounds specifically inhibit CDK9. In other words, the CDK9 inhibitors are preferably selective CDK9 inhibitors.

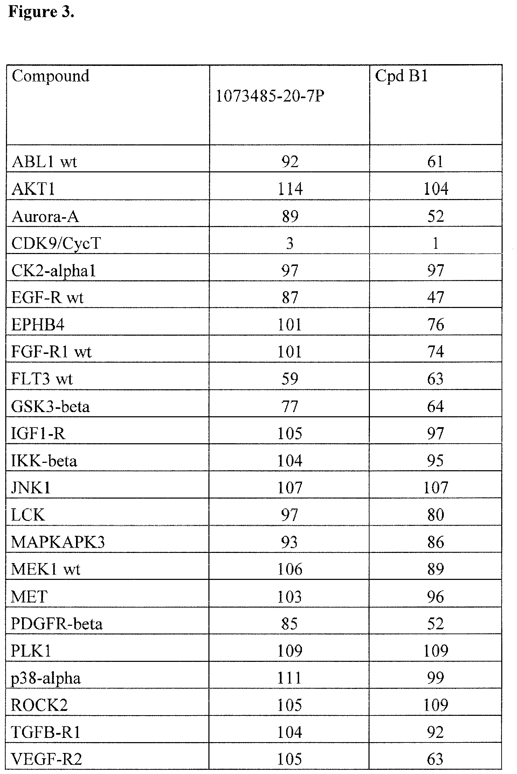

This is inter alia shown in Figure 3 where the inhibiting effect of exemplary compounds on CDK9 is demonstrated.

A radiometric protein kinase assay (33PanQinase® Activity Assay) was used for measuring the kinase activity of protein kinases employing exemplary CDK9 inhibitors to be used in the present invention (see Figure **3**). The low kinase activities of CDK9 show that exemplary compounds potently inhibit CDK9. Activities of other kinases are not inhibited.

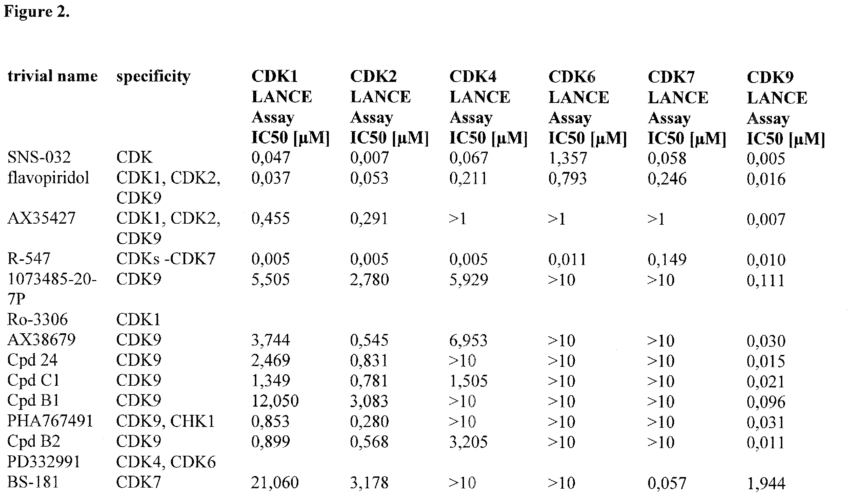

In the experimental part selectivity of the herein provided inhibitors for CDK9 is shown using, inter alia, the well known Lance Assay; see Figure **2**. The Lance assay has been described for example in Moshinsky et al.; 2003 (A widely applicable, high-throughput TR-FRET assay for the measurement of kinase autophosphorylation: VEGFR-2 as a prototype. Moshinsky DJ, Ruslim L, Blake RA, Tang F. J Biomol Screen. 2003 Aug;8(4):447-52). The Lance Ultra KinaSelect Assay may be used to determine half maximal inhibitory concentration ($IC_{50}$) of inhibitor compounds and CDK/Cyclin complexes. The principle behind this enzymatic assay is based upon the phosphorylation of the Ulight-Peptide Substrat. It is detected by using a specific EU-labeled anti-phospho peptide antibody. The binding of the Eu labeled anti-phospho peptide antibody to the phosphorylated U*Light* labeled peptide gives rise to a FRET-signal. Binding of an inhibitor to the kinase prevents phosphorylation of the U*light*-MBP Substrat, resulting in a loss of FRET. Based on these results, the IC50 value can be determined.

The Lance assay and the $^{33}$PanQinase® assay may be performed as follows:

Typically such experiments are started by generation of compounds which are serially diluted in multi titer plates

in dimethylsulfoxide (DMSO). In the next step, working solutions for the enzymes, the substrates (protein and ATP separately) are generated in enzyme buffer. The preparation of the assay plate (definition of positive and negative control, reference inhibitors, test compounds and the pipetting of all solutions and compounds except the ATP working solution) is done within the next step. Finally the reaction is started by the addition of the ATP working solution. All pipetting steps can be done manually or by the help of robotics. Within the incubation of 1 h at room temperature the enzyme catalyzes the generation of phosphorylated substrate. This reaction is more ore less inhibited by the added compounds. Finally, to stop the reaction and to detect phosphorylated substrate detection buffer (see material and methods) is added followed by another incubation of 1 h. The data is evaluated by measuring the FRET-Signal. Data is processed by subtraction of the backgroung signal (negative control) from all investigated activities. These activities are set into relation to the positive control. Altogether this is shown by the following equation:

resulting activity (%) = 100 X [(signal of compound – signal of negative control) / (signal of positive control – signal of negative control)]

Further analysis steps include the determination of IC50 values by using the activities of a dose response experiment and an algorithm (equation #205 in Excel fit) for calculation.

A similar experimental procedure is performed when the resulting activity within $^{33}$PanQinase® assay is done. In advance buffers are prepared but in this case the pipetting sequence is first ATP solution diluted with assay buffer, DMSO or compound solution. The reaction (1h at 30°C) is started by addition of a substrate-kinase mix. During the incubation the kinase phosphorylates the substrate (different for each kinase). Due to the fact that the ATP solution contains $^{33}$P-labelled ATP the substrate proteins are labeled with $^{33}$P. The reaction is stopped by addition of excess $H_3PO_4$. If the reaction is performed in plates binding substrate proteins, said plates are washed to reduce unspecific signals (mainly not used ATP). The incorporation of $^{33}$P into substarte proteins is a direct measure of activity of the respective kinase. Therefore, the incorporated radioactivity is detected by scintillation counting. Data is evaluated, processed and analyzed as described for the LANCE assays.

From Figure 2 it can be deduced that a known CDK7-inhibitor (BS-181) has an IC50 value of 1.944 in the CDK9 Lance Assay. As shown in the experimental part, the IC50 value determined for exemplary selective CDK9 inhibitors, for example according to the Lance Assay, is low, preferably below 0.2 μM, more preferably, below 0.15 μM, 0.14 μM, 0.13 μM, 0.12 μM or even lower. More preferably, the IC50 value is below 0.1 μM, 0.095 μM, 0.090 μM, 0.085 μM, 0.080 μM, 0.075 μM, 0.070 μM, 0.065 μM, 0.060 μM, 0.055 μM, 0.050 μM, 0.045 μM, 0.040 μM , 0.035 μM, 0.030 μM, or even below 0.025 μM, wherein the lower values are preferred over the higher values. Even more preferably, the IC50 value is below 0.024 μM, 0.023 μM, 0.022 μM, 0.021 μM, 0.020 μM, 0.019 μM, 0.018 μM, 0.017 μM, 0.016 μM, 0.015 μM, 0.014 μM, 0.013 μM, 0.012 μM, or 0.011 μM. The IC50 value may even be lower, for example, below 0.010 μM, 0.009 μM, 0.008 μM, 0.007 μM, 0.006 μM, or 0.005 μM. Generally, the lower values are preferred herein over the higher values.

It is preferred herein that the ratio of IC50 values of selective CDK9-inhibitors determined according to the CDK9 Lance assay and IC50 values of selective CDK9-inhibitors determined according to the CDK1 Lance assay, CDK2 Lance assay, CDK4 Lance assay, and/or the CDK6 Lance assay is about 1:10 or lower. A ratio of 1:10 or lower also indicates selectivity of the inhibitor for CDK9. More preferred is a ratio of 1:10, 1:20, 1:30, 1:40, 1:50, 1:60, 1:70, 1:80, 1:90 or 1:100 or even lower.

The following selective CDK9 inhibitors are preferably used in accordance with the present invention; these and further selective CDK9 inhibitors for use in the present invention are described in PCT/EP2011/001445, EP10075131.2 (filing date 22.03.2010) EP11075037.9 (filing date 02.03.2011) and EP11075038.7 (filing date 02.03.2011) which are incorporated herein by reference in their entirety.

The disubstituted triazine compounds to be used according to the present invention are defined by the general formula (I)

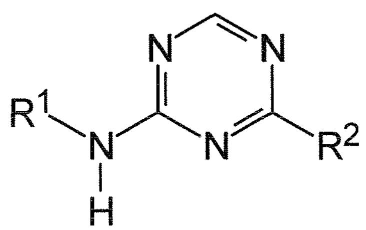

Formula (I)

wherein
**$R^1$** is

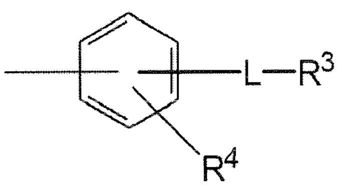 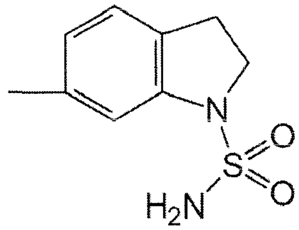 or 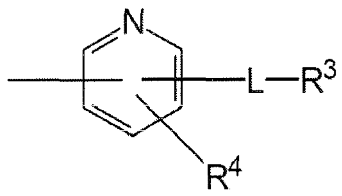

**L** is a bond or $-CR^5R^6-$, $-CR^5R^6-CR^7R^8-$, $-CR^5R^6-CR^7R^8-CR^9R^{10}-$, $-CR^5R^6-CR^7R^8-CR^9R^{10}-CR^{11}R^{12}-$;
**$R^5$-$R^{12}$** represent independently of each other -H, $-CH_3$, $-C_2H_5$, $-C_3H_7$, -F, -Cl, -Br, -I;
**$R^3$** is selected from -H, $-NO_2$, $-NH_2$, -CN, -F, -Cl, -Br, -I, $-CH_3$, $-C_2H_5$, -Ph, $-C_3H_7$, $-CH(CH_3)_2$, $-C_4H_9$, $-CH_2-CH(CH_3)_2$, $-CH(CH_3)-C_2H_5$, $-C(CH_3)_3$, $-O-CH_3$, $-O-C_2H_5$, $-O-C_3H_7$, $-O-CH(CH_3)_2$, $-O-C_4H_9$, $-O-CH_2-CH(CH_3)_2$, $-O-CH(CH_3)-C_2H_5$, $-O-C(CH_3)_3$, $-CR^{13}R^{14}R^{21}$, $-CR^{13}R^{14}-CR^{15}R^{16}R^{21}$, $-O-CR^{13}R^{14}R^{21}$, $-CR^{13}R^{14}-CR^{15}R^{16}-CR^{17}R^{18}R^{21}$, $-CR^{13}R^{14}-CR^{15}R^{16}-CR^{17}R^{18}-CR^{19}R^{20}R^{21}$, $-O-CR^{13}R^{14}-CR^{15}R^{16}R^{21}$, $-O-CR^{13}R^{14}-CR^{15}R^{16}-CR^{17}R^{18}R^{21}$, $-SO_2R^{22}$, $-CONR^{23}R^{24}$, $-NR^{25}COR^{22}$, $-O-CR^{13}R^{14}-CR^{15}R^{16}-CR^{17}R^{18}-CR^{19}R^{20}R^{21}$, $-NR^{25}SO_2NR^{23}R^{24}$, $-NR^{25}SO_2R^{22}$, $-NR^{25}CONR^{23}R^{24}$, $-SO_2NR^{23}R^{24}$, $-SO(NR^{26})R^{27}$, NH-CO-NH-Ph;
**$R^{13}$- $R^{21}$, $R^{29}$ - $R^{32}$** and **$R^{33}$ - $R^{48}$** represent independently of each other -H, -F, -Cl, -Br, -I;
**$R^{26}$** is -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CR^{13}R^{14}R^{21}$, $-COR^{28}$, $-CR^{13}R^{14}-CR^{15}R^{16}R^{21}$, $-CR^{13}R^{14}-CR^{15}R^{16}-CR^{17}R^{18}-CR^{19}R^{20}-CR^{29}R^{30}R^{21}$, $-CR^{13}R^{14}-CR^{15}R^{16}-CR^{17}R^{18}R^{21}$, $-CR^{13}R^{14}-C^{15}R^{16}-CR^{17}R^{18}-CR^{19}R^{20}R^{21}$, $-CR^{13}R^{14}-CR^{15}R^{16}-CR^{17}R^{18}-CR^{19}R^{20}-CR^{29}R^{30}-CR^{31}R^{32}R^{21}$, $-COOR^{28}$,

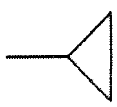, 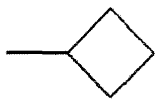, 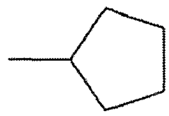, 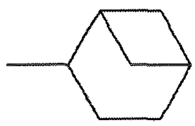,

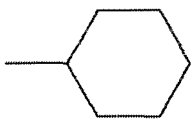, 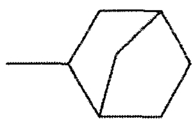, 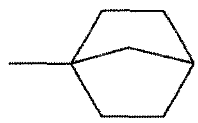, 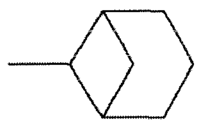,

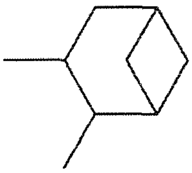, 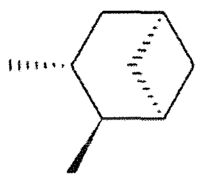, 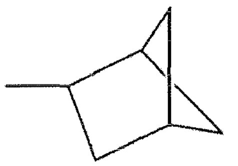, 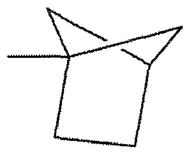,

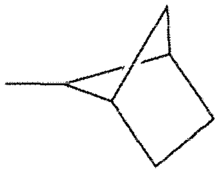,
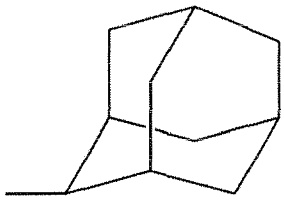,
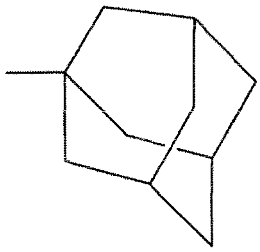,

these $C_3$-$C_6$-cycloalkyl groups may further be substituted by one, two, three, four, five or more substituents selected from the group consisting of $R^{33}$ -$R^{48}$;

**$R^{22}$**, **$R^{27}$**, and **$R^{28}$** are independently selected from $-CR^{49}R^{50}R^{51}$, $-CR^{49}R^{50}-CR^{52}R^{53}R^{51}$, $-CR^{49}R^{50}-CR^{52}R^{53}-CR^{54}R^{55}-CR^{56}R^{57}-CR^{58}R^{59}R^{51}$, $-CR^{49}R^{50}-CR^{52}R^{53}-CR^{54}R^{55}R^{51}$, $-CR^{49}R^{50}-CR^{52}R^{53}-CR^{54}R^{55}-CR^{56}R^{57}R^{51}$, $-CR^{49}R^{50}-CR^{52}R^{53}-CR^{54}R^{55}-CR^{56}R^{57}-CR^{58}R^{59}-CR^{60}R^{61}R^{51}$, $-CH_2Ph$; $-CH_2Ph$ the phenyl group of which may further be substituted by one, two, three, four or five substituents selected from the group consisting of $R^5$ - $R^{12}$;

$C_3$-$C_6$-cycloalkyl groups listed for $R^{26}$, which may further be substituted by one, two, three, four, five or more substituents selected from the group consisting of $R^{33}$ - $R^{48}$;

**$R^{49}$ - $R^{61}$** represent independently of each other -H, $-CH_3$, $-C_2H_5$, $-C_3H_7$, $-C_4H_9$, -F, -Cl, -Br, -I, -OH, $-NO_2$, $NH_2$;

**$R^{23}$** and **$R^{24}$** are independently selected from -H, $-CR^{49}R^{50}R^{51}$, $-CR^{49}R^{50}-CR^{52}R^{53}R^{51}$, $-CR^{49}R^{50}-CR^{52}R^{53}-CR^{54}R^{55}-CR^{56}R^{57}-CR^{58}R^{59}R^{51}$, $-CR^{49}R^{50}-CR^{52}R^{53}-CR^{54}R^{55}R^{51}$, $-CR^{49}R^{50}-CR^{52}R^{53}-CR^{54}R^{55}-CR^{56}R^{57}R^{51}$, $-CR^{49}R^{50}-CR^{52}R^{53}-CR^{54}R^{55}-CR^{56}R^{57}-CR^{58}R^{59}-CR^{60}R^{61}R^{51}$ ,- $CR^{49}R^{50}-CR^{52}R^{53}-O-R^{51'}$, $-CR^{49}R^{50}-CR^{52}R^{53}-CR^{54}R^{55}-O-R^{51'}$, $-CR^{49}R^{50}-CR^{52}R^{53}-NR^{51'}-R^{51''}$, $-CR^{49}R^{50}-CR^{52}R^{53}-CR^{54}R^{55}-NR^{51'}R^{51''}$, $-CR^{49}R^{50}-CR^{52}R^{53}-CR^{54}R^{55}-CR^{56}R^{57}-NR^{51'}-R^{51''}$, $-CR^{49}R^{50}-CR^{52}R^{53}-CR^{54}R^{55}-CR^{56}R^{57}-CR^{58}R^{59}-NR^{51'}R^{51''}$, phenyl, substituted phenyl, benzyl, substituted benzyl, or both residues **$R^{23}$** and **$R^{24}$** together form with the nitrogen atom to which they are attached a azetidine, pyrrolidine, piperidine, piperazine, azepane, or morpholine ring;

**$R^{51'}$** and **$R^{51''}$** represent independently of each other -H, $-CH_3$, $-C_2H_5$, $-C_3H_7$, $-C_4H_9$, $-CH_2Ph$, $-COOC(CH_3)_3$, $-COOCH_3$, $-COOCH_2CH_3$, $-COOCH_2CH_2CH_3$, $-COOCH(CH_3)_2$, $-COOCH_2Ph$, $-COCH_3$;

and **$R^{25}$** is selected from -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$ or $-C(CH_3)_3$;

**$R^4$** is selected from -H, $NO_2$, $NH_2$, -CN, -F, -Cl, -Br, -I, $-CONH_2$, $-SO_2CH_3$, $-SO_2C_2H_5$, $-SO_2C_3H_7$, $-NH-SO_2-CH_3$, $-NH-SO_2-C_2H_5$, $-NH-SO_2-C_3H_7$, $-NHCO-CH_3$, $-NHCO-C_2H_5$, $-NHCO-C_3H_7$, $-SO_2NR^{23}R^{24}$, $-CH_2-SO_2NR^{23}R^{24}$, $-C_2H_4-SO_2-NR^{23}R^{24}$, $C_3H_6-SO_2-NR^{23}R^{24}$, $SO_2NH_2$, $CH_2-SO_2NH_2$, $C_2H_4-SO_2NH_2$, $-C_3H_6-SO_2NH_2$, $-C_3H_6-SO_2NR^{23}R^{24}$, $-SO_2NH_2$, $-CH_2-SO_2NH_2$, $-C_2H_4-SO_2NH_2$, $-C_3H_6-SO_2NH_2$,

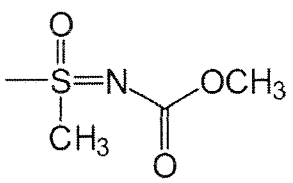
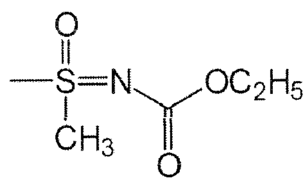
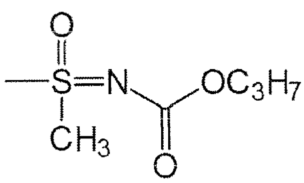

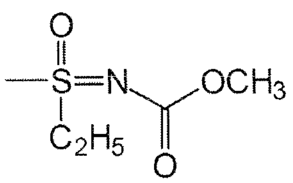
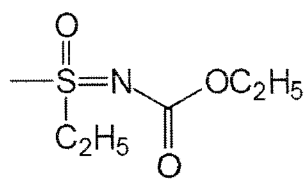
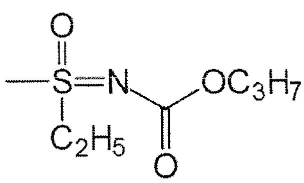

$-CR^{62}R^{63}R^{64}$, $-CR^{62}R^{63}-CR^{65}R^{66}-CR^{67}R^{68}-CR^{69}R^{70}R^{64}$, $-O-CR^{62}R^{63}-CR^{65}R^{66}R^{64}$, $-O-CR^{62}R^{63}-R^{65}R^{66}-CR^{67}R^{68}R^{64}$, $-CR^{62}R^{63}-CR^{65}R^{66}-CR^{67}R^{68}R^{64}$, $-O-CR^{62}R^{63}-CR^{65}R^{66}-CR^{67}R^{68}-CR^{69}R^{70}R^{64}$, $-CR^{62}R^{63}-CR^{65}R^{66}R^{64}$, $-O-CR^{62}R^{63}-CR^{65}R^{66}-CR^{67}R^{68}-CR^{69}R^{70}-CR^{71}R^{72}R^{64}$, $-O-CR^{62}R^{63}R^{64}$, $-O-CR^{62}R^{63}-CR^{65}R^{66}-CR^{67}R^{68}-CR^{69}R^{70}-CR^{71}R^{72}-CR^{73}R^{74}R^{64}$, $-CR^{62}R^{63}-CR^{65}R^{66}-CR^{67}R^{68}-CR^{69}R^{70}-CR^{71}R^{72}R^{64}$, $-CR^{62}R^{63}-CR^{65}R^{66}-CR^{67}R^{68}-CR^{69}R^{70}-CR^{71}R^{72}-CR^{73}R^{74}R^{64}$, $-OCH_2Ph$,

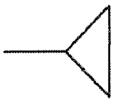 , 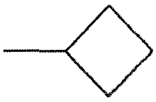 , 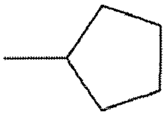 , 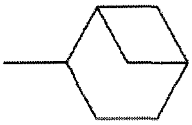 ,

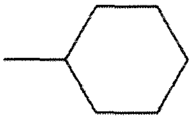 , 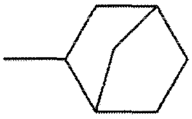 , 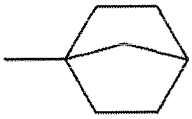 , 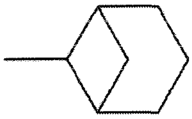 ,

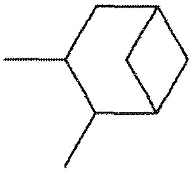 , 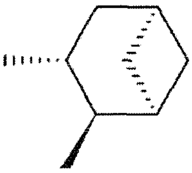 , 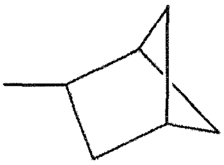 , 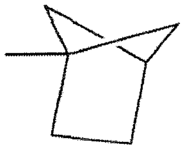 ,

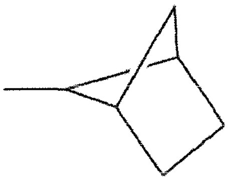 , 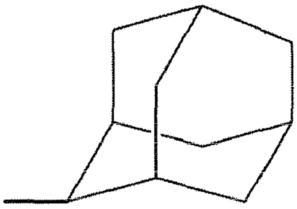 , 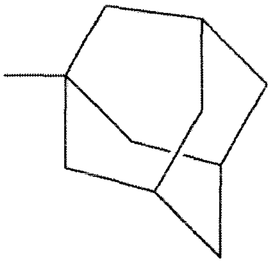 ,

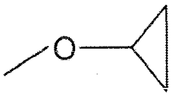 , 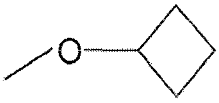 , 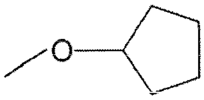 , 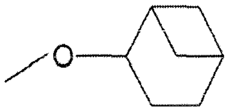 ,

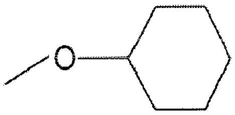 , 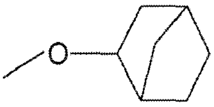 , 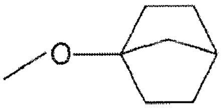 , 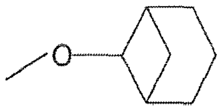 ,

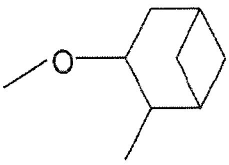 , 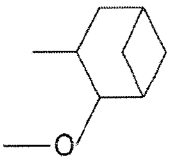 , 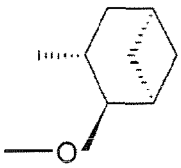 , 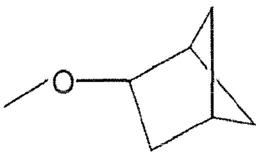 ,

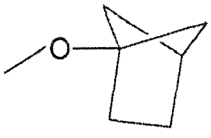,
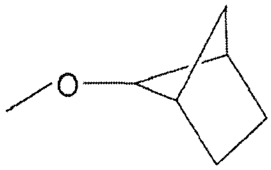
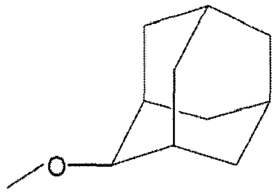,
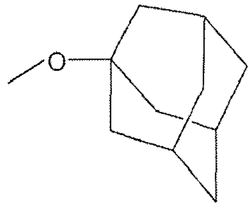,

these $C_3$-$C_6$-cycloalkoxy groups and $C_3$-$C_6$-cycloalkyl groups may further be substituted by one, two, three, four, five or more substituents selected from the group consisting of $R^{33}$ - $R^{48}$;

**$R^{62}$ - $R^{74}$** represent independently of each other -H, -cyclo- $C_3H_5$, -cyclo- $C_4H_7$, -cyclo- $C_5H_9$, $-CR^{75}R^{76}R^{77}$, $-CR^{75}R^{76}-CR^{78}R^{79}R^{77}$, $-CR^{75}R^{76}-CR^{78}R^{79}-CR^{80}R^{81}R^{77}$, $-CR^{75}R^{76}-CR^{78}R^{79}-CR^{80}R^{79}-CR^{82}R^{81}R^{77}$, -F, -Cl, -Br, -I, -Ph;

**$R^{75}$ - $R^{82}$** represent independently of each other -H, -F, -Cl, -Br, -I, $NH_2$;

**$R^4$** together with $R^{22}$, $R^{23}$, $R^{24}$, or $R^{25}$ may form a group $-CH_2CH_2-$ or $-CH_2CH_2CH_2-$ if $R^4$ is attached ortho to -L- $R^3$;

**$R^2$** is

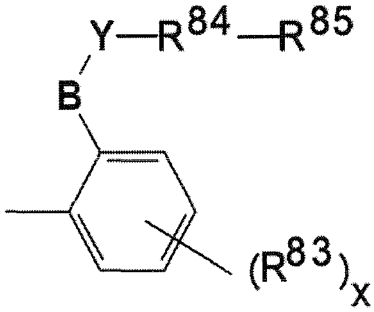

or

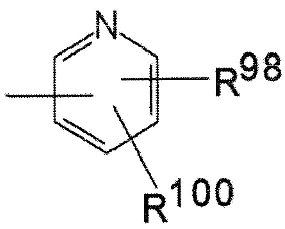

;

**$R^{83}$** is selected from -H, -OH, $-NO_2$, -CN, -F, -Cl, -Br, -I, $-NR^{23'}R^{24'}$ $-CF_3$, $-CR^{62}$ $R^{63}R^{64}$, $-CR^{62}R^{63}-NR^{23'}R^{24'}$, $-CR^{62}R^{63}-CR^{65}R^{66}R^{64}$, $-CR^{62}R^{63}-CR^{65}R^{66}-NR^{23'}R^{24'}$, $-CR^{62}R^{63}-CR^{65}R^{66}-CR^{67}R^{68}R^{64}$, $-CR^{62}R^{63}-CR^{65}R^{66}-CR^{67}R^{68}-NR^{23'}R^{24'}$, -O- $CR^{62}R^{63}R^{64}$, -O- $CR^{62}R^{63}$ $-CR^{63}R^{66}R^{64}$, -O- $CR^{62}R^{63}-CR^{65}R^{66}-CR^{67}R^{68}R^{64}$, -CHO, $-CH_2OH$, $-CR^{23'}O$, $-CH_2OR^{23'}$;

**$R^{23'}$** and **$R^{24'}$** represent independently of each other -H, $-CH_3$, $-C_2H_5$, $-C_3H_7$, -CH $(CH_3)_2$, $-C_4H_9$, $-CH_2$-CH $(CH_3)_2$, -CH $(CH_3)$-$C_2H_5$, -C $(CH_3)_3$; -(cyclo- $C_3H_5$);

**x** is a value between 0 and 3;

**B** is a bond, $-CR^{86}R^{87}-$, $-CR^{86}R^{87}-CR^{88}R^{89}-$ , $-CR^{86}R^{87}-CR^{88}R^{89}-CR^{90}R^{91}-$, $-CR^{86}R^{87}-CR^{88}R^{89}-CR^{90}R^{91}-CR^{92}R^{93}-$, $-CR^{86}R^{87}-CR^{88}R^{89}-CR^{90}R^{91}-CR^{92}R^{93}-CR^{94}R^{95}-$, $-CR^{86}R^{87}-CR^{88}R^{89}-CR^{90}R^{91}-CR^{92}R^{93}-CR^{94}R^{95}-CR^{96}R^{97}-$,

**$R^{86}$ - $R^{97}$** represent independently of each other -H, $-CH_3$, $-C_2H_5$, $-C_3H_7$, $-C_4H_9$, -F, -Cl, -Br, -I;

**Y** is a bond, -O-, -S-, -SO-, $-SO_2-$, $-SO_2NH-$, $-NHSO_2-$, -CO-, -COO-, -OOC-, -CONH-, NHCO-, NH-, -N $(CH_3)$-, NH- CO- NH-, -O- CO- NH-, -NH- CO- O-;

**$R^{84}$** is selected from a bond, $-CR^{86}R^{87}-$, $-CR^{86}R^{87}-CR^{88}R^{89}-CR^{90}R^{91}-$, $-CR^{86}R^{87}-CR^{88}R^{89}-CR^{90}R^{91}-CR^{92}R^{93}-$, $-CR^{86}R^{87}-CR^{88}R^{89}-CR^{90}R^{91}-CR^{92}R^{93}-CR^{94}R^{95}-$, $-CR^{86}R^{87}-CR^{88}R^{89}-$, $-CR^{86}R^{87}-CR^{88}R^{89}-CR^{90}R^{91}-CR^{92}R^{93}-CR^{94}-R^{95}-CR^{96}R^{97}-$;

**$R^{85}$** is selected from

(i) -H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -Ph, -OPh, $-OCH_2$-Ph, $-OCPh_3$, -SH, $-SCH_3$, $-SC_2H_5$, $-SC_3H_7$, -S- cyclo- $C_3H_5$, -SCH $(CH_3)_2$, -SC $(CH_3)_3$, $-SC_4H_9$, $-NO_2$, -F, -Cl, -Br, -I, -P (O) $(OH)_2$, -P (O) $(OCH_3)_2$, -P (O) $(OC_2H_5)_2$, -P (O) (OCH $(CH_3)_2)_2$,, -Si $(CH_3)_2$ (C $(CH_3)_3$), -si $(C_2H_5)_3$, -Si $(CH_3)_3$, -CN, -CHO, $-COCH_3$, $-COC_2H_5$, $-COC_3H_7$, -CO- cyclo- $C_3H_5$, -COCH $(CH_3)_2$, -COC $(CH_3)_3$, $-COC_4H_9$, -COOH, $-COOCH_3$, $-COOC_2H_5$, $-COOC_3H_7$, $-COOC_4H_9$, -COO- cyclo- $C_3H_5$, -COOCH $(CH_3)_2$, -COOC $(CH_3)_3$, -OOC- $CH_3$, -OOC- $C_2H_5$, -OOC- $C_3H_7$, -OOC- $C_4H_9$, -OOC- cyclo- $C_3H_5$, -OOC- CH $(CH_3)_2$, -OOC- C $(CH_3)_3$, $-CONR^{23'}R^{24'}$, $NHCOCH_3$, $NHCOC_2H_5$, $-NHCOC_3H_7$, NHCO- cyclo- $C_3H_5$, -NHCO- CH $(CH_3)_2$, $-NHCOC_4H_9$, -NHCO- C $(CH_3)_3$, NHCO- $OCH_3$, -NHCO- $OC_2H_5$, NHCO- $OC_3H_7$, -NHCO- O- cyclo- $C_3H_5$, -NHCO- $OC_4H_9$, -NHCO- OCH $(CH_3)_2$, -NHCO- OC $(CH_3)_3$, -NHCO- $OCH_2Ph$, $-NR^{23}R^{24}$, $-CF_3$, $-SOCH_3$, $-SOC_2H_5$, $-SOC_3H_7$, -SO- cyclo- $C_3H_5$, -SOCH $(CH_3)_2$, -SOC $(CH_3)_3$, $-SO_2CH_3$, $-SO_2C_2H_5$, $-SO_2C_3H_7$, $-SO_2$-cyclo- $C_3H_5$, $-SO_2CH$ $(CH_3)_2$, $-SO_2C_4H_9$, $-SO_2C$ $(CH_3)_3$, $-SO_3H$, $-SO_2NR^{23'}R^{24'}$, $-OCF_3$, $-OC_2F_5$, -O- $COOCH_3$, -O- $COOC_2H_5$, -O- $COOC_3H_7$, -O- COO- cyclo- $C_3H_5$, -O- $COOC_4H_9$, -O- COOCH $(CH_3)_2$, -O- $COOCH_2Ph$, -O- COOC $(CH_3)_3$, NH- CO- $NH_2$, -NH- CO- $NHCH_3$, NH- CO-

$NHC_2H_5$, -NH- CO- $NHC_3H_7$, -NH- CO- $NHC_4H_9$, -NH- CO- NH- cyclo- $C_3H_5$, -NH- CO- NH [CH $(CH_3)_2$], -NH- CO- NH [C $(CH_3)_3$], NH- CO- N $(CH_3)_2$, NH- CO- N $(C_2H_5)_2$, -NH- CO- N $(C_3H_7)_2$, -NH- CO- N $(C_4H_9)_2$, -NH- CO- N (cyclo- $C_3H_5)_2$, -NH- CO- N [CH $(CH_3)_2]_2$, NH- CO- N [C $(CH_3)_3]_2$, -NH- C (=NH)-$NH_2$, NH- C (=NH)-$NHCH_3$, -NH- C (=NH)-$NHC_2H_5$, -NH- C (=NH)-$NHC_3H_7$, -NH- C (=NH)-$NHC_4H_9$, -NH- C (=NH)-NH- cyclo- $C_3H_5$, -$OCH_2$-cyclo-$C_3H_5$, -NH- C (=NH)-NH [CH $(CH_3)_2$], -NH- C (=NH)-NH [C $(CH_3)_3$], -NH- C (=NH)-N $(CH_3)_2$, -NH- C (=NH)-N $(C_2H_5)_2$, - NH- C (=NH)-N $(C_3H_7)_2$, -NH- C (=NH)-N (cyclo- $C_3H_5)_2$, -NH- C (=NH)-N $(C_4H_9)_2$, -NH- C (=NH)-N [CH $(CH_3)_2]_2$, -NH- C (=NH)-N [C $(CH_3)_3]_2$, -O- CO- $NH_2$, -O- CO- $NHCH_3$, -O- CO- $NHC_2H_5$, -O- CO- $NHC_3H_7$, -O- CO- $NHC_4H_9$, -O- CO- NH- cyclo- $C_3H_5$, -O- CO- NH [CH $(CH_3)_2$], -O- CO- NH [C $(CH_3)_3$], -O- CO- N $(CH_3)_2$, -O- CO- N $(C_2H_5)_2$, -O- CO- N $(C_3H_7)_2$, -O- CO- N $(C_4H_9)_2$, -O- CO- N (cyclo- $C_3H_5)_2$, -O- CO- N [CH $(CH_3)_2]_2$, -O- CO- N [C $(CH_3)_3]_2$,

(ii) an aromatic or heteroaromatic mono- or bicyclic ring selected from 2-thienyl, 3-thienyl, 2-furanyl, 3-furanyl, 2-oxazolyl, 3- oxazolyl, 4- oxazolyl, 2- thiazolyl, 3- thiazolyl, 4- thiazolyl, 1- pyrazolyl, 3- pyrazolyl, 4- pyrazolyl, 5-pyrazolyl, 1- imidazolyl, 2- imidazolyl, 4- imidazolyl, 5- imidazolyl, phenyl, 1- naphthyl, 2- naphthyl, 2- pyridyl, 3-pyridyl, 4- pyridyl, 2- pyrimidinyl, 4- pyrimidinyl, 5- pyrimidinyl, 2- pyrazinyl, 3- pyridazinyl, 4- pyridazinyl, 1,3,5- triazin-2- yl,

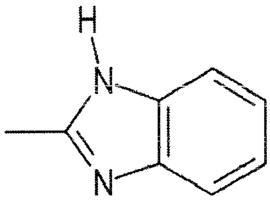 , 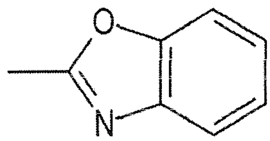 , 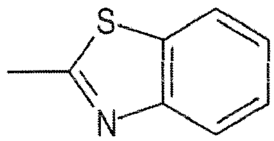 ,

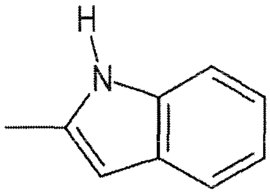 , 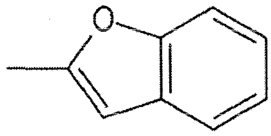 , 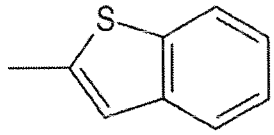 ,

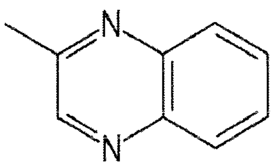 , 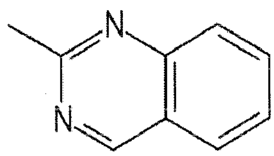 , 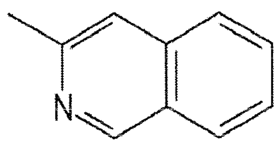 ,

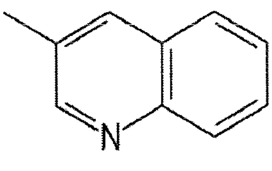 , 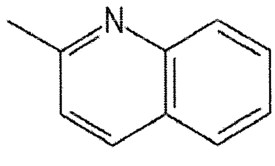 , 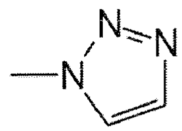 ,

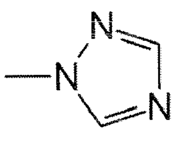 , 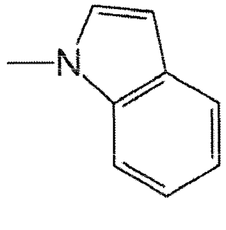 , 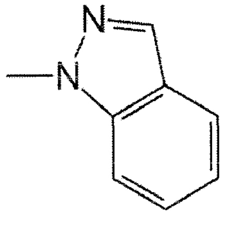 , 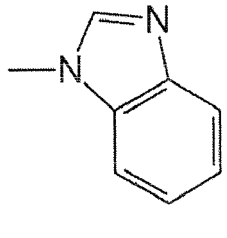 ,

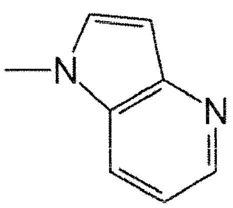 , 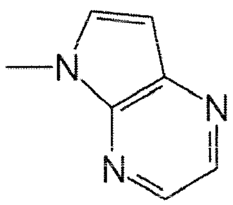 , 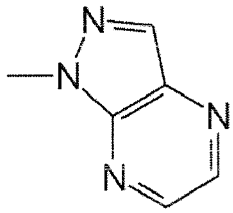 , 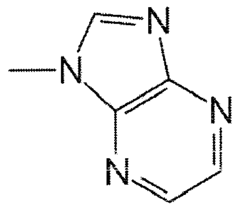 ,

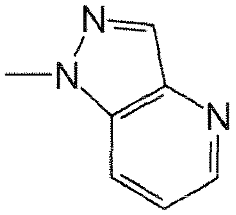 , 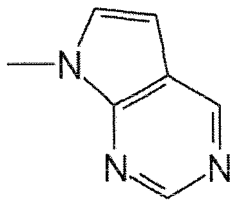 , 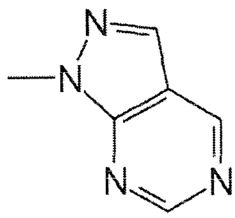 , 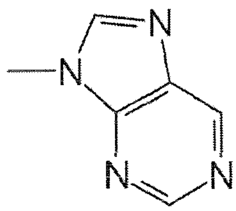 ,

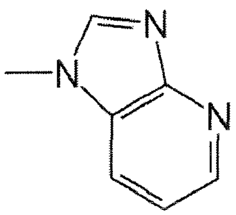 , 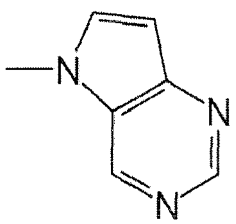 , 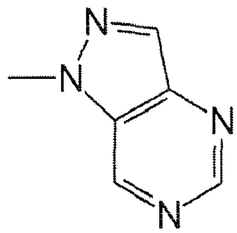 , 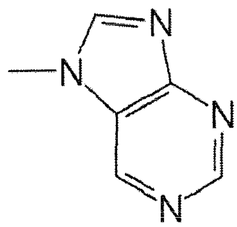 ,

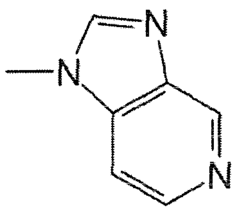 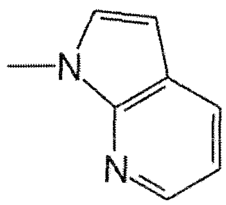 , 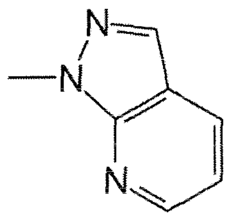 , 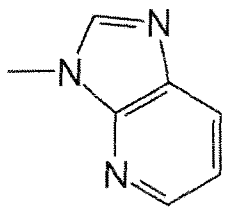 ,

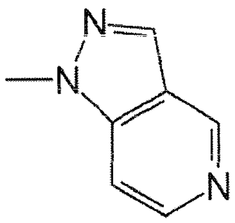 , 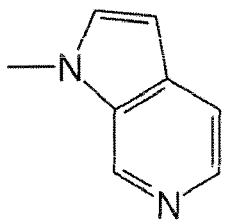 , 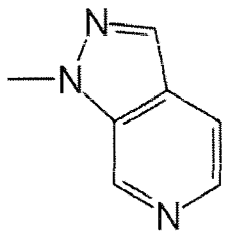 , 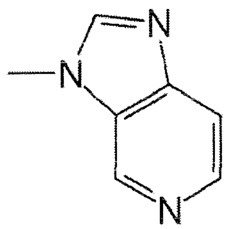 ,

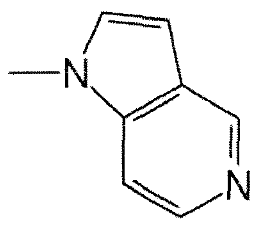, 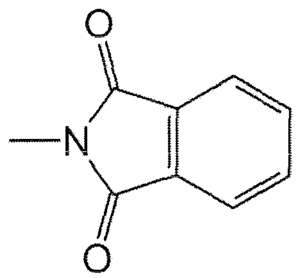

which optionally may be substituted by one or two substituents selected from -F, -Cl, -Br, -I, $-OCH_3$, $-CH_3$, $NO_2$, -CN, $-CF_3$;

(iii) a saturated ring selected from

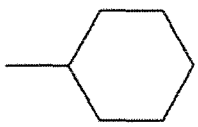, 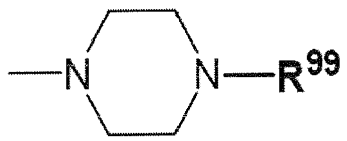, 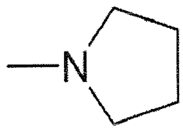,

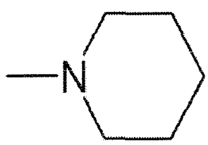, 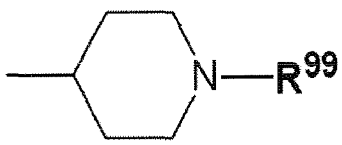, 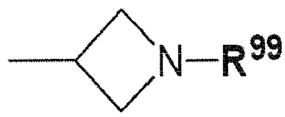,

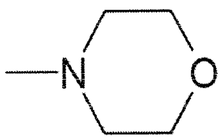, 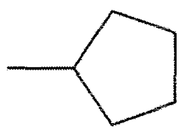, 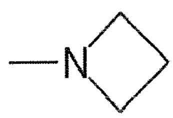;

**$R^{99}$** represents -H, $-CH_3$, $-CH_2Ph$, $-COOC(CH_3)_3$, $-COOCH_3$, $-COOCH_2CH_3$, $-COOCH_2CH_2CH_3$, -COOCH $(CH_3)_2$, $-COOCH_2Ph$, $-COCH_3$;
the group -B- Y- $R^{84}$-$R^{85}$ together with one substituent $R^{83}$ may form a group $-OCH_2O-$ if $R^{83}$ is attached in position ortho to -B- Y- $R^{84}$-$R^{85}$;
with the proviso that $R^{83}$ is not -H, if the group -B- Y- $R^{84}$-$R^{85}$ is hydrogen.
**$R^{98}$** is selected from $-NO_2$, -CN, -F, -Cl, -Br, -I, $-NH_2$, -OH, $-CR^{62}R^{63}-CR^{65}R^{66}-CR^{67}R^{68}-CR^{69}R^{70}R^{64}$, -O- $CR^{62}R^{63}-R^{64}$, -O- $CR^{62}R^{63}-CR^{65}R^{66}R^{64}$ -O- $CR^{62}R^{63}CR^{65}R^{66}-CR^{67}R^{68}R^{64}$ -O- $CR^{62}R^{63}-CR^{65}R^{66}-CR^{67}R^{68}-CR^{69}R^{70}R^{64}$, -O- $CR^{62}R^{63}-CR^{65}R^{66}-CR^{67}R^{68}-CR^{69}R^{70}-CR^{71}R^{72}R^{64}$, $-CR^{62}R^{63}-CR^{65}R^{66}-CR^{67}R^{68}R^{64}$, -O- $CR^{62}R^{63}-CR^{65}R^{66}-CR^{67}R^{68}-CR^{69}R^{70}-CR^{71}R^{72}-CR^{73}R^{74}R^{64}$, $-CR^{62}R^{63}-CR^{65}R^{66}R^{64}$, $-CR^{62}R^{63}$-O- $CR^{65}R^{66}-CR^{67}R^{68}-CR^{69}R^{70}R^{64}$, $-CR^{62}R^{63}$-O- $CR^{65}R^{66}-CR^{67}R^{68}R^{64}$, $-CR^{62}R^{63}$-O- $CR^{65}R^{66}-CR^{67}R^{68}-CR^{69}R^{70}-CR^{71}R^{72}R^{64}$, $-CR^{62}R^{63}$-O- $CR^{65}-R^{66}R^{64}$, $-CR^{62}R^{63}$-O- $CR^{65}R^{66}-CR^{67}R^{68}-CR^{69}R^{70}-CR^{71}R^{72}-CR^{73}R^{74}R^{64}$, $-CR^{62}R^{63}R^{64}$, $-CR^{62}R^{63}-CR^{65}R^{66}-CR^{67}-R^{68}-CR^{69}R^{70}-CR^{71}R^{72}R^{64}$, $-OCH_2Ph$, $-OCH_2-CH_2-Ph$, $-CH_2$-O- $CH_2$-Ph, $-CR^{62}R^{63}-CR^{65}R^{66}-CR^{67}R^{68}-CR^{69}R^{70}-CR^{71}R^{72}-CR^{73}R^{74}R^{64}$;
with the proviso that $R^{98}$ is attached to a position ortho to the bond between the pyridine and the triazine ring if $R^{98}$ is not an amino group in para position to the bond between the pyridine and the triazine ring;
**$R^{100}$** is selected from -H, $-NO_2$, -CN, -F, -Cl, -Br, -I, $-NH_2$, -OH, $-CF_3$, $-CH_3$, $-CH_2CH_3$, $-CH_2CH_2CH_3$, -CH $(CH_3)_2$, $-OCH_3$, $-OCH_2CH_3$, $-OCH_2CH_2CH_3$, -OCH $(CH_3)_2$, $-OCF_3$, $-OCH_2Ph$;
and with the proviso that if $R^1$ is a phenyl moiety and $R^2$ is also a phenyl moiety a chloro substituent is only allowed on the $R^1$ phenyl moiety or on the $R^2$ phenyl moiety but not on both simultaneously;
and with the proviso that the compound 4-[4-(2- benzoylaminophenyl)-[1,3,5] triazin- 2- ylamino] benzamide is

excluded;

and enantiomers, stereoisomeric forms, mixtures of enantiomers, diastereomers, mixtures of diastereomers, prodrugs, hydrates, solvates, acid salt forms, tautomers, and racemates of the above mentioned compounds and pharmaceutically acceptable salts or salts of solvates thereof.

The expression prodrug is defined as a substance, which is applied in an inactive or significantly less active form. Once applied and incorporated, the prodrug is metabolized in the body *in vivo* into the active compound.

The expression tautomer is defined as an organic compound that is interconvertible by a chemical reaction called tautomerization. Tautomerization can be catalyzed preferably by bases or acids or other suitable compounds.

Preferred are compounds having the general formula (I):

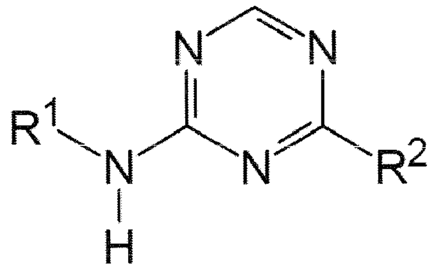

Formula (I)

wherein

$R^1$ represents

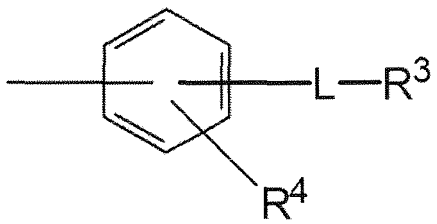

in which

**L** is a bond, $-CH_2-$, $-CH_2CH_2-$, or $-CF_2-$, particularly preferred $-CH_2-$;

$\mathbf{R^3}$ is $-SO_2NH_2$, $-SO_2NH(CH_3)$, $-SO_2N(CH_3)_2$, $-SO_2NH(CH_2CH_2OCH_3)$, $-NHSO_2CH_3$, $NHSO_2CH_2CH_3$, $-NHSO_2CH_2CH_2CH_3$, $NHSO_2CF_3$, $-SO_2CH_3$, $NHSO_2NH_2$, $-SO(NH)CH_3$, particularly preferred $-SO_2NH_2$;

$\mathbf{R^4}$ is -H, $-CH_3$, -F, -Cl, or $-CF_3$, particularly preferred -H;

$\mathbf{R^1}$ represents

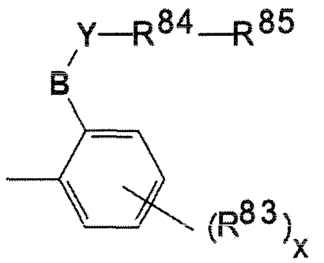

or

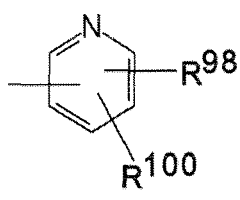

;

in which the group **-B-Y-$\mathbf{R^{84}}$-$\mathbf{R^{85}}$** is $-OCH_3$, $-OCH_2CH_3$, $-OCH_2CH_2CH_3$, $-OCH_2CH_2CH_2CH_3$, $-OCH(CH_3)_2$, -OPh, $-OCH_2Ph$, $-OCH_2$(4-pyridyl), particularly preferred $-OCH_3$;

$\mathbf{R^{83}}$ is -H, -F, or -Cl;

x is 0, 1, or 2;

$\mathbf{R^{98}}$ is $-OCH_3$ and $\mathbf{R^{100}}$ is -H, provided that $R^{98}$ is attached to a position ortho to the bond between the pyridine and the triazine ring.

In more preferred compounds of Formula (I) the substituent **-L-R³** is $-SO_2NH_2$, $-CH_2SO_2NH_2$, $-CH_2CH_2SO_2NH_2$, $-CF_2SO_2NH_2$, $NHSO_2NH_2$, $-CH_2NHSO_2NH_2$, $-SO_2CH_3$, $-SO(NH)CH_3$, $-CH_2SO(NH)CH_3$,
and $\mathbf{R^4}$ is -H;
$\mathbf{R^2}$ is 2-methoxyphenyl, 4-fluoro-2-methoxyphenyl, or 6-fluoro-2-methoxyphenyl.

Preferred are compounds of general formula (I), wherein $R^1$ is

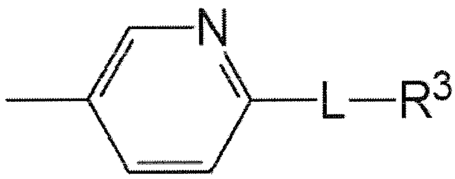

and wherein L is a bond or is $-CH_2-$ or $-CH_2CH_2-$ and $R^3$ has the meanings as defined heren and more preferably $R^3$ represents $-SO_2R^{22}$ or $-SO_2NR^{23}R^{24}$, wherein $R^{22}$, $R^{23}$ and $R^{24}$ have the meanings as defined herein and preferably $R^{22}$, $R^{23}$ and $R^{24}$ represent independently of each other -H, $-CF_3$, $-CH_3$, $-CH_2CH_3$, $-CH_2CH_2CH_3$, $-CH_2CH_2CH_2CH_3$, $-CH(CH_3)_2$, $-CH_2-NH_2$, $-CH_2-CH_2-NH_2$, $-CH_2-CH_2-CH_2-NH_2$, $-CH_2-CH_2-CH_2-CH_2-NH_2$, $-CH_2-NH-CO-O-C(CH_3)_3$, $-CH_2-CH_2-NH-CO-O-C(CH_3)_3$, $-CH_2-CH_2-CH2-NH-CO-O-C(CH_3)_3$, $-CH_2-CH_2-CH_2-CH_2{}_-NH-CO-O-C(CH_3)_3$.

Also preferred are compounds of general formula (I), wherein **L** is a bond, $-CH_2-$, $-CH_2CH_2-$, $-CH_2CH_2CH_2-$, or $-CF_2-$, more preferred $-CH_2-$ or $-CH_2CH_2-$.

Preferred are compounds of general formula (I), wherein $\mathbf{R^2}$ is

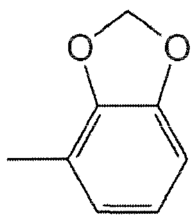 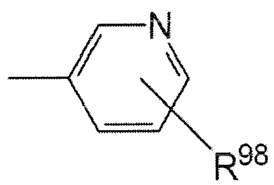 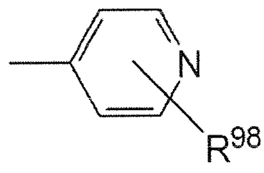

If residue $\mathbf{R^2}$ is a phenyl ring, it is preferred that the substituent $\mathbf{B\text{-}Y\text{-}R^{84}\text{-}R^{85}}$ in ortho position of the linkage to the triazine core is not hydrogen and if that substituent is hydrogen, $\mathbf{R^{83}}$ is not hydrogen and moreover that at least one substituent $\mathbf{R^{83}}$ is in ortho position of the linkage to the triazine core. Thus one substituent of $\mathbf{B\text{-}Y\text{-}R^{84}\text{-}R^{85}}$ and $\mathbf{R^{83}}$ has to be different from hydrogen so that $\mathbf{R^2}$ cannot be an unsubstituted phenyl ring. Moreover it is preferred that $R^{85}$ is not -H, if B, Y and $R^{84}$ are bonds and $R^{83}$ is different from hydrogen. If two substituents are present, it is preferred that the second substituent is in meta position or para position of the linkage to the triazine core. If a third substituent is present the substitution pattern 2,3,5 or 2,3,4 are preferred. Fluorine is a preferred second and/or third substituent and is preferably in meta or para position of the linkage to the triazine core. Thus, the following residues $\mathbf{R^2}$ are preferred:

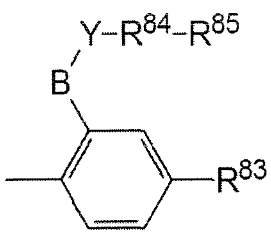 , 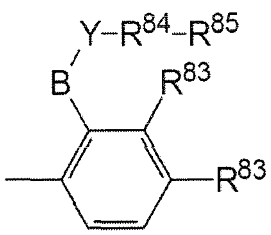 , 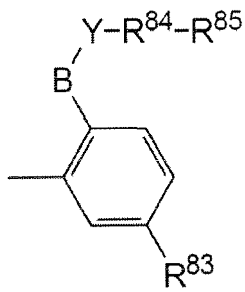 , 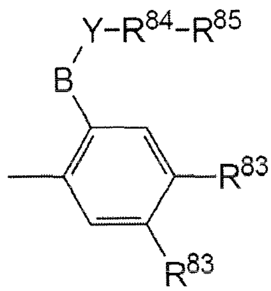 .

If residue $\mathbf{R^2}$ is a pyridyl ring it is preferred that one substituent of $\mathbf{R^{98}}$ is in ortho position of the linkage to the triazine core. Preferred are the following $\mathbf{R^2}$ residues:

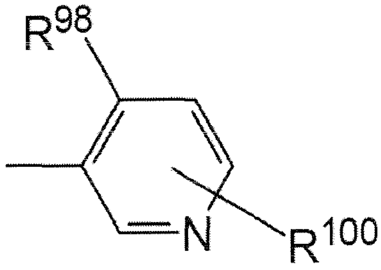
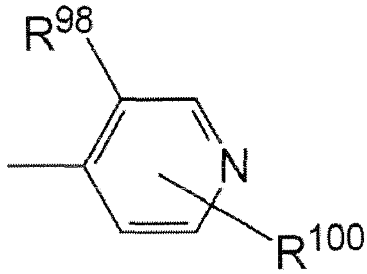
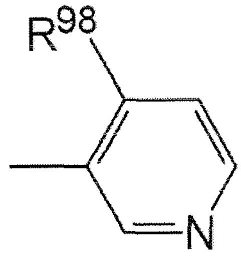

Also preferred are compounds of general formula (I), wherein $\mathbf{R^{85}}$ is

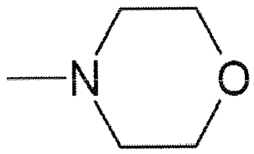
,
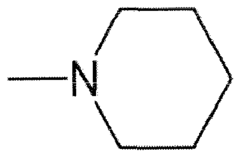
,
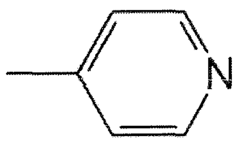
.

$\mathbf{R^3}$ is preferably selected from -H, $NO_2$, $-NH_2$, -CN, -F, -Cl, -Br, -I, $-CH_3$, $-C_2H_5$, -Ph, $-C_3H_7$, $-CH(CH_3)_2$, $-C_4H_9$, $-CH_2-CH(CH_3)_2$, $-CH(CH_3)-C_2H_5$, $-C(CH_3)_3$, $-O-CH_3$, $-O-C_2H_5$, $-O-C_3H_7$, $-O-CH(CH_3)_2$, $-O-C_4H_9$, $-O-CH_2-CH(CH_3)_2$, $-O-CH(CH_3)-C_2H_5$, $-O-C(CH_3)_3$, $-SO_2R^{22}$ and $-SO_2NR^{23}R^{24}$.

$\mathbf{R^{26}}$ is preferably selected from -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, -cyclo-$C_3H_5$, -cyclo-$C_4H_7$ and -cyclo-$C_5H_9$.

Moreover compounds of general formula (I), wherein $\mathbf{R^{22}}$, $\mathbf{R^{23}}$, $\mathbf{R^{24}}$, $\mathbf{R^{27}}$ and $\mathbf{R^{28}}$ are independently of each other selected from -H, $-CH_3$, $-C_2H_5$, $-C_3H_7$, $-C_4H_9$ or $-CH_2Ph$.

Preferably $\mathbf{R^{62}}$ **-** $\mathbf{R^{74}}$ represent independently of each other -H, -Ph, -cyclo-$C_3H_5$, -cyclo-$C_4H_7$, $-CH_3$, $-C_2H_5$, $-C_3H_7$, $-C_4H_9$, -cyclo-$C_5H_9$, -F, -Cl, -Br or -I.

Furthermore preferred are compounds of general formula (I), herein $\mathbf{R^4}$ is selected from -H, $-NO_2$, $-NH_2$, -CN, F, -Cl, -Br, -I, -cyclo-$C_3H_5$, cyclo-$C_4H_7$, -cyclo-$C_5H_9$, $-CH_3$, $-C_2H_5$, $-C_3H_7$, $-CH(CH_3)_2$, $-C_4H_9$, $-CONH_2$, $-SO_2CH_3$, $-SO_2C_2H_5$, $-SO_2C_3H_7$, $-NH-SO_2-CH_3$, $-NH-SO_2-C_2H_5$, $-NH-SO_2-C_3H_7$, $-NHCO-CH_3$, $-NHCO-C_2H_5$, $-NHCO-C_3H_7$, $-SO_2NR^{23}R^{24}$, $-CH_2-SO_2NR^{23}R^{24}$, $-C_2H_4-SO_2NR^{23}R^{24}$, $-C_3H_6-SO_2NR^{23}R^{24}$, $-SO_2NH_2$, $-CH_2-SO_2NH_2$, $-C_2H_4-SO_2NH_2$, $-C_3H_6-SO_2NH_2$,

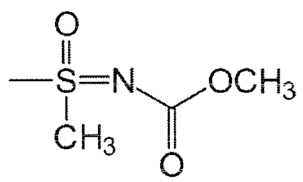
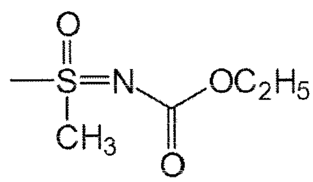
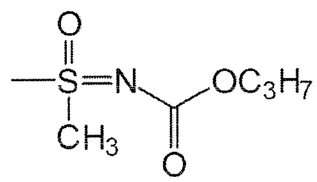

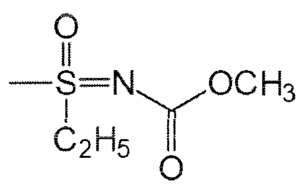
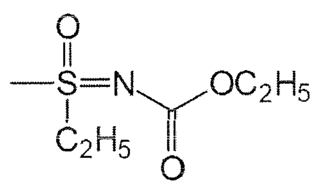
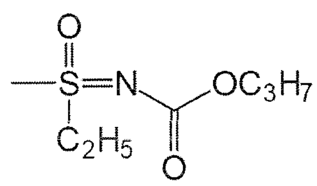

$-CH(CH_3)-C_2H_5$, $-C(CH_3)_3$, $-C_5H_{11}$, $-CH_2-CH_2-CH_2-CH_2R^{64}$, $-O-CH_2-CH_2R^{64}$, $-CH_2R^{64}$, $-O-CH_2-CH_2-CH_2R^{64}$, $-CH_2-CH_2-CH_2R^{64}$, $-O-CH_2-CH_2-CH_2-CH_2R^{64}$, $-CH_2-CH_2R^{64}$, $-O-CH_2-CH_2-CH_2-CH_2-CH_2R^{64}$, $-CH_2-CH_2-CH_2-CH_2-CH_2R^{64}$, $-O-CH_2-CH_2-CH_2-CH_2-CH_2-CH_2R^{64}$, $-CH_2-CH_2-CH_2-CH_2-CH_2-CH_2R^{64}$, $-OCH_2Ph$, $-O-CH_2R^{64}$, wherein $\mathbf{R^{64}}$ represents -Ph, -F, -Cl, -Br or -I. Preferred are compounds wherein, $\mathbf{R^4}$ is selected from $-NO_2$, $NH_2$, $-CONH_2$, $-SO_2CH_3$, $-SO_2C_2H_5$, $-SO_2C_3H_7$, $-NH-SO_2-CH_3$, $-NH-SO_2-C_2H_5$, $-NH-SO_2-C_3H_7$, $-NHCO-CH_3$, $-NHCO-C_2H_5$, $-NHCO-C_3H_7$, $-SO_2NR^{23}R^{24}$,

$-CH_2-SO_2NR^{23}R^{24}$, $-C_2H_4-SO_2NR^{23}R^{24}$, $-C_3H_6-SO_2NR^{23}R^{24}$, $-SO_2NH_2$, $-CH_2-SO_2NH_2$, $-C_2H_4-SO_2NH_2$, $-C_3H_6-SO_2-NH_2$,

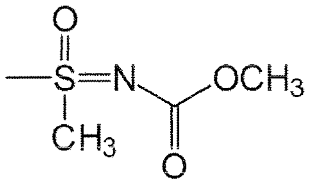
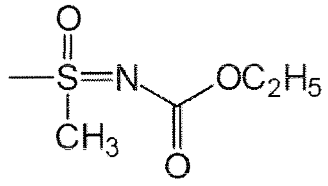
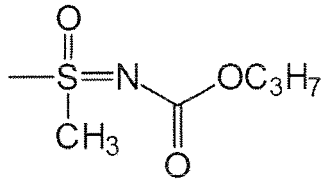

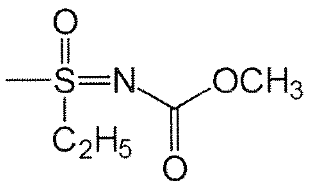
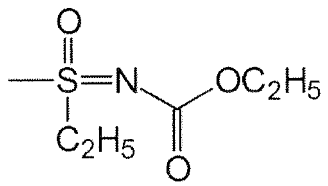
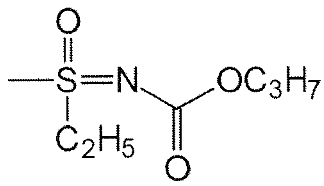

Moreover it is especially preferred that not both substituents $-L-R^3$ and $-R^4$ are hydrogen. Thus it is preferred that the phenyl substituent $R^1$ and the pyridyl substituent $R^1$ have at least one substituent and preferably one substituent in meta position and most preferably the preferred substituents mentioned above for $-L-R^3$ and $-R^4$ in meta position and especially preferred for $-R^4$ in meta position. Consequently the following $\mathbf{R^1}$ residues are preferred and especially preferred are the following substituents $\mathbf{R^1}$ with the preferred substituents for $-L-R^3$ and $-R^4$:

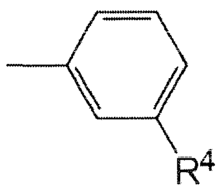
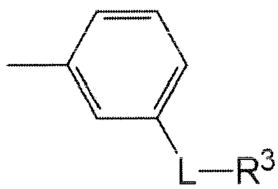

Also preferred are compounds of general formula (I), wherein $\mathbf{R^{83}}$ is -H, -OH, $-NO_2$, -CN, -F, -Cl, -Br, -I, $-NH_2$, $-NH(CH_3)$, $-N(CH_3)_2$, $-NH(C_2H_5)$, $-N(C_2H_5)_2$, $-CF_3$, $-CH_3$, $-C_2H_5$, $-C_3H_7$, $-CH(CH_3)_2$, $-C_4H_9$, $-C(CH_3)_3$, $-CH_2-NH_2$, $-CH_2-NH(CH_3)$, $-CH_2-N(CH_3)_2$, $-CH_2-NH(C_2H_5)$, $-CH_2-N(C_2H_5)_2$, $-CH_2-CH_2-NH_2$, $-CH_2-CH_2-NH(CH_3)$, $-CH_2-CH_2-N(CH_3)_2$, $-CH_2-CH_2-NH(C_2H_5)$, $-CH_2CH_2-N(C_2H_5)_2$, $-CH_2-CH_2-CH_2-NH_2$, $-CH_2-CH_2-CH_2-NH(CH_3)$, $-CH_2-CH_2-CH_2-N(CH_3)_2$, $-CH_2-CH_2-CH_2-NH(C_2H_5)$, $-CH_2-CH_2-CH_2-N(C_2H_5)_2$, $-O-CH_3$, $-O-CH_2-CH_3$, $-O-CH_2-CH_2-CH_3$, -CHO, $-CH_2OH$, $-CO-CH_3$, $-CO-CH_2-CH_3$, $-CO-CH_2-CH_2-CH_3$, $-CO-CH_2-CH_2-CH_2-CH_3$, $-CH_2O-CH_3$, $-CH_2O-CH_2-CH_3$, $-CH_2O-CH_2-CH_2-CH_3$, $-CH_2F$, $-CH_2Cl$, $-CH_2Br$, $-CH_2-CH_2F$, $-CH_2-CH_2Cl$, $-CH_2-CH_2Br$, $-CH_2-CH_2-CH_2F$, $-CH_2-CH_2-CH_2Cl$, $-CH_2-CH_2-CH_2Br$.

Moreover compounds of general formula (I) are preferred, wherein **B** represents a bond, $-CH_2-$, $-CH_2CH_2-$, $-CH_2CH_2CH_2-$, $-CH_2CH_2CH_2CH_2-$, $-CH_2CH_2CH_2CH_2CH_2-$ and/or wherein **Y** represents a bond, -O-, or -NH-.

In addition compounds of general formula (I) are preferred, wherein $\mathbf{R^{84}}$ represents a bond, $-CH_2-$, $-CH_2CH_2-$, $-CH_2CH_2CH_2-$, $-CH_2CH_2CH_2CH_2-$, $-CH_2CH_2CH_2CH_2CH_2-$.

Preferred are also compounds of general formula (I), wherein $\mathbf{R^{85}}$ is -H, -OH, $-OCH_3$, $-OC_2H_5$, $-OC_3H_7$, $-O-cyclo-C_3H_5$, $-OCH(CH_3)_2$, $-OC(CH_3)_3$, $-OC_4H_9$, -Ph, -OPh, $-OCH_2-Ph$, $-OCPh_3$, $NO_2$, -F, -Cl, -Br, -I, -CN, -CHO, $-COCH_3$, $-COC_2H_5$, $-COC_3H_7$, $-CO-cyclo-C_3H_5$, $-COCH(CH_3)_2$, $-COC(CH_3)_3$, $-COC_4H_9$, -COOH, $-COOCH_3$, $-COOC_2H_5$, $-COOC_3H_7$, $-COOC_4H_9$, $-COO-cyclo-C_3H_5$, $-COOCH(CH_3)_2$, $-COOC(CH_3)_3$, $-OOC-CH_3$, $-OOC-C_2H_5$, $-OOC-C_3H_7$, $-OOC-C_4H_9$, $-OOC-cyclo-C_3H_5$, $-OOC-CH(CH_3)_2$, $-OOC-C(CH_3)_3$, $-CONR^{23'}R^{24'}$, $-NHCOCH_3$, $-NHCOC_2H_5$, $-NHCOC_3H_7$, $-NHCO-cyclo-C_3H_5$, $-NHCO-CH(CH_3)_2$, $-NHCOC_4H_9$, $-NHCO-C(CH_3)_3$, $-NHCO-OCH_3$, $-NHCO-OC_2H_5$, $-NHCO-OC_3H_7$, $-NHCO-O-cyclo-C_3H_5$, $-NHCO-OC_4H_9$, $-NHCO-OCH(CH_3)_2$, $-NHCO-OC(CH_3)_3$, $NHCO-OCH_2Ph$, $-NR^{23}R^{24}$, $-CF_3$, $-SOCH_3$, $-SOC_2H_5$, $-SOC_3H_7$, $-SO-cyclo-C_3H_5$, $-SOCH(CH_3)_2$, $-SOC(CH_3)_3$, $-SO_2CH_3$, $-SO_2C_2H_5$, $-SO_2C_3H_7$, $-SO_2-cyclo-C_3H_5$, $-SO_2CH(CH_3)_2$, $-SO_2C_4H_9$, $-SO_2C(CH_3)_3$, $-SO_3H$, $-SO_2NR^{23'}R^{24'}$, $-OCF_3$, $-OC_2F_5$, $-NH-CO-NH_2$, $-NH-CO-NHCH_3$, $-NH-CO-NHC_2H_5$, $-NH-CO-NHC_3H_7$, $NH-CO-NHC_4H_9$, $-NH-CO-NH-cyclo-C_3H_5$, $-NH-CO-NH[CH(CH_3)_2]$, $-NH-CO-NH[C(CH_3)_3]$, $NH-CO-N(CH_3)_2$, $-NH-CO-N(C_2H_5)_2$, $NH-CO-N(C_3H_7)_2$, $-O-CO-NH_2$, $-O-CO-NHCH_3$, $-O-CO-NHC_2H_5$, $-O-CO-NHC_3H_7$, $-O-CO-NHC_4H_9$, $-O-CO-NH-cyclo-C_3H_5$ $-O-CO-NH[CH(CH_3)_2]$, $-O-CO-NH[C(CH_3)_3]$, $-O-CO-N(CH_3)_2$, $-O-CO-N(C_2H_5)_2$, $-O-CO-N(C_3H_7)_2$, $-O-CO-N(C_4H_9)_2$, $-O-CO-N(cyclo-C_3H_5)_2$, $-O-CO-N[CH(CH_3)_2]_2$, $-O-CO-N[C(CH_3)_3]_2$, 2-thienyl, 3-thienyl, 2-furanyl, 3-furanyl, 2-oxazolyl, 3-oxazolyl, 4-oxazolyl, 2-thiazolyl, 3-thiazolyl, 4-thiazolyl, 1-pyrazolyl, 3-pyrazolyl, 4-pyrazolyl, 5-pyrazolyl, 1-imidazolyl, 2-imidazolyl, 4-imidazolyl, 5-imidazolyl, phenyl, 1-naphthyl, 2-naphthyl, 2-pyridyl, 3-pyridyl, 4-pyridyl, 2-pyrimidinyl, 4-pyrimidinyl, 5-pyrimidinyl, 2-pyrazinyl, 3-pyridazinyl, 4-pyridazinyl, 1,3,5-triazin-2-yl,

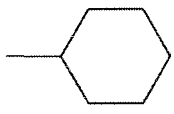
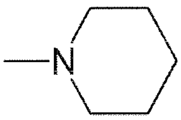
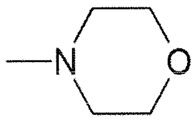
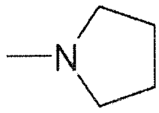
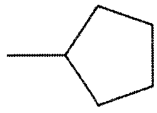

with the proviso that $R^{83}$ is not -H, if the group -B-Y-$R^{84}$-$R^{85}$ is hydrogen.

Also preferred are compounds of general formula (I), wherein $\mathbf{R^{98}}$ is $-NO_2$, -CN, -F, -Cl, - Br, -I, $NH_2$, -OH, $-CF_3$, $-CH_3$, $-C_2H_5$, $-C_3H_7$, $-CH(CH_3)_2$, $-C_4H_9$, $-C(CH_3)_3$, $-CH_2-NH_2$, $-CH_2-NH(CH_3)$, $-CH_2-N(CH_3)_2$, $-CH_2-NH(C_2H_5)$, $-CH_2-N(C_2H_5)_2$, $-CH_2-CH_2-NH_2$, $-CH_2-CH_2-NH(CH_3)$, $-CH_2-CH_2-N(CH_3)_2$, $-CH_2-CH_2-NH(C_2H_5)$, $-CH_2-CH_2-N(C_2H_5)_2$, $-CH_2-CH_2-CH_2-NH_2$, $-CH_2-CH_2-CH_2-NH(CH_3)$, $-CH_2-CH_2-CH_2-N(CH_3)_2$, $-CH_2-CH_2-CH_2-NH(C_2H_5)$, $-CH_2-CH_2-CH_2-N(C_2H_5)_2$, $-O-CH_3$, $-O-CH_2-CH_3$, $-O-CH_2-CH_2-CH_3$, $-CH_2O-CH_3$, $-CH_2O-CH_2-CH_3$, $-CH_2O-CH_2-CH_2-CH_3$, $-CH_2F$, $-CH_2Cl$, $-CH_2Br$, - $CH_2-CH_2F$, $-CH_2-CH_2Cl$, $-CH_2-CH_2Br$, $-CH_2-CH_2-CH_2F$, $-CH_2-CH_2-CH_2Cl$, - $CH_2-CH_2-CH_2Br$, $-OCH_2Ph$, $-OCH_2-CH_2-Ph$, $-CH_2-O-CH_2-Ph$.

Moreover especially preferred are compounds of the general formula (I), wherein
$\mathbf{R^1}$ is

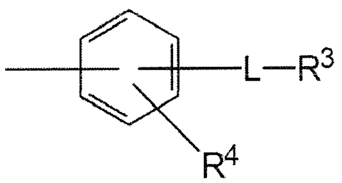
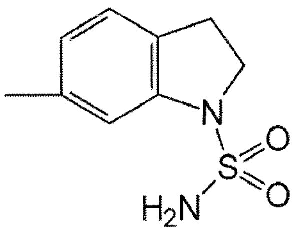
or
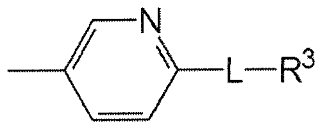

**L** is a bond, $-CH_2-$, or $-CH_2CH_2-$;
$\mathbf{R^3}$ is -H, $-SO_2NR^{23}R^{24}$, $-CONR^{23}R^{24}$, $-NO_2$, $NH_2$, $-NHSO_2R^{22}$, $NHCOR^{22}$, $-SO_2R^{22}$, -NH-CO-NH-Ph, or -Ph,
$\mathbf{R^4}$ is -H, $-CH_2-SO_2NR^{23}R^{24}$, $-SO_2NR^{23}R^{24}$, $-CONH_2$, $-C_2H_4-SO_2NR^{23}R^{24}$, $-NH-SO_2-CH_3$, $NH-SO_2-C_2H_5$, $-NH-SO_2-C_3H_7$, $-NHCO-CH_3$, $-NHCO-C_2H_5$, $-NO_2$, $-NH_2$, $-SO_2CH_3$, or

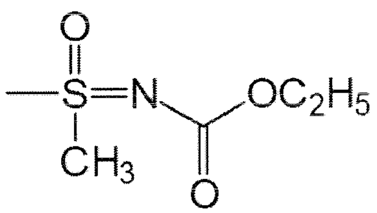

$\mathbf{R^{23}}$ and $\mathbf{R^{24}}$ are independently selected from -H, $-CH_3$, $-C_2H_5$, $-C_3H_7$, -(cyclo-$C_3H_5$), $-CH_2-CH_2-CH_2-CH_2-NH_2$, or $-CH_2-CH_2-CH_2-CH_2-NH-COOC(CH_3)_3$,
$\mathbf{R^2}$ represents

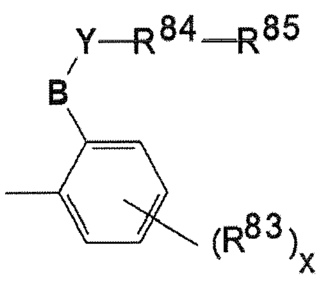
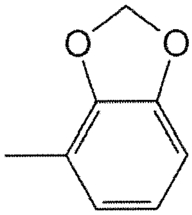
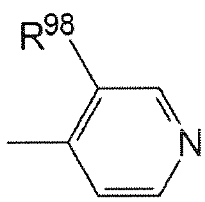

or

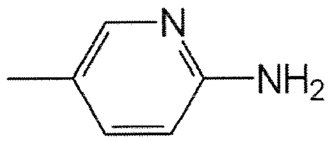

**B** is a bond or $-CH_2-$;
**Y** is a bond, -O-, or NH-;
**$R^{83}$** is selected from -H, -CN, -F, -Cl, $-O-CR^{62}R^{63}R^{64}$, $-CF_3$, $-CH_2OR^{23'}$, $-CR^{23'}O$, $-CR^{62}R^{63}-NR^{23'}R^{24'}$, $-CR^{62}R^{63}R^{64}$;
**$R^{23'}$** and **$R^{24'}$** represent independently of each other -H, $-CH_3$, -(cyclo-$C_3H_5$);
**$R^{62}$ - $R^{64}$** represent independently of each other -H, $-CH_3$, -Ph, -F, -(cyclo-$C_3H_5$); **$R^{84}$** is selected from a bond, $-CH_2-$, or $-CH_2-CH_2-CH_2-CH_2-$;
**$R^{85}$** is selected from -H, $-CF_3$, $-OCH_3$, $-OCH(CH_3)_2$, -CN, $NHCOCH_3$, $-OCH_2$-(cyclo-$C_3H_5$), $-NR^{23}R^{24}$, -Ph, -OPh, -NHCO-$OC(CH_3)_3$,

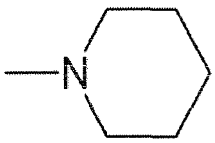
,
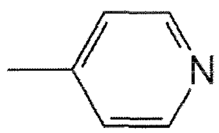
,
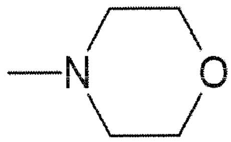
;

**$R^{98}$** represents $-OCH_3$;
and salts, solvates or salts of solvates of the afore-mentioned compounds and especially the hydrochloride salt or the trifluoroacetate salt of these compounds.

Moreover especially preferred are compounds of the general formula (I), wherein
**$R^1$** is

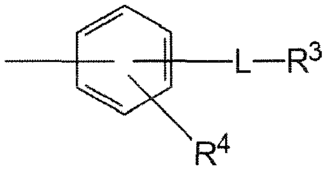
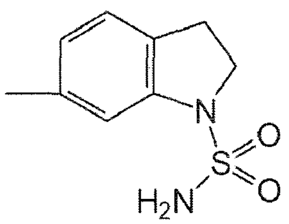
or
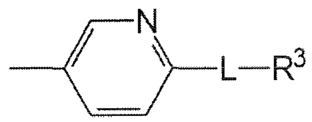

**L** is a bond, $-CH_2-$, or $-CH_2CH_2-$;
**$R^3$** is -H, $-SO_2NH_2$, $-CONH_2$, $-NO_2$, $-NH_2$, $-NH-SO_2-CH_3$, $-NH-SO_2-C_3H_7$, $-NHCO-CH_3$, $-SO_2CH_3$, -Ph, $-SO_2-NH-CH_2-CH_2-CH_2-CH_2-NH-COOC(CH_3)_3$, NH-CO-NH-Ph, or $-SO_2-NH-CH_2-CH_2-CH_2-CH_2-NH_2$,
**$R^4$** is -H, $-CH_2-SO_2NH_2$, $-SO_2NH_2$, $-C_2H_4-SO_2NH_2$, $-CONH_2$, $-NH-SO_2-CH_3$, $NH-SO_2-C_3H_7$, $-NHCO-CH_3$, $-NO_2$, $NH_2$, $-SO_2CH_3$, or

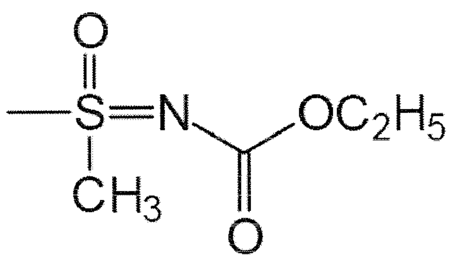

**$R^2$** represents

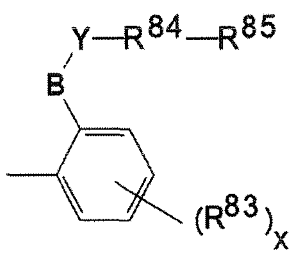 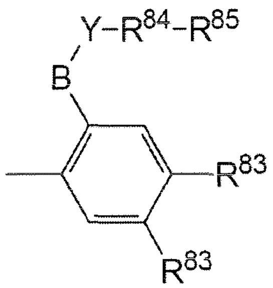 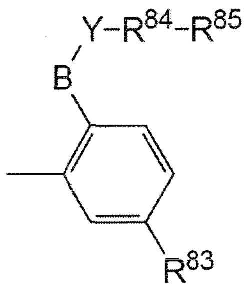 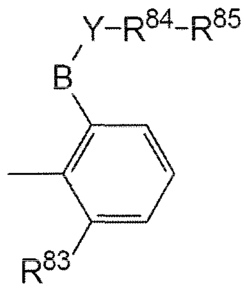

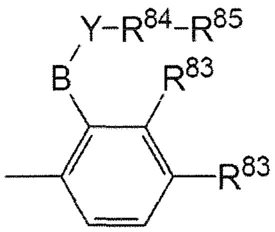 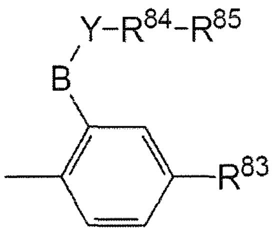 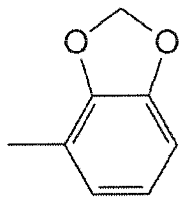 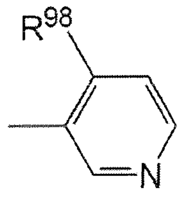

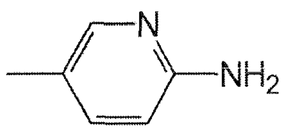

or

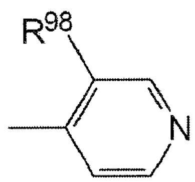

**B** is a bond or $-CH_2-$;
**Y** is a bond, -O-, or -NH-;
**R⁸³** is selected from -H, -F, -Cl, $-O-CH_3$, $-O-C_2H_5$, $-OCH_2-(cyclo-C_3H_5)$, -CN, $-CF_3$, $-CH_2OH$, -CHO, $-CH_2-NH(cyclo-C_3H_5)$, $-CH_2-NH(CH_3)$, $-CF_3$;
**$R^{84}$** is selected from a bond, $-CH_2-$, or $-CH_2-CH_2-CH_2-CH_2-$;
**$R^{85}$** is selected from -H, $-CF_3$, $-OCH_3$, $-OCH(CH_3)_2$, -CN, $NHCOCH_3$, $-OCH_2-(cyclo-C_3H_5)$, $NH_2$, $-NH-(cyclo-C_3H_5)$, -Ph, -OPh, $-NHCO-OC(CH_3)_3$,

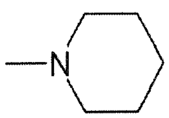 , 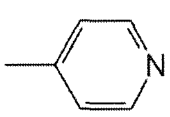 , 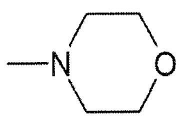 ;

**$R^{98}$** represents $-OCH_3$; and salts, solvates or salts of solvates of the afore-mentioned compounds and especially the hydrochloride salt or the trifluoroacetate salt of these compounds.

In a particularly preferred embodiment the present invention concerns compounds of formula (I), wherein **$R^1$** represents

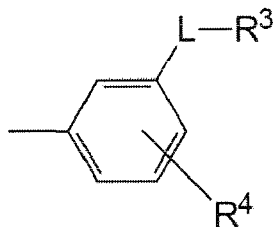

in which
the substituent $-L-R^3$ is $-SO_2NH_2$ or $-CH_2SO_2NH_2$,
$R^4$ is -H;
$R^2$ represents 2-methoxyphenyl, 4-fluoro-2-methoxyphenyl or 2-benzyloxyphenyl,
or their salts, solvates or salts of solvates and especially the hydrochloride salt or the trifluoroacetate salt.

In another particularly preferred embodiment the present invention concerns compounds of formula (I) selected from 3-[(4-(2-Methoxyphenyl)-1,3,5-triazin-2-yl)amino]benzenemethanesulfonamide (B1), 3-[(4-(2-Methoxyphenyl)-1,3,5-triazin-2-yl)amino]benzenesulfonamide (C1), 3-[(4-(4-Fluoro-2-methoxyphenyl)-1,3,5-triazin-2-yl)amino]benzenemethanesulfonamide (B2), 3-[(4-(2-Benzyloxyphenyl)-1,3,5-triazin-2-yl)amino]benzenemethanesulfonamide (B13), or their salts, solvates or salts of solvates and especially the hydrochloride salt or the trifluoroacetate salt.

In another particularly preferred embodiment the present invention concerns 3-[(4-(4-Fluoro-2-methoxyphenyl)-1,3,5-triazin-2-yl)amino]benzenemethanesulfonamide, or its salts, solvates or salts of solvates and especially the hydrochloride salt or the trifluoroacetate salt.

In another particularly preferred embodiment the present invention concerns 1-(3-{[4-(4-fluoro-2-methoxyphenyl)-1,3,5-triazin-2-yl]amino}phenyl)methanesulfonamide hydro-chloride.

Excluded are these compounds from the present invention, wherein $-R^4$ and $-L-R^3$ are methoxy or ethoxy groups.

Excluded from the present invention are also compounds, wherein $\mathbf{R^1}$ is

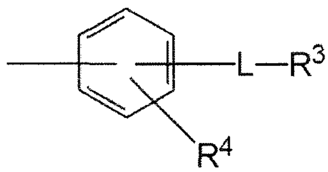

and wherein $R^2$ is

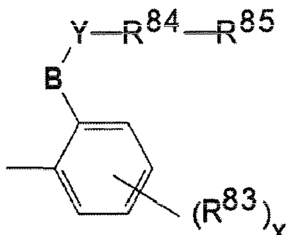

and wherein one of
$-R^4$ and $-L-R^3$ is a chloro substituent and wherein one of $B-Y-R^{84}-R^{85}$ and $-R^{83}$ is also a chloro substituent. More general, compounds of general formula (I) with two or more chloro substituents are not preferred and might be excluded.

If the group $B-Y-R^{84}-R^{85}$ represents the substituent NH-CO-Ph, the phenyl moiety $R^1$ has at least one substituent which is not in para position to the bond between the phenyl moiety $R^1$ and the triazine ring or the substituent $-L-R^3$, wherein L is a bond is different from the substituent - $CO-NH_2$. In addition the following compound is excluded from the scope of the present invention by disclaimer:

4-[4-(2-benzoylaminophenyl)-[1,3,5]triazin-2-ylamino]benzamide

152

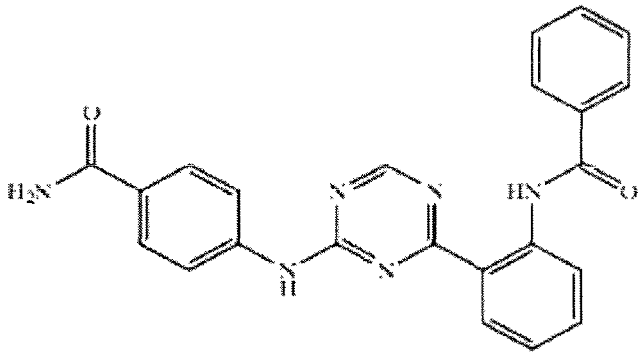

In a further aspect of the present invention, the novel compounds according to the general formula (I) represent chiral compounds. The novel compounds according to the general formula (I) represent a racemate, or a S or a R enantiomer or a mixture of isomers.

In yet another preferred embodiment of the present invention, the compound according to the general formula (I) is selected from the group of compounds depicted in the following Table 1.

**Table 1**

| Compound No. | Structure | Nomenclature |
|---|---|---|
| **B1** | 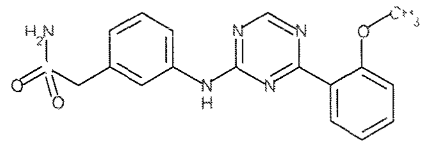 | 3-[(4-(2-Methoxyphenyl)-1,3,5-triazin-2-yl)amino] benzenemethanesulfonamide |
| **B2** | 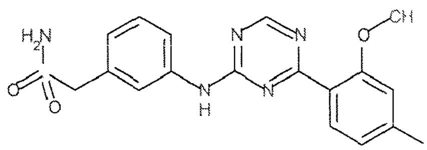 | 3-[(4-(4-Fluoro-2-methoxyphenyl)-1,3,5-triazin-2-yl)amino] benzenemethanesulfonamide |
| **B3** | 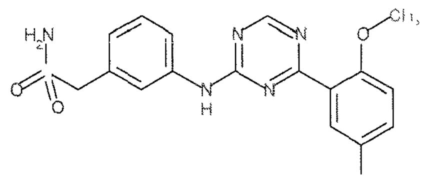 | 3-[(4-(5-Fluoro-2-methoxyphenyl)-1,3,5-triazin-2-yl)amino] benzenemethanesulfonamide |
| **B4** | 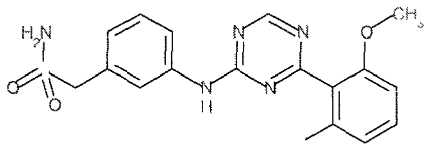 | 3-[(4-(6-Fluoro-2-methoxyphenyl)-1,3,5-triazin-2-yl)amino] benzenemethanesulfonamide |
| **B5** | 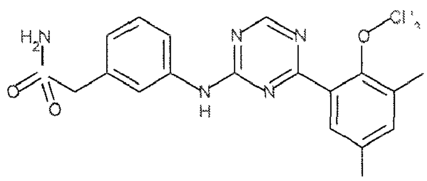 | 3-[(4-(3,5-Difluoro-2-methoxyphenyl)-1,3,5-triazin-2-yl)amino] benzenemethanesulfonamide |
| **B6** | 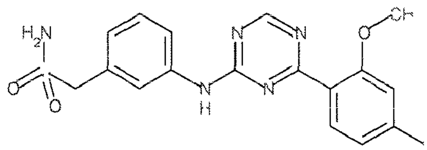 | 3-[(4-(4-Chloro-2-methoxyphenyl)-1,3,5-triazin-2-yl)amino] benzenemethanesulfonamide |

(continued)

| Compound No. | Structure | Nomenclature |
|---|---|---|
| **B7** | 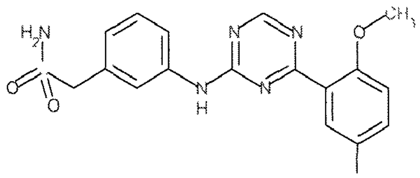 | 3-[(4-(5-Chloro-2-methoxyphenyl)-1,3,5-triazin-2-yl)amino] benzenemethanesulfonamide |
| **B8** | 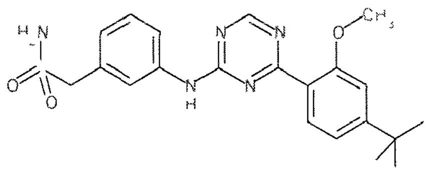 | 3-[(4-(2-Methoxy-4-trifluoromethyl-phenyl)-1,3,5-triazin-2-yl)amino] benzenemethanesulfonamide |
| **B9** | 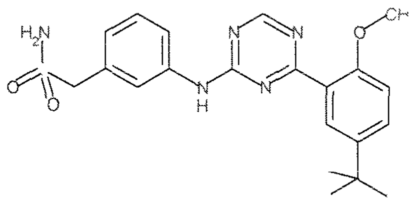 | 3-[(4-(2-Methoxy-5-trifluoromethyl-phenyl)-1,3,5-triazin-2-yl)amino] benzenemethanesulfonamide |
| **B10** | 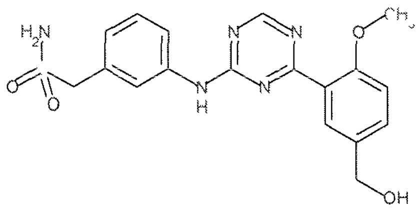 | 3-[(4-(5-Hydroxymethyl-2-methoxyphenyl)-1,3,5-triazin-2-yl)amino] benzenemethanesulfonamide |
| **B11** | 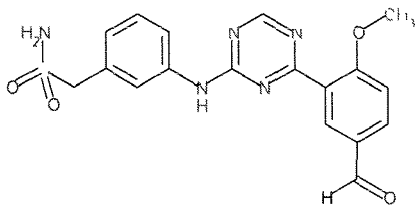 | 3-[(4-(5-Formyl-2-methoxyphenyl)-1,3,5-triazin-2-yl)amino] benzenemethanesulfonamide |
| **B12** | 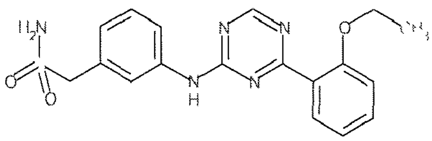 | 3-[(4-(2-Ethoxyphenyl)-1,3,5-triazin-2-yl)amino] benzenemethanesulfonamide |
| **B13** | 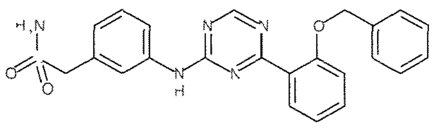 | 3-[(4-(2-Benzyloxyphenyl)-1,3,5-triazin-2-yl) amino] benzenemethanesulfonamide |
| **B14** | 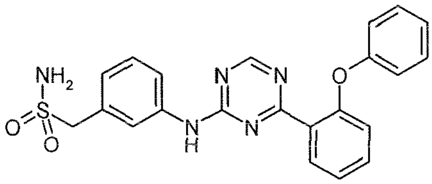 | 1-(3-{[4-(2-phenoxyphenyl)-1,3,5-triazin-2-yl] amino}phenyl)methanesulfonamide |

(continued)

| Compound No. | Structure | Nomenclature |
|---|---|---|
| **B15** | 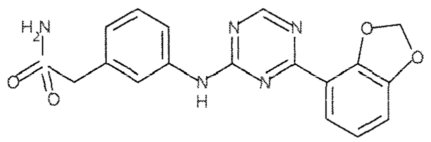 | 3-[(4-(1,3-Benzodioxol-4-yl)-1,3,5-triazin-2-yl) amino]benzenemethanesulfonamide |
| **B16** | 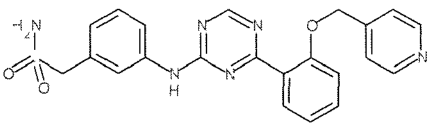 | 3-[(4-(2-((4-Pyridinyl)methoxy)phenyl)-1,3,5-triazin-2-yl)amino]benzenemethanesulfonamide |
| **B17** | 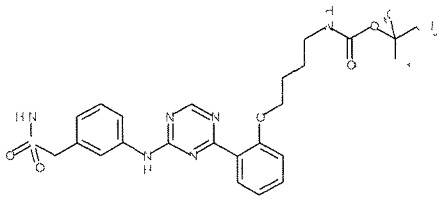 | 3-[(4-(2-(4-(tert-Butoxycarbonylamino)butoxy) phenyl)-1,3,5-triazin-2-yl)amino] benzenemethanesulfonamide |
| **B18** | 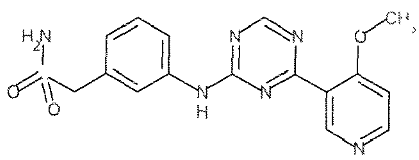 | 3-[(4-(4-Methoxypyridin-3-yl)-1,3,5-triazin-2-yl) amino]benzenemethanesulfonamide |
| **B19** | 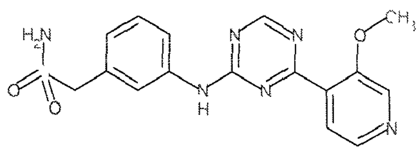 | 3-[(4-(3-Methoxypyridin-4-yl)-1,3,5-triazin-2-yl) amino]benzenemethanesulfonamide |
| **B20** | 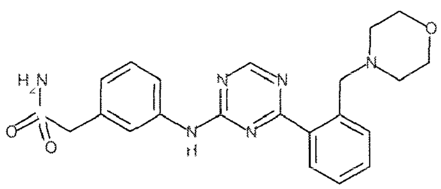 | 3-[(4-(2-((Morpholin-4-yl)methyl)phenyl)-1,3,5-triazin-2-yl)amino]benzenemethanesulfonamide |
| **B21** | 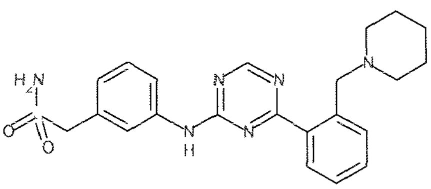 | 3-[(4-(2-((Piperidin-1-yl)methyl)phenyl)-1,3,5-triazin-2-yl)amino]benzenemethanesulfonamide |
| **B22** | 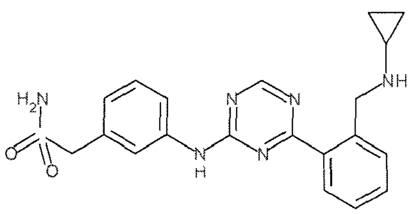 | 3-[(4-(2-(Cyclopropylamino-methyl)phenyl)-1,3,5-triazin-2-yl)amino] benzenemethanesulfonamide |

(continued)

| Compound No. | Structure | Nomenclature |
|---|---|---|
| **B23** | 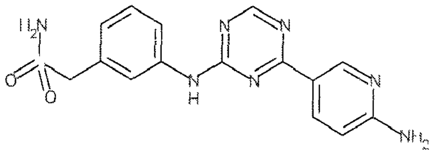 | 3-[(4-(6-Aminopyridin-3-yl)-1,3,5-triazin-2-yl) amino]benzenemethanesulfonamide |
| **B24** | 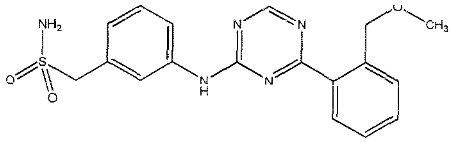 | 3-[(4-(2-(Methoxymethyl)phenyl)-1,3,5-triazin-2-yl)amino]benzenemethanesulfonamide |
| **C1** | 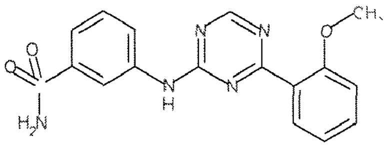 | 3-[(4-(2-Methoxyphenyl)-1,3,5-triazin-2-yl)amino] benzenesulfonamide |
| **D1** | 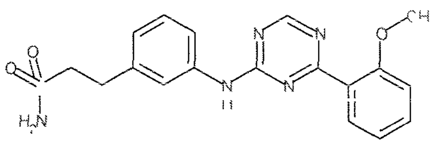 | 2-[3-((4-(2-Methoxyphenyl)-1,3,5-triazin-2-yl) amino)phenyl]ethanesulfonamide |
| **D2** | 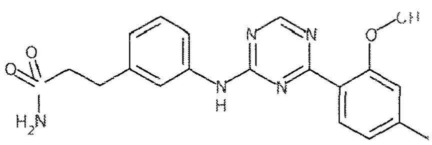 | 2-[3-((4-(4-Fluoro-2-methoxyphenyl)-1,3,5-triazin-2-yl)amino)phenyl]ethanesulfonamide |
| **E1** | 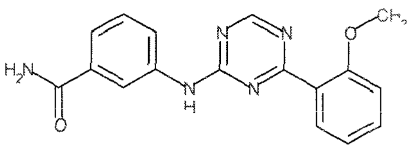 | 3-[(4-(2-Methoxyphenyl)-1,3,5-triazin-2-yl)amino] benzamide |
| **F1** | 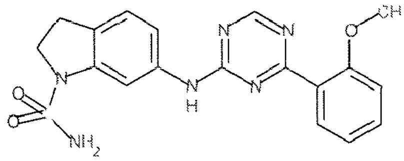 | 6-[(4-(2-Methoxyphenyl)-1,3,5-triazin-2yl)amino]-2,3-dihydro-1H-indole-1-sulfonamide |
| **G1** | 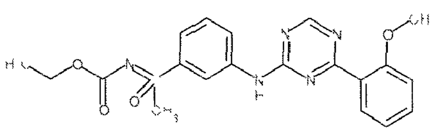 | rac-S-[3-((4-(2-Methoxyphenyl)-1,3,5-triazin-2-yl) amino)phenyl]-N-ethoxycarbonyl-S-methyl-sulfoximide |
| **H1** | 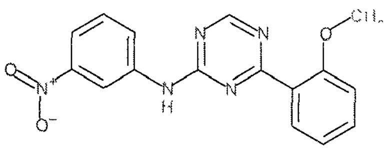 | 4-(2-Methoxyphenyl)-N-(3-nitrophenyl)-1,3,5-triazine-2-amine |

(continued)

| Compound No. | Structure | Nomenclature |
|---|---|---|
| I1 | 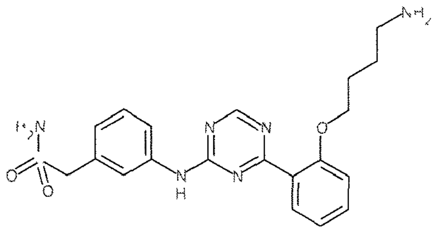 | 3-[(4-(2-(4-Aminobutoxy)phenyl)-1,3,5-triazin-2-yl)amino]benzenemethanesulfonamide |
| J1 | 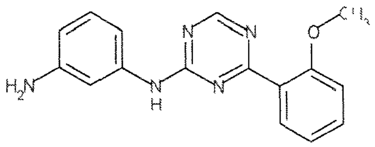 | N-(3-Aminophenyl)-4-(2-methoxyphenyl)-1,3,5-triazine-2-amine |
| K1 | 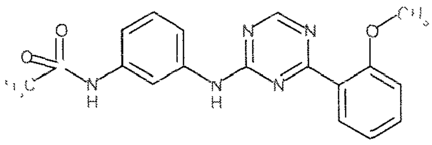 | N-[3-((4-(2-Methoxyphenyl)-1,3,5-triazin-2-yl) amino)phenyl]-methanesulfonamide |
| L1 | 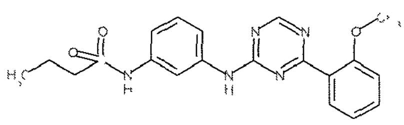 | N-[3-((4-(2-Methoxyphenyl)-1,3,5-triazin-2-yl) amino)phenyl]-propanesulfonamide |
| M1 | 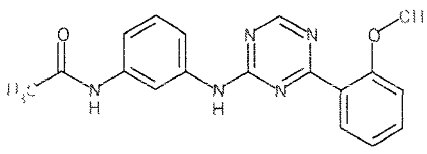 | N-[3-((4-(2-Methoxyphenyl)-1,3,5-triazin-2-yl) amino)phenyl]acetamide |
| N1 | 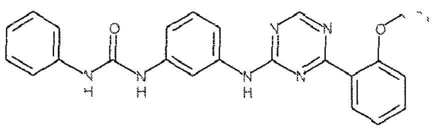 | N-[3-((4-(2-Methoxyphenyl)-1,3,5-triazin-2-yl) amino)phenyl]-N'-phenyl-urea |
| O1 | 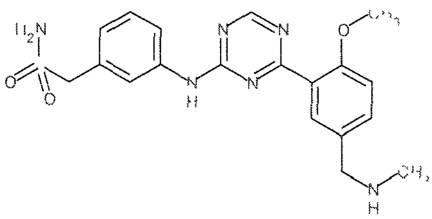 | 3-[(4-(2-Methoxy-5-(methylamino-methyl) phenyl)-1,3,5-triazin-2-yl)amino] benzenemethanesulfonamide |
| P1 | 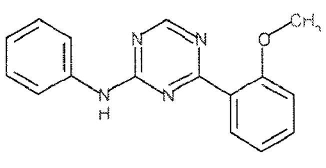 | 4-(2-Methoxyphenyl)-N-phenyl-1,3,5-triazine-2-amine |
| Q1 | 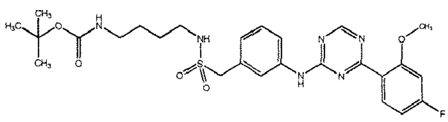 | tert-Butyl [4-((3-((4-(4-Fluoro-2-methoxyphenyl)-1,3,5-triazin-2-yl)amino)phenyl) methylsulfonamido) butyl]carbamate |

(continued)

| Compound No. | Structure | Nomenclature |
|---|---|---|
| R1 | 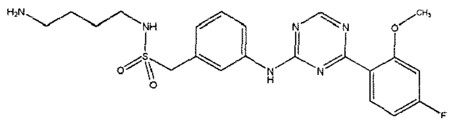 | N-(4-Aminobutyl)-1-[3-((4-(4-fluoro-2-methoxyphenyl)-1,3,5-triazin-2-yl)amino)phenyl] methanesulfonamide |
| S1 | 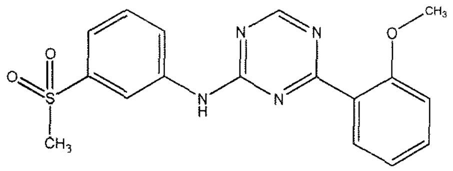 | 4-(2-Methoxyphenyl)-N-(3-(methylsulfonyl) phenyl)-1,3,5-triazin-2-amine |
| T1 | 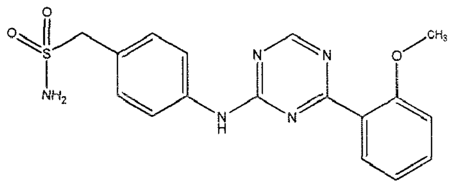 | 4-[(4-(2-Methoxyphenyl)-1,3,5-triazin-2-yl)amino] benzenemethane-sulfonamide |
| U1 | 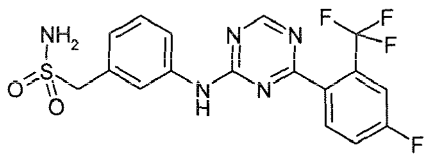 | 1-[3-({4-[4-fluoro-2-(trifluoromethyl)phenyl]-1,3,5-triazin-2-yl}amino)phenyl]methanesulfonamide |
| U2 | 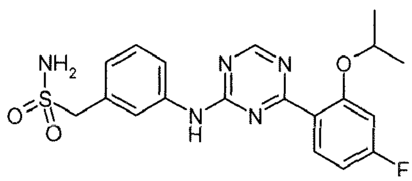 | 1-[3-({4-[4-fluoro-2-(propan-2-yloxy)phenyl]-1,3,5-triazin-2-yl}amino)phenyl] methanesulfonamide |
| U3 | 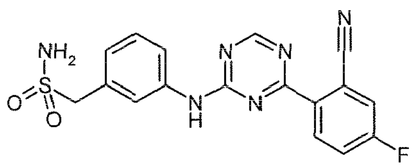 | 1-(3-{[4-(2-cyano-4-fluorophenyl)-1,3,5-triazin-2-yl]amino}phenyl)methanesulfonamide |
| U4 | 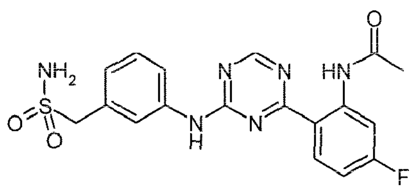 | N-[5-fluoro-2-(4-{[3-(sulfamoylmethyl)phenyl] amino}-1,3,5-triazin-2-yl)phenyl]acetamide |
| U5 | 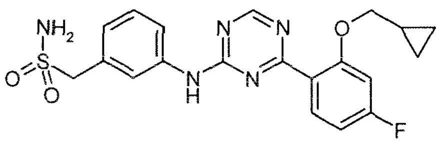 | 1-[3-({4-[2-(cyclopropylmethoxy)-4-fluorophenyl]-1,3,5-triazin-2-yl}amino)phenyl] methanesulfonamide |
| U6 | 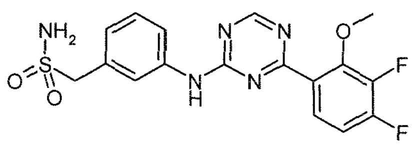 | 1-(3-{[4-(3,4-difluoro-2-methoxyphenyl)-1,3,5-triazin-2-yl]amino}phenyl)methanesulfonamide |

(continued)

| Compound No. | Structure | Nomenclature |
|---|---|---|
| **U7** | 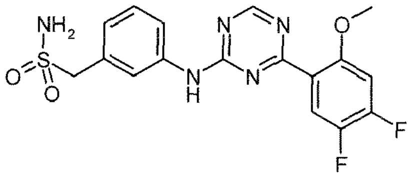 | 1-(3-{[4-(4,5-difluoro-2-methoxyphenyl)-1,3,5-triazin-2-yl]amino}phenyl)methanesulfonamide |
| **U8** | 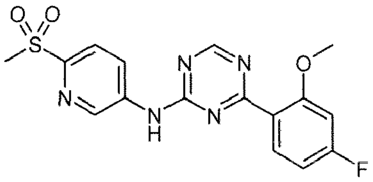 | 4-(4-fluoro-2-methoxyphenyl)-N-[6-(methylsulfonyl)pyridin-3-yl]-1,3,5-triazin-2-amine |
| **B1'** | 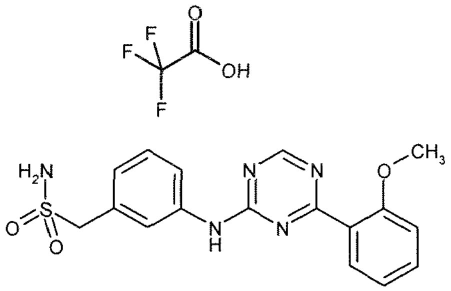 | 3-[(4-(2-Methoxyphenyl)-1,3,5-triazin-2-yl)amino] benzenemethanesulfonamide trifluoroacetic acid salt |
| **B2'** | HCl 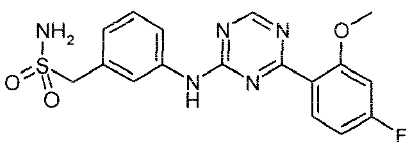 | 1-(3-{[4-(4-fluoro-2-methoxyphenyl)-1,3,5-triazin-2-yl]amino}phenyl)methanesulfonamide hydrochloride |
| **B13'** | 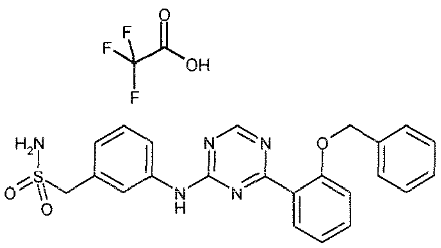 | 3-[(4-(2-Benzyloxyphenyl)-1,3,5-triazin-2-yl) amino]benzenemethanesulfon-amide trifluoroacetic acid salt |
| **C1'** | 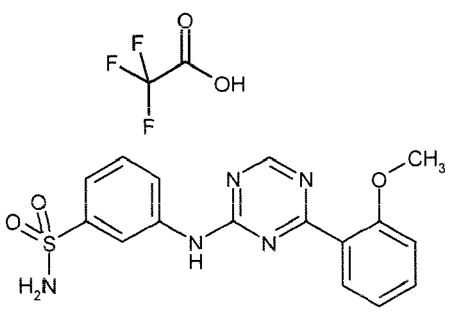 | 3-[(4-(2-Methoxyphenyl)-1,3,5-triazin-2-yl)amino] benzenesulfonamide trifluoroacetic acid salt |

(continued)

| Compound No. | Structure | Nomenclature |
|---|---|---|
| B2" | $CF_3COOH$ 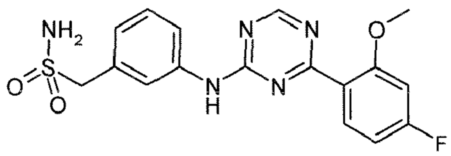 | 1-(3-{[4-(4-fluoro-2-methoxyphenyl)-1,3,5-triazin-2-yl]amino}phenyl)methanesulfonamide trifluoroacetic acid salt |

It is particularly preferred to use the following selective CDK9 inhibitors shown in Table 2 in accordance with the present invention:

**Table 2.**

| Compound No. | Structure | Nomenclature |
|---|---|---|
| B1 | 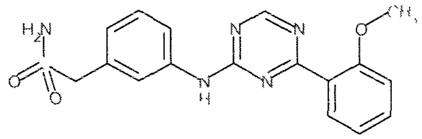 | 3-[(4-(2-Methoxyphenyl)-1,3,5-triazin-2-yl)amino] benzenemethanesulfonamide |
| B2 | 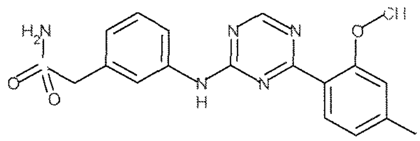 | 3-[(4-(4-Fluoro-2-methoxyphenyl)-1,3,5-triazin-2-yl)amino] benzenemethanesulfonamide |
| B3 | 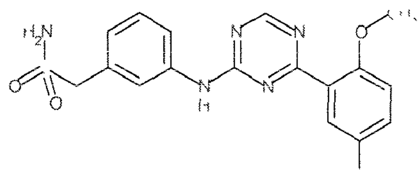 | 3-[(4-(5-Fluoro-2-methoxyphenyl)-1,3,5-triazin-2-yl)amino] benzenemethanesulfonamide |
| B4 | 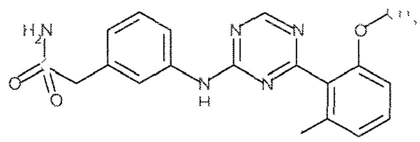 | 3-[(4-(6-Fluoro-2-methoxyphenyl)-1,3,5-triazin-2-yl)amino] benzenemethanesulfonamide |
| B6 | 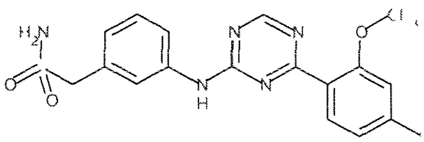 | 3-[(4-(4-Chloro-2-methoxyphenyl)-1,3,5-triazin-2-yl)amino] benzenemethanesulfonamide |
| B7 | 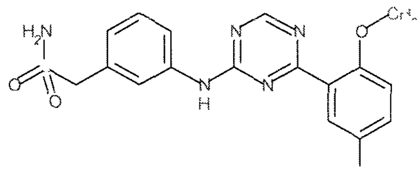 | 3-[(4-(5-Chloro-2-methoxyphenyl)-1,3,5-triazin-2-yl)amino] benzenemethanesulfonamide |
| B12 | 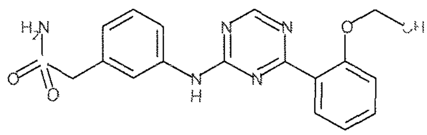 | 3-[(4-(2-Ethoxyphenyl)-1,3,5-triazin-2-yl)amino] benzenemethanesulfonamide |

(continued)

| Compound No. | Structure | Nomenclature |
|---|---|---|
| **B13** | 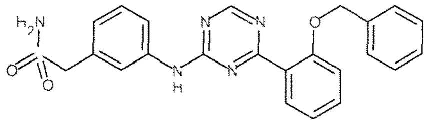 | 3-[(4-(2-Benzyloxyphenyl)-1,3,5-triazin-2-yl) amino] benzenemethanesulfonamide |
| **B14** | 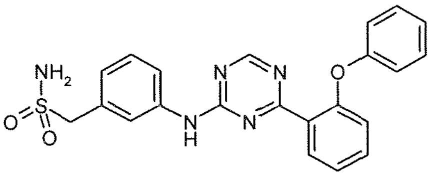 | 1-(3-{[4-(2-phenoxyphenyl)-1,3,5-triazin-2-yl] amino}phenyl)methanesulfonamide |
| **B16** | 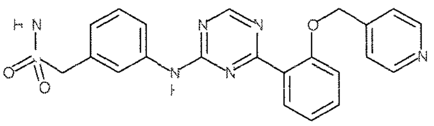 | 3-[(4-(2-((4-Pyridinyl)methoxy)phenyl)-1,3,5-triazin-2-yl)amino]benzenemethanesulfonamide |
| **B17** | 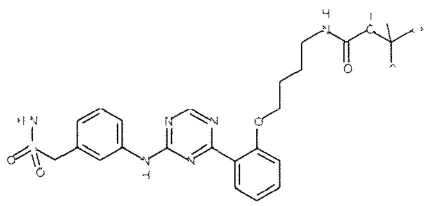 | 3-[(4-(2-(4-(tert-Butoxycarbonylamino)butoxy) phenyl)-1,3,5-triazin-2-yl)amino] benzenemethanesulfonamide |
| **B18** | 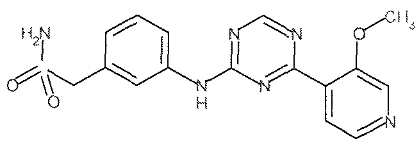 | 3-[(4-(3-Methoxypyridin-4-yl)-1,3,5-triazin-2-yl) amino]benzenemethanesulfonamide |
| **B23** | 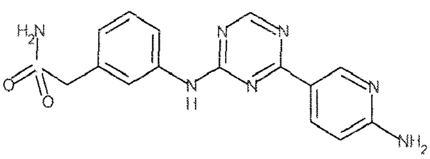 | 3-[(4-(6-Aminopyridin-3-yl)-1,3,5-triazin-2-yl) amino]benzenemethanesulfonamide |
| **B24** | 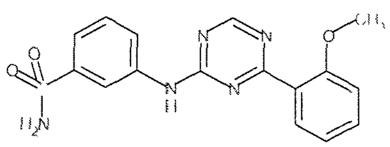 | 3-[(4-(2-Methoxyphenyl)-1,3,5-triazin-2-yl)amino] benzenesulfonamide |
| **C1** | 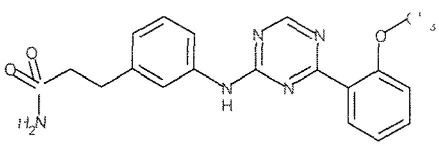 | 2-[3-((4-(2-Methoxyphenyl)-1,3,5-triazin-2-yl) amino)phenyl]ethanesulfonamide |
| **D1** | 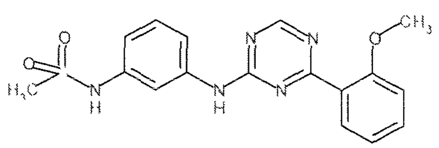 | N-[3-((4-(2-Methoxyphenyl)-1,3,5-triazin-2-yl) amino)phenyl]-methanesulfonamide |
| **L1** | 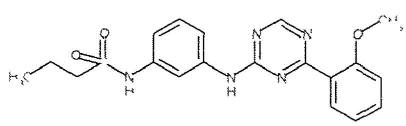 | N-[3-((4-(2-Methoxyphenyl)-1,3,5-triazin-2-yl) amino)phenyl]-propanesulfonamide |

(continued)

| Compound No. | Structure | Nomenclature |
|---|---|---|
| **Q1** | 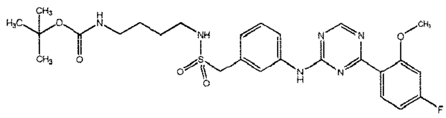 | tert-Butyl [4-((3-((4-(4-Fluoro-2-methoxyphenyl)-1,3,5-triazin-2-yl)amino)phenyl) methylsulfonamido) butyl]carbamate |
| **R1** | 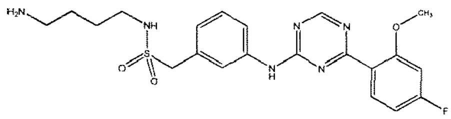 | N-(4-Aminobutyl)-1-[3-((4-(4-fluoro-2-methoxyphenyl)-1,3,5-triazin-2-yl)amino)phenyl] methanesulfonamide |
| **S1** | 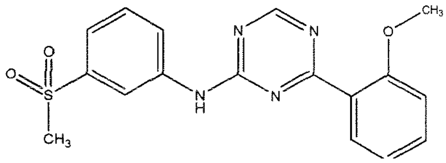 | 4-(2-Methoxyphenyl)-N-(3-(methylsulfonyl) phenyl)-1,3,5-triazin-2-amine |
| **U2** | 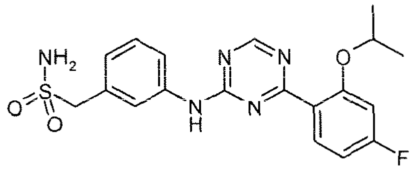 | 1-[3-({4-[4-Fluoro-2-(propan-2-yloxy)phenyl]-1,3,5-triazin-2-yl}amino)phenyl] methanesulfonamide |
| **U5** | 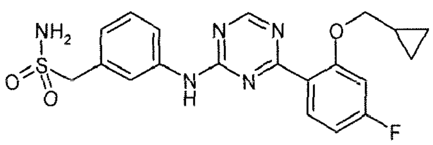 | 1-[3-({4-[2-(Cyclopropylmethoxy)-4-fluorophenyl]-1,3,5-triazin-2-yl}amino)phenyl] methanesulfonamide |
| **U7** | 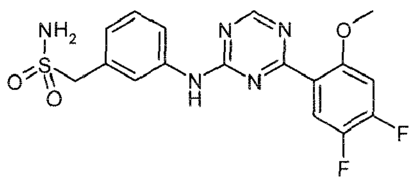 | 1-(3-{[4-(4,5-Difluoro-2-methoxyphenyl)-1,3,5-triazin-2-yl]amino}phenyl)methanesulfonamide |
| **24** | 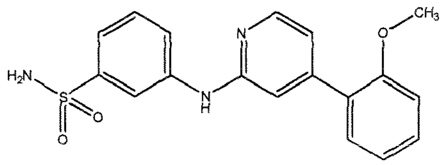 | 3-[(4-(2-Methoxyphenyl)pyridin-2-yl)amino] benzenesulfonamide |
| **25** | 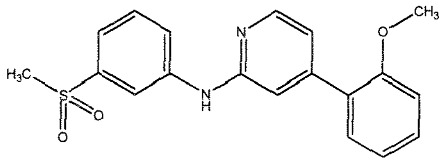 | 4-(2-Methoxyphenyl)-N-(3-(methylsulfonyl) phenyl)pyridin-2-amine |
| **26** | 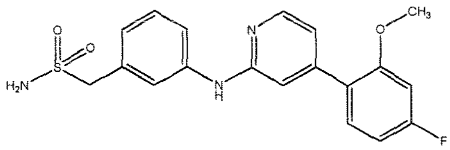 | [3-((4-(4-Fluoro-2-methoxyphenyl)pyridin-2-yl) amino)phenyl]methanesulfonamide |
| **27** | 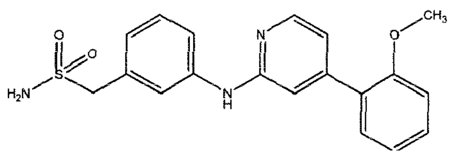 | [3-((4-(2-Methoxyphenyl)pyridin-2-yl)amino) phenyl]methanesulfonamide |

(continued)

| Compound No. | Structure | Nomenclature |
|---|---|---|
| 28 | 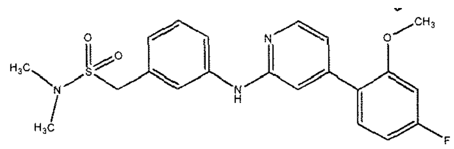 | 1-[3-((4-(4-Fluoro-2-methoxyphenyl)pyridin-2-yl) amino)phenyl]-N,N-dimethylmethanesulfonamide |
| 29 | 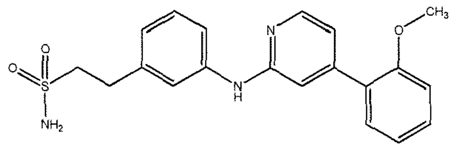 | 2-[3-((4-(2-Methoxyphenyl)pyridin-2-yl)amino) phenyl]ethanesulfonamide |
| 30 | 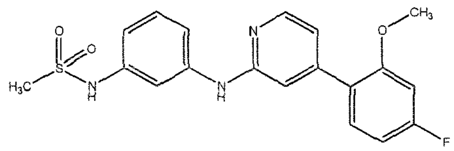 | N-[3-((4-(4-Fluoro-2-methoxyphenyl)pyridin-2-yl) amino)phenyl]methanesulfonamide |
| 31 | 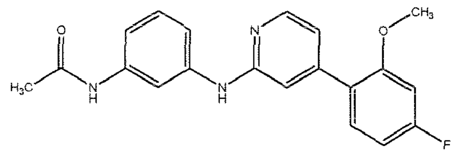 | N-[3-((4-(4-Fluoro-2-methoxyphenyl)pyridin-2-yl) amino)phenyl]acetamide |
| 32 | 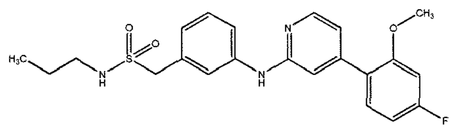 | 1-[3-((4-(4-Fluoro-2-methoxyphenyl)pyridin-2-yl) amino)phenyl]-N-propylmethanesulfonamide |
| 33 | 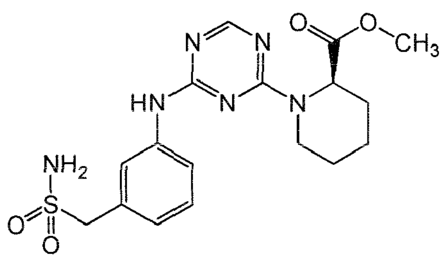 | (R)-Methyl 1-[4-((3-(Sulfamoylmethyl)phenyl) amino)-1,3,5-triazin-2-yl]piperidine-2-carboxylate |
| 34 | 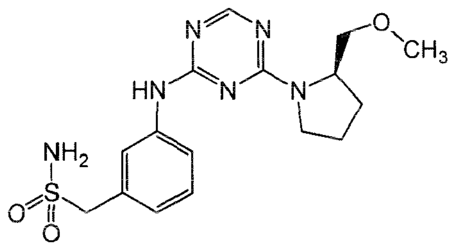 | (R)-3-[(4-(2-(Methoxymethyl)pyrrolidin-1-yl)-1,3,5-triazin-2-yl)amino] benzenemethanesulfonamide |
| 35 | 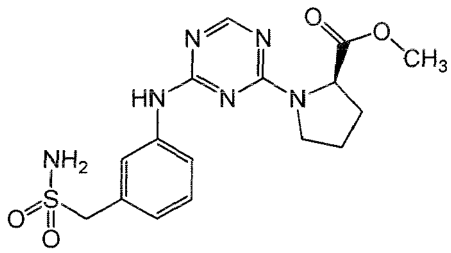 | (R)-Methyl 1-[4-((3-(Sulfamoylmethyl)phenyl) amino)-1,3,5-triazin-2-yl]pyrrolidine-2-carboxylate |

(continued)

| Compound No. | Structure | Nomenclature |
|---|---|---|
| **36** | 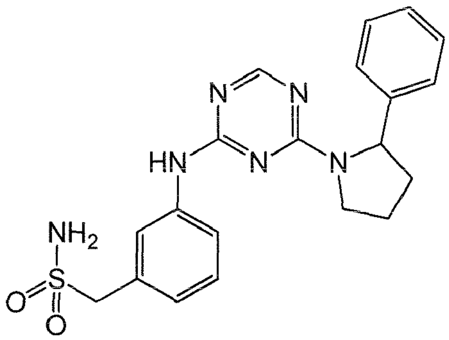 | rac-3-[(4-(2-Phenylpyrrolidin-1-yl)-1,3,5-triazin-2-yl)amino]benzenemethanesulfonamide |
| **37** | 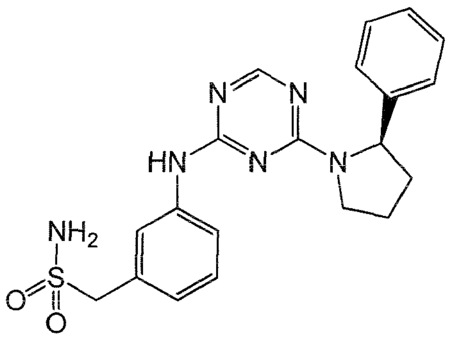 | (R)-3-[(4-(2-Phenylpyrrolidin-1-yl)-1,3,5-triazin-2-yl)amino]benzenemethanesultonamide |
| **38** | 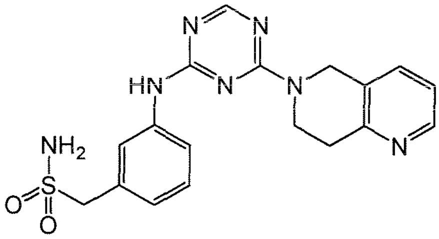 | 3-[(4-(7,8-Dihydro-1,6-naphthyridin-6(5H)-yl)-1,3,5-triazin-2-yl)amino] benzenemethanesulfonamide |
| **39** | 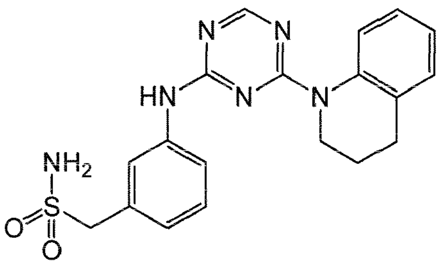 | 3-[(4-(3,4-Dihydroquinolin-1 (2H)-yl)-1,3,5-triazin-2-yl)amino]benzenemethanesulfonamide |
| **40** | 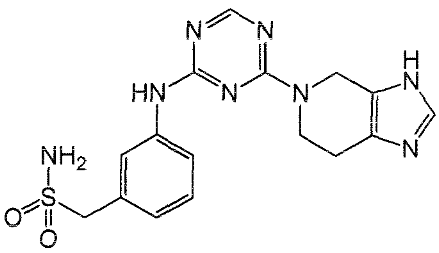 | 3-[(4-(6,7-Dihydro-3H-imidazo[4,5-c]pyridin-5 (4H)-yl)-1,3,5-triazin-2-yl)amino] benzenemethanesulfonamide |

(continued)

| Compound No. | Structure | Nomenclature |
|---|---|---|
| **41** | 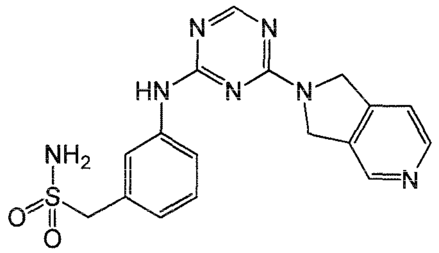 | 3-[(4-(1H-Pyrrolo[3,4-c]pyridin-2(3H)-yl)-1,3,5-triazin-2-yl)amino]benzenemethanesulfonamide |
| **42** | 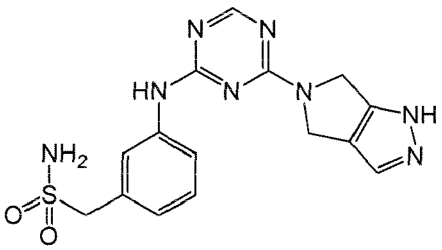 | 3-[(4-(Pyrrolo[3,4-c]pyrazol-5(1H,4H,6H)-yl)-1,3,5-triazin-2-yl)amino] benzenemethanesulfonamide |
| **43** | 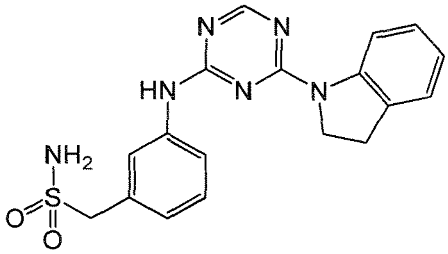 | 3-[(4-(Indolin-1-yl)-1,3,5-triazin-2-yl)amino] benzenemethanesulfonamide |
| **44** | 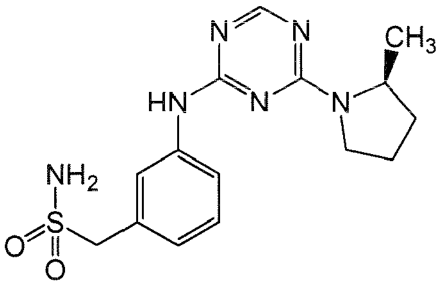 | (S)-3-[(4-(2-Methylpyrrolidin-1-yl)-1,3,5-triazin-2-yl)amino]benzenemethanesulfonamide |

The above and further compounds which can be used in accordance with the present invention are also disclosed in PCT/EP2011/001445, EP10075131.2 (filing date 22.03.2010) EP11075037.9 (filing date 02.03.2011) and EP11075038.7 (filing date 02.03.2011) which are incorporated herein by reference in their entirety.

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form with a sufficient amount of the desired acid to produce a salt in the conventional manner. Preferred is the mesylate salt, hydrochloride salt and the trifluoroacetate salt and especially preferred is the trifluoroacetate salt and the hydrochloride salt.

In the case the inventive compounds bear acidic groups, salts could also be formed with inorganic or organic bases. Examples for suitable inorganic or organic bases are, for example, NaOH, KOH, $NH_4OH$, tetraalkylammonium hydroxide, lysine or arginine and the like. Salts may be prepared in a conventional manner using methods well known

in the art, for example by treatment of a solution of the compound of the general formula (I) with a solution of an acid, selected out of the group mentioned above.

Syntheses of compounds

The synthesis of the inventive disubstituted triazines according to the present invention is preferably carried out according to the general synthetic sequences, shown in Schemes 1 to 3.

**Scheme 1**

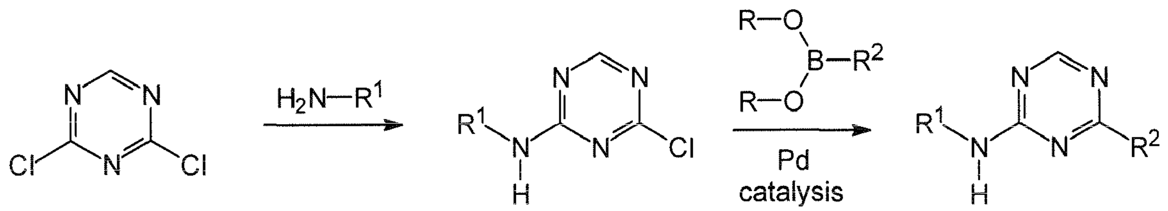


In a first step 2,4-Dichloro-1,3,5-triazine is reacted with anilines $R^1NH_2$ to give 2-arylamino-4-chloro-1,3,5-triazines. The reaction is carried out with one equivalent of the aniline in an inert solvent like DMF, THF, DME, dioxane or an alcohol like isopropanol, or mixtures of such solvents. Preferably the reaction is carried out at a temperature below room temperature in such a way that the reaction mixture is kept homogenous. Preferred conditions use an additional base like triethylamine or N,N-diisopropylethylamine.

In a second step the intermediate 2-arylamino-4-chloro-1,3,5-triazine is reacted with a boronic acid derivative $R^2$-$B(OR)_2$ to give compounds of Formula (I). The boronic acid derivative may be a boronic acid (R = -H) or an ester of the boronic acid, e.g. its isopropyl ester (R = - $CH(CH_3)_2$), preferably an ester derived from pinacol in which the boronic acid intermediate forms a 2-aryl-4,4,5,5-tetramethyl-1,3,2-dioxaborolane (R-R = $-C(CH_3)_2-C(CH_3)_2-$). Both R represent independently of each other preferably hydrogen or an alkyl chain with 1-10 carbon atoms or a cycloalkyl chain with 3 to 12 carbon atoms or both residues R represent together a residue derived from pinacol. The coupling reaction is catalyzed by Pd catalysts, e.g. by Pd(0) catalysts like tetrakis(triphenylphosphine)palladium(0) $[Pd(PPh_3)_4]$, tris(dibenzylideneacetone)di-palladium(0) $[Pd_2(dba)_3]$, or by Pd(II) catalysts like dichlorobis(triphenylphosphine)-palladium(II) $[Pd(PPh_3)_2Cl_2]$, palladium(II) acetate and triphenylphosphine or more preferred by [1,1'-bis(diphenylphosphino)ferrocene] palladium dichloride. The reaction is preferably carried out in a mixture of a solvent like dioxane, DMF, DME, THF, or isopropanol with water and in the presence of a base like aqueous sodium bicarbonate or $K_3PO_4$.

**Scheme 2**

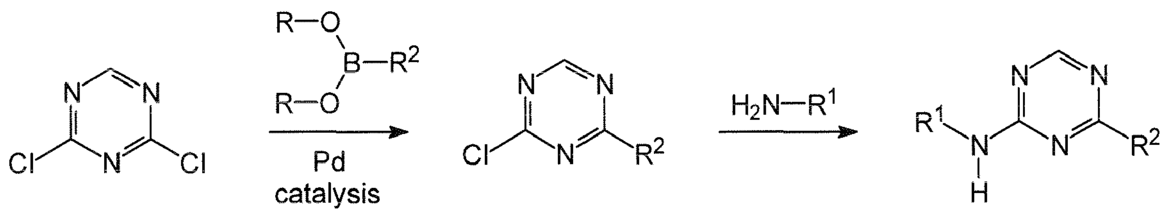


The synthesis of 1,3,5-triazines of Formula (I) starting from 2,4-dichloro-1,3,5-triazine may be carried out in the inverse order of the reaction steps compared to Scheme 1, in such a manner that in a first step the reaction of a triazine with a boronic acid derivative is followed in a second step by the reaction of the intermediate triazine with an aniline. Preferred conditions for the coupling reaction of the first step are heating the reacting agents in toluene with dichlorobis(triphenylphosphine)palladium(II) $[Pd(PPh_3)_2Cl_2]$ as a catalyst in the presence of sodium or potassium carbonate as a base.

## Scheme 3

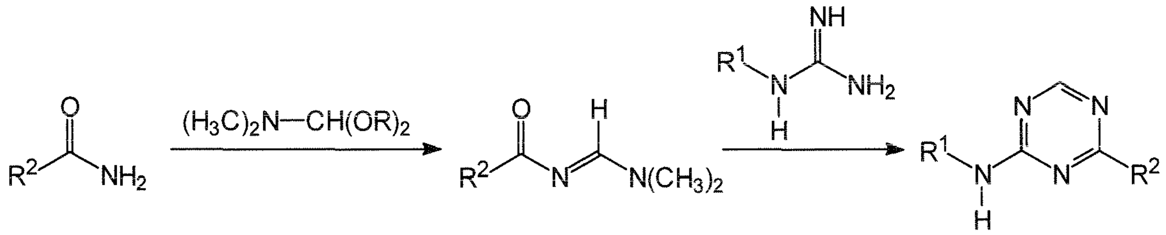


Compounds of Formula (I) may be prepared by the methodology described in J. Org. Chem. 60 (1995), 8428-8430. Primary amides $R^2$-CONH2 are heated with acetals and preferably dialkylacetals of N,N-dimethylformamide, preferably with its dimethyl or diethyl acetal, in particular with the dimethyl acetal (R = $-CH_3$). The intermediate N-acylformamidine is not isolated and subsequently converted to 1,3,5-triazines of Formula (I) by heating with a guanidine $R^1$-NH-C(NH)$NH_2$. Preferably the reaction is carried out by heating the reacting agents in dioxane in the presence of a base like potassium tert-butoxide.

Several compounds of Formula (I) may be prepared by converting substituents which are attached to the aromatic rings $R^1$ and/or $R^2$ to other substituents using standard reactions which are known to the person skilled in the art. For example, a nitro group can be reduced to an amino group, such an amino group can be converted to a sulfonamide by reaction with a sulfonyl chloride, to a carboxamide by reaction with a carbonyl chloride or another activated derivative of a carboxylic acid, to an urea by reaction with an isocyanate. Carbamate substituents may be cleaved to amino groups, in particular tert-butyl carbamates by reaction with acids like trifluoroacetic acid or hydrochloric acid. Formyl groups may be converted to aminomethyl groups by reaction with primary amines under conditions of a reductive amination; see, for example, synthesis of the compounds as shown in Table 2.

Further CDK9 inhibitors to be used in accordance with the present invention are well known in the art and are, for example, described in Krystof (2009) Medicinal Research Reviews, DOI 10.1002/med.24172, as well as in international patent applications published as WO 2009/047359, WO 2010/003133, WO 2008/79933 and WO 2011/012661. All these documents are incorporated herein by reference in their entirety.

Potential CDK9 inhibitors, especially selective CDK9 inhibitors, as defined herein above may be screened/identified by routine assays, such as a radiometric protein kinase assay (33PanQinase® Activity Assay; and/or the well known Lance Assay.

The following exemplary inhibitors can be used in accordance with the present invention, for example, in cotherapy as described herein: SNS-032: Piperidine-4-carboxylic acid [5-(5-tert-butyl-oxazol-2-ylmethylsulfanyl)-thiazol-2-yl]-amide; Misra RN et al. J Med Chem. 2004, 47(7):1719-28;

flavopiridol: 2-(2-Chloro-phenyl)-5,7-dihydroxy-8-(3-hydroxy-1-methyl-piperidin-4-yl)-chromen-4-one, Lloyd R Kelland. Expert Opinion on Investigational Drugs. 2000, 9(12):2903-2911;

AX35427: N-(5-((6-(3-aminophenyl)pyrimidin-4-yl)amino)-2-methylphenyl)propane-1-sulfonamide, 848637-29-6P in WO 2005026129;

R-547: [4-amino-2-(1-methanesulfonylpiperidin-4-ylamino)pyrimidin-5-yl]-(2,3-difluoro-6-methoxyphenyl)methanone, DePinto W et al., Mol Cancer Ther 2006, 5:2644-2658;

1073485-20-7P: 3-[[6-(2-methoxyphenyl)-4-pyrimidinyl]amino]-Benzenemethanesulfonamide compound 1073485-20-7P in WO 2008132138.

AX38679: 3-((6-(2-methoxyphenyl)pyrimidin-4-yl)amino)benzenesulfonamide, 848637-62-7P in WO 2005026129;

PHA767491: 1,5,6,7-tetrahydro-2-(4-pyridinyl)-4H-pyrrolo[3,2-c]pyridin-4-one, Montagnoli, A. Nat Chem Biol 2008, 4(6) 357-365;

BS181: N5-(6-aminohexyl)-3-(1-methylethyl)-N7-(phenylmethyl), Ali S et al. Cancer Res. 2009, 69(15):6208-15);

DRB:

5,6-dichloro-1-b-ribofuranosyl-benzimidazole; Mancebo HS, Lee G, Flygare J, Tomassini J, Luu P, Zhu Y, Peng J, Blau C, Hazuda D, Price D, Flores O. P-TEFb kinase is required for HIV Tat transcriptional activation in vivo and in vitro. Genes Dev 1997; 11:2633-2644;

Roscovitine: 6-Benzylamino-2[(R)-(1'-ethyl-2'-hydroxyethylamino)]-9-isopropylpurine, Meijer, Laurent; Bisagni, Emile; Legraverend, Michel. Purine derivatives with antiproliferative properties, their preparation, and biological uses thereof. PCT Int. Appl. (1997), 52 pp. WO 9720842 A1;

AG-012986:

4-[[4-Amino-5-(2,6-difluorobenzoyl)thiazol-2-yl]amino]-N-((R)-2-dimethylamino-1-methylethyl)benzamide Zhang C, Lundgren K, Yan Z, Arango ME, Price S, Huber A, Higgins J, Troche G, Skaptason J, Koudriakova T, Nonomiya J, Yang M, O'Connor P, Bender S, Los G, Lewis C, Jessen B. Pharmacologic properties of AG-012986, a pan-cyclin-dependent kinase inhibitor with antitumor efficacy. Mol Cancer Ther 2008;7:818-828;

P276-00:

4H-1-Benzopyran-4-one, 2-(2-chlorophenyl)-5,7-dihydroxy-8-[(2R,3S)-2-(hydroxymethyl)-1-methyl-3-pyrrolidinyl]-, hydrochloride (1:1); Joshi, Kalpana S.; Rathos, Maggie J.; Joshi, Rajendra D.; Sivakumar, Meenakshi; Mascarenhas, Malcolm; Kamble, Shrikant; Lal, Bansi; Sharma, Somesh. In vitro antitumor properties of a novel cyclin-dependent kinase inhibitor, P276-00. Molecular Cancer Therapeutics (2007), 6(3), 918-925. CODEN: MCTOCF ISSN: 1535-7163. CAN 146:513967 AN 2007:289479:

ZK 304709:

4-[3-Chloro-5-(4-methylpiperazin-1-yl)benzoylamino]-1H-pyrazole-3-carboxylic acid cyclohexylamide Siemeister, G.; Luecking, U.; Wagner, C.; Detjen, K.; Mc Coy, C.; Bosslet, Klaus. Molecular and pharmacodynamic characteristics of the novel multi-target tumor growth inhibitor ZK304709. Biomedicine & Pharmacotherapy (2006), 60(6), 269-272. CODEN: BIPHEX ISSN:0753-3322. CAN 146:176348 AN 2006:831518;

EXEL-8647 and/or EXEL-3700:

Heuer TS. Discovery of Selective CDK9 Small Molecule Inhibitors: CDK9 Inhibition in Tumor Cells is Associated with Inhibition of Proliferation and Induction of Apoptosis. AACR-NCIEORTC International Conference on Molecular Targets and Cancer Therapeutics. Geneva, Switzerland; 21-24 October 2008;

AT7519:

4-(2,6-Dichlorobenzoylamino)-1H-pyrazole-3-carboxylic acid N-(piperidin-4-yl)amide Squires MS, Feltell RE, Lock V, Smith D, Lewis EJ, Higgins J, Yule M, Thompson NT, Cooke L, Croce Della K, Qi W, Lyons JF, Mahadevan D. AT7519, a potent CDK inhibitor, is active in leukemia models and primary CLL patient samples. 49th Annual Meeting and Exposition of American Society for Hematology, Atlanta, GA, 8-11 December 2007;

Compound 7d:

N-[2-(dimethylamino)ethyl]-2-fluoro-4-[[5-fluoro-4-[2-methyl-1-(1-methylethyl)-1H-imidazol-5-yl]-2-pyrimidinyl]amino] ; Jones CD, Andrews DM, Barker AJ, Blades K, Daunt P, East S, Geh C, Graham MA, Johnson KM, Loddick SA, McFarland HM, McGregor A, Moss L, Rudge DA, Simpson PB, Swain ML, Tam KY, Tucker JA, Walker M. The discovery of AZD5597, a potent imidazole pyrimidine amide CDK inhibitor suitable for intravenous dosing. Bioorg Med Chem Lett 2008;18:6369-6373;

AZD5597:

[4-[[5-fluoro-4-[2-methyl-1-(1-methylethyl)-1H-imidazol-5-yl]-2-pyrimidinyl]amino]phenyl][(3S)-3-(methylamino)-1-pyrrolidinyl]- Jones CD, Andrews DM, Barker AJ, Blades K, Daunt P, East S, Geh C, Graham MA, Johnson KM, Loddick SA, McFarland HM, McGregor A, Moss L, Rudge DA, Simpson PB, Swain ML, Tam KY, Tucker JA, Walker M. The discovery of AZD5597, a potent imidazole pyrimidine amide CDK inhibitor suitable for intravenous dosing. Bioorg Med Chem Lett 2008;18:6369-6373;

RGB-286638:

N-[1,4-dihydro-3-[4-[[4-(2-methoxyethyl)-1-piperazinyl]methyl]phenyl]-4-oxoindeno[1,2-c]pyrazol-5-yl]-N'-4-morpholinyl-, hydrochloride (1:2) Pharmacological targeting of CDK9 in cardiac hypertrophy Krystof V, Chamrád I, Jorda R, Kohoutek J. Med Res Rev. 2010 Jul;30(4):646-66. Review;

LCQ195/AT9311:

4-(2,6-dichlorobenzamido)-N-(1-(methylsulfonyl)piperidin-4-yl)-1H-pyrazole-3-carboxamide McMillin, Douglas W.; Delmore, Jake; Negri, Joseph; Buon, Leutz; Jacobs, Hannah M.; Laubach, Jacob; Jakubikova, Jana; Ooi, Melissa; Hayden, Patrick; Schlossman, Robert; Munshi, Nikhil c.; Lengauer, Christoph; Richardson, Paul G.; Anderson, Kenneth C.; Mitsiades, Constantine S. Molecular and cellular effects of multi-targeted cyclin-dependent kinase inhibition in myeloma: biological and clinical implications. British Journal of Haematology (2011), 152(4), 420-432. CODEN: BJHEAL ISSN:0007-1048. AN 2011:307444 CAPLUS.

Particularly preferred compounds for use in the present invention are Cpd 24, Cpd C1, Cpd B1 and Cpd B2 as described and defined herein above.

Also siRNAs/RNAis, antisense molecules and ribozymes directed against nucleic acid molecules encoding CDK9 or its activators Cyclin T or Cyclin K are envisaged as (an) CDK9 inhibitor(s) for the use and the method of the present invention. The above-mentioned antagonist/inhibitor of CDK9 may also be a co-suppressive nucleic acid.

An siRNA approach is, for example, disclosed in Elbashir ((2001), Nature 411, 494-498)). It is also envisaged in accordance with this invention that for example short hairpin RNAs (shRNAs) are employed in accordance with this invention as pharmaceutical composition. The shRNA approach for gene silencing is well known in the art and may comprise the use of st (small temporal) RNAs; see, inter alia, Paddison (2002) Genes Dev. 16, 948-958.

As mentioned above, approaches for gene silencing are known in the art and comprise "RNA"-approaches like RNAi (iRNA) or siRNA. Successful use of such approaches has been shown in Paddison (2002) loc. cit., Elbashir (2002) Methods 26, 199-213; Novina (2002) Mat. Med. June 3, 2002; Donze (2002) Nucl. Acids Res. 30, e46; Paul (2002) Nat. Biotech 20, 505-508; Lee (2002) Nat. Biotech. 20, 500-505; Miyagashi (2002) Nat. Biotech. 20, 497-500; Yu (2002) PNAS 99, 6047-6052 or Brummelkamp (2002), Science 296, 550-553. These approaches may be vector-based, e.g. the pSUPER vector, or RNA polIII vectors may be employed as illustrated, inter alia, in Yu (2002) loc. cit.; Miyagishi (2002) loc. cit. or Brummelkamp (2002) loc. cit.

However, use of CDK9 inhibitors in accordance with the present invention is not limited to known CDK9 inhibitors. Accordingly, also yet unknown CDK9 inhibitors may be used in accordance with the present invention. Such inhibitors may be identified by the methods described and provided herein and methods known in the art, like high-throughput screening using biochemical assays for inhibition of CDK9. Assays for screening of potential CDK9 inhibitors and, in particular, for identifying selective CDK9 inhibitors as defined herein are shown in the experimental part and described herein above. For example, a radiometric protein kinase assay (33PanQinase® Activity Assay; see Figure 3. In addition/in the alternative, the well known Lance Assay can also be used; see Figure 2. Based on his general knowledge a person skilled in the art is easily in the position to identify inhibitors or verify the inhibiting activity of compounds suspected of being CDK9 inhibitors. These tests may be employed on cell(s) or cell culture(s) described in the appended example, but also further cell(s)/tissue(s)/cell culture(s) may be used, such as cell(s)/ tissue(s)/cell culture(s) derived from biopsies.

The following refers to screening or validating potential CDK9 inhibitors, especially selective CDK9 inhibitos to be used in accordance with the present invention. For example, the activity/level of expression of CDK9 may be determined, wherein a lower activity/level of expression of CDK9 compared to a control is indicative for the capacity of a candidate molecule/substance to selectively inhibit CDK9. The term "activity of CDK9" used herein refers to the activity of a CDK9 protein (protein encoded by a CDK9 gene). The term "expression of CDK9" is used herein interchangeably with "expression of CDK9 gene" and refers to the expression of the CDK9 gene. It is to be understood that the activity/ expression level of CDK9 determined in (a) cell(s), (a) tissue(s) or (a) cell culture(s) contacted with/exposed to an CDK9 inhibitor is compared with the activity/expression level of CDK9 in (a) control cell(s), (a) tissue(s) or (a) cell culture(s), i.e. cell(s), (a) tissue(s) or (a) cell culture(s) not contacted with/exposed to an CDK9 inhibitor. A skilled person will be aware of means and methods for performing such tests and selecting appropriate controls. Preferably, the control cell (s), (a) tissue(s) or (a) cell culture(s) will be identical to the cell(s), (a) tissue(s) or (a) cell culture(s) to be tested as described herein with the only exception that the control (s), (a) tissue(s) or (a) cell culture(s) are not contacted with/ exposed to the CDK9 inhibitor.

Preferably, decreased CDK9 activity/expression levels of CDK9 proteins/polypeptides and/or CDK9 polynucleotides/nacleic acid molecules are indicative of the capacity of a candidate molecule/substance to selectively inhibit CDK9. It is preferred herein that the CDK9 activity/expression level is decreased by at least 10 %, 20 %, 30 %, 40 %, 50 %, 60 %, 70 %, 80 % and most preferably by at least 90 % in cell(s), (a) tissue(s) or (a) cell culture(s) contacted with/ exposed to an CDK9 inhibitor compared with the activity/expression level of CDK9 in (a) control cell(s), (a) control tissue (s) or (a) control cell culture(s). It is of note that the CDK9 activity must not necessarily correlate with the expression level. Thus, it may be, that CDK9 acitivity is decreased in the presence of an CDK9 inhibitor even though CDK9 expression is the same or even increased. However, a person skilled of the art will be aware of this and preferably evaluate CDK9 activity (i.e. activity/function of the CDK9 protein) when determining the capacitiy of a candidate substance to inhibit CDK9.

As mentioned, a person skilled in the art will be aware of corresponding means and methods for detecting and evaluating the CDK9 activity/expression level. Exemplary methods to be used include but are not limited to molecular assessments such as Western Blots, Northern Blots, Real-Time PCR and the like.

If the gene product is an RNA, in particular an mRNA (e.g. unspliced, partially spliced or spliced mRNA), determination can be performed by taking advantage of northern blotting techniques, hybridization on microarrays or DNA chips equipped with one or more probes or probe sets specific for mRNA transcripts or PCR techniques referred to above, like, for example, quantitative PCR techniques, such as Real time PCR. These and other suitable methods for binding (specific) mRNA are well known in the art and are, for example, described in Sambrook and Russell (2001, loc. cit.). A skilled person is capable of determining the amount of the component, in particular said gene products, by taking advantage of a correlation, preferably a linear correlation, between the intensity of a detection signal and the amount of the gene product to be determined.

In case the component is a polypeptide/protein, quantification can be performed by taking advantage of the techniques referred to above, in particular Western blotting techniques. Generally, the skilled person is aware of methods for the quantitation of (a) polypeptide(s)/protein(s). Amounts of purified polypeptide in solution can be determined by physical methods, e.g. photometry. Methods of quantifying a particular polypeptide in a mixture rely on specific binding, e.g of antibodies. Specific detection and quantitation methods exploiting the specificity of antibodies comprise for example immunohistochemistry (*in situ*). Western blotting combines separation of a mixture of proteins by electrophoresis and specific detection with antibodies. Electrophoresis may be multi-dimensional such as 2D electrophoresis. Usually, polypeptides are separated in 2D electrophoresis by their apparent molecular weight along one dimension and by their isoelectric point along the other direction. Alternatively, protein quantitation methods may involve but are not limited to mass spectrometry or enzyme-linked immunosorbant assay methods.

Also the use of high throughput screening (HTS) is envisaged in context of the present invention, in particular the screening methods for potential CDK9 inhibitors to be used herein. Suitable (HTS) approaches are known in the art and a person skilled in the art is readily in the position to adapt such protocols or known HTS approaches to the performance of the methods of the present invention.

Screening-assays are usually performed in liquid phase, wherein for each cell/tissue/cell culture to be tested at least one reaction batch is made. Typical containers to be used are micro titer plates having for example, 384, 1536, or 3456 wells (i.e. multiples of the "original" 96 reaction vessels).

Robotics, data processing and control software, and sensitive detectors, are further commonly used components of a HTS device. Often robot system are used to transport micro titer plates from station to station for addition and mixing of sample(s) and reagent(s), incubating the reagents and final readout (detection). Usually, HTS can be used in the simultaneous preparation, incubation and analysis of many plates.

The assay can be performed in a singly reaction (which is usually preferred), may, however, also comprise washing and/or transfer steps. Detection can be performed taking advantage of radioactivity, luminescence or fluorescence, like fluorescence-resonance-energytransfer (FRET) and fluorescence polarisation (FP) and the like. The biological samples described herein can also be used in such a context. In particular cellular assays and in vivo assays can be employed in HTS. Cellular assays may also comprise cellular extracts, i.e. extracts from cells, tissues and the like. However, preferred herein is the use of cell(s) or tissue(s) as biological sample (in particular a sample obtained from a patient/subject suffering or being prone to suffer from midline carcinoma, especially NMC), whereas in vivo assays (wherein suitable animal models are employed, e.g. the herein described mouse models) are particularly useful in the validation of potential CDK9 inhibitors. Depending on the results of a first assay, follow up assays can be performed by re-running the experiment to collect further data on a narrowed set (e.g. samples found "positive" in the first assay), confirming and refining observations.

HTS is useful in identifying further CDK9 inhibitors to be used herein. The screening of compound libraries with usually several hundred thousands of substances takes usually between days and weeks. An experimental high throughput screen may be supplemented (or even be replaced) by a virtual screen. For example, if the structure of the target molecule (e.g. CDK9) is known, methods can be employed, which are known under the term "docking". If the structure of several target-binding molecules is known (e.g. the herein described CDK9) methods for Pharmacophor-Modelling can be used aiming at the development new substances which also bind to the target molecule. A suitable readout in animal (in vivo) models is tumor growth (or respectively the complete or partial inhibition of tumor growth and/or its remission). High-throghput methods for the detection of mutations involve massively parallel sequencing approaches, such as the "picotiter plate pyrosequencing". This approach relies on emulsion PCR-based clonal amplification of a DNA library adapted onto micron-sized beads and subsequent pyrosequencing-by-synthesis (Thomas RK et al. Nature Med 2007) of each clonally amplified template in a picotiter plate, generating over 200,000 unique clonal sequencing reads per experiment. Furthermore, mass spectrometric genotyping approaches (Thomas RK et al.; Nat Gen 2007) and other next generation sequencing methods (Marguerat S et al.; Biochem Soc Trans 2008) may be employed.

The meaning of the terms "cell(s)", "tissue(s)" and "cell culture(s)" is well known in the art and may, for example, be deduced from "The Cell" (Garland Publishing, Inc., third edition). Generally, the term "cell(s) used herein refers to a single cell or a plurality of cells. The term "plurality of cells" means in the context of the present invention a group of cells comprising more than a single cell. Thereby, the cells out of said group of cells may have a similar function. Said cells may be connected cells and/or separate cells. The term "tissue" in the context of the present invention particularly means

a group of cells that perform a similar function. The term "cell culture(s)" means in context of the present invention cells as defined herein above which are grown/cultured under controlled conditions. Cell culture(s) comprise in particular cells (derived/obtained) from multicellular eukaryotes, preferably animals as defined elsewhere herein. It is to be understood that the term "cell culture(s)" as used herein refers also "tissue culture (s)" and/or "organ culture(s)", an "organ" being a group of tissues which perform the some function.

Preferably, the cell(s), tissue(s) or cell culture(s) to be contacted with/exposed to a selective CDK9 inhibitor comprise/are derived from or are (a) tumor cell(s). The tumor cells may, for example, be obtained from a biopsy, in particular a biopsy/biopsies from a patient/subject suffering from midline carcinoma, like NMC or, though less preferred a patient/subject being prone to suffer from midline carcinoma, like NMC. It is preferred herein that said subject is a human. The term "mammalian tumor cell(s)" used herein refers to (a) tumor cell(s) which is derived from or is a tumor cell from a mammal, the term mammal being derived herein below. As described herein above in respect of "cell(s)", "tissue(s)" and "cell culture(s)" the "mammalian tumor cells" may be obtained from a biopsy, in particular a biopsy/ biopsies from a patient/subject suffering from midline carcinoma, like NMC or, though less preferred a patient/subject being prone to suffer from midline carcinoma, like NMC. The term "tumor cell" also relates to "cancer cells".

Generally, said tumor cell or cancer cell may be obtained from any biological source/organism, particularly any biological source/organism, suffering from the above-mentioned midline carcinoma, like NMC.

Preferably, the (tumor) cell(s) or (cancer) cell to be contacted is (are) obtained/derived from an animal. More preferably, said (tumor)/(cancer) cell(s) is (are) derived from a mammal. The meaning of the terms "animal" or "mammal" is well known in the art and can, for example, be deduced from Wehner und Gehring (1995; Thieme Verlag). Non-limiting examples for mammals are even-toed ungulates such as sheep, cattle and pig, odd-toed angulates such as horses as well as carnivors such as cats and dogs. In the context of this invention, it is particularly envisaged that DNA samples are derived from organisms that are economically, agronomically or scientifically important. Scientifically or experimentally important organisms include, but are not limited to, mice, rats, rabbits, guinea pigs and pigs.

The tumor cell(s) may also be obtained from primates which comprise lemurs, monkeys and apes. The meaning of the terms "primate", "lemur", "monkey" and "ape" is known and may, for example, be deduced by an artisan from Wehner und Gehring (1995, Thieme Verlag). As mentioned above, the tumor or cancer cell(s) is (are) most preferably derived from a human being suffering from the above-mentioned NMCs. In context of this invention particular useful cells, in particular tumor or cancer cells, are, accordingly, human cells. These cells can be obtained from e.g. biopsies or from biological samples but the term "cell" also relates to in vitro cultured cells.

A preferred, however non-limiting cell(s) or cell culture(s) also used in the appended example is cell line 143100 (showing a t15;19 translocation resulting in the formation of a BRD4-NUT-fusion protein). A further cell line to be used in accordance with the present invention is HCC2429 (showing NOTCH3 overexpression in addition to the 115;19 translocation). Further cell lines that can be used include HCC1143 (NOTCH3 overexpression), PC9 (EGFRmut) or A549 (KRAS mut). These cell lines are well known in the art and may be obtained from ATCC and/ or DSMZ and/or from the U.S. National Cancer Institute (www.lgcpromochem-atcc.com/; www.dsmz.de/; dtp.nci.nih.gov/docs/misc/ common_files/cell _list .html).

The following explanations refer, inter alia, to rearrangements in the NUT gene which is characteristic especially for NUT midline carcinoma.

The below explanations concerning the NUT gene and NUT protein, apply, mutatis mutandis, to other nucleic acid sequences and amino acid sequences to be employed in context of the present invention, such as partner genes of NUT in NUT fusion genes like BRD4-NUT fusion genes, BRD3-NUT fusion genes or NUT-variant fusion genes characteristic for NMC. Accordingly, the explanations apply, mutatis mutandis, to members of the BET family (BRD2, BRDT and, in particular, human BRD3 gene and BRD3 protein (SEQ ID NOs: 5 and 6, respectively) and human BRD4 gene and BRD4 protein (SEQ ID NOs: 3 and 4, respectively). The explanations apply also to human CDK9 gene and CDK9 protein (SEQ ID NOs: 7 and 8, respectively), in particular CDK9 proteins to be used in the screening and/or validation of potential selective CDK9 inhibitors as described herein.

The term "NUT gene" ("nuclear protein in testis") as used in context of this invention refers to a gene encoding an unstructured polypeptide of unknown function that is highly expressed in normal spermatids; see Schwartz, loc. cit. It has been reported that the NUT protein binds to the histone acetyltransferase (HAT) p300; see Schwartz, loc. cit.

The nucleic acid sequence of the human NUT gene and the corresponding amino acid sequence is shown in SEQ ID NO: 1 and SEQ ID NO: 2, respectively. Generally, the term NUT used herein refers to any amino acid sequence having (partial) NUT activity as described herein and nucleic acid sequence(s) encoding such (an) amino acid sequence (s).

The nucleic acid sequences of NUT of other mammalian or non-mammalian species (in particular mouse, rat, chimpanzee) than the herein provided sequences for human NUT can be identified by the skilled person using methods known in the art, e.g. by nucleic acid sequencing or using hybridization assays or by using alignments, either manually or by using computer programs such as those mentioned herein below in connection with the definition of the term "hybridization" and degrees of homology. In one embodiment, the nucleic acid sequence encoding for orthologs of human

NUT gene is at least 40% homologous to the nucleic acid sequences as shown in SEQ ID NO: 1. More preferably, the nucleic acid sequence encoding for orthologs of the human NUT gene is at least 45%, 50%, 55%, 60%, 65%, 70%, 75%, 80%, 85%, 90%, 91%, 92%, 93%, 94%, 95%, 96%, 97% or 98% homologous to the nucleic acid sequence as shown in SEQ ID NO. 1, wherein the higher values are preferred. Most preferably, the nucleic acid sequence encoding for orthologs of the human NUT gene is at least 99% homologous to the nucleic acid sequence as shown in SEQ ID NO. 1.

Hybridization assays for the characterization of orthologs of known nucleic acid sequences/promoters are well known in the art; see e.g. Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989). The term "hybridization" or "hybridizes" as used herein may relate to hybridizations under stringent or non-stringent conditions. If not further specified, the conditions are preferably non-stringent. Said hybridization conditions may be established according to conventional protocols described, e.g., in Sambrook (2001) loc. cit.; Ausubel (1989) loc. cit., or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as, for example, the highly stringent hybridization conditions of 0.1 x SSC, 0.1% SDS at 65°C or 2 x SSC, 60°C, 0.1 % SDS. Low stringent hybridization conditions for the detection of homologous or not exactly complementary sequences may, for example, be set at 6 x SSC, 1% SDS at 65°C. As is well known, the length of the probe and the composition of the nucleic acid to be determined constitute further parameters of the hybridization conditions.

In accordance with the present invention, the terms "homology" or "percent homology" or "identical" or "percent identity" or "percentage identity" or "sequence identity" in the context of two or more nucleic acid sequences refers to two or more sequences or subsequences that are the same, or that have a specified percentage of nucleotides that are the same (preferably at least 40% identity, more preferably at least 45%, 50%, 55%, 60%, 65%, 70%, 75%, 80%, 85%, 90%, 91%, 92%, 93%, 94%, 95%, 96%, 97% or 98% identity, most preferably at least 99% identity), when compared and aligned for maximum correspondence over a window of comparison, or over a designated region as measured using a sequence comparison algorithm as known in the art, or by manual alignment and visual inspection. Sequences having, for example, 75% to 90% or greater sequence identity may be considered to be substantially identical. Such a definition also applies to the complement of a test sequence. Preferably the described identity exists over a region that is at least about 15 to 25 nucleotides in length, more preferably, over a region that is at least about 50 to 100 nucleotides in length, more preferably at least about 200 to 400 nucleotides, at least about 300 to 500 nucleotides, at least about 400 to 600 nucleotides in length, at least about 500 to 1000 nucleotides, at least about 800 to 1500 nucleotides, at least about 1000 to 2000 nucleotides, at least about 1500 to 2500 nucleotides or at least about 2000 to 3000 nucleotides. Even more preferably, the described identity exists over a region that is at least about 3000 to 4200 nucleotides in length, more preferably at least about 3200 to 4000 nucleotides, more preferably at least about 3300 to 3900 nucleotides. Most preferably, the described identity exists over a region that is at least about 3350 to 3850 nucleotides in length. In a most preferred embodiment, the described identity exists over the entire length of the nucleotide sequence shown in SEQ ID NO. 1, preferably the region thereof encoding the NUT protein. The coding region ranges from nucleotide 156 to nucleotide 3554 of the nucleotide sequence shown in SEQ ID NO: 1. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul, (1997) Nucl. Acids Res. 25:3389-3402; Altschul (1993) J. Mol. Evol. 36:290-300; Altschul (1990) J. Mol. Biol. 215:403-410). The BLASTN program for nucleic acid sequences uses as defaults a word length (W) of 11, an expectation (E) of 10, M=5, N=4, and a comparison of both strands. The BLOSUM62 scoring matrix (Henikoff (1989) PNAS 89:10915) uses alignments (B) of 50, expectation (E) of 10, M=5, N=4, and a comparison of both strands.

In order to determine whether a nucleotide residue in a nucleic acid sequence corresponds to a certain position in the nucleotide sequence of e.g. SEQ ID NO 1, the skilled person can use means and methods well-known in the art, e.g., alignments, either manually or by using computer programs such as those mentioned herein. For example, BLAST 2.0, which stands for Basic Local Alignment Search Tool BLAST (Altschul (1997), loc. cit.; Altschul (1993), loc. cit.; Altschul (1990), loc. cit.), can be used to search for local sequence alignments. BLAST, as discussed above, produces alignments of nucleotide sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold

or cut-off score set by the user. The BLAST approach is to look for HSPs between a query sequence and a database sequence, to evaluate the statistical significance of any matches found, and to report only those matches, which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\%\ \text{sequence identity}\ \ x\ \ \%\ \text{maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules. Another example for a program capable of generating sequence alignments is the CLUSTALW computer program (Thompson (1994) Nucl. Acids Res. 2:4673-4680) or FASTDB (Brutlag (1990) Comp. App. Biosci. 6:237-245), as known in the art.

Also envisaged herein is not only the use of nucleic acid sequences encoding the NUT gene but also amino acid sequences of NUT protein. In line with the above explanations concerning orthologs/homologs of human NUT gene as shown in SEQ ID NO: 2, also orthologous/homologous amino acid sequences of the human NUT protein may be employed in accordance with the present invention. Accordingly, the terms "homology" or "percent homology" or "identical" or "percent identity" or "percentage identity" or "sequence identity" refer in the context of two or more amino acid sequences to two or more sequences or subsequences that are the same, or that have a specified percentage of amino acids that are the same (preferably at least 40% identity, more preferably at least 45%, 50%, 55%, 60%, 65%, 70%, 75%, 80%, 85%, 90%, 91%, 92%, 93%, 94%, 95%, 96%, 97% or 98% identity, most preferably at least 99% identity) compared to the amino acid sequence of human NUT protein as shown in SEQ ID NO. 2.

As mentioned above, the term "rearrangement in the NUT gene" used herein refers to any rearrangement in the NUT gene that is characteristic for NUT midline carcinoma (NMC). Exemplary "rearrangments in the NUT gene" as well as methods for their detection are known in the art (see, for example, French (2010) J Clin Pathol, loc. cit.). For example, the rearrangement can be or can be caused by a translocation of the NUT gene (or a part or fragment thereof).

One particularly preferred translocation is the t15;19 translocation known in the art. This translocation has resulted in the formation of a fusion gene of NUT, the so called BRD4-NUT fusion gene. Accordingly, rearrangement in the NUT gene which are or are caused/associated by the formation of a BRD4/NUT fusion gene are particularly preferred in context of the present invention. Also envisaged in this context is the formation of a fusion gene comprising a sequence encoding the complete BRD4 gene, and/or one or more parts or fragments thereof and comprising a sequence encoding the complete NUT gene and/or one or more parts or fragments thereof. The exemplary BRD4-NUT fusion protein is composed of the N-terminal of BRD4 (amino acids 1-720 out of 1372) and almost the entire protein sequence of NUT (amino acids 6-1127). The N-terminal of BRD4 includes bromodomains 1 and 2 and other, less well characterized functional domains.

Though the majority of known NMC cases are associated with the formation of a BRD4-NUT fusion gene, further rearrangments in the NUT gene have been described in the art, and the term "NUT variant fusion gene" has been coined in the art to cover the remaining NMC subtypes.

One exemplary "NUT variant fusion gene" is the so called "BRD3-NUT fusion gene". Accordingly, rearrangements in the NUT gene which are or are caused/associated by the formation of a BRD3/NUT fusion gene are also envisaged in context of the present invention. Again, the formation of a fusion gene comprising a sequence encoding the complete BRD3 gene, and/or one or more parts or fragments thereof and comprising comprising a sequence encoding the complete NUT gene and/or one or more parts or fragments thereof is envisaged herein.

The rearrangements in the NUT gene and optionally mutations/rearrangments/aberrant expression of further genes can be detected by methods known in the art. Such methods are, for example described in French CA, 2010 (NUT midline carcinoma. French CA. Cancer Genet Cytogenet. 2010 Nov;203(1):16-20.). A person skilled in the art is in the position to adapt the methods for detecting rearrangments in genes described in the above-mentioned documents to the rearrangements in the NUT gene described herein and further rearrangments in this gene known in the art. A person skilled in the art will readily understand that also rearrangements in said gene not described herein but known

in the art or mutations yet to be identified may also be used in the context of the present invention.

Exemplary, non-limiting methods to be used in the detection of rearrangements in the NUT gene are described in WO 2010/011700, Haack (2009), loc. cit., French (2010) Cancer Genet Cytogenet (loc. cit.) and French (2010) Clin Pathol (loc. cit.).

Particularly preferred is diagnosis via in situ hybridisation (FISH, CISH, SISH and the like), since these methods can detect any rearrangement in the NUT gene. However, also detection of a gene product of the above described NUT fusion genes is envisaged using routine techniques like immunohistochemical methods, Northern Blot, Real time PCR and the like. This especially useful in cases where said rearrangement in the NUT gene is reflected in expression of the formed NUT fusion gene, as the expression level of the formed NUT fusion gene may be detected. Such methods are particularly envisaged in the detection of BRD3-NUT transcripts and/or BRD4-NUT transcripts. Also immunohistochemical methods (or other routine methods like Western Blots etc.) may be employed to detect expression products on a protein level. For example, antibodies French (2010) Cancer Genet Cytogenet (loc. cit.) or Haack (2009), loc. cit. describe the use of a diagnostic NUT specific monoclonal antibody, taking advantage of the fact the the native protein is not expressed outside of the testis.

Further methods which are useful for detecting mutations or rearrangments are methods for sequencing of nucleic acids (e.g. Sanger di-deoxy sequencing), "next generation" methods, single molecule sequencing, methods enabling detection of variant alleles/mutations, such as Real-time PCR, PCR-RFLP assay (see Cancer Research 59 (1999), 5169-5175), mass-spectrometric genotyping (e.g. MALDI-TOF), HPLC, enzymatic methods and SSPC (single strand conformation polymorphism analysis; see Pathol Int (1996) 46, 801-804).

In particular, such methods may include enzymatic amplification of DNA or cDNA fragments using oligonucleotides specifically hybridizing to exonic or intronic parts of the rearranged NUT gene by PCT. Such amplifications may be carried out in two reactions when employing genomic DNA or even in only a single reaction when employing cDNA. The resulting PCR products may be subjected to either conventional Sanger-based dideoxy nucleotide sequencing methods or employing novel parallel sequencing methods ("next generation sequencing") such as those marketed by Roche (454 technology), Illumina (Solexa technology) or ABI (Solid technology). Rearrangements or mutations may be identified from sequence reads by comparison with publicly available gene sequence data bases. Alternatively, mutations may be identified by allele-specific incorporation of probes that can either be detected using enzymatic detection reactions, fluorescence, mass spectrometry or others; see Vogeser (2007) Dtsch Arztebl 104 (31-32), A2194-200.

Paraffin-embedded clinical material may be used in the detection of rearrangements in the NUT gene. Detection may comprise a histolopathology review of the sample to be tested to ensure tumour tissue is present. A commercially available Kit to be used in the detection method is the AllPrep DNA/RNA FFPE Kit form Quiagen (Germany). Further kits to be used for detecting rearrangements in the NUT gene are commercially available.

A positive result in the detection method indicates the presence of (a) rearrangement(s) in the NUT gene.

In one embodiment of the present invention, the tumor or cancer cell is not only characterized by the presence of at least one rearrangement in the NUT gene, but also, as a further option, by expression of the NOTCH3 gene. It has been shown in the appended examples that cell lines having a NOTCH3 overexpression in addition to a rearrangement in the NUT gene are particularly susceptible to a CDK9 inhibitor. A nucleic acid sequence of the human NOTCH3 gene and a corresponding Amino acid sequence are depicted in SEQ ID NOs: 11 and 12, respectively.

As mentioned above, (a) tumor cell(s)/tumor(s) with (a) rearrangement(s) in the NUT gene and overexpression of the NOTCH3 gene is (are) sensitive to treatment with selective CDK9 inhibitors. Therefore, it is envisaged that (a) tumor cell(s)/tumor(s) with (a) with (a) rearrangement(s) in the NUT gene and, optionally, overexpression of the NOTCH3 gene might be particularly sensitive to treatment with CDK9 inhibitors. Therefore, (a) cell(s), (a) tissue(s) or (a) cell culture selected in accordance with the present method with at least one rearrangement in the NUT gene and overexpression of the NOTCH3 gene might be particularly susceptible to a selective CDK9 inhibitor. Accordingly, treatment of patients with a selective CDK9 inhibitor (the patients suffering from NMC) may be particularly successful in respect of, for example, prognosis or survival rate.

Patient(s) may also be subject to co-therapy/co-treatment with a CDK9 inhibitor and a further compound/drug (e.g. (a) NUT inhibitor(s)). Patients suffering from cancer characterized by the presence of at least one rearrangement in the NUT gene (e.g. NMC) and (a) mutation(s) or overexpreesion of a further gene (e.g. NOTCH3) may only be treated with a CDK9 inhibitor but not in co-therapy with NOTCH3 inhibitor and a selective CDK9 inhibitor if the patients are known to be resistant to such NOTCH3 inhibitor. Of course, co-therapy/combination therapy to be used in context of the present invention may also comprise radiation therapy, conventional chemotherapy and the like.

The following relates to pharmaceutical compositions and drug combinations. In one embodiment, the present invention relates to a CDK9 inhibitor, such as a selective CDK9 inhibitor, as defined herein for use in treating, ameliorating and/or preventing midline carcinoma, like NUT midline carcinoma (NMC). Accordingly, also the use of a CDK9 inhibitor, such as a selective CDK9 inhibitor, for the preparation of a pharmaceutical composition for the treatment, amelioration and/or prevention of midline carcinoma, like NUT midline carcinoma (NMC), is envisaged in context of the present invention.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a subject and includes:

(a) preventing a disease related to an insufficient immune response from occurring in a subject which may be predisposed to the disease; (b) inhibiting the disease, i.e. arresting its development; or (c) relieving the disease, i.e. causing regression of the disease.

A "patient" or "subject" for the purposes of the present invention includes both humans and other animals, particularly mammals, and other organisms. Thus, the methods are applicable to both human therapy and veterinary applications. In the preferred embodiment the patient is a mammal, and in the most preferred embodiment the patient is human.

In accordance with the above, the present invention relates to drug combinations and pharmaceutical compositions comprising at least one CDK9 inhibitor, such as (a) compound(s) of general formula (I) as active ingredient together with at least one pharmaceutically acceptable carrier, excipient and/or diluent and optionally together with one or more other anti-tumor agents As used herein the term "drug combination" refers to a combination of at least to pharmaceutically active agents or therapeutic agents with or without further ingredients, carrier, diluents and/or solvents. As used herein the term "pharmaceutical composition" refers to a galenic formulation of at least one pharmaceutically active agent together with at least one further ingredient, carrier, diluent and/or solvent.

CDK9 inhibitors, such as compounds of formula (I) may be administered as the sole pharmaceutical agent or in combination with one or more additional therapeutic agents, wherein the drug combination causes no unacceptable adverse effects. This combination therapy includes administration of a single pharmaceutical dosage formulation, which contains a CDK9 inhibitor and one or more additional therapeutic agents in form of a single pharmaceutical composition, as well as administration of a CDK9 inhibitor and each additional therapeutic agent in its own separate pharmaceutical dosage formulation, i.e. in its own separate pharmaceutical composition. For example, a CDK9 inhibitor and a therapeutic agent may be administered to the patient together in a single oral dosage composition such as a tablet or capsule, or each agent may be administered in separate pharmaceutical compositions.

Where separate pharmaceutical compositions are used, a CDK9 inhibitor and one or more additional therapeutic agents may be administered at essentially the same time (*e.g.*, concurrently) or at separately staggered times (*e.g.*, sequentially).

In particular, the CDK9 inhibitors to be used in accordance withrthe present invention may be used in fixed or separate pharmaceutical compositions with other anti-tumor agents such as alkylating agents, anti-metabolites, plant-derived anti-tumor agents, hormonal therapy agents, topoisomerase inhibitors, camptothecin derivatives, kinase inhibitors, targeted drugs, antibodies, interferons and/or biological response modifiers, anti-angiogenic compounds, and other anti-tumor drugs. In this regard, the following is a non-limiting list of examples of secondary agents that may be used in combination with the CDK9 inhibitors:

- Alkylating agents include, but are not limited to, nitrogen mustard *N*-oxide, cyclophosphamide, ifosfamide, thiotepa, ranimustine, nimustine, temozolomide, altretamine, apaziquone, brostallicin, bendamustine, carmustine, estramustine, fotemustine, glufosfamide, mafosfamide, and mitolactol; platinum-coordinated alkylating compounds include, but are not limited to, cisplatin, carboplatin, eptaplatin, lobaplatin, nedaplatin, oxaliplatin, and satraplatin;
- Anti-metabolites include, but are not limited to, methotrexate, 6-mercaptopurine riboside, mercaptopurine, 5-fluorouracil alone or in combination with leucovorin, tegafur, doxifluridine, carmofur, cytarabine, cytarabine ocfosfate, enocitabine, gemcitabine, fludarabin, 5-azacitidine, capecitabine, cladribine, clofarabine, decitabine, eflornithine, ethynylcytidine, cytosine arabinoside, hydroxyurea, melphalan, nelarabine, nolatrexed, ocfosfite, disodium premetrexed, pentostatin, pelitrexol, raltitrexed, triapine, trimetrexate, vidarabine, vincristine, and vinorelbine;
- Hormonal therapy agents include, but are not limited to, exemestane, Lupron, anastrozole, doxercalciferol, fadrozole, formestane, 11-beta hydroxysteroid dehydrogenase 1 inhibitors, 17-alpha hydroxylase/17,20 lyase inhibitors such as abiraterone acetate, 5-alpha reductase inhibitors such as finasteride and epristeride, anti-estrogens such as tamoxifen citrate and fulvestrant, Trelstar, toremifene, raloxifene, lasofoxifene, letrozole, anti-androgens such as bicalutamide, flutamide, mifepristone, nilutamide, Casodex, and anti-progesterones and combinations thereof;
- Plant-derived anti-tumor substances include, *e.g.*, those selected from mitotic inhibitors, for example epothilones such as sagopilone, ixabepilone and epothilone B, vinblastine, vinflunine, docetaxel, and paclitaxel;
- Cytotoxic topoisomerase inhibiting agents include, but are not limited to, aclarubicin, doxorubicin, amonafide, belotecan, camptothecin, 10-hydroxycamptothecin, 9-aminocamptothecin, diflomotecan, irinotecan, topotecan, edotecarin, epimbicin, etoposide, exatecan, gimatecan, lurtotecan, mitoxantrone, pirambicin, pixantrone, rubitecan, sobuzoxane, tafluposide, and combinations thereof;

- Immunologicals include interferons such as interferon alpha, interferon alpha-2a, interferon alpha-2b, interferon beta, interferon gamma-1a and interferon gamma-n1, and other immune enhancing agents such as L19-IL2 and other IL2 derivatives, filgrastim, lentinan, sizofilan, TheraCys, ubenimex, aldesleukin, alemtuzumab, BAM-002, dacarbazine, daclizumab, denileukin, gemtuzumab, ozogamicin, ibritumomab, imiquimod, lenograstim, lentinan, melanoma vaccine (Corixa), molgramostim, sargramostim, tasonermin, tecleukin, thymalasin, tositumomab, Vimlizin, epratuzumab, mitumomab, oregovomab, pemtumomab, and Provenge; Merial melanoma vaccine;
- Biological response modifiers are agents that modify defense mechanisms of living organisms or biological responses such as survival, growth or differentiation of tissue cells to direct them to have anti-tumor activity; such agents include, *e.g.*, krestin, lentinan, sizofiran, picibanil, ProMune, and ubenimex;
- Anti-angiogenic compounds include, but are not limited to, acitretin, aflibercept, angiostatin, aplidine, asentar, axitinib, recentin, bevacizumab, brivanib alaninat, cilengtide, combretastatin, DAST, endostatin, fenretinide, halofuginone, pazopanib, ranibizumab, rebimastat, removab, revlimid, sorafenib, vatalanib, squalamine, sunitinib, telatinib, thalidomide, ukrain, and vitaxin;
- Antibodies include, but are not limited to, trastuzumab, cetuximab, bevacizumab, rituximab, ticilimumab, ipilimumab, lumiliximab, catumaxomab, atacicept, oregovomab, and alemtuzumab;
- VEGF inhibitors such as, *e.g.*, sorafenib, DAST, bevacizumab, sunitinib, recentin, axitinib, aflibercept, telatinib, brivanib alaninate, vatalanib, pazopanib, and ranibizumab; Palladia
- EGFR (HER1) inhibitors such as, *e.g.*, cetuximab, panitumumab, vectibix, gefitinib, erlotinib, and Zactima;
- HER2 inhibitors such as, *e.g.*, lapatinib, tratuzumab, and pertuzumab;
- mTOR inhibitors such as, *e.g.*, temsirolimus, sirolimus/Rapamycin, and everolimus;
- c-Met inhibitors;
- PI3K and AKT inhibitors;
- CDK inhibitors;
- Spindle assembly checkpoints inhibitors and targeted anti-mitotic agents such as PLK inhibitors, Aurora inhibitors (*e.g.* Hesperadin), checkpoint kinase inhibitors, and KSP inhibitors;
- HDAC inhibitors such as, *e.g.*, panobinostat, vorinostat, MS275, belinostat, and LBH589;
- HSP90 and HSP70 inhibitors;
- Proteasome inhibitors such as bortezomib and carfilzomib;
- Serine/threonine kinase inhibitors including MEK inhibitors (such as e.g. RDEA 119) and Raf inhibitors such as sorafenib;
- Farnesyl transferase inhibitors such as, *e.g.*, tipifarnib;
- Tyrosine kinase inhibitors including, *e.g.*, dasatinib, nilotibib, DAST, bosutinib, sorafenib, bevacizumab, sunitinib, AZD2171, axitinib, aflibercept, telatinib, imatinib mesylate, brivanib alaninate, pazopanib, ranibizumab, vatalanib, cetuximab, panitumumab, vectibix, gefitinib, erlotinib, lapatinib, tratuzumab, pertuzumab, and c-Kit inhibitors; Palladia, masitinib
- Vitamin D receptor agonists;
- Bcl-2 protein inhibitor such as obatoclax, oblimersen sodium, and gossypol;
- Cluster of differentiation 20 receptor antagonists such as, *e.g.*, rituximab;
- Ribonucleotide reductase inhibitors such as, *e.g.*, gemcitabine;
- Tumor necrosis apoptosis inducing ligand receptor 1 agonists such as, *e.g.*, mapatumumab;
- 5-Hydroxytryptamine receptor antagonists such as, *e.g.*, rEV598, xaliprode, palonosetron hydrochloride, granisetron, Zindol, and AR-1001;
- Integrin inhibitors including alpha5-beta1 integrin inhibitors such as, *e.g.*, E7820, JSM 6425, volociximab, and endostatin;
- Androgen receptor antagonists including, *e.g.*, nandrolone decanoate, fluoxymesterone, Android, Prost-aid, andromustine, bicalutamide, flutamide, apo-cyproterone, apo-flutamide, chlormadinone acetate, Androcur, Tabi, cyproterone acetate, and nilutamide;
- Aromatase inhibitors such as, *e.g.*, anastrozole, letrozole, testolactone, exemestane, amino-glutethimide, and formestane;
- Matrix metalloproteinase inhibitors;
- Other anti-cancer agents including, *e.g.*, alitretinoin, ampligen, atrasentan bexarotene, bortezomib, bosentan, calcitriol, exisulind, fotemustine, ibandronic acid, miltefosine, mitoxantrone, l-asparaginase, procarbazine, dacarbazine, hydroxycarbamide, pegaspargase, pentostatin, tazaroten, velcade, gallium nitrate, canfosfamide, darinaparsin, and tretinoin.

The CDK9 inhibitors may also be employed in cancer treatment in conjunction with radiation therapy and/or surgical intervention.

Furthermore, the CDK9 inhibitors may be utilized, as such or in compositions, in research and diagnostics, or as analytical reference standards, and the like, which are well known in the art.

Thus, another aspect of the present invention relates to drug combinations comprising at least one inventive CDK9 inhibitor, such as a compound according to general formula (I) and/or pharmaceutically acceptable salts thereof together with at least one anti-retroviral drug, especially at least one of the drugs mentioned above.

The pharmaceutical compositions according to the present invention comprise at least one CDK9 inhibitor according to the present invention as an active ingredient together with at least one pharmaceutically acceptable (i.e. non-toxic) carrier, excipient and/or diluent. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one CDK9 inhibitor according to the present invention and/or a pharmaceutical acceptable salt thereof as active ingredient.

The pharmaceutical compositions according to the present invention containing at least one CDK9 inhibitor according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the CDK9 inhibitors (such as 2,4,6-disubstituted pyrimdine derivative according to the general formula (I) or analogues compound thereof) or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The CDK9 inhibitors according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation,

dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to 10 weight %. Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 about weight %.

The present invention is further described by reference to the following non-limiting figures and examples

The Figures show:

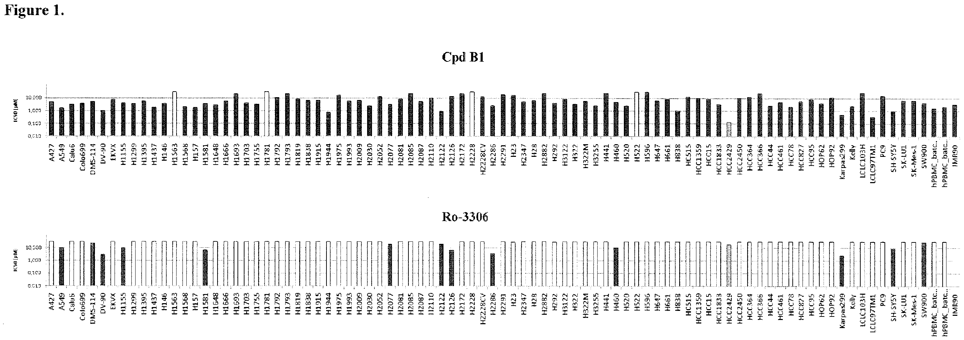

**Figure 1. Proliferation inhibition profile.**

Figure 1 shows a proliferation inhibition profile of a potent specific CDK9 inhibitor (Cpd B1) and a selective CDK1 inhibitor (Ro-3306).

Proliferation assays were performed as described below under materials and methods. Three compounds (Cpd B1 and Ro-3306) were applied at concentrations between 30 and 0,0137 μM. After 72h incubation with compounds ATP content/proliferation was determined employing CTG (Promega). Relative proliferation values (compared to vehicle control) were used to calculate $IC_{50}$ values (Excel fit; algorithm #205). $IC_{50}$s of the respective compound (Y-axis; logarithmic scale) on proliferation of various cell lines (X-axis) are depicted in black bars.White bars indicate that an $IC_{50}$ could not be determined due to too low activity and therefore was higher than the higest applied concentration in the assays (30 μM). $IC_{50}$s of compounds on CDK9 inhibitor sensitive cell line HCC2429 are presented in grey bars. The $IC_{50}$s of Cpd B1 was determined at 0,151 μM. The specific CDK1 inhibitor Ro-3306 does not affect said cell line potently ($IC_{50}$ initially higher 10μM).

**Figure 2. CDK9 inhibition.**

Figure 2 shows CDK9 inhibition by and selectivity of described compounds. The figure summarizes $IC_{50}$ values of 12 compounds on CDK1/CyclinB1, CDK2/CyclinA, CDK4/CyclinD1, CDK6/CyclinD3, CDK7/CyclinH/Mat1 and CDK9/CyclinT1 activity (methods are described below).

Compounds were either already described (SNS-032: Piperidine-4-carboxylic acid [5-(5-tert-butyl-oxazol-2-ylmethylsulfanyl)-thiazol-2-yl]-amide; Misra RN et al. J Med Chem. 2004, 47(7):1719-28; flavopiridol: 2-(2-Chloro-phenyl)-5,7-dihydroxy-8-(3-hydroxy-1-methyl-piperidin-4-yl)-chromen-4-one, Lloyd R Kelland. Expert Opinion on Investigational Drugs. 2000, 9(12):2903-2911; AX35427: N-(5-((6-(3-aminophenyl)pyrimidin-4-yl)amino)-2-methylphenyl)propane-1-sulfonamide, 848637-29-6P in WO 2005026129; R-547: [4-amino-2-(1-methanesulfonylpiperidin-4-ylamino)pyrimidin-5-yl]-(2,3-difluoro-6-methoxyphenyl)methanone, DePinto W et al., Mol Cancer Ther 2006, 5:2644-2658; 3-[[6-(2-methoxyphenyl)-4-pyrimidinyl]amino]-Benzenemethanesulfonamide compound 1073485-20-7P in WO 2008132138.

AX38679: 3-((6-(2-methoxyphenyl)pyrimidin-4-yl)amino)benzenesulfonamide, 848637-62-7P in WO 2005026129; PHA767491: 1,5,6,7-tetrahydro-2-(4-pyridinyl)-4H-pyrrolo[3,2-c]pyridin-4-one, Montagnoli, A. Nat Chem Biol 2008, 4(6) 357-365; BS181: N5-(6-aminohexyl)-3-(1-methylcthyl)-N7-(phenylmethyl), Ali S et al. Cancer Res. 2009, 69(15):6208-15) or mentioned above (Cpd 24, Cpd C1, Cpd B1 and Cpd B2).

**Figure 3. CDK9 inhibitors 1073485-20-7P and Cpd B1.**

Figure 3 shows general kinase selectivity of two typical selective CDK9 inhibitors 1073485-20-7P and Cpd B1. Employing enzymatic in vitro assays on the activity of 23 different kinases incubated with 10 μM Cpd B1 or DMSO as control resulted in the depicted residual activities (compared to the vehicle control). As a result it is demonstrated that Cpd B1 inhibits only CDK9 with high potency and is a selective CDK9 kinase inhibitor in accordance with the present invention.

**Figure 4.**

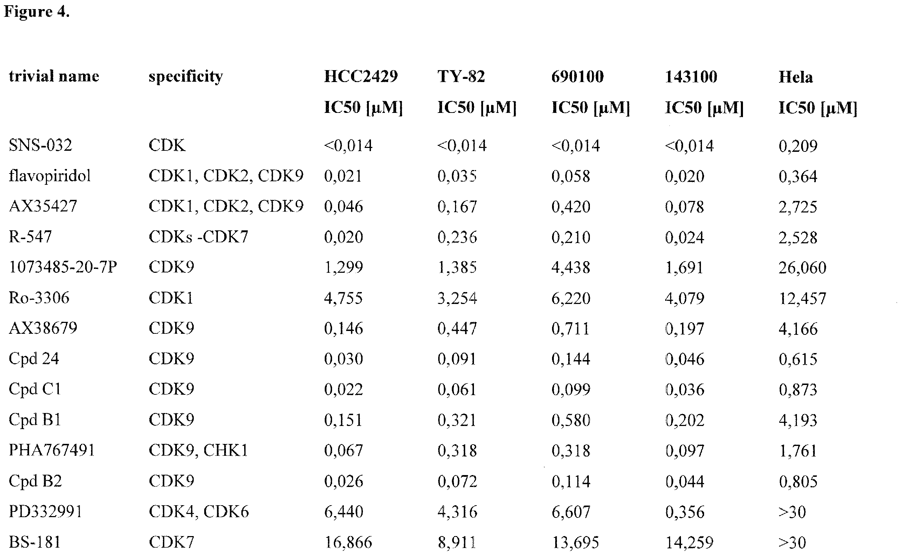

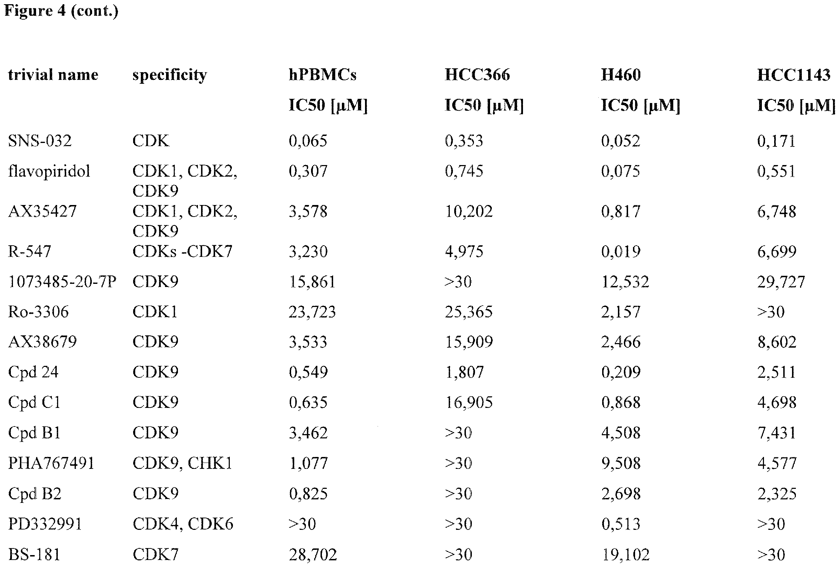

Figure 4 displays proliferation assay results of selected CDK9 inhibitors as well as other CDK standard inhibitors on various Brd4-Nut mutated as well as wild type cell lines. The proliferation results are presented as $IC_{50}$ in μM. First, the anti-proliferative effect of CDK9 inhibitors is much more pronounced on BrdNut mutated cell lines (=sensitive cell lines). Second, within the table it is clearly visible, that the anti-proliferative effect of the compounds is directly correlated to the Even with relatively weak CDK9 Inhibitors like 1073485-20-7P proliferation inhibition is much more potent in mutated, sensitive cell lines (HCC2429, TY-82, 690100, 143100) than in normal wilde type cell lines (Hela, HCC366, H460 HCC1143 and hPBMCs).

**Figure 5.**

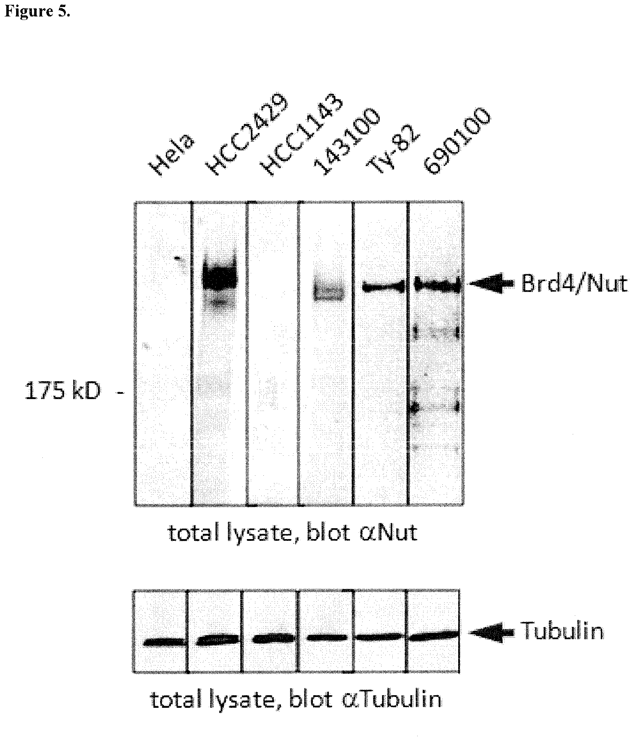

Figure 5 shows the expression of BrdNut fusion proteins in various cell lines (Hela, HCC2429, Ty-82, 143100, 69100 and HCC1143). Cell lysates were analyzed as described in materials and methods. Fusion proteins were detected as high molecular weight bands employing an antibody directed against Nut proteins. As a loading control same lysates were analyzed for their tubulin content.

The Examples illustrate the invention.

**Example 1:**

<u>**Material and Methods**</u>

***Material***

NSCLC cells (A427, A549, Calu6, Colo699, DMS-114, DV-90, EKVX, H1155, H1299, H1395, H1437, H146, H1563, H1568, H157, H1581, H1648, H1666, H1693, H1703, H1755, H1781, H1792, H1793, H1819, H1838, H1915, H1944, H1975, H1993, H2009, H2030, H2052, H2077, H2081, H2085, H2087, H2110, H2122, H2126, H2172, H2228, H2228CV, H2286, H2291, H23, H2347, > H28 > H2882 H292, > H3122 H322, H322M, > H3255 H441, H460, > H520 > H522 H596, H647, H661, H838, HC515, HCC1359, HCC15, HCC1143, HCC1833, HCC2429, HCC2450, HCC364, HCC366, HCC44, HCC461, HCC78, HCC827, HCC95, HOP62, HOP92, Karpas299, Kelly, LCLC103H, LCLC97TM1, PC9, SH-SY5Y, SK-LU1, SK-Mes-1, SW900, 690100 and 143100) are described in PCT patent application WO 2010/020618 and WO 2010/020619. TY-82 cells were purchased from Health Science Research Resource Bank (Osaka, Japan). Peripheral blood mononuclear cells (hPBMCs) were provided by the Blutspendedienst Hagen (DRK West,

Hagen). All other cell lines (e.g. Hela cells) have been purchased from LGC Standards (ATCC, Wesel) or the DSMZ (Deutsche Sammlung von Mikroorganismen und Zellkulturen, Braunschweig).

***Cell Viability Assays***

Cell lines were maintained in RPMI 1640 cell culture medium + glutamine (PAN Biotech GmbH, Aidenbach, Germany; order no. P04-22100; P04-05500) supplemented with 10% fetal calf serum "Gold" (PAA Laboratories GmbH, Pasching, Austria; order no. A15-151) and grown in a humidified atmosphere at 37 °C, 5 % CO2. As a first step, an optimal cell density for each cell line was determined to guarantee linearity. For viability assays with compounds, cells were then seeded at a density of 200 to 1000 per well in 25 μl in 384-well plates (Greiner Bio-One, Frickenhausen, Germany; order no. 781080). After overnight incubation at 37 °C/5 % CO2, 25nl or 75nl compounds were added to each sample well by using BIOMEK $FX^P$ Laboratory Automation Workstation (Beckman Coulter, USA). Wells with cells and 0.1 % or 0,3 % DMSO in culture medium were used as positive controls, wells with cells and 10μM staurosporine (Selleck Chemicals, Huston, USA) in culture medium were used as negative controls. Upon incubation with compounds for 72 h at 37 °C/5 % CO2 25 μl Cell Titer Glo reagent (Promega, Madison, USA, order no. G7573; 1:2 diluted with cell culture medium) was added to each well to determine cell viability. 384well-plates were placed for 2 min on an orbital microplate shaker and incubated for further 10 min at room temperature resulting in a stabilization of light signal. Luminescence was measured by Envision Plate Reader (Perkin Elmer, USA). IC50 values were calculated with the software Excel Fit (IDBS, Guildford, UK) from 3-fold dilution series comprising 8 concentrations in duplicates.

***Immunoblot analysis***

Cellular proteins were solubilized in CLB-A buffer (Zeptosens, Switzerland). After addition of 3xLämmli buffer (30 % v/v glycerol, 6 % SDS, 150 mM Tris/HCl [pH 6,8], 0,3 % w/v bromophenol blue, 300 mM DTT) proteins were denatured by incubation at 95 °C for 5 min. Thereon, proteins were separated on SDS-PAGE and transferred to PVDF membranes (Immobilon®, Milipore, Schwalbach). After blocking, bound proteins were incubated with antibodies against □-tubulin (T59840R, Biozol; clone B-5-1-2, Sigma-Aldrich) or Nut (C52B1, Cell Signaling, Frankfurt a. M.) diluted in blocking buffer (L1-Cor biosciences, Bad Homburg, Germany).

***Measurement of hat maximal inhibitory concentration to CDKs***

This protocol describes how the Lance Ultra KinaSelect Assay was performed to determine half maximal inhibitory concentration ($IC_{50}$) of compounds of general formula (I) and CDK/Cyclin complexes. The principle behind this enzymatic assay is based upon the phosphorylation of the Ulight-Peptide Substrat. It is detected by using a specific EU-labeled anti-phospho peptide antibody. The binding of the Eu labeled anti-phospho peptide antibody to the phosphorylated *ULight* labeled peptide gives rise to a FRET-signal. Binding of an inhibitor to the kinase prevents phosphorylation of the U*light*-MBP Substrat, resulting in a loss of FRET.

**Table 3** Reagents, stock concentrations and final assay concentrations

| Kinase | Supplier | Kinase-conc.[nM] | ATP-conc.[μM] | Substrate | Supplier | Substrat-conc. [nM] | Antibody | Supplier | Antibody-conc. [nM] |
|---|---|---|---|---|---|---|---|---|---|
| **CDK1/CyclinB1 (91 kDa]** | Carna | 2 | 20 | Ulight MBP | Perkin Elmer | 50 | Eu-anti-P-MBP | Perkin Elmer | 0,25 |
| **CDK2/CyclinA [135 kDa]** | Proginase | 5 | 3 | Ulight MBP | Perkin Elmer | 50 | Eu-anti-P-MBP | Perkin Elmer | 0,25 |
| **CDK4/CyclinD1 (123 kDa)** | Invitrogen | 10 | 90 | Ulight MBP | Perkin Elmer | 50 | Eu-anti-P-MBP | Perkin Elmer | 0,25 |
| **CDK6/CyclinD3 (123 kDa)** | Carna | 5 | 55 | Ulight MBP | Perkin Elmer | 50 | Eu-anti-P-MBP | Perkin Elmer | 0,025 |
| **CDK7/CylinH/ MAT1 (126 kDa)** | Invitrogen | 10 | 25 | Ulight MBP | Perkin Elmer | 50 | Eu-anti-P-MBP | Perkin Elmer | 0,25 |
| **CDK9/CyclinT1 (132 kDa)** | Invitrogen | 10 | 25 | Ulight MBP | Perkin Elmer | 50 | Eu-anti-P-MBP | Perkin Elmer | 0,25 |
| **CDK9/CyclinK (92 kDa)** | Invitrogen | 10 | 125 | Ulight MBP | Perkin Elmer | 50 | Eu-anti-P-MBP | Perkin Elmer | 0,25 |

The selective CDK9 inhibitors described herein above (see also Table 1 and Table 2) were diluted from a 10 mM DMSO stock solution 1:10 in a total volume of 15 μl DMSO. This compound predilution was then serial diluted 1:3 over 8 steps in DMSO and briefly spun down.

Each compound solution was now diluted 1:20 in Enzymatic Buffer (HEPES: 50 mM, pH: 7.5; $MgCl_2$: 10 mM; EGTA: 1 mM; DTT: 2mM; Tween-20: 0.01%), mixed thoroughly and spun down. For every sample, 2 μl of the diluted compound were mixed with 6 μl CDK/Cyclin/Substrat solution and 2 μl ATP solution in a well of a small volume 384 well plate (Corning Incorporated, Corning, NY, USA; order no. 3673). The CDK/Cyclin was diluted to the appropriate concentration (see Table 3 and the ATP concentration was adjusted to its $IC_{50}$ concentration for the CDK/Cyclin, which was 3 μM for CDK2/ Cyclin A, 20 μM for CDK1/ Cyclin B1, 25 μM CDK7/Cyclin H and CDK9/Cyclin T1, 55 μM CDK6/Cyclin D3, 90 μM CDK4/Cyclin D1 and 125 μM for CDK9/Cyclin K. For negative controls, in each well 2 μl of DMSO solution (1% final DMSO assay concentration) was mixed with 6 μl substrat solution (50 nM Ulight MBP final assay concentration) and 2 μl ATP solution (appropriate final concentration see Table 3. For positive controls, in each well 2 μl of DMSO solution (1% final DMSO assay concentration) was mixed with 6 μl CDK/Cyclin/Substrat (appropriate final concentration see Table 3 and 2 μl Tracer ATP solution (appropriate final concentration see Table 3. Positive and negative controls were calculated from at least 8 different sample wells. The 384 well plates were mixed in a Teleshaker plate mixer (Beckman Coulter, Brea, CA, USA) at 2000 rpm for 40 sec, and incubated for 1h at room temperature. Before reading, 10 μl the detection buffer (Lance Detection Buffer 1X ; EDTA: 20nM; Eu-Anti-P-MBP: see Table 3 was added. The FRET signal was measured at 340 nm excitation, 665 nm and 615 nm emission (for the kinase tracer and LanthaScreen Eu-AB, respectively) with an Envision spectrophotometer (Perkin Elmer, Waltham, MA, USA) with 50 μs delay and 300 μs integration time. $IC_{50}$ values were determined from the sigmoidal dose response curves with the software Quattro Workflow (Quattro GmbH, Munich, Germany).

***Other Kinase assays:***

A radiometric protein kinase assay (33PanQinase® Activity Assay) was used for measuring the kinase activity of the 333 protein kinases. All kinase assays were performed in 96-well FlashPlatesTM from Perkin Elmer (Boston, MA, USA) in a 50 μl reaction volume. The reaction cocktail was pipetted in 4 steps in the following order:

- 10 μl of non-radioactive ATP solution (in H2O)
- 25 μl of assay buffer/ [γ-33P]-ATP mixture
- 5 μl of test sample in 10% DMSO
- 10 μl of enzyme/substrate mixture

The assay for all enzymes contained 70 mM HEPES-NaOH, pH 7.5, 3 mM MgCl2, 3 mM MnCl2, 3 μM Na-orthovanadate, 1.2 mM DTT, ATP/[γ-33P]-ATP (variable amounts, corresponding to the apparent ATP-Km of the respective kinase, see Table 4 below / approx. 8 x 1005 cpm per well), protein kinase (variable amounts; see Table 4), and substrate (variable amounts; see Table 4). All protein kinases provided by ProQinase were expressed in Sf9 insect cells or in E.coli as recombinant GST-fusion proteins or His-tagged proteins. All kinases were produced from human cDNAs. Kinases were purified by affinity chromatography using either GSH-agarosc or Ni-NTA-agarose. The purity of the protein kinases was examined by SDS-PAGE/Coomassie staining. The identity of the protein kinases was checked by mass spectroscopy. The concentrations of enzymes and substrates used for the assays are shown in Table 4 below.

The reaction cocktails were incubated at 30° C for 60 minutes. The reaction was stopped with 50 μl of 2 % (v/v) H3PO4, plates were aspirated and washed two times with 200 μl 0.9 % (w/v) NaCl. All assays were performed with a BeckmanCoulter Biomek 2000/SL robotic system. Incorporation of 33Pi (counting of "cpm") was determined with a microplate scintillation counter (Microbeta, Wallac).

Table 4: Conditions of additional kinase assays

| **Kinase** | | | **Kinase Conc.** | | **ATP Cone.** | **Substrate** | | |
|---|---|---|---|---|---|---|---|---|
| **Name** | **PQ Lot** | **External Vendor Lot** | **ng/50μl** | **nM*** | **μM** | **Name** | **Lot** | **μg/50/μl** |
| ABL1 wt | 5 | | 25 | 6,6 | 0,3 | Poly(Ala,Glu,Lys,Tyr) 6:2:5:1 | SIG 53H5516 | 0,125 |
| AKT1 | 7 | | 25 | 6,4 | 3 | GSK3(14-27) | 11 | 2 |

(continued)

| Kinase | | | Kinase Conc. | | ATP Cone. | Substrate | | |
|---|---|---|---|---|---|---|---|---|
| Name | PQ Lot | External Vendor Lot | ng/50μl | nM* | μM | Name | Lot | μg/50/μl |
| Aurora-A | 4 | | 50 | 13,1 | 10 | tetra(LRRWSLG) | 6 | 0,5 |
| CDK9/CycT | 4 | | 15 | 1,9 | 1 | RBER-CHKtide | 25 | 1 |
| CK2-alphal | 3 | | 20 | 5,6 | 0,1 | Casein | SIG_83K7430 | 1 |
| EGF-R wt | 17 | | 20 | 4,5 | 0,3 | Poly(Glu,Tyr)4:1 | SIG_20K5903 | 0,125 |
| EPHB4 | 7 | | 10 | 2,6 | 1 | Poly(Glu,Tyr)4:1 | SIG 20K5903 | 0,125 |
| FGF-R1 | 1 | INV 31517 | 3 | 1,2 | 1 | Poly(Glu, Tyr)4:1 | SIG_20K5903 | 0,125 |
| FLT3 wt | 11 | | 25 | 6,1 | 3 | Poly(Ala,Glu,Lys,Tyr) 6:2:5:1 | SIG 53H5516 | 0,125 |
| GSK3-beta | 3 | | 50 | 13,1 | 0,3 | RBER-CHKtide | 24 | 1 |
| IGF1-R | 12 | | 10 | 2,6 | 1 | Poly(Glu,Tyr)4:1 | SIG_20K5903 | 0,125 |
| IKK-beta | 8 | | 50 | 8,3 | 0,3 | RBER-CHKtide | 24 | 2 |
| JNK1 | 5 | | 5 | 2,3 | 0,3 | ATF2 | 11 | 2 |
| LCK | 3 | | 20 | 4,3 | 0,3 | Poly(Glu,Tyr)4:1 | SIG 20K5903 | 0,125 |
| MAPKAPK3 | 1 | | 10 | 4,6 | 0,1 | tetra(LRRWSLG) | 6 | 0,25 |
| MEEK1 | 2 | 50 | 23 | 3 | | ERK2 K54R (kinase-dead) | 6 | 2 |
| MET wt | 12 | | 20 | 5,1 | 0,3 | Poly(Ala,Glu/Lys,Tyr) 6:2:5:1 | SIG 53H5516 | 0,125 |
| PDGFR-beta | 13 | | 50 | 11,4 | 0,3 | Poly(Ala,Glu,Lys,Tyr) 6:2:5:1 | SIG 53H5516 | 0,125 |
| PLK1 | 13 | | 15 | 3,0 | 1 | RBER-CHKtide | 25 | 2 |
| p38-alpha | 5 | | 10 | 4,8 | 1 | ATF2 | 10 | 2 |
| ROCK2 | 2 | | 5 | 1,1 | 0,3 | S6-Peptide | 6 | 1 |
| TGFB-R1 | 3 | | 5 | 1,6 | 1 | GSK3(14-27) | 9 | 1 |
| VEGF-R2 | 15 | | 25 | 5,7 | 1 | Poly(Glu,Tyr)4:1 | SIG 20K5903 | 0,125 |

**Results**

Within a screening approach to profile more than 1600 kinase inhibitors and other compounds on proliferation of 86 cell lines we identified HCC2429 cells to be sensitive for CDK9 inhibitors (specific as well as unspecific). This initial finding was verified by dose response experiments (see figure 1). In these experiments selective CDK9 inhibitors (Cpd B1) potently affected proliferation of HCC2429 cells whereas a specific CDK1 inhibitor (Ro-3306) did not show comparable effects (figure 1). HCC2429 cells overexpress Notch3 and are known to contain a t(15, 19) translocation. The later one results in the expression of a Brd4/Nut fusion protein. We confirmed the selectivity of a set of additional compounds by in vitro kinase assays (figure 2). To this end we performed assays for CDKs as well as for other kinases (ABL1 wt, AKT1, Aurora-A, CDK1/CycA, CDK1/CycE, CDK2/CycA, CDK3/CycE, CDK4/CycD1, CDK6/CycD1, CDK7/CycH/MATI, CDK9/CycT, CK2-alphal, EGF-R wt, EPHB4, FGF-R1 wt, FLT3 wt, GSK3-beta, IGF1-R, IKK-beta, JNK1, LCK, MAPKAPK3, MEK1 wt, MET, PDGFR-beta, PLK1, p38-alpha, ROCK2, TGFB-R1, VEGF-R2). Alltogether these experiments corroborate the enormous selectivity of the pyridine, triazine and pyrimidine compounds.Profiling of selected CDK9 inhibitors on cells bearing a similar t(15, 19) translocation (Ty-82, 690100, 143100 together with HCC2429) or

control cells (HCC1143, H3122, PC9, A549, Hela, H460 and hPBMCs) confirmed that this rearrangement sensitizes for CDK9 inhibition (figure 4). Interestingly, overexpression of Notch3 did not result in sensitization as shown by Notch3 overexpressing cell lines HCC1143 and others (H1793, H1819, H441 and H647; data not shown). The expression of the fusion gene was confirmed in said cells by western blot analysis (figure 5).

The present invention refers to the following nucleotide and amino acid sequences:

The sequences provided herein are available in the NCBI database and can be retrieved from www.ncbi.nlm.nih.gov/sites/entrez?db=gene; Theses sequences also relate to annotated and modified sequences. The present invention also provides techniques and methods wherein homologous sequences, and also genetic allelic variants and the like of the concise sequences provided herein are used. Preferably, such "variants" are genetic variants.

SEQ ID No. 1:

Nucleotide sequence encoding homo sapiens nut gene (alias Homo sapiens chromosome 15 open reading frame 55 (C15orf55); accession number NM_175741.1):

```
  1 gagttccgta ttctagttct gtgtgatctg atctttacct tcccttcctt ggatccctgt
 61 gcacctactg gagccaggtt actctgggtc ctggacctga ctgcctcatt ctggaggctt
121 ccagacagcc acagttagtg cccaaacctg agaggatggc ttcagatgga gcatctgcat
181 tgccgggacc ggatatgagc atgaaaccta gtgccgccct gtctccatcc cctgcacttc
```

241 cctttctccc accaacttct gacccaccag accacccacc cagggagcca cctccacagc
301 ccatcatgcc ttcagtattc tctccagaca accctctgat gctctctgct ttccccagct
361 cactgttggt gacaggggac gggggccctt gcctcagtgg ggctggggct ggcaaggtca
421 ttgtcaaagt caagacagaa gggggtcag ctgagccctc tcaaactcag aactttatcc
481 ttactcagac tgccctcaat tcgactgccc cgggcactcc ctgtggaggc cttgagggtc
541 ctgcacctcc atttgtgaca gcatctaatg tgaagaccat tctgccctct aaggctgttg
601 gtgtcagcca ggagggtcct ccaggccttc cgcctcagcc tccaccacca gttgctcaac
661 tggtccccat tgtgcccctg gaaaaagctt ggccagggcc acatgggaca accggggaag
721 gaggtcctgt ggccactcta tccaagcctt ccctaggtga ccgctccaaa atttccaagg
781 acgtttatga gaacttccgt cagtggcagc gttacaaagc cttggcccgg aggcacctat
841 cccagagtcc tgacacagaa gctctttcct gtttcttat cccagtgctt cgttccctgg
901 cccggctgaa gcccactatg accctggagg agggactgcc attggctgtg caggagtggg
961 agcacaccag caactttgac cggatgatct tttatgagat ggcagaaagg ttcatggagt
1021 ttgaggctga ggagatgcag attcagaaca cacagctgat gaatgggtct cagggcctgt
1081 ctcctgcaac cccttgaaa cttgatcctc tagggcccct ggcctctgag gtttgccagc
1141 agccagtgta cattccgaag aaggcagcct ccaagacacg ggcccccgc cggcgtcagc
1201 gtaaagccca gagacctcct gctcctgagg cacccaagga gatcccacca gaagctgtga
1261 aggagtatgt tgacatcatg gaatggctgg tggggactca cttggccact ggggagtcag
1321 atggaaaaca agaggaagaa gggcagcagc aggaggagga agggatgtat ccagatccag
1381 gtctcctgag ctacatcaat gagctgtgtt ctcagaaggt ctttgtctcc aaggtggagg
1441 ctgtcattca ccctcaattt ctggcagatc tgctgtcccc agaaaaacag agagatccct
1501 tggccttaat tgaggagcta gagcaagaag aaggactcac tcttgcccag ctggtccaga
1561 agcgactcat ggccttggaa gaggaggaag atgcagaggc gcctccaagt ttcagtggcg
1621 ctcagttgga ctcaagtcct tctggttctg ttgaggatga agatggggat gggcggcttc
1681 ggccctcacc tgggcttcag gggctgggg gcgccgcttg ccttggaaag gtttcttctt
1741 caggaaaacg ggcaagagaa gtgcatggtg ggcaggagca agccctagat agccccagag
1801 ggatgcacag ggatgggaac actctgccat cccccagcag ctgggacctg cagccagaac
1861 ttgcagctcc acagggaact ccgggaccct tgggtgtgga gaggagaggg tctgggaagg
1921 ttataaacca ggtatctcta catcaggatg gccatctagg aggcgctggg cctcctgggc
1981 actgcctggt ggctgatagg acttcagagg ctctgcccct ttgttggcag ggaggcttcc
2041 agcctgagag cactcccagt ttggatgctg gacttgcaga gctggctcct ctgcaaggac
2101 aagggttaga aaagcaagtc ctgggattgc agaaaggaca acaaacaggg ggtcgtggag
2161 tgcttcctca agggaaggag cctttagcag tgccctggga aggctcttca ggagccatgt
2221 ggggagatga cagaggtacc cccatggctc agagttatga tcagaatcct tcccctagag
2281 cagctgggga gagggacgat gtctgtctca gcccaggagt ttggctgagc agtgagatcg
2341 atgctgtagg cttggagctg cctgtacaaa tagaggaggt catagagagc ttccaagttg
2401 agaagtgtgt aactgagtat caggaaggct gccagggact gggctccagg ggcaacattt
2461 ccctgggtcc tggagaaacc ctagtacctg gggatacgga gagcagtgtg attccctgtg
2521 gaggcacagt tgcggcagct gccctagaaa agagaaacta ttgcagcttg ccaggacctt
2581 tgagggccaa cagcccaccc ttgaggtcca aagaaaatca agaacagagc tgtgaaaccg
2641 tagggcatcc cagtgatctg tgggcagaag gttgcttccc attgctagaa agtggtgatt
2701 ccacactggg gtcttccaaa gaaacccttc cacccacatg ccaaggcaat ctccttatca
2761 tggggactga ggatgcctcc tccttgcctg aagccagtca agaggcaggg agcagaggca
2821 attccttttc tcctctgttg gaaaccatag aacctgtcaa catactagat gttaaagatg
2881 actgtggcct ccaactaagg gtcagcgagg acacctgccc actgaatgtt cattcttatg
2941 acccccaagg agaaggcagg gtggatcctg atctgtccaa gcctaaaaac cttgctcctt
3001 tacaagagag tcaggagtct tacacaactg ggactcccaa agcaacatct tctcaccagg
3061 gccttggaag cactttgcct agaaggggaa ccaggaatgc catagttccg agagaaactt
3121 ctgttagtaa aacacacagg tcagcagaca gggccaaagg aaaggagaaa aagaaaaagg
3181 aagcagagga agaggatgag gaactctcca actttgctta cctcttggcc tctaaactta
3241 gcctctcacc aagggagcat cccctcagtc ctcaccatgc ctcaggaggt cagggcagcc
3301 agagagcatc ccacctgctc cctgctggag caaaaggccc cagcaaactt ccatatcctg
3361 ttgccaagtc tgggaagcga gctctagctg gaggtccagc ccctactgaa aagacacccc
3421 actcaggagc tcaacttggg gtccccaggg agaaacccct agctctggga gtagttcgac
3481 cctcacagcc tcgtaaaagg cggtgtgaca gttttgtcac gggcagaagg aagaaacgac
3541 gtcgtagcca gtagggagca gcgggaccat ctgaccccac ttgccagtcc ctaaaggtgg
3601 gtgccccaga gtagattcca cccctgctgc ccaccaatgg agaatcccaa tgttgaatct
3661 catcccaatg ttgttttgtt gttctgcaaa agtggcaagc atggagagag aggtcagact
3721 ggctaggctg caggggaat tacctttgga aggagctata tagaaaaaaa atgaataaag
3781 tgttttgttg gaaaa

The coding region ranges from nucleotide 156 to nucleotide 3554.

SEQ ID No. 2:

Amino acid sequence of homo sapiens Nut protein:

```
MASDGASALPGPDMSMKPSAALSPSPALPFLPPTSDPPDHPPREPPPQPIMPSVFSPDNPLMLSAFPSSLLVTGDGG
PCLSGAGAGKVIVKVKTEGGSAEPSQTQNFILTQTALNSTAPGTPCGGLEGPAPPFVTASNVKTILPSKAVGVSQEG
PPGLPPQPPPPVAQLVPIVPLEKAWPGPHGTTGEGGPVATLSKPSLGDRSKISKDVYENFRQWQRYKALARRHLSQS
PDTEALSCFLIPVLRSLARLKPTMTLEEGLPLAVQEWEHTSNFDRMIFYEMAERFMEFEAEEMQIQNTQLMNGSQGL
SPATPLKLDPLGPLASEVCQQPVYIPKKAASKTRAPRRRQRKAQRPPAPEAPKEIPPEAVKEYVDIMEWLVGTHLAT
GESDGKQEEEGQQQEEEGMYPDPGLLSYINELCSQKVFVSKVEAVIHPQFLADLLSPEKQRDPLALIEELEQEEGLT
LAQLVQKRLMALEEEEDAEAPPSFSGAQLDSSPSGSVEDEDGDGRLRPSPGLQGAGGAACLGKVSSSGKRAREVHGG
QEQALDSPRGMHRDGNTLPSPSSWDLQPELAAPQGTPGPLGVERRGSGKVINQVSLHQDGHLGGAGPPGHCLVADRT
SEALPLCWQGGFQPESTPSLDAGLAELAPLQGQGLEKQVLGLQKGQQTGGRGVLPQGKEPLAVPWEGSSGAMWGDDR
GTPMAQSYDQNPSPRAAGERDDVCLSPGVWLSSEMDAVGLELPVQIEEVIESFQVEKCVTEYQEGCQGLGSRGNISL
GPGETLVPGDTESSVIPCGGTVAAAALEKRNYCSLPGPLRANSPPLRSKENQEQSCETVGHPSDLWAEGCFPLLESG
DSTLGSSKETLPPTCQGNLLIMGTEDASSLPEASQEAGSRGNSFSPLLETIEPVNILDVKDDCGLQLRVSEDTCPLN
VHSYDPQGEGRVDPDLSKPKNLAPLQESQESYTTGTPKATSSHQGLGSTLPRRGTRNAIVPRETSVSKTHRSADRAK
GKEKKKKEAEEEDEELSNFAYLLASKLSLSPREHPLSPHHASGGQGSQRASHLLPAGAKGPSKLPYPVAKSGKRALA
GGPAPTEKTPHSGAQLGVPREKPLALGVVRPSQPRKRRCDSFVTGRRKKRRRSQ
```

SEQ ID No. 3:

Nucleotide sequence encoding homo sapiens brd4 (accession number NM_058243.2; ):

```
   1 attctttgga atactactgc tagaagtctg acttaagacc cagcttatgg gccacatggc
  61 acccagctgc ttctgcagag aaggcaggcc actgatgggt acagcaaagt gtggtgctgc
 121 tggccaagcc aaagacccgt gtaggatgac tgggcctctg ccccttgtgg gtgttgccac
 181 tgtgcttgag tgcctggtga agaatgtgat gggatcacta gcatgtctgc ggagagcggc
 241 cctgggacga gattgagaaa tctgccagta atgggggatg gactagaaac ttcccaaatg
 301 tctacaacac aggcccaggc ccaaccccag ccagccaacg cagccagcac caaccccccg
 361 cccccagaga cctccaaccc taacaagccc aagaggcaga ccaaccaact gcaatacctg
 421 ctcagagtgg tgctcaagac actatggaaa caccagtttg catggccttt ccagcagcct
 481 gtggatgccg tcaagctgaa cctccctgat tactataaga tcattaaaac gcctatggat
 541 atgggaacaa taaagaagcg cttggaaaac aactattact ggaatgctca ggaatgtatc
 601 caggacttca acactatgtt tacaaattgt tacatctaca acaagcctgg agatgacata
 661 gtcttaatgg cagaagctct ggaaaagctc ttcttgcaaa aaataaatga gctacccaca
 721 gaagaaaccg agatcatgat agtccaggca aaaggaagag gacgtgggag gaaagaaaca
 781 gggacagcaa aacctggcgt ttccacggta ccaaacacaa ctcaagcatc gactcctccg
 841 cagacccaga cccctcagcc gaatcctcct cctgtgcagg ccacgcctca ccccttccct
 901 gccgtcaccc cggacctcat cgtccagacc cctgtcatga cagtggtgcc tccccagcca
 961 ctgcagacgc cccgccagt gcccccccag ccacaacccc cacccgctcc agctccccag
1021 cccgtacaga gccacccacc catcatcgcg gccaccccac agcctgtgaa gacaaagaag
1081 ggagtgaaga ggaaagcaga caccaccacc cccaccacca ttgaccccat tcacgagcca
1141 ccctcgctgc ccccggagcc caagaccacc aagctgggcc agcggcggga gagcagccgg
1201 cctgtgaaac ctccaaagaa ggacgtgccc gactctcagc agcacccagc accagagaag
1261 agcagcaagg tctcggagca gctcaagtgc tgcagcggca tcctcaagga gatgtttgcc
1321 aagaagcacg ccgcctacgc ctggcccttc tacaagcctg tggacgtgga ggcactgggc
1381 ctacacgact actgtgacat catcaagcac cccatggaca tgagcacaat caagtctaaa
1441 ctggaggccc gtgagtaccg tgatgctcag gagtttggtg ctgacgtccg attgatgttc
1501 tccaactgct ataagtacaa ccctcctgac catgaggtgg tggccatggc ccgcaagctc
1561 caggatgtgt tcgaaatgcg ctttgccaag atgccggacg agcctgagga gccagtggtg
1621 gccgtgtcct ccccggcagt gccccctccc accaaggttg tggccccgcc ctcatccagc
1681 gacagcagca gcgatagctc ctcggacagt gacagttcga ctgatgactc tgaggaggag
```

```
1741 cgagcccagc ggctggctga gctccaggag cagctcaaag ccgtgcacga gcagcttgca
1801 gccctctctc agccccagca gaacaaacca aagaaaaagg agaaagacaa gaaggaaaag
1861 aaaaaagaaa agcacaaaag gaaagaggaa gtggaagaga ataaaaaaag caaagccaag
1921 gaacctcctc ctaaaaagac gaagaaaaat aatagcagca acagcaatgt gagcaagaag
1981 gagccagcgc ccatgaagag caagcccct cccacgtatg agtcggagga agaggacaag
2041 tgcaagccta tgtcctatga ggagaagcgg cagctcagct tggacatcaa caagctcccc
2101 ggcgagaagc tgggccgcgt ggtgcacatc atccagtcac gggagccctc cctgaagaat
2161 tccaaccccg acgagattga aatcgacttt gagaccctga agccgtccac actgcgtgag
2221 ctggagcgct atgtcacctc ctgtttgcgg aagaaaagga aacctcaagc tgagaaagtt
2281 gatgtgattg ccggctcctc caagatgaag ggcttctcgt cctcagagtc ggagagctcc
2341 agtgagtcca gctcctctga cagcgaagac tccgaaacag agatggctcc gaagtcaaaa
2401 aagaaggggc accccgggag ggagcagaag aagcaccatc atcaccacca tcagcagatg
2461 cagcaggccc cggctcctgt gccccagcag ccgcccccgc ctccccagca gcccccaccg
2521 cctccacctc cgcagcagca acagcagccg ccacccccgc ctcccccacc ctccatgccg
2581 cagcaggcag ccccggcgat gaagtcctcg cccccaccct tcattgccac ccaggtgccc
2641 gtcctggagc cccagctccc aggcagcgtc tttgacccca tcggccactt cacccagccc
2701 atcctgcacc tgccgcagcc tgagctgccc cctcacctgc cccagccgcc tgagcacagc
2761 actccacccc atctcaacca gcacgcagtg gtctctcctc cagctttgca caacgcacta
2821 ccccagcagc catcacggcc cagcaaccga gccgctgccc tgcctcccaa gcccgcccgg
2881 cccccagccg tgtcaccagc cttgacccaa acacccctgc tcccacagcc ccccatggcc
2941 caaccccccc aagtgctgct ggaggatgaa gagccacctg ccccacccct cacctccatg
3001 cagatgcagc tgtacctgca gcagctgcag aaggtgcagc cccctacgcc gctactccct
3061 tccgtgaagg tgcagtccca gcccccaccc cccctgccgc ccccacccca cccctctgtg
3121 cagcagcagc tgcagcagca gccgccacca cccccaccac cccagcccca gcctccaccc
3181 cagcagcagc atcagccccc tccacggccc gtgcacttgc agcccatgca gttttccacc
3241 cacatccaac agcccccgcc accccagggc cagcagcccc cccatccgcc cccaggccag
3301 cagccacccc cgccgcagcc tgccaagcct cagcaagtca tccagcacca ccattcaccc
3361 cggcaccaca agtcggaccc ctactcaacc ggtcacctcc gcgaagcccc ctccccgctt
3421 atgatacatt ccccccagat gtcacagttc cagagcctga cccaccagtc tccaccccag
3481 caaaacgtcc agcctaagaa acaggagctg cgtgctgcct ccgtggtcca gccccagccc
3541 ctcgtggtgg tgaaggagga gaagatccac tcacccatca tccgcagcga gcccttcagc
3601 ccctcgctgc ggccggagcc ccccaagcac ccggagagca tcaaggcccc cgtccacctg
3661 ccccagcggc cggaaatgaa gcctgtggat gtcgggaggc ctgtgatccg gcccccagag
3721 cagaacgcac cgccaccagg ggcccctgac aaggacaaac agaaacagga gccgaagact
3781 ccagttgcgc ccaaaaagga cctgaaaatc aagaacatgg gctcctgggc cagcctagtg
3841 cagaagcatc cgaccacccc ctcctccaca gccaagtcat ccagcgacag cttcgagcag
3901 ttccgccgcg ccgctcggga gaaagaggag cgtgagaagg ccctgaaggc tcaggccgag
3961 cacgctgaga aggagaagga gcggctgcgg caggagcgca tgaggagccg agaggacgag
4021 gatgcgctgg agcaggcccg gcgggcccat gaggaggcac gtcggcgcca ggagcagcag
4081 cagcagcagc gccaggagca acagcagcag cagcaacagc aagcagctgc ggtggctgcc
4141 gccgccaccc cacaggccca gagctcccag ccccagtcca tgctggacca gcagagggag
4201 ttggcccgga agcgggagca ggagcgaaga cgccgggaag ccatggcagc taccattgac
4261 atgaatttcc agagtgatct attgtcaata tttgaagaaa atcttttctg agcgcaccta
4321 ggtggcttct gactttgatt ttctggcaaa acattgactt tccatagtgt taggggcggt
4381 ggtggaggtg ggatcagcgg ccaggggatg cctcagggcc tggccctcct gcatgctatg
4441 cccggggcag gcctgacggg cagctgagga ttgcagagcc tgtctgcctt acggccagtc
4501 ggacagacgt cccgccaccc accacccctc acaggacgtc cgctcagcac acgccttgtt
4561 acgagcaagt gccggctgga cccaagccct gcatccccac atgcggggca gaggcccttc
4621 tctccgccaa atgtctacac agtatacaca ggacatcgtt gctgccgccg tgactggttt
4681 tctgtcccca agaacgtgac gttcgtgatg tcctgcccgc cgggagtctt tccccacacc
4741 ccagccatcg ccgcccgctc ccaggaggcc agggcaggcc tgcgtggcct ggaggcgggc
4801 gaggccggcc caccccctcg ctggcactga ctttgccttg aacagacccc ccgaccctcc
4861 cccacaagcc tttaattgag agccgctctc tgtaagtgtt tgcttgtgca aaagggaata
4921 gtgccgtgga ggtgtgtgtg tccatggcat ccggagcgag gcgactgtcc tgcgtgggta
4981 gccctcggcc ggggagtgag gccaccaacc aaagtcagtt ccttcccacc tgtgtttctg
5041 tttcgttttt ttttttcttt tttttctata tatatttttt gttgaattct attttatttt
5101 taattctctc ttctcctcca gacacaatgg cactgcttat ctccgaaatg gtgtgatcgt
5161 ctcctcattg agcagcggct gccaccgcgc tgtgggta
```

The coding region ranges from nucleotide 223 to nucleotide 4311.

SEQ ID No. 4:

Amino acid sequence of homo sapiens Brd4 protein:

```
MSAESGPGTRLRNLPVMGDGLETSQMSTTQAQAQPQPANAASTNPPPPETSNPNKPKRQTNQLQYLLRVVLKTLWKH
QFAWPFQQPVDAVKLNLPDYYKIIKTPMDMGTIKKRLENNYYWNAQECIQDFNTMFTNCYIYNKPGDDIVLMAEALE
KLFLQKINELPTEETEIMIVQAKGRGRGRKETGTAKPGVSTVPNTTQASTPPQTQTPQPNPPPVQATPHPFPAVTPD
LIVQTPVMTVVPPQPLQTPPPVPPQPQPPPAPAPQPVQSHPPIIAATPQPVKTKKGVKRKADTTTPTTIDPIHEPPS
LPPEPKTTKLGQRRESSRPVKPPKKDVPDSQQHPAPEKSSKVSEQLKCCSGILKEMFAKKHAAYAWPFYKPVDVEAL
GLHDYCDIIKHPMDMSTIKSKLEAREYRDAQEFGADVRLMFSNCYKYNPPDHEVVAMARKLQDVFEMRFAKMPDEPE
EPVVAVSSPAVPPPTKVVAPPSSSDSSSDSSSDSDSSTDDSEEERAQRLAELQEQLKAVHEQLAALSQPQQNKPKKK
EKDKKEKKKEKHKRKEEVEENKKSKAKEPPPKKTKKNNSSNSNVSKKEPAPMKSKPPPTYESEEEDKCKPMSYEEKR
QLSLDINKLPGEKLGRVVHIIQSREPSLKNSNPDEIEIDFETLKPSTLRELERYVTSCLRKKRKPQAEKVDVIAGSS
KMKGFSSSESESSSESSSSDSEDSETEMAPKSKKKGHPGREQKKHHHHHHQQMQQAPAPVPQQPPPPPQQPPPPPPP
QQQQQPPPPPPPPPSMPQQAAPAMKSSPPPFIATQVPVLEPQLPGSVFDPIGHFTQPILHLPQPELPPHLPQPPEHST
PPHLNQHAVVSPPALHNALPQQPSRPSNRAAALPPKPARPPAVSPALTQTPLLPQPPMAQPPQVLLEDEEPPAPPLT
SMQMQLYLQQLQKVQPPTPLLPSVKVQSQPPPPLPPPPHPSVQQQLQQQPPPPPPPQPQPPPQQQHQPPPRPVHLQP
MQFSTHIQQPPPPQGQQPPHPPPGQQPPPPQPAKPQQVIQHHHSPRHHKSDPYSTGHLREAPSPLMIHSPQMSQFQS
LTHQSPPQQNVQPKKQELRAASVVQPQPLVVVKEEKIHSPIIRSEPFSPSLRPEPPKHPESIKAPVHLPQRPEMKPV
DVGRPVIRPPEQNAPPPGAPDKDKQKQEPKTPVAPKKDLKIKNMGSWASLVQKHPTTPSSTAKSSSDSFEQFRRAAR
EKEEREKALKAQAEHAEKEKERLRQERMRSREDEDALEQARRAHEEARRRQEQQQQRQEQQQQQQQAAAVAAAT
PQAQSSQPQSMLDQQRELARKREQERRRREAMAATIDMNFQSDLLSIFEENLF
```

SEQ ID No. 5:

Nucleotide sequence encoding homo sapiens brd3 (accession number NM_007371.3).

```
   1 tgccggggcc ggcgagccaa agaggagccg gccgcgcggg ccgggagggg acggccgccg
  61 gagccgcgag gccaactgtc gcctggttgg gcccggaaat gggacgtcgc gctttctcag
 121 ggagcgtaga agcagccagg gcctctccaa gccgctgctg tgacagaaag tgagtgagct
 181 gccggaggat gtccaccgcc acgacagtcg cccccgcggg gatcccggcg accccgggcc
 241 ctgtgaaccc accccccccg gaggtctcca accccagcaa gcccggccgc aagaccaacc
 301 agctgcagta catgcagaat gtggtggtga agacgctctg gaaacaccag ttcgcctggc
 361 ccttctacca gcccgtggac gcaatcaaat tgaacctgcc ggattatcat aaaataatta
 421 aaaacccaat ggatatgggg actattaaga agagactaga aaataattat tattggagtg
 481 caagcgaatg tatgcaggac ttcaacacca tgtttacaaa ttgttacatt tataacaagc
 541 ccacagatga catagtgcta atggcccaag ctttagagaa aatttttcta caaaaagtgg
 601 cccagatgcc ccaagaggaa gttgaattat taccccctgc tccaaagggc aaaggtcgga
 661 agccggctgc gggagcccag agcgcaggta cacagcaagt ggcggccgtg tcctctgtct
 721 ccccagcgac cccctttcag agcgtgcccc ccaccgtctc ccagacgccc gtcatcgctg
 781 ccacccctgt accaaccatc actgcaaacg tcacgtcggt cccagtcccc ccagctgccg
 841 ccccacctcc tcctgccaca cccatcgtcc ccgtggtccc tcctacgccg cctgtcgtca
 901 agaaaaaggg cgtgaagcgg aaagcagaca caaccactcc cacgacgtcg gccatcactg
 961 ccagccggag tgagtcgccc ccgccgttgt cagaccccaa gcaggccaaa gtggtggccc
1021 ggcgggagag tggtggccgc cccatcaagc ctcccaagaa ggacctggag gacggcgagg
1081 tgccccagca cgcaggcaag aagggcaagc tgtcggagca cctacgctac tgcgacagca
1141 tcctcaggga gatgctatcc aagaagcacg cggcctacgc ctggcccttc tacaagccag
1201 tggatgccga ggccctggag ctgcacgact accacgacat catcaagcac ccgatggacc
1261 tcagcaccgt gaaaaggaag atggatggcc gagagtaccc agacgcacag ggctttgctg
1321 ctgatgtccg gctgatgttc tcgaattgct acaaatacaa tcccccagac cacgaggttg
1381 tggccatggc ccggaagctc caggacgtgt ttgagatgag gtttgccaag atgccagatg
1441 agcccgtgga ggcaccggcg ctgcctgccc ccgcggcccc catggtgagc aagggcgctg
1501 agagcagccg tagcagtgag gagagctctt cggactcagg cagctcggac tcggaggagg
1561 agcgggccac caggctggcg gagctgcagg agcagctgaa ggccgtgcac gagcagctgg
1621 ccgccctgtc tcaggcccca gtaaacaaac caaagaagaa gaaggagaag aaggagaagg
1681 agaagaagaa gaaggacaag gagaaggaga aggagaagca caaagtgaag gccgaggaag
1741 agaagaaggc caaggtggct ccgcctgcca agcaggctca gcagaagaag gctcctgcca
1801 agaaggccaa cagcacgacc acggccggca gacagctgaa gaaaggcggc aagcaggcat
1861 ctgcctccta cgactcagag gaagaggagg agggcctgcc catgagctac gatgaaaagc
1921 gccagcttag cctggacatc aaccggctgc ccggggagaa gctgggccgg gtagtgcaca
```

1981 tcatccaatc tcgggagccc tcgctcaggg actccaaccc cgacgagata gaaattgact
2041 ttgagactct gaaacccacc actttgcggg aactggagag atatgtcaag tcttgtttac
2101 agaaaaagca aaggaaaccg ttctcagcaa gcgggaagaa acaggcagcc aagtcgaaag
2161 aggagctagc tcaggaaaag aagaaggagc tggaaaagcg tctgcaggat gtcagcgggc
2221 agctgagcag cagcaagaag cccgcccgga aagagaagcc cggctcagca ccctcagggg
2281 gcccgtccag gctcagcagc agcagctcct ccgagtctgg gagcagcagc tccagcgggt
2341 ccagctctga cagcagtgac tcagaatgaa ctggcttcgg acagaacagg acagatggat
2401 gtcgcacacg ccgagactct gccgtacccc tctgtggttc atattactac ttctgttcca
2461 tggtgtgcag gtctgcttct taattcagtg ttatgatatc ttccagtttt tgctttcata
2521 ggtcagagat ctatcttgtg tgtgcgttag acttgatgag aaggtgtgaa ctctgcagaa
2581 agtctcttct tcatcactga attcagtcac ttggagatga caacttcaaa tgctaacccg
2641 atgaccccag aaaaccgtgt gagattcgta ccgaagaacc ttgtggaatc cctttgctta
2701 ggcccaacct ggtcgatagc tcgagaaaga attttttcca aggaaatgtc tcggatatgg
2761 gtactgtatt tgaaagctgt tagctttgtc aacacgcatt gtccttgtca tttgggcccc
2821 gagctctgac cctcgtgtct gacgcggcca cctctttctg gaggggctga ggacagatgt
2881 gcctgcttgt ggagaccagg ctgggcctaa gcgaagggtc atcgcagccc cagcccggag
2941 cgtggagccc ttgggggtg gtcgggtggg atgtgcgttc tccgctcgtg gtgatgtcag
3001 gagctcctcg gagggaacag agcggctgtg tatgcagcct gcaggtttcc atacactgaa
3061 gcttttacct caactttaaa aaaaaaaaaa gaagaaaaga tttaaaaaaa aaaaaaaagg
3121 agactttttt tgtaaggttc ggcaattttc tacggaagtc cagcccgctg tgagtccccg
3181 cctggctagc gctgcccctg ctgctgctgc cgcgccaccc ttgggaacca cttcccgagc
3241 ttatgtgtat ataaatagtt atttattaac atcattggtc atttttaaaa aaaagaaaat
3301 aagaaaaaac cgcagaagaa atgcattcac acagtcgcag agatgcaggc cttgccagtg
3361 gtgtgccggg cgcgggtcct gtctggcggc ggcctgtcgt ctccaggtgc taactcctgc
3421 caccgcgcgg tgctcaccca cgtctgttcg cgcgctcgcc cctgggtttg ttgggttttt
3481 tggttttttt ctttgtggtt tttttttttt tttttttttg tatgaaactt ggaggcttac
3541 aggtatagac agctttcagc tacagcacat tctaattttt tattttgttt agttcttttg
3601 tattcacttc tggtctcttt aagactgttt taaaagaaat caatttaggg aaccccagtt
3661 atataatata aactttgtaa tctgagagaa aaaatgtata gtaaatctaa gtcttgattt
3721 ttaactttct attgtaaaaa ataataatat acagagttta atagaaggtg atgttttggt
3781 tttgttttcc cagaggctgc catatggtct ttgagtacgg ggatgtccca aactggccca
3841 ccaatgagca tggcggctcc ggccaggaat gccagagtta gcctcccagg cttgcgggtg
3901 gacatgcctg ctccctgcca gcctccagtg gcctggccag gccctcccga gcctgtctgc
3961 cctccccagg ggtggaggag tctctgggcc ccaggaggat tccctcccgg agactcgcac
4021 ggtgctccct gctcacgcgt tgtcacagtt agtccggaaa tgactgaaac caggcattct
4081 cccggacctc agcgtggggg agcctccagg cagacgctgg gtatggagct gtggtgtggt
4141 ctgtcctgta tggtggccag tgctttctgc cagcatttct ggatggatat agggactatc
4201 attagtatcc taatacacgg tgattttaaa acaaccataa aattgattca gagtccactg
4261 acccttacag atgtaggtat acccttactg gagagggaac tctgatgagg agatgctggt
4321 aaattatcat tttttaaatt gctggtgagt ctgacacttg gtgagttttc agccagtttg
4381 ttaaactttt aattaagttt tgtttataat aaaaatataa atggatttga aagtttccat
4441 tttttaaagt taccctcgtt ttcaaaggta ttttctaaac agatctttaa tggactattt
4501 aaaccgaatt taaggaattc acacacgaca gttgacaggt cttcacgcag gctggttggt
4561 aacgtgctgc cagcacaggg ctgggtgata cgtacaccct aagccggggg tgcctggggc
4621 tgggggcgc tccttgcaat gcccctccag ccacagggca gtgaggtgct gcctgtgtga
4681 gccgtcgggg gagcggccgg ctgtggggc agcgcagcag gagcatcgtg ggcctttcc
4741 ttctcggctg gttctctgtg acggtggcgt cggctcgcct ctgctccttt catctagaaa
4801 gaagccactg accctgacag cccacggcgg gtacactgag cagctgcatt ggtgctgtca
4861 ctttttttaag gctttctgtc cagacttcaa cactggtttc ttttcagagt ttcgaaggat
4921 taatgacttc ctcagcgccc ttgctggcgg gctgagggtg acagtcacgt ccgtttcttc
4981 tgtattagaa ggctgcggtg attcaattag attgtcccac tgctgagacc tgtagggcag
5041 cttctaacat gcttttttca aggggagagg agtagtgaca agtcgtgtgt cggaattgga
5101 tttgagaaca ctctgaatga cccctggagg ccgagggggc aggcttcggg cgtgaactga
5161 actccagacc cctctttgtg ttgggcagtg tcatcttgct tacaaactgt aagacacatt
5221 tttttgtgtg tttgtttttg ttgttgttct tttgcagcac tcacgcctct gacagtcttt
5281 tgggaaagag taacacccac atacagaatt tgtcacatcc agagtagcac tgttccttaa
5341 tactggcata atgcttccag gaagtttttc tttttttatat ttaaaatgtt acttttctgt
5401 atgatgtgca tgcaagttta ccgtaacttt tcttaaactt tttagtgccg tttctagtat
5461 attcctgtaa atgtcagtta ctgaaaatga gtccaatgta agtagtttag cttgtttatt
5521 gcaatgctgg cctcaacaca acagaataaa aatggtagaa agtactcttt gatgtttctg

```
5581 gtaatcatgg acccttctcc tggggcattt gttttgtttt cataataaaa agcaaaaaaa
5641 aaaaaaaaaa aaaaaaaaaa aaaaaaaaaa aaa
```

The coding region ranges from nucleotide 189 to nucleotide 2369.

SEQ ID No. 6:

Amino acid sequence of homo sapiens Brd3:

```
MSTATTVAPAGIPATPGPVNPPPPEVSNPSKPGRKTNQLQYMQNVVVKTLWKHQFAWPFYQPVDAIKLNLPDYHKII
KNPMDMGTIKKRLENNYYWSASECMQDFNTMFTNCYIYNKPTDDIVLMAQALEKIFLQKVAQMPQEEVELLPPAPKG
KGRKPAAGAQSAGTQQVAAVSSVSPATPFQSVPPTVSQTPVIAATPVPTITANVTSVPVPPAAAPPPPATPIVPVVP
PTPPVVKKKGVKRKADTTTPTTSAITASRSESPPPLSDPKQAKVVARRESGGRPIKPPKKDLEDGEVPQHAGKKGKL
SEHLRYCDSILREMLSKKHAAYAWPFYKPVDAEALELHDYHDIIKHPMDLSTVKRKMDGREYPDAQGFAADVRLMFS
NCYKYNPPDHEVVAMARKLQDVFEMRFAKMPDEPVEAPALPAPAAPMVSKGAESSRSSEESSSDSGSSDSEEERATR
LAELQEQLKAVHEQLAALSQAPVNKPKKKKEKKEKEKKKKDKEKEKEKHKVKAEEEKKAKVAPPAKQAQQKKAPAKK
ANSTTTAGRQLKKGGKQASASYDSEEEEEGLPMSYDEKRQLSLDINRLPGEKLGRVVHIIQSREPSLRDSNPDEIEI
DFETLKPTTLRELERYVKSCLQKKQRKPFSASGKKQAAKSKEELAQEKKKELEKRLQDVSGQLSSSKKPARKEKPGS
APSGGPSRLSSSSSSESGSSSSSGSSSDSSDSE
```

SEQ ID No. 7:

Nucleotide sequence encoding homo sapiens cdk9 (accession number NM_001261.3, alias TAK, C-2k, CDK1, CDC2L4, PITALRE):

```
   1 aagtggccgtggaggcggaagtggcgcggccgcggaggggcctggagtgcggcggcgg
  61 ggacccggagcaggagcggcggcagcagcgactgggggcggcggcggcgcgttggaggcg
 121 gccatggcaaagcagtacgactcggtggagtgccctttttgtgatgaagtttccaaatac
 181 gagaagctcgccaagatcggccaaggcaccttcggggaggtgttcaaggccaggcaccgc
 241 aagaccggccagaaggtggctctgaagaaggtgctgatggaaaacgagaaggaggggttc
 301 cccattacagccttgcgggagatcaagatccttcagcttctaaaacacgagaatgtggtc
 361 aacttgattgagatttgtcgaaccaaagcttcccctataaccgctgcaagggtagtata
 421 tacctggtgttcgacttctgcgagcatgaccttgctgggctgttgagcaatgttttggtc
 481 aagttcacgctgtctgagatcaagagggtgatgcagatgctgcttaacggcctctactac
 541 atccacagaaacaagatcctgcatagggacatgaaggctgctaatgtgcttatcactcgt
 601 gatggggtcctgaagctggcagactttggctggcccgggccttcagcctggccaagaac
 661 agccagcccaaccgctacaccaaccgtgtggtgacactctggtaccggcccccggagctg
 721 ttgctcggggagcgggactacggcccccccattgacctgtggggtgctgggtgcatcatg
 781 gcagagatgtggacccgcagccccatcatgcagggcaacacggagcagcaccaactcgcc
 841 ctcatcagtcagctctgcggctccatcacccctgaggtgtggccaaacgtggacaactat
 901 gagctgtacgaaaagctggagctggtcaagggccagaagcggaaggtgaaggacaggctg
 961 aaggcctatgtgcgtgacccatacgcactggacctcatcgacaagctgctggtgctggac
1021 cctgcccagcgcatcgacagcgatgacgccctcaaccacgacttcttctggtccgacccc
1081 atgccctccgacctcaagggcatgctctccacccacctgacgtccatgttcgagtacttg
1141 gcaccaccgcgccggaagggcagccagatcacccagcagtccaccaaccagagtcgcaat
1201 cccgccaccaccaaccagacggagtttgagcgcgtcttctgagggccggcgcttgccact
1261 agggctcttgtgtttttttcttctgctatgtgacttgcatcgtggagacagggcatttg
1321 agtttatatctctcatgcatattttatttaatccccaccctgggctctgggagcagcccg
1381 ctgagtggactggagtggagcattggctgagagaccaggagggcactggagctgtcttgt
1441 ccttgctggttttctggatggttcccagagggtttccatggggtaggaggatgggctcgc
1501 ccaccagtgactttttctaagagctcccggcgtggtggaagaggggacaggtccctcacc
1561 cacccacaatcctattctcgggctgagaaccctgcgtggggacagggctcgcctcaggaa
1621 tgggctgtttttggcctaaccctcagaaacactggggctggcacaaactcttggtttctt
1681 caacaggagaattttactgtgtttcttttggttccattgtttggagacattcctgggcac
1741 agtttggtccgttagaattaaaagttgaatttttttttttttaaattttttttttcct
1801 ccaggacttgtgtgttttgttctgcgcacacaccgccaactgttcccccacagtcagcag
1861 caggttgggcctgaccattgggacttgattgtcaagtcactggaggtcttgacttttta
1921 tctcagttcatgttctcttccataattggaaaggacctttgtctgtttttcctcttgggt
1981 gccttccagaacgcatctcatgtccctggtgagggaattggtgagggcctgctgtgagct

2041 gctgtggctgcgatggtcacccagctgggcaaatcactggagtgacaatttgacctgtca
2101 cctgagaaggatggtccctcagactgctgggtagagggcctggggcaggctggagagaga
2161 aagtgggcagagggtgaagggatcacaggggtcttggaaggtggcagtagtttggacggg
2221 ggtggggagtatgtgggagaaaaaaacagactgaaggtagaatccttgggaacctttgag
2281 gagcggcacattctggcaggcacgttttctgtgagcctgaagttaggaagagacattctg
2341 gaggtcaatttcctacatcctcttacaggcggagaccttgaagtggggccaggaaggaag
2401 gttggcaaaacctttgaccagaactgtccttcatttacagaaactgacccagaccacaac
2461 acaaaaggccag
```

The coding region ranges from nucleotide 124 to nucleotide 1242.

SEQ ID No. 8:

Amino acid sequence of homo sapiens CDK9:

```
MAKQYDSVECPFCDEVSKYEKLAKIGQGTFGEVFKARHRKTGQKVALKKVLMENEKEGFPITALREIKILQLLKHEN
VVNLIEICRTKASPYNRCKGSIYLVFDFCEHDLAGLLSNVLVKFTLSEIKRVMQMLLNGLYYIHRNKILHRDMKAAN
VLITRDGVLKLADFGLARAFSLAKNSQPNRYTNRVVTLWYRPPELLLGERDYGPPIDLWGAGCIMAEMWTRSPIMQG
NTEQHQLALISQLCGSITPEVWPNVDNYELYEKLELVKGQKRKVKDRLKAYVRDPYALDLIDKLLVLDPAQRIDSDD
ALNHDFFWSDPMPSDLKGMLSTHLTSMFEYLAPPRRKGSQITQQSTNQSRNPATTNQTEFERVF
```

SEQ ID No. 9:

Nucleotide sequence encoding homo sapiens cyclinT1 (accession number NMC_001240.2):

```
   1 gggaagatac acggttttta agaaagaact tctaacccca ctccaccgcc caacggtcac
  61 gtgacgcgcc tgcgtcctcg cctcaaggtt ttttctggtc gcatattggc tgttaactcg
 121 gctacggggt gtagcgaggt gcattccgga agtaggtcct ttgacttttg cttcctctac
 181 ggaggcaagt aacaacccgg cggtcgacgc ttagcggaag ttcgtcaagt cgcagttgcc
 241 cccgcacagc ggatgtgggt cgcctcttag gtgacgctgg gaagtgcctg caaccttcgc
 301 cgctgccttc tggttgaagc actatggagg gagagaggaa gaacaacaac aaacggtggt
 361 atttcactcg agaacagctg gaaaatagcc catcccgtcg ttttggcgtg gacccagata
 421 aagaactttc ttatcgccag caggcggcca atctgcttca ggacatgggg cagcgtctta
 481 acgtctcaca attgactatc aacactgcta tagtatacat gcatcgattc tacatgattc
 541 agtccttcac acagttccct ggaaattctg tggctccagc agccttgttt ctagcagcta
 601 aagtggagga gcagcccaaa aaattggaac atgtcatcaa ggtagcacat acttgtctcc
 661 atcctcagga atcccttcct gatactagaa gtgaggctta tttgcaacaa gttcaagatc
 721 tggtcatttt agaaagcata attttgcaga ctttaggctt tgaactaaca attgatcacc
 781 cacatactca tgtagtaaag tgcactcaac ttgttcgagc aagcaaggac ttagcacaga
 841 cttcttactt catggcaacc aacagcctgc atttgaccac atttagcctg cagtacacac
 901 ctcctgtggt ggcctgtgtc tgcattcacc tggcttgcaa gtggtccaat tgggagatcc
 961 cagtctcaac tgacgggaag cactggtggg agtatgttga cgccactgtg accttggaac
1021 ttttagatga actgacacat gagtttctac agattttgga gaaaactccc aacaggctca
1081 aacgcatttg gaattggagg gcatgcgagg ctgccaagaa aacaaaagca gatgaccgag
1141 gaacagatga aaagacttca gagcagacaa tcctcaatat gatttcccag agctcttcag
1201 acacaaccat tgcaggttta atgagcatgt caacttctac cacaagtgca gtgccttccc
1261 tgccagtctc cgaagagtca tccagcaact taaccagtgt ggagatgttg ccgggcaagc
1321 gttggctgtc ctcccaacct tctttcaaac tagaacctac tcagggtcat cggactagtg
1381 agaatttagc acttacagga gttgatcatt ccttaccaca ggatggttca aatgcattta
1441 tttcccagaa gcagaatagt aagagtgtgc catcagctaa agtgtcactg aaagaatacc
1501 gcgcgaagca tgcagaagaa ttggctgccc agaagaggca actggagaac atggaagcca
1561 atgtgaagtc acaatatgca tatgctgccc agaatctcct ttctcatcat gatagccatt
1621 cttcagtcat tctaaaaatg cccatagagg gttcagaaaa ccccgagcgg ccttttctgg
1681 aaaaggctga caaaacagct ctcaaaatga gaatcccagt ggcaggtgga gataaagctg
1741 cgtcttcaaa accagaggag ataaaaatgc gcataaaagt ccatgctgca gctgataagc
1801 acaattctgt agaggacagt gttacaaaga gccgagagca caaagaaaag cacaagactc
1861 acccatctaa tcatcatcat catcataatc accactcaca caagcactct cattcccaac
1921 ttccagttgg tactgggaac aaacgtcctg gtgatccaaa acatagtagc cagacaagca
1981 acttagcaca taaaacctat agcttgtcta gttctttttc ctcttccagt tctactcgta

2041 aaaggggacc ctctgaagag actggagggg ctgtgtttga tcatccagcc aagattgcca
2101 agagtactaa atcctcttcc ctaaatttct ccttcccttc acttcctaca atgggtcaga
2161 tgcctgggca tagctcagac acaagtggcc tttccttttc acagcccagc tgtaaaactc
2221 gtgtccctca ttcgaaactg gataaagggc ccactggggc caatggtcac aacacgaccc
2281 agacaataga ctatcaagac actgtgaata tgcttcactc cctgctcagt gcccagggtg
2341 ttcagcccac tcagcccact gcatttgaat ttgttcgtcc ttatagtgac tatctgaatc
2401 ctcggtctgg tggaatctcc tcgagatctg gcaatacaga caaaccccgg ccaccacctc
2461 tgccatcaga acctcctcca ccacttccac cccttcctaa gtaaaaaaag aaaaagaaga
2521 ggagaaaaaa acttctttaa aaaaacacat aatttttctt tttttttt
```

The coding region ranges from nucleotide 324 to nucleotide 2504.

SEQ ID No. 10:

Amino acid sequence of homo sapiens CyclinT1:

MEGERKNNNKRWYFTREQLENSPSRRFGVDPDKELSYRQQAANLLQDMGQRLNVSQLTINTAIVYMHRFYMIQSFTQ
FPGNSVAPAALFLAAKVEEQPKKLEHVIKVAHTCLHPQESLPDTRSEAYLQQVQDLVILESIILQTLGFELTIDHPH
THVVKCTQLVRASKDLAQTSYFMATNSLHLTTFSLQYTPPVVACVCIHLACKWSNWEIPVSTDGKHWWEYVDATVTL
ELLDELTHEFLQILEKTPNRLKRIWNWRACEAAKKTKADDRGTDEKTSEQTILNMISQSSSDTTIAGLMSMSTSTTS
AVPSLPVSEESSSNLTSVEMLPGKRWLSSQPSFKLEPTQGHRTSENLALTGVDHSLPQDGSNAFISQKQNSKSVPSA
KVSLKEYRAKHAEELAAQKRQLENMEANVKSQYAYAAQNLLSHHDSHSSVILKMPIEGSENPERPFLEKADKTALKM
RIPVAGGDKAASSKPEEIKMRIKVHAAADKHNSVEDSVTKSREHKEKHKTHPSNHHHHHNHHSHKHSHSQLPVGTGN
KRPGDPKHSSQTSNLAHKTYSLSSSFSSSSSTRKRGPSEETGGAVFDHPAKIAKSTKSSSLNFSFPSLPTMGQMPGH
SSDTSGLSFSQPSCKTRVPHSKLDKGPTGANGHNTTQTIDYQDTVNMLHSLLSAQGVQPTQPTAFEFVRPYSDYLNP
RSGGISSRSGNTDKPRPPPLPSEPPPPLPPLPK

SEQ ID No. 11:

Nucleotide sequence encoding homo sapiens Notch3.

```
   1 gcggcgcgga ggctggcccg ggacgcgccc ggagcccagg gaaggaggga ggaggggagg
  61 gtcgcggccg gccgccatgg ggccggggc ccgtggccgc cgccgccgcc gtcgcccgat
 121 gtcgccgcca ccgccaccgc cacccgtgcg ggcgctgccc ctgctgctgc tgctagcggg
 181 gccggggct gcagcccccc cttgcctgga cggaagcccg tgtgcaaatg gaggtcgttg
 241 cacccagctg ccctcccggg aggctgcctg cctgtgcccg cctggctggg tgggtgagcg
 301 gtgtcagctg gaggacccct gtcactcagg cccctgtgct ggccgtggtg tctgccagag
 361 ttcagtggtg gctggcaccg cccgattctc atgccggtgc cccgtggct tccgaggccc
 421 tgactgctcc ctgccagatc cctgcctcag cagcccttgt gcccacggtg cccgctgctc
 481 agtgggcccc gatggacgct tcctctgctc ctgcccacct ggctaccagg gccgcagctg
 541 ccgaagcgac gtggatgagt gccgggtggg tgagccctgc cgccatggtg gcacctgcct
 601 caacacacct ggctccttcc gctgccagtg tccagctggc tacacagggc cactatgtga
 661 gaaccccgcg gtgccctgtg caccctcacc atgccgtaac gggggcacct gcaggcagag
 721 tggcgacctc acttacgact gtgcctgtct tcctgggttt gagggtcaga attgtgaagt
 781 gaacgtggac gactgtccag gacaccgatg tctcaatggg gggacatgcg tggatggcgt
 841 caacacctat aactgccagt gccctcctga gtggacaggc cagttctgca cggaggacgt
 901 ggatgagtgt cagctgcagc ccaacgcctg ccacaatggg ggtacctgct tcaacacgct
 961 gggtggccac agctgcgtgt gtgtcaatgg ctggacaggc gagagctgca gtcagaatat
1021 cgatgactgt gccacagccg tgtgcttcca tggggccacc tgccatgacc gcgtggcttc
1081 tttctactgt gcctgcccca tgggcaagac tggcctcctg tgtcacctgg atgacgcctg
1141 tgtcagcaac ccctgccacg aggatgctat ctgtgacaca aatccggtga acggccgggc
1201 catttgcacc tgtcctcccg gcttcacggg tggggcatgt gaccaggatg tggacgagtg
1261 ctctatcggc gccaacccct gcgagcactt gggcaggtgc gtgaacacgc agggctcctt
1321 cctgtgccag tgcggtcgtg gctacactgg acctcgctgt gagaccgatg tcaacgagtg
1381 tctgtcgggg ccctgccgaa accaggccac gtgcctcgac cgcataggcc agttcacctg
1441 tatctgtatg gcaggcttca caggaaccta ttgcgaggtg gacattgacg agtgtcagag
1501 tagcccctgt gtcaacggtg ggtctgcaa ggaccgagtc aatggcttca gctgcacctg
1561 cccctcgggc ttcagcggct ccacgtgtca gctggacgtg gacgaatgcg ccagcacgcc
1621 ctgcaggaat ggcgccaaat gcgtggacca gcccgatggc tacgagtgcc gctgtgccga
```

1681 gggctttgag ggcacgctgt gtgatcgcaa cgtggacgac tgctcccctg acccatgcca
1741 ccatggtcgc tgcgtggatg gcatcgccag cttctcatgt gcctgtgctc ctggctacac
1801 gggcacacgc tgcgagagcc aggtggacga atgccgcagc cagccctgcc gccatggcgg
1861 caaatgccta gacctggtgg acaagtacct ctgccgctgc ccttctggga ccacaggtgt
1921 gaactgcgaa gtgaacattg acgactgtgc cagcaacccc tgcacctttg gagtctgccg
1981 tgatggcatc aaccgctacg actgtgtctg ccaacctggc ttcacagggc cccttgtaa
2041 cgtggagatc aatgagtgtg cttccagccc atgcggcgag ggaggttcct gtgtggatgg
2101 ggaaaatggc ttccgctgcc tctgcccgcc tggctccttg cccccactct gcctcccccc
2161 gagccatccc tgtgcccatg agccctgcag tcacggcatc tgctatgatg cacctggcgg
2221 gttccgctgt gtgtgtgagc ctggctggag tggcccccgc tgcagccaga gcctggcccg
2281 agacgcctgt gagtcccagc cgtgcagggc cggtgggaca tgcagcagcg atggaatggg
2341 tttccactgc acctgcccgc ctggtgtcca gggacgtcag tgtgaactcc tctcccccctg
2401 caccccgaac cctgtgagc atgggggccg ctgcgagtct gcccctggcc agctgcctgt
2461 ctgctcctgc cccagggct ggcaaggccc acgatgccag caggatgtgg acgagtgtgc
2521 tggccccgca ccctgtggcc ctcatggtat ctgcaccaac ctggcaggga gtttcagctg
2581 cacctgccat ggagggtaca ctggcccttc ctgcgatcag gacatcaatg actgtgaccc
2641 caacccatgc ctgaacggtg gctcgtgcca agacggcgtg ggctcctttt cctgctcctg
2701 cctccctggt ttcgccggcc cacgatgcgc ccgcgatgtg gatgagtgcc tgagcaaccc
2761 ctgcggcccg ggcacctgta ccgaccacgt ggcctccttc acctgcacct gcccgccagg
2821 ctacggaggc ttccactgcg aacaggacct gcccgactgc agccccagct cctgcttcaa
2881 tggcgggacc tgtgtggacg gcgtgaactc gttcagctgc ctgtgccgtc ccggctacac
2941 aggagcccac tgccaacatg aggcagaccc ctgcctctcg cggccctgcc tacacggggg
3001 cgtctgcagc gccgcccacc ctggcttccg ctgcacctgc ctcgagagct tcacgggccc
3061 gcagtgccag acgctggtgg attggtgcag ccgccagcct tgtcaaaacg gggtcgctg
3121 cgtccagact ggggcctatt gcctttgtcc ccctggatgg agcggacgcc tctgtgacat
3181 ccgaagcttg ccctgcaggg aggccgcagc ccagatcggg gtgcggctgg agcagctgtg
3241 tcaggcgggt gggcagtgtg tggatgaaga cagctcccac tactgcgtgt gcccagaggg
3301 ccgtactggt agccactgtg agcaggaggt ggacccctgc ttggcccagc cctgccagca
3361 tggggggacc tgccgtggct atatgggggg ctacatgtgt gagtgtcttc ctggctacaa
3421 tggtgataac tgtgaggacg acgtggacga gtgtgcctcc cagccctgcc agcacgggggg
3481 ttcatgcatt gacctcgtgg cccgctatct ctgctcctgt cccccaggaa cgctgggggt
3541 gctctgcgag attaatgagg atgactgcgg cccaggccca ccgctggact cagggccccg
3601 gtgcctacac aatggcacct gcgtggacct ggtgggtggt ttccgctgca cctgtccccc
3661 aggatacact ggtttgcgct gcgaggcaga catcaatgag tgtcgctcag gtgcctgcca
3721 cgcggcacac acccgggact gcctgcagga cccaggcgga ggtttccgtt gcctttgtca
3781 tgctggcttc tcaggtcctc gctgtcagac tgtcctgtct ccctgcgagt cccagccatg
3841 ccagcatgga ggccagtgcc gtcctagccc gggtcctggg ggtgggctga ccttcacctg
3901 tcactgtgcc cagccgttct ggggtccgcg ttgcgagcgg gtggcgcgct cctgccggga
3961 gctgcagtgc ccggtgggcg tcccatgcca gcagacgccc cgcgggccgc gctgcgcctg
4021 ccccccaggg ttgtcgggac cctcctgccg cagcttcccg gggtcgccgc cgggggccag
4081 caacgccagc tgcgcggccg cccctgtct ccacgggggc tcctgccgcc ccgcgccgct
4141 cgcgcccttc ttccgctgcg cttgcgcgca gggctggacc gggccgcgct gcgaggcgcc
4201 cgccgcggca cccgaggtct cggaggagcc gcggtgcccg cgcgccgcct gccaggccaa
4261 gcgcggggac cagcgctgcg accgcgagtg caacagccca ggctgcggct gggacggcgg
4321 cgactgctcg ctgagcgtgg gcgacccctg gcggcaatgc gaggcgctgc agtgctggcg
4381 cctcttcaac aacagccgct gcgaccccgc ctgcagctcg cccgcctgcc tctacgacaa
4441 cttcgactgc cacgccggtg gccgcgagcg cacttgcaac ccggtgtacg agaagtactg
4501 cgccgaccac tttgccgacg gccgctgcga ccagggctgc aacacggagg agtgcggctg
4561 ggatgggctg gattgtgcca gcgaggtgcc ggccctgctg gcccgcggcg tgctggtgct
4621 cacagtgctg ctgccgccag aggagctact gcgttccagc gccgactttc tgcagcggct
4681 cagcgccatc ctgcgcacct cgctgcgctt ccgcctggac gcgcacggcc aggccatggt
4741 cttcccttac caccggccta gtcctggctc cgaaccccgg gcccgtcggg agctggcccc
4801 cgaggtgatc ggctcggtag taatgctgga gattgacaac cggctctgcc tgcagtcgcc
4861 tgagaatgat cactgcttcc ccgatgccca gagcgccgct gactacctgg gagcgttgtc
4921 agcggtggag cgcctggact tcccgtaccc actgcgggac gtgcgggggg agccgctgga
4981 gcctccagaa cccagcgtcc cgctgctgcc actgctagtg gcgggcgctg tcttgctgct
5041 ggtcattctc gtcctgggtg tcatggtggc ccggcgcaag cgcgagcaca gcaccctctg
5101 gttccctgag ggcttctcac tgcacaagga cgtggcctct ggtcacaagg gccggcggga
5161 acccgtgggc caggacgcgc tgggcatgaa gaacatggcc aagggtgaga gcctgatggg
5221 ggaggtggcc acagactgga tggacacaga gtgcccagag gccaagcggc taaaggtaga

```
5281 ggagccaggc atgggggctg aggaggctgt ggattgccgt cagtggactc aacaccatct
5341 ggttgctgct gacatccgcg tggcaccagc catggcactg acaccaccac agggcgacgc
5401 agatgctgat ggcatggatg tcaatgtgcg tggcccagat ggcttcaccc cgctaatgct
5461 ggcttccttc tgtggggggg ctctggagcc aatgccaact gaagaggatg aggcagatga
5521 cacatcagct agcatcatct ccgacctgat ctgccagggg gctcagcttg ggcacggac
5581 tgaccgtact ggcgagactg ctttgcacct ggctgcccgt tatgcccgtg ctgatgcagc
5641 caagcggctg ctggatgctg ggcagacac caatgcccag gaccactcag gccgcactcc
5701 cctgcacaca gctgtcacag ccgatgccca gggtgtcttc cagattctca tccgaaaccg
5761 ctctacagac ttggatgccc gcatggcaga tggctcaacg gcactgatcc tggcggcccg
5821 cctggcagta gagggcatgg tggaagagct catcgccagc catgctgatg tcaatgctgt
5881 ggatgagctt gggaaatcag ccttacactg ggctgcggct gtgaacaacg tggaagccac
5941 tttggccctg ctcaaaaatg gagccaataa ggacatgcag gatagcaagg aggagacccc
6001 cctattcctg gccgcccgcg agggcagcta tgaggctgcc aagctgctgt tggaccactt
6061 tgccaaccgt gagatcaccg accacctgga caggctgccg cgggacgtag cccaggagag
6121 actgcaccag gacatcgtgc gcttgctgga tcaacccagt gggccccgca gcccccccgg
6181 tccccacggc ctggggcctc tgctctgtcc tccaggggcc ttcctccctg gcctcaaagc
6241 ggcacagtcg gggtccaaga agagcaggag gccccccggg aaggcggggc tggggccgca
6301 ggggccccgg gggcggggca agaagctgac gctggcctgc ccgggccccc tggctgacag
6361 ctcggtcacg ctgtcgcccg tggactcgct ggactccccg cggcctttcg gtgggccccc
6421 tgcttcccct ggtggcttcc cccttgaggg gccctatgca gctgccactg ccactgcagt
6481 gtctctggca cagcttggtg gcccaggccg ggcgggtcta gggcgccagc cccctggagg
6541 atgtgtactc agcctgggcc tgctgaaccc tgtggctgtg cccctcgatt gggcccggct
6601 gcccccacct gcccctccag gcccctcgtt cctgctgcca ctggcgccgg gaccccagct
6661 gctcaaccca gggacccccg tctccccgca ggagcggccc ccgccttacc tggcagtccc
6721 aggacatggc gaggagtacc cggcggctgg ggcacacagc agcccccaa aggcccgctt
6781 cctgcgggtt cccagtgagc acccttacct gaccccatcc cccgaatccc ctgagcactg
6841 ggccagcccc tcacctccct ccctctcaga ctggtccgaa tccacgccta gcccagccac
6901 tgccactggg gccatggcca ccaccactgg ggcactgcct gcccagccac ttcccttgtc
6961 tgttcccagc tcccttgctc aggcccagac ccagctgggg ccccagccgg aagttacccc
7021 caagaggcaa gtgttggcct gagacgctcg tcagttctta gatcttgggg gcctaaagag
7081 acccccgtcc tgcctccttt ctttctctgt ctcttccttc cttttagtct tttcatcct
7141 cttctctttc caccaaccct cctgcatcct tgccttgcag cgtgaccgag ataggtcatc
7201 agcccagggc ttcagtcttc ctttatttat aatgggtggg ggctaccacc caccctctca
7261 gtcttgtgaa gagtctggga cctccttctt ccccacttct ctcttccctc attcctttct
7321 ctctccttct ggcctctcat ttccttacac tctgacatga atgaattatt attattttta
7381 tttttctttt tttttttaca ttttgtatag aaacaaattc atttaaacaa acttattatt
7441 attatttttt acaaaatata tatatggaga tgctccctcc ccctgtgaac cccccagtgc
7501 ccccgtgggg ctgagtctgt gggcccattc ggccaagctg gattctgtgt acctagtaca
7561 caggcatgac tgggatcccg tgtaccgagt acacgaccca ggtatgtacc aagtaggcac
7621 ccttgggcgc acccactggg gccaggggtc gggggagtgt tgggagcctc ctccccaccc
7681 cacctccctc acttcactgc attccagatg ggacatgttc catagccttg ctggggaagg
7741 gcccactgcc aactccctct gccccagccc caccccttggc catctccctt tgggaactag
7801 ggggctgctg gtgggaaatg ggagccaggg cagatgtatg cattcctttg tgtccctgta
7861 aatgtgggac tacaagaaga ggagctgcct gagtggtact ttctcttcct ggtaatcctc
7921 tggcccagcc tcatggcaga atagaggtat ttttaggcta tttttgtaat atggcttctg
7981 gtcaaaatcc ctgtgtagct gaattcccaa gccctgcatt gtacagcccc ccactcccct
8041 caccacctaa taaaggaata gttaacactc aaaaaaaaaa aaaaaaaaa
```

The coding region ranges from nucleotide 77 to nucleotide 7042.

SEQ ID No. 12:

Amino acid sequence of homo sapiens Notch3:

```
MGPGARGRRRRRRPMSPPPPPPPVRALPLLLLLAGPGAAAPPCLDGSPCANGGRCTQLPSREAACLCPPGWVGERCQ
LEDPCHSGPCAGRGVCQSSVVAGTARFSCRCPRGFRGPDCSLPDPCLSSPCAHGARCSVGPDGRFLCSCPPGYQGRS
CRSDVDECRVGEPCRHGGTCLNTPGSFRCQCPAGYTGPLCENPAVPCAPSPCRNGGTCRQSGDLTYDCACLPGFEGQ
```

NCEVNVDDCPGHRCLNGGTCVDGVNTYNCQCPPEWTGQFCTEDVDECQLQPNACHNGGTCFNTLGGHSCVCVNGWTG
ESCSQNIDDCATAVCFHGATCHDRVASFYCACPMGKTGLLCHLDDACVSNPCHEDAICDTNPVNGRAICTCPPGFTG
GACDQDVDECSIGANPCEHLGRCVNTQGSFLCQCGRGYTGPRCETDVNECLSGPCRNQATCLDRIGQFTCICMAGFT
GTYCEVDIDECQSSPCVNGGVCKDRVNGFSCTCPSGFSGSTCQLDVDECASTPCRNGAKCVDQPDGYECRCAEGFEG
TLCDRNVDDCSPDPCHHGRCVDGIASFSCACAPGYTGTRCESQVDECRSQPCRHGGKCLDLVDKYLCRCPSGTTGVN
CEVNIDDCASNPCTFGVCRDGINRYDCVCQPGFTGPLCNVEINECASSPCGEGGSCVDGENGFRCLCPPGSLPPLCL
PPSHPCAHEPCSHGICYDAPGGFRCVCEPGWSGPRCSQSLARDACESQPCRAGGTCSSDGMGFHCTCPPGVQGRQCE
LLSPCTPNPCEHGGRCESAPGQLPVCSCPQGWQGPRCQQDVDECAGPAPCGPHGICTNLAGSFSCTCHGGYTGPSCD
QDINDCDPNPCLNGGSCQDGVGSFSCSCLPGFAGPRCARDVDECLSNPCGPGTCTDHVASFTCTCPPGYGGFHCEQD
LPDCSPSSCFNGGTCVDGVNSFSCLCRPGYTGAHCQHEADPCLSRPCLHGGVCSAAHPGFRCTCLESFTGPQCQTLV
DWCSRQPCQNGGRCVQTGAYCLCPPGWSGRLCDIRSLPCREAAAQIGVRLEQLCQAGGQCVDEDSSHYCVCPEGRTG
SHCEQEVDPCLAQPCQHGGTCRGYMGGYMCECLPGYNGDNCEDDVDECASQPCQHGGSCIDLVARYLCSCPPGTLGV
LCEINEDDCGPGPPLDSGPRCLHNGTCVDLVGGFRCTCPPGYTGLRCEADINECRSGACHAAHTRDCLQDPGGGFRC
LCHAGFSGPRCQTVLSPCESQPCQHGGQCRPSPGPGGGLTFTCHCAQPFWGPRCERVARSCRELQCPVGVPCQQTPR
GPRCACPPGLSGPSCRSFPGSPPGASNASCAAAPCLHGGSCRPAPLAPFFRCACAQGWTGPRCEAPAAAPEVSEEPR
CPRAACQAKRGDQRCDRECNSPGCGWDGGDCSLSVGDPWRQCEALQCWRLFNNSRCDPACSSPACLYDNFDCHAGGR
ERTCNPVYEKYCADHFADGRCDQGCNTEECGWDGLDCASEVPALLARGVLVLTVLLPPEELLRSSADFLQRLSAILR
TSLRFRLDAHGQAMVFPYHRPSPGSEPRARRELAPEVIGSVVMLEIDNRLCLQSPENDHCFPDAQSAADYLGALSAV
ERLDFPYPLRDVRGEPLEPPEPSVPLLPLLVAGAVLLLVILVLGVMVARRKREHSTLWFPEGFSLHKDVASGHKGRR
EPVGQDALGMKNMAKGESLMGEVATDWMDTECPEAKRLKVEEPGMGAEEAVDCRQWTQHHLVAADIRVAPAMALTPP
QGDADADGMDVNVRGPDGFTPLMLASFCGGALEPMPTEEDEADDTSASIISDLICQGAQLGARTDRTGETALHLAAR
YARADAAKRLLDAGADTNAQDHSGRTPLHTAVTADAQGVFQILIRNRSTDLDARMADGSTALILAARLAVEGMVEEL
IASHADVNAVDELGKSALHWAAAVNNVEATLALLKNGANKDMQDSKEETPLFLAAREGSYEAAKLLLDHFANREITD
HLDRLPRDVAQERLHQDIVRLLDQPSGPRSPPGPHGLGPLLCPPGAFLPGLKAAQSGSKKSRRPPGKAGLGPQGPRG
RGKKLTLACPGPLADSSVTLSPVDSLDSPRPFGGPPASPGGFPLEGPYAAATATAVSLAQLGGPGRAGLGRQPPGGC
VLSLGLLNPVAVPLDWARLPPPAPPGPSFLLPLAPGPQLLNPGTPVSPQERPPPYLAVPGHGEEYPAAGAHSSPPKA
RFLRVPSEHPYLTPSPESPEHWASPSPPSLSDWSESTPSPATATGAMATTTGALPAQPLPLSVPSSLAQAQTQLGPQ
PEVTPKRQVLA

All references cited herein are fully incorporated by reference. Having now fully described the invention, it will be understood by a person skilled in the art that the invention may be practiced within a wide and equivalent range of conditions, parameters and the like, without affecting the spirit or scope of the invention or any embodiment thereof.

## Claims

1. A CDK9 inhibitor for use in treating, ameliorating and/or preventing midline carcinoma.

2. A method for treating, preventing or ameliorating midline carcinoma comprising the administration of a CDK9 inhibitor to a subject in need of such a treatment, prevention or amelioration.

**3.** The CDK9 inhibitor for use in treating, ameliorating and/or preventing midline carcinoma according to claim 1, or the method for treating, preventing or ameliorating midline carcinoma according to claim 2, wherein said midline carcinoma is NUT midline carcinoma (NMC).

**4.** The CDK9 inhibitor for use in treating, ameliorating and/or preventing midline carcinoma according to claim 1 or 3, or the method for treating, preventing or ameliorating midline carcinoma according to claim 2 or 3, wherein said CDK9 inhibitor is a selective CDK9 inhibitor.

**5.** The CDK9 inhibitor for use according to claim 4, or the method according to claim 4, wherein said selective CDK9 inhibitor is a compound having the general formula (I)

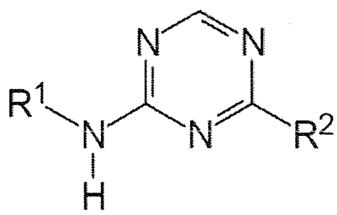

Formula (I)

wherein
$R^1$ is

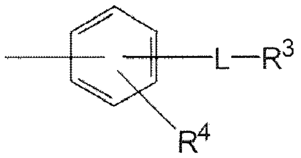 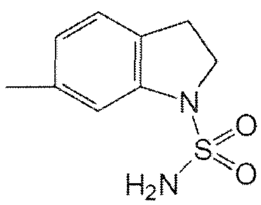 or 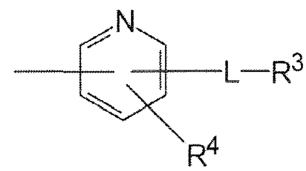

**L** is a bond or $-CR^5R^6-$, $-CR^5R^6-CR^7R^8-$, $-CR^5R^6-CR^7R^8-CR^9R^{10}-$, $-CR^5R^6-CR^7R^8-CR^9R^{10}-CR^{11}R^{12}-$,
**$R^5$-$R^{12}$** represent independently of each other -H, $-CH_3$, $-C_2H_5$, $-C_3H_7$, -F, -Cl, -Br, -I;
**$R^3$** is selected from -H, $NO_2$, $-NH_2$, -CN, -F, -Cl, -Br, -I, $-CH_3$, $-C_2H_5$, -Ph, $-C_3H_7$, $-CH(CH_3)_2$, $-C_4H_9$, $-CH_2-CH(CH_3)_2$, $-CH(CH_3)-C_2H_5$, $-C(CH_3)_3$, $-O-CH_3$, $-O-C_2H_5$, $-O-C_3H_7$, $-O-CH(CH_3)_2$, $-O-C_4H_9$, $-O-CH_2-CH(CH_3)_2$, $-O-CH(CH_3)-C_2H_5$, $-O-C(CH_3)_3$, $-CR^{13}R^{14}R^{21}$, $-CR^{13}R^{14}-CR^{15}R^{16}R^{21}$, $-O-CR^{13}R^{14}R^{21}$, $-CR^{13}R^{14}-CR^{15}R^{16}-CR^{17}R^{18}R^{21}$, $-CR^{13}R^{14}-CR^{15}R^{16}-CR^{17}R^{18}-CR^{19}R^{20}R^{21}$, $-O-CR^{13}R^{14}-CR^{15}R^{16}R^{12}$, $-O-CR^{13}R^{14}-CR^{15}R^{16}-CR^{17}R^{18}R^{21}$, $-SO_2-R^{22}$, $-CONR^{13}R^{24}$, $-NR^{25}COR^{22}$, $-O-CR^{13}R^{14}-R^{15}R^{16}-CR^{17}R^{18}-CR^{19}R^{20}R^{21}$, $-NR^{25}SO_2NR^{23}R^{24}$, $-NR^{25}SO_2R^{22}$, $-NR^{25}CONR^{23}R^{24}$, $-SO_2NR^{23}R^{24}$, $-SO(NR^{26})R^{27}$, -NH-CO-NH-Ph;
**$R^{13}$ - $R^{21}$, $R^{29}$ - $R^{32}$** and **$R^{33}$ - $R^{48}$** represent independently of each other -H, -F, -Cl, -Br, -I;
**$R^{26}$** is -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CR^{13}R^{14}R^{21}$, $-COR^{28}$, $-CR^{13}R^{14}-CR^{15}R^{16}R^{21}$, $-CR^{13}R^{14}-CR^{15}R^{16}-CR^{17}R^{18}-CR^{19}R^{20}-CR^{29}R^{30}R^{21}$, $-CR^{13}R^{14}-CR^{15}R^{16}-CR^{17}R^{18}R^{21}$, $-CR^{13}R^{14}-CR^{15}R^{16}-CR^{17}R^{18}-CR^{19}R^{20}R^{21}$, $-CR^{13}R^{14}-CR^{15}R^{16}-CR^{17}R^{18}-CR^{19}R^{20}-CR^{29}R^{30}-R^{31}R^{32}R^{21}$, $-COOR^{28}$,

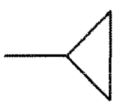 , 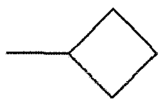 , 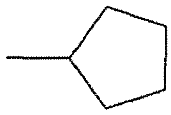 , 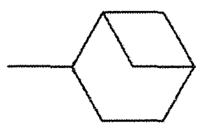 ,

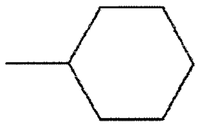, 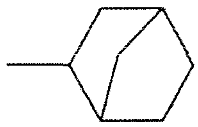, 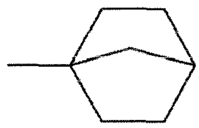, 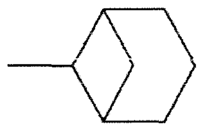,

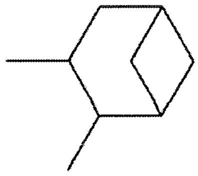, 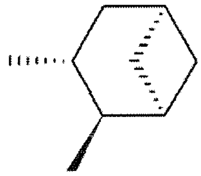, 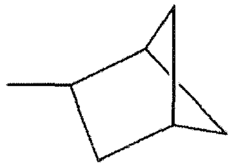, 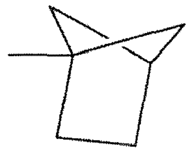,

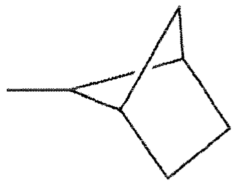, 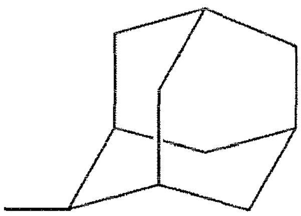, 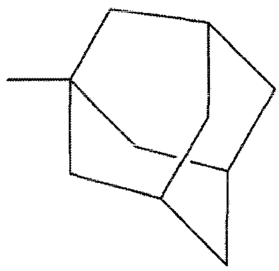,

these $C_3$-$C_6$-cycloalkyl groups may further be substituted by one, two, three, four, five or more substituents selected from the group consisting of $R^{33}$ - $R^{48}$;

**$R^{22}$**, **$R^{27}$**, and **$R^{28}$** are independently selected from $-CR^{49}R^{50}R^{51}$, $-CR^{49}R^{50}$-$CR^{52}R^{53}R^{51}$, $-CR^{49}R^{50}$-$CR^{52}R^{53}$-$CR^{54}R^{55}$-$CR^{56}R^{57}$-$CR^{58}R^{59}R^{51}$, $-CR^{49}R^{50}$-$CR^{52}R^{53}$-$CR^{54}R^{55}R^{51}$, $-CR^{49}R^{50}$-$CR^{52}R^{53}$-$CR^{54}R^{55}$-$CR^{56}R^{57}R^{51}$, $-CR^{49}R^{50}$-$CR^{52}R^{53}$-$CR^{54}R^{55}$-$CR^{56}R^{57}$-$CR^{58}R^{59}$-$CR^{60}R^{61}R^{51}$, $-CH_2Ph$, $-CH_2Ph$ the phenyl group of which may further be substituted by one, two, three, four or five substituents selected from the group consisting of $R^5$ - $R^{12}$; $C_3$-$C_6$-cycloalkyl groups listed for $R^{26}$, which may further be substituted by one, two, three, four, five or more substituents selected from the group consisting of $R^{33}$ - $R^{48}$;

**$R^{49}$ - $R^{61}$** represent independently of each other -H, $-CH_3$, $-C_2H_5$, $-C_3H_7$, $-C_4H_9$, -F, -Cl, -Br, -I, -OH, $-NO_2$, $-NH_2$;

**$R^{23}$** and **$R^{24}$** are independently selected from -H, $-CR^{49}R^{50}R^{51}$ $-CR^{49}R^{50}$-$CR^{52}R^{53}R^{51}$, $-CR^{49}R^{50}$-$CR^{52}R^{53}$-$CR^{54}R^{55}$-$CR^{56}R^{57}$-$CR^{58}R^{59}R^{51}$, $-CR^{49}R^{50}$-$CR^{52}R^{53}$-$CR^{54}R^{55}R^{51}$, $-CR^{49}R^{50}$-$CR^{52}R^{53}$-$CR^{54}R^{55}$-$CR^{56}R^{57}R^{51}$, $-CR^{49}R^{50}$-$CR^{52}R^{53}$-$CR^{54}R^{55}$-$CR^{56}R^{57}$-$CR^{58}R^{59}$-$CR^{60}R^{61}R^{51}$, $-CR^{49}R^{50}$-$CR^{52}R^{53}$-O-$R^{51'}$, $-CR^{49}R^{50}$-$CR^{52}R^{53}$-$CR^{54}R^{55}$-O-$R^{51'}$, $-CR^{49}R^{50}$-$CR^{52}R^{53}$-$NR^{51'}R^{51''}$, $-CR^{49}R^{50}$-$CR^{52}R^{53}$-$CR^{54}R^{55}$-$NR^{51'}R^{51''}$,- $CR^{49}R^{50}$-$CR^{52}R^{53}$-$CR^{54}R^{55}$-$CR^{56}$-$R^{57}$-$NR^{51'}R^{51''}$, $-CR^{49}R^{50}$-$CR^{52}R^{53}$-$CR^{54}R^{55}$-$CR^{56}R^{57}$-$CR^{58}R^{59}$-$NR^{51'}R^{51''}$, phenyl, substituted phenyl, benzyl, substituted benzyl, or both residues **$R^{23}$** and **$R^{24}$** together form with the nitrogen atom to which they are attached a azetidine, pyrrolidine, piperidine, piperazine, azepane, or morpholine ring;

**$R^{51'}$** and **$R^{51''}$** represent independently of each other -H, $-CH_3$, $-C_2H_5$, $-C_3H_7$, $-C_4H_9$, $-CH_2Ph$, $-COOC(CH_3)_3$, $-COOCH_3$, $-COOCH_2CH_3$, $-COOCH_2CH_2CH_3$, $-COOCH(CH_3)_2$, $-COOCH_2Ph$, $-COCH_3$;

and **$R^{25}$** is -H, $-CH_3$, $-C_2H_5$, $-C_3H_7$, $-CH(CH_3)_2$, $-C_4H_9$, $-CH_2$-$CH(CH_3)_2$, $-CH(CH_3)$-$C_2H_5$, $-C(CH_3)_3$;

**$R^4$** is selected from -H, $-NO_2$, $NH_2$, -CN, -F, -Cl, $-B_r$, -I, $-CR^{62}R^{63}R^{64}$,

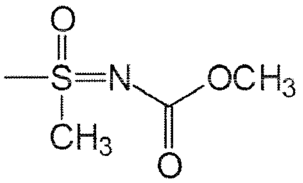 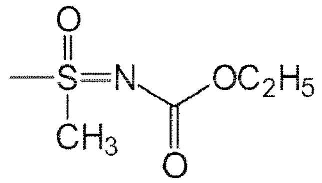 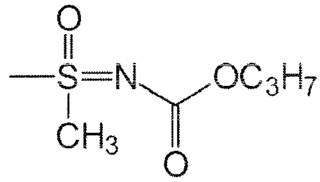

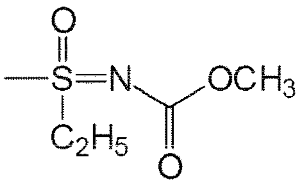 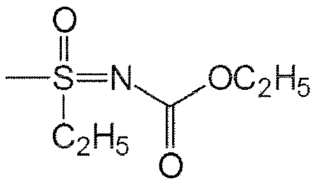 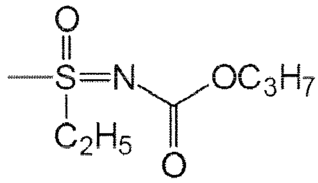

$-CONH_2$, $-SO_2CH_3$, $-S0_2C_2H_5$, $-SO_2C_3H_7$, $-NH-SO_2-CH_3$, $NH-SO_2-C_2H_5$, $-NH-SO_2-C_3H_7$, $-NHCO-CH_3$, $-NHCO-C_2H_5$, $NH-CO-C_3H_7$, $-SO_2NR^{23}R^{24}$, $-CH_2-SO_2NR^{23}R^{24}$,$-C_2H_4-SO_2NR^{23}R^{24}$, $-C_3H_6-SO_2NR^{23}R^{24}$ $-SO_2NH_2$, $-CH_2-SO_2NH_2$, $-C_2H_4-SO_2NH_2$, $-C_3H_6-SO_2NH_2$, $-O-CR^{62}R^{63}-CR^{65}R^{66}R^{64}$, $-O-CR^{62}R^{63}-CR^{65}R^{66}-CR^{67}R^{68}R^{64}$, $-CR^{62}R^{63}-CR^{65}R^{66}-CR^{67}R^{68}-CR^{69}R^{70}R^{64}$, $-O-CR^{62}R^{63}-CR^{65}R^{66}-CR^{67}R^{68}-CR^{69}R^{70}R^{64}$, $-CR^{62}R^{63}-CR^{65}R^{66}-CR^{67}R^{68}R^{64}$, $-O-CR^{62}R^{63}-CR^{65}R^{66}-CR^{67}R^{68}-CR^{69}R^{70}-CR^{71}R^{72}R^{64}$, $-CR^{62}R^{63}-CR^{65}R^{66}R^{64}$, $-O-CR^{62}R^{63}-CR^{65}R^{66}-CR^{67}R^{68}-CR^{69}R^{70}-CR^{71}R^{72}-CR^{73}R^{74}R^{64}$, $-O-CR^{62}R^{63}R^{64}$, $-CR^{62}R^{63}-CR^{65}R^{66}-CR^{67}R^{68}-CR^{69}R^{70}-CR^{71}R^{72}R^{64}$, $-CR^{62}R^{63}-CR^{65}R^{66}-CR^{67}R^{68}-CR^{69}R^{70}-CR^{71}R^{72}-CR^{73}R^{74}R^{64}$, $-OCH_2Ph$,

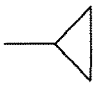 , 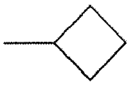 , 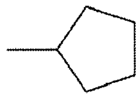 , 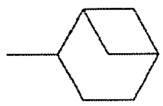 ,

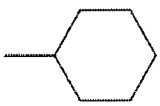 , 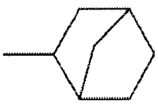 , 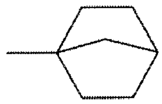 , 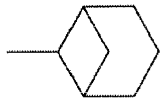 ,

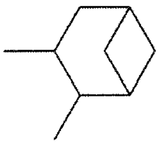 , 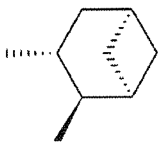 , 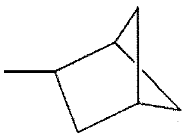 , 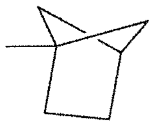 ,

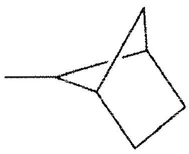 , 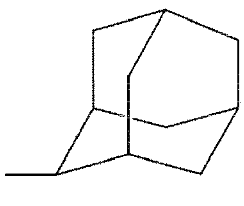 , 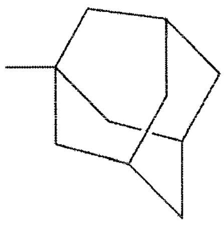 ,

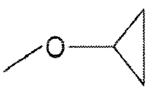 , 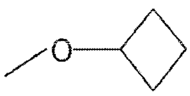 , 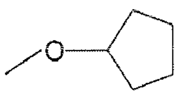 , 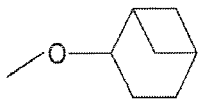 ,

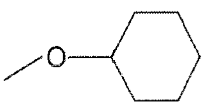 . 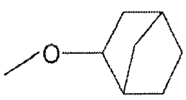 , 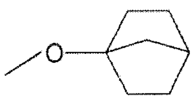 , 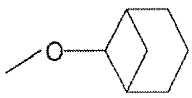 ,

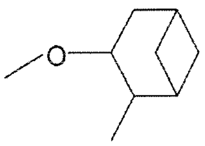,

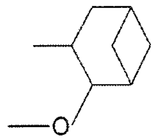,

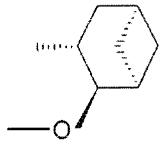,

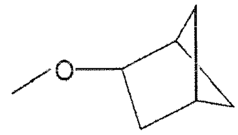,

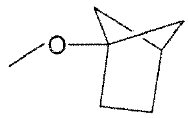,

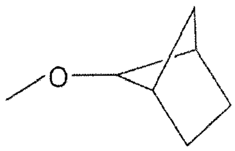

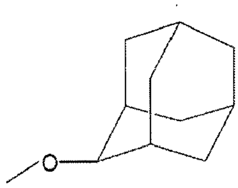,

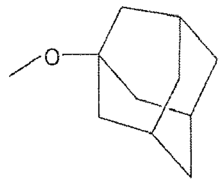,

these $C_3$-$C_6$-cycloalkoxy groups and $C_3$-$C_6$-cycloalkyl groups may further be substituted by one, two, three, four, five or more substituents selected from the group consisting of $R^{33}$-$R^{48}$;

**$R^{62}$-$R^{74}$** represent independently of each other -H, -cyclo-$C_3H_5$, -cyclo-$C_4H_7$, - cyclo-$C_5H_9$, $-CR^{75}R^{76}R^{77}$, $-CR^{75}R^{76}$-$CR^{78}R^{79}R^{77}$, $-CR^{75}R^{76}CR^{78}R^{79}$-$CR^{80}R^{81}R^{77}$, - $CR^{75}R^{76}$-$CR^{78}R^{79}$-$CR^{80}R^{79}$-$CR^{82}R^{82}R^{77}$, -F, -Cl, -Br, -1, -Ph;

**$R^{75}$-$R^{82}$** represent independently of each other -H, -F, -Cl, -Br, -I, $-NH_2$;

**$R^4$** together with $R^{22}$, $R^{23}$, $R^{24}$, or $R^{25}$ may form a group $-CH_2CH_2$- or $-CH_2CH_2CH_2$- if $R^4$ is attached ortho to -L-$R^3$;

**$R^2$** is

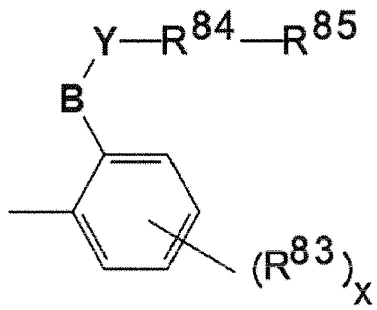

or

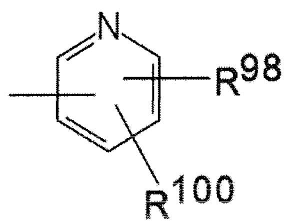

;

**$R^{83}$** is selected from -H, -OH, $-NO_2$, -CN, -F, -Cl, -Br, -I, $-CF_3$, $-NR^{23'}R^{24'}$, $-CR^{62}R^{63}R^{64}$, $-CR^{62}R^{63}$-$NR^{23'}R^{24'}$, $-CR^{62}$-$R^{63}$-$CR^{65}R^{66}R^{64}$, $-CR^{62}R^{63}$-$CR^{65}R^{66}$-$NR^{23'}R^{24'}$, $-CR^{62}R^{63}$-$CR^{65}R^{66}$-$CR^{67}R^{68}R^{64}$, $-CR^{62}R^{63}$-$CR^{65}R^{66}$-$CR^{67}R^{68}$-$NR^{23'}R^{24'}$, -O-$CR^{62}R^{63}R^{64}$, -O-$CR^{62}R^{63}$-$CR^{63}R^{66}R^{64}$, -O-$CR^{62}R^{63}$-$CR^{65}R^{66}$-$CR^{67}R^{68}R^{64}$, -CHO, $-CH_2OH$, $-CR^{23'}$-O, $-CH_2OR^{23'}$;

**$R^{23'}$** and **$R^{24'}$** represent independently of each other -H, $-CH_3$, $-C_2H_5$, $-C_3H_7$, $-CH(CH_3)_2$, $-C_4H_9$, $-CH_2$-$CH(CH_3)_2$, $-CH(CH_3)$-$C_2H_5$, $-C(CH_3)_3$; -(cyclo-$C_3H_5$);

**x** is a value between 0 and 3;

**B** is a bond, $-CR^{86}R^{87}$-, $-CR^{86}R^{87}$-$CR^{88}R^{89}$-, $-CR^{86}R^{87}$-$CR^{88}R^{89}$-$CR^{90}R^{91}$-, $-CR^{86}R^{87}$-$CR^{88}R^{89}$-$CR^{90}R^{91}$-$CR^{92}$-$R^{93}$-, $-CR^{86}R^{87}$-$CR^{88}R^{89}$-$CR^{90}R^{91}$-$CR^{92}R^{93}$-$CR^{94}R^{95}$-, $-CR^{86}R^{87}$-$CR^{88}R^{89}$-$CR^{90}R^{91}$-$CR^{92}R^{93}$-$CR^{94}R^{95}$-$CR^{96}$-$R^{97}$-;

**$R^{86}$-$R^{97}$** represent independently of each other -H, $-CH_3$, $-C_2H_5$, $-C_3H_7$, $-C_4H_9$, -F, -Cl, -Br, -I;

**Y** is a bond, -O-, -S-, -SO-, $-SO_2-$, $-SO_2NH-$, $-NHSO_2-$, -CO-, -COO-, -OOC-, -CONH-, -NHCO-, -NH-, $-N(CH_3)-$, -NH-CO-NH-, -O-CO-NH-, -NH-CO-O-;

$\mathbf{R^{84}}$ is selected from a bond, $-CR^{86}R^{87}-$, $-CR^{86}R^{87}-CR^{88}R^{89}-CR^{90}R^{91}-$, $-CR^{86}R^{87}-CR^{88}R^{89}-CR^{90}R^{91}-CR^{92}R^{93}-$, $-CR^{86}R^{87}-CR^{88}R^{89}-CR^{90}R^{91}-CR^{92}R^{93}-CR^{94}R^{95}-$, $-CR^{86}R^{87}-CR^{88}R^{89}-$, $-CR^{86}R^{87}-CR^{88}R^{89}-CR^{90}R^{91}-CR^{92}R^{93}-CR^{94}R^{95}-CR^{96}R^{97}-$;

$\mathbf{R^{85}}$ is selected from

(i) -H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -Ph, -OPh, $-OCH_2$-Ph, $-OCPh_3$, -SH, $-SCH_3$, $-SC_2H_5$, $-SC_3H_7$, -S-cyclo-$C_3H_5$, $-SCH(CH_3)_2$, $-SC(CH_3)_3$, $-SC_4H_9$, $NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-Si(CH_3)_2(C(CH_3)_3)$, $-Si(C_2H_5)_3$, $-Si(CH_3)_3$, -CN, -CHO, $-COCH_3$, $-COC_2H_5$, $-COC_3H_7$, -CO-cyclo-$C_3H_5$, $-COCH(CH_3)_2$, $-COC(CH_3)_3$, $-COC_4H_9$, -COOH, $-COOCH_3$, $-COOC_2H_5$, $-COOC_3H_7$, $-COOC_4H_9$, -COO-cyclo-$C_3H_5$, $-COOCH(CH_3)_2$, $-COOC(CH_3)_3$, -OOC-$CH_3$, -OOC-$C_2H_5$, -OOC-$C_3H_7$, -OOC-$C_4H_9$, -OOC-cyclo-$C_3H_5$, -OOC-$CH(CH_3)_2$, -OOC-$C(CH_3)_3$, -CO-$NR^{23'}R^{24'}$, $-NHCOCH_3$, $-NHCOC_2H_5$, $-NHCOC_3H_7$, -NHCO-cyclo-$C_3H_5$, -NHCO-$CH(CH_3)_2$, $-NHCOC_4H_9$, -NH-CO-$C(CH_3)_3$, -NHCO-$OCH_3$, -NHCO-$OC_2H_5$, -NHCO-$OC_3H_7$, -NHCO-O-cyclo-$C_3H_5$, -NHCO-$OC_4H_9$, -NHCO-$OCH(CH_3)_2$, -NHCO-$OC(CH_3)_3$, -NHCO-$OCH_2$Ph, $-NR^{23}R^{24}$, $-CF_3$, $-SOCH_3$, $-SOC_2H_5$, $-SOC_3H_7$, -SO-cyclo-$C_3H_5$, $-SOCH(CH_3)_2$, $-SOC(CH_3)_3$, $-SO_2CH_3$, $-SO_2C_2H_5$, $-SO_2C_3H_7$, $-SO_2$-cyclo-$C_3H_5$, $-SO_2CH(CH_3)_2$, $-SO_2C_4H_9$, $-SO_2C(CH_3)_3$, $-SO_3H$, $-SO_2NR^{23'}R^{24'}$, $-OCF_3$, $-OC_2F_5$, -O-$COOCH_3$, -O-$COC_2H_5$, -O-$COOC_3H_7$, -O-COO-cyclo-$C_3H_5$, -O-$COOC_4H_9$, -O-$COOCH(CH_3)_2$, -O-$COOCH_2$Ph, -O-$COOC(CH_3)_3$, -NH-CO-$NH_2$, -NH-CO-$NHCH_3$, -NH-CO-$NHC_2H_5$, -NH-CO-$NHC_3H_7$, -NH-CO-$NHC_4H_9$, -NH-CO-NH-cyclo-$C_3H_5$, $-OCH_2$-cyclo-$C_3H_5$, -NH-CO-NH[$CH(CH_3)_2$], NH-CO-NH[$C(CH_3)_3$], NH-CO-$N(CH_3)_2$, -NH-CO-$N(C_2H_5)_2$, -NH-CO-$N(C_3H_7)_2$, NH-CO-$N(C_4H_9)_2$, -NH-CO-N(cyclo-$C_3H_5$)$_2$, -NH-CO-N[$CH(CH_3)_2$]$_2$, -NH-CO-N[$C(CH_3)_3$]$_2$, -NH-C(=NH)-$NH_2$, -NH-C(=NH)-$NHCH_3$, -NH-C(=NH)-$NHC_2H_5$, -NH-C(=NH)-$NHC_3H_7$, -NH-C(=NH)-$NHC_4H_9$, -NH-C(=NH)-NH-cyclo-$C_3H_5$, -NH-C(=NH)-NH[$CH(CH_3)_2$], -NH-C(=NH)-NH[$C(CH_3)_3$], -NH-C(=NH)-$N(CH_3)_2$, -NTH-C(=NH)-,N ($C_2H_5$)$_2$, -NH-C(=NH)-$N(C_3H_7)_2$, -NH-C(=NH)-N(cyclo-$C_3H_5$)$_2$, -NH-C(=NH)-$N(C_4H_9)_2$, -NH-C(=NH)-N[CH ($CH_3$)$_2$]$_2$, -NH-C(=NH)-N[$C(CH_3)_3$]$_2$, -O-C;O-$NH_2$, -O-CO-$NHCH_3$, -O-CO-$NHC_2H_5$, -O-CO-$NHC_3H_7$, -O-CO-$NHC_4H_9$, -O-CO-NH-cyclo-$C_3H_5$,- O-CO-NH[$CH(CH_3)_2$], -O-CO-NH[$C(CH_3)_3$], -O-CO $N(CH_3)_2$, -O-CO-N ($C_2H_5$)$_2$, -O-CO-$N(C_3H_7)_2$, -O-CO-$N(C_4H_9)_2$, -O-CO-N(cyclo-$C_3H_5$)$_2$, -O-CO-N[$CH(CH_3)_2$]$_2$, -O-CO-N[C ($CH_3$)$_3$]$_2$,

(ii) an aromatic or heteroaromatic mono- or bicyclic ring selected from 2-thienyl, 3-thienyl, 2-furanyl, 3-furanyl, 2-oxazolyl, 3-oxazolyl, 4-oxazolyl, 2-thiazolyl, 3-thiazolyl, 4-thiazolyl, 1-pyrazolyl, 3-pyrazolyl, 4-pyrazolyl, 5-pyrazolyl, 1-imidazolyl, 2-imidazolyl, 4-imidazolyl, 5-imidazolyl, phenyl, 1-naphthyl, 2-naphthyl, 2-pyridyl, 3-pyridyl, 4-pyridyl, 2-pyrimidinyl, 4-pyrimidinyl, 5-pyrimidinyl, 2-pyrazinyl, 3-pyridazinyl, 4-pyridazinyl, 1,3,5-triazin-2-yl,

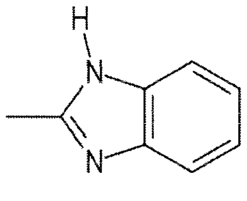 , 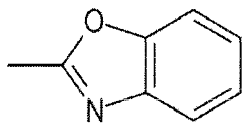 , 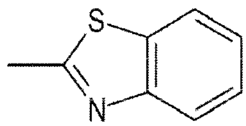 ,

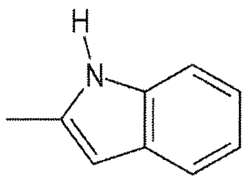 , 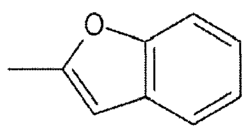 , 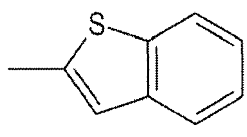 ,

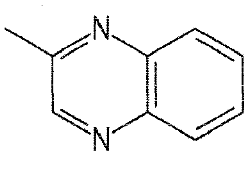 , 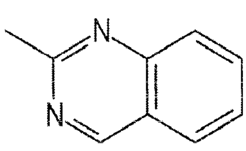 , 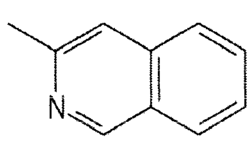 ,

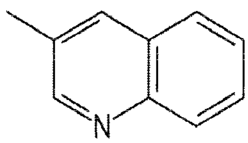, 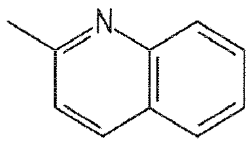, 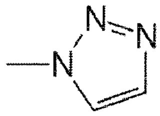,

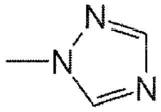, 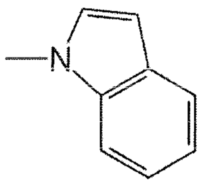, 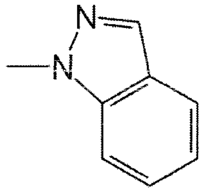, 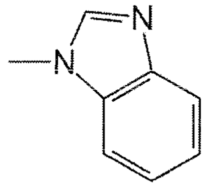,

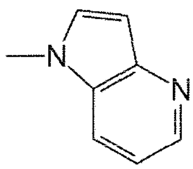, 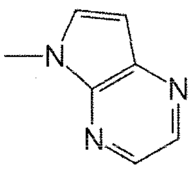, 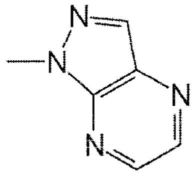, 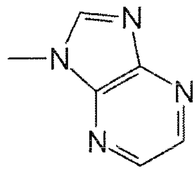,

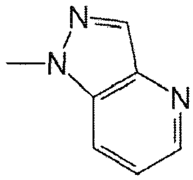, 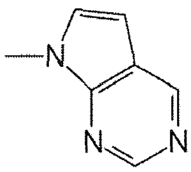, 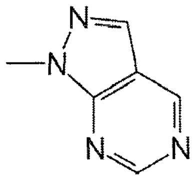, 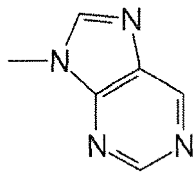,

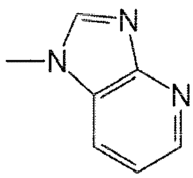, 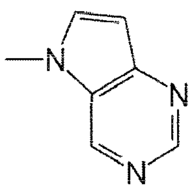, 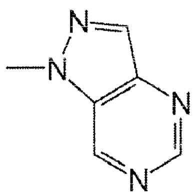, 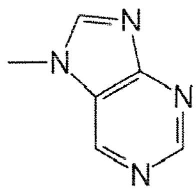,

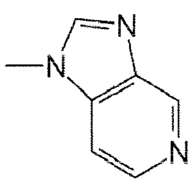 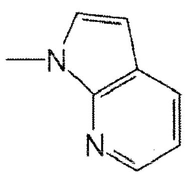, 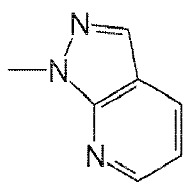, 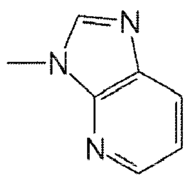,

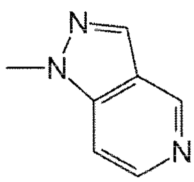, 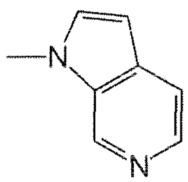, 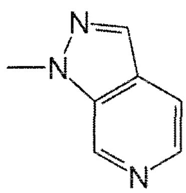, 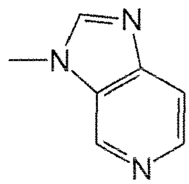,

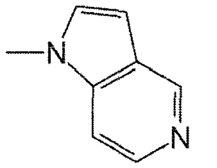, 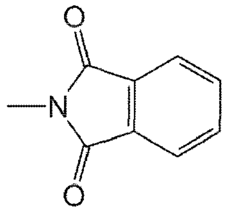.

which optionally may be substituted by one or two substituents selected from -F, -Cl, -Br, -I, $-OCH_3$, $-CH_3$, $NO_2$, -CN, $-CF_3$;

(iii) a saturated ring selected from cyclopentyl, azetidin-1-yl,

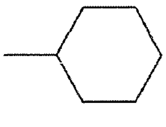, 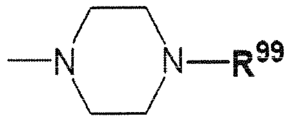, 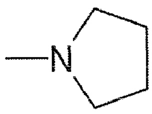,

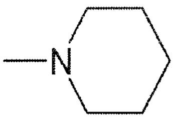, 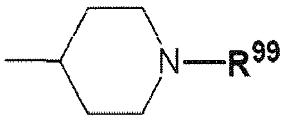, 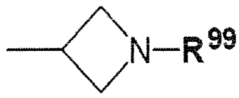,

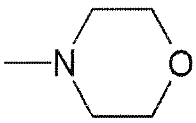; 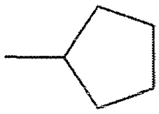, 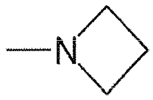;

**R⁹⁹** represents -H, $-CH_3$, $-CH_2Ph$, $-COOC(CH_3)_3$, $-COOCH_3$, $-COOCH_2CH_3$, $-COOCH_2CH_2CH_3$, $-COOCH(CH_3)_2$, $-COOCH_2Ph$, $-COCH_3$;

the group $-B-Y-R^{84}-R^{85}$ together with one substituent $R^{83}$ may form a group $-OCH_2O-$, if $R^{83}$ is attached in position ortho to $-B-Y-R^{84}-R^{85}$;

with the proviso that $R^{83}$ is not -H, if the group $-B-Y-R^{84}-R^{85}$ is hydrogen.

**P⁹⁸** is selected from $-NO_2$, -CN, -F, -Cl, -Br, -I, $-NH_2$, -OH, $-CR^{62}R^{63}R^{64}$, $-CR^{62}R^{63}-CR^{65}R^{66}R^{64}$, $-CR^{62}R^{63}-CR^{65}R^{66}-CR^{67}R^{68}R^{64}$, $-CR^{62}R^{63}-CR^{65}R^{66}-CR^{67}R^{68}-CR^{69}R^{70}R^{64}$, $-O-CR^{62}R^{63}R^{64}$, $-O-CR^{62}R^{63}-CR^{65}R^{66}R^{64}$, $-O-CR^{62}R^{63}-CR^{65}R^{66}-CR^{67}R^{68}R^{64}$, $-O-CR^{62}R^{63}-R^{65}R^{66}-CR^{67}R^{68}-CR^{69}R^{70}R^{64}$, $-O-CR^{62}R^{63}-CR^{65}R^{66}-CR^{67}R^{68}-CR^{69}R^{70}-CR^{71}R^{72}R^{64}$, $-O-CR^{62}R^{63}-CR^{65}R^{66}-CR^{67}R^{68}-CR^{69}R^{70}-CR^{71}R^{72}-CR^{73}R^{74}R^{64}$, $-CR^{62}R^{63}-O-CR^{65}R^{66}R^{64}$, $-CR^{62}R^{63}-O-CR^{65}R^{66}-CR^{67}R^{68}R^{64}$, $-CR^{62}R^{63}-O-CR^{65}R^{66}-CR^{67}R^{68}-CR^{69}R^{70}R^{64}$, $-CR^{62}R^{63}-O-CR^{65}R^{66}-CR^{67}R^{68}-CR^{69}R^{70}-CR^{71}R^{72}R^{64}$, $-CR^{62}R^{63}-O-CR^{65}R^{66}-CR^{67}R^{68}-CR^{69}R^{70}-CR^{71}R^{72}-CR^{73}R^{74}R^{64}$, $-CR^{62}R^{63}-CR^{65}R^{66}-CR^{67}R^{68}-CR^{69}R^{70}-CR^{71}R^{72}R^{64}$, $-CR^{62}R^{63}-CR^{65}R^{66}-CR^{67}R^{68}-CR^{69}R^{70}-CR^{71}R^{72}-CR^{73}R^{74}R^{64}$, $-OCH_2Ph$, $-OCH_2-CH_2-Ph$, $-CH_2-O-CH_2-Ph$;

with the proviso that $R^{98}$ is attached to a position ortho to the bond between the pyridine and the triazine ring if $R^{98}$ is not an amino group in para position to the bond between the pyridine and the triazine ring;

**R¹⁰⁰** is selected from -H, $-NO_2$, -CN, -F, -Cl, -Br, -I, $NH_2$, -OH, $-CF_3$, $-CH_3$, $-CH_2CH_3$, $-CH_2CH_2CH_3$, $-CH(CH_3)_2$, $-OCH_3$, $-OCH_2CH_3$, $-OCH_2CH_2CH_3$, $-OCH(CH_3)_2$, $-OCF_3$, $-OCH_2Ph$;

and with the proviso that if $R^1$ is a phenyl moiety and $R^2$ is also a phenyl moiety a chloro substituent is only allowed on the $R^1$ phenyl moiety or on the $R^2$ phenyl moiety but not on both simultaneously;

and with the proviso that the compound 4-[4-(2-benzoylaminophenyl)-[1,3,5]triazin-2-ylamino]benzamide is excluded;

and enantiomers, stereoisomeric forms, mixtures of enantiomers, diastereomers, mixtures of diastereomers, prodrugs, hydrates, solvates, acid salt forms, tautomers, and racemates of the above mentioned compounds and pharmaceutically acceptable salts thereof or salts of solvates thereof.

**6.** The CDK9 inhibitor for use according to claim 5, or the method according to claim 5,

wherein
**R¹** represents

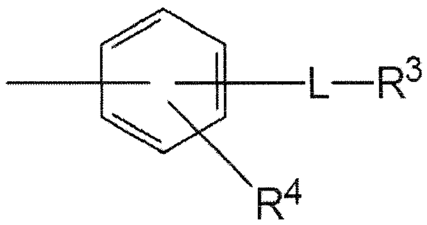

in which
**L** is a bond, $-CH_2-$, $-CH_2CH_2-$, or $-CF_2-$;
**$R^3$** is $-SO_2NH_2$, $-SO_2NH(CH_3)$, $-SO_2N(CH_3)_2$, $-SO_2NH(CH_2CH_2OCH_3)$, $-NHSO_2CH_3$, $-NHSO_2CH_2CH_3$, $-NHSO_2-CH_2CH_2CH_3$, $-NHSO_2CF_3$, $-SO_2CH_3$, $-NHSO_2NH_2$, $-SO(NH)CH_3$;
**$R^4$** is -H, $-CH_3$, -F, -Cl, or $-CF_3$;
**$R^2$** represents

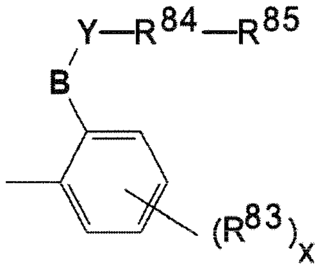

or

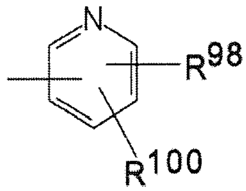

;

in which the group **$-B-Y-R^{84}-R^{85}$** is $-OCH_3$, $-OCH_2CH_3$, $-OCH_2CH_2CH_3$, $-OCH_2CH_2CH_2CH_3$, $-OCH(CH_3)_2$, -OPh, $-OCH_2Ph$, $-OCH_2$(4-pyridyl);
**$R^{83}$** is -H, -F, or -Cl;
x is 0,1, or 2;
**$R^{98}$** is $-OCH_3$ and **$R^{100}$** is -H,
with the proviso that $R^{98}$ is attached to a position ortho to the bond between the pyridine and the triazine ring.

**7.** The CDK9 inhibitor for use according to claim 5 or 6, or the method according to claim 5 or 6, wherein
**$R^1$** represents

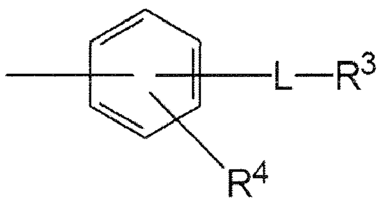

in which
the substituent **$-L-R^3$** is $-SO_2NH_2$, $-CH_2SO_2NH_2$, $-CH_2CH_2SO_2NH_2$, $-CF_2SO_2NH_2$, $-NHSO_2NH_2$, $-CH_2NHSO_2NH_2$, $-SO_2CH_3$, $-SO(NH)CH_3$, $-CH_2SO(NH)CH_3$;
**$R^4$** is -H;
**$R^2$** represents 2-methoxyphenyl, 4-fluoro-2-methoxyphenyl, or 6-fluoro-2-methoxyphenyl.

**8.** The CDK9 inhibitor for use according to claim 5, or the method according to claim 5,
wherein
**$R^1$** is

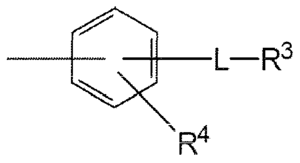 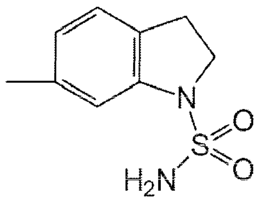 or 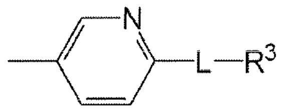

**L** is a bond, $-CH_2-$, or $-CH_2CH_2-$;
**R³** is -H, $-SO_2NR^{23}R^{24}$, $-CONR^{23}R^{24}$, $-NO_2$, $-NH_2$, $-NHSO_2R^{22}$, $-NHCOR^{22}$, $-SO_2R^{22}$, NH-CO-NH-Ph, or -Ph,
**R⁴** is -H, $-CH_2-SO_2-NR^{23}R^{24}$, $-SO_2NR^{23}R^{24}$, $-CONH_2$, $-C_2H_4-SO_2NR^{23}R^{24}$, $-NH-SO_2-CH_3$, $-NH-SO_2-C_2H_5$, $-NH-SO_2-C_3H_7$, $-NHCO-CH_3$, $-NHCO-C_2H_5$, $-NO_2$, $-NH_2$, $-SO_2CH_3$, or

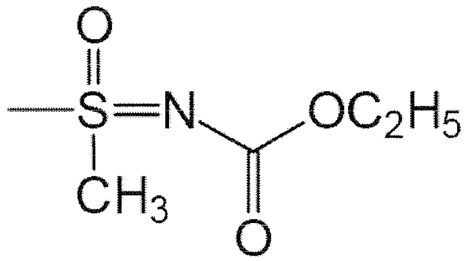

$R^{23}$ and $R^{24}$ are independently selected from -H, $-CH_3$, $-C_2H_5$, $-C_3H_7$, -(cyclo-$C_3H_5$),$-CH_2-CH_2-CH_2-CH_2-NH_2$, or $-CH_2-CH_2-CH_2-CH_2-NH-COOC(CH_3)_3$,
**$R^2$** represents

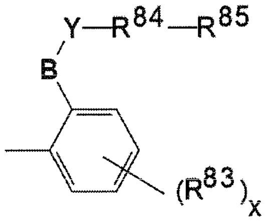 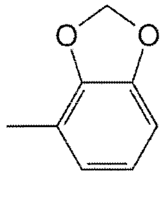 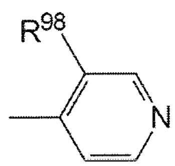 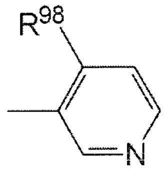

or

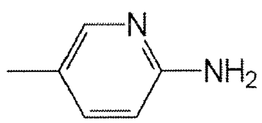

**B** is a bond or $-CH_2-$;
**Y** is a bond, -O-, or -NH-;
**$R^{83}$** is selected from -H, -CN, -F, -Cl, $-O-CR^{62}R^{63}R^{64}$, $-CF_3$, $-CH_2OR^{23'}$, $-CR^{23'}O$, $-CR^{62}R^{63}-NR^{23'}R^{24'}$, $-CR^{62}R^{63}R^{64}$;
**$R^{23'}$** and **$R^{24'}$** represent independently of each other -H, $-CH_3$, -(cyclo-$C_3H_5$);
**$R^{62}$ - $R^{64}$** represent independently of each other -H, $-CH_3$, -Ph, -F, -cyclo-$C_3F_5$;
**$R^{84}$** is selected from a bond, $-CH_2-$, or $-CH_2-CH_2-CH_2-CH_2-$;
**$R^{85}$** is selected from -H, $-CF_3$, $-OCH_3$, $-OCH(CH_3)_2$, -CN, $-NHCOCH_3$, $-OCH_2$-cyclo-$C_3H_5$, $-NR^{23}R^{24}$, -Ph, -OPh, $-NHCO-OC(CH_3)_3$,

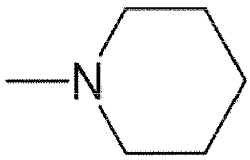, 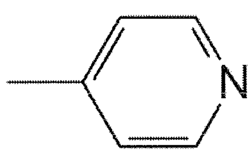, 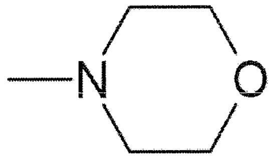;

**$R^{98}$** represents $-OCH_3$;
and salts, solvates or salts of solvates of the afore-mentioned compounds and especially the hydrochloride salt or the trifluoroacetate salt of these compounds.

**9.** The CDK9 inhibitor for use according to claim 5, or the method according to claim 5,
wherein
**$R^1$** represents

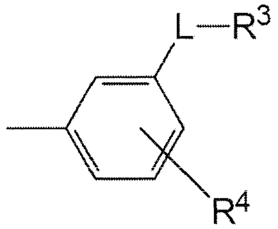

in which
the substituent $-E-R^3$ is $-SO_2NH_2$ or $-CH_2SO_2NH_2$,
$R^4$ is -H;
$R^2$ represents 2-methoxyphenyl, 4-fluoro-2-methoxyphenyl or 2-benzyloxyphenyl,
or their salts, solvates or salts of solvates and especially the hydrochloride salt or the trifluoroacetate salt.

**10.** The CDK9 inhibitor for use according to claim 5, or the method according to claim 5, wherein the compound is selected from the group consisting of

3-[(4-(2-Methoxyphenyl)-1,3,5-triazin-2-yl)amino]benzenemethanesulfonamide,
3-[(4-(4-Fluoro-2-methoxyphenyl)-1,3,5-triazin-2-yl)amino]benzenemethane-sulfonamide,
3-[(4-(5-Fluoro-2-methoxyphenyl)-1,3,5-triazin-2-yl)amino]benzenemethane-sulfonamide,
3-[(4-(6-Fluoro-2-methoxyphenyl)-1,3,5-triazin-2-y1)amino]benzenemethane-sulfonamide,
3-[(4-(3,5-Difluoro-2-methoxyphenyl)-1,3,5-triazin-2-yl)amino]benzenemethanesulfonamide,
3-[(4-(4-Chloro-2-methoxyphenyl)-1,3,5-triazin-2-yl)amino]benzenemethanesulfonamide,
3-[(4-(5-Chloro-2-methoxyphenyl)-1,3,5-triazin-2-yl)amino]benzenemethanesulfonamide,
3-[(4-(2-Methoxy-4-trifluoromethyl-phenyl)-1,3,5-triazin-2-yl)amino]benzenemethanesulfonamide,
3-[(4-(2-Methoxy-5-trifluoromethyl-phenyl)-1,3,5-triazin-2-yl)amino]benzenemethanesulfonamide,
3-[(4-(5-Hydroxymethyl-2-methoxyphenyl)-1,3,5-triazin-2-yl)amino]benzenemethanesulfonamide,
3-[(4-(5-Formyl-2-methoxyphenyl)-1,3,5-triazin-2-yl)amino]benzenemethanesulfonamide,
3-[(4-(2-Ethoxyphenyl)-1,3,5-triazin-2-yl)amino]benzenemethanesulfonamide,
3-[(4-(2-Benzyloxyphenyl)-1,3,5-triazin-2-yl)amino] benzenemethanesulfonamide,
1-(3-{[4-(2-phenoxyphenyl)-1,3,5-triazin-2-yl]amino}phenyl)methanesulfonamide,
3-[(4-(1,3-Benzodioxol-4-yl)-1,3,5-triazin-2-yl)amino]benzenemethanesulfonamide,
3-[(4-(2-((4-Pyridinyl)methoxy)phenyl)-1,3,5-triazin-2-yl)amino]benzenemethanesulfonamide,
3-[(4-(2-(4-(tert-Butoxycarbonylamino)butoxy)phenyl)-1,3,5-triazin-2-yl)amino]-benzenemethanesulfonamide,
3-[(4-(4-Methoxypyridin-3-yl)-1,3,5-triazin-2-yl)amino]benzenemethane-sulfonamide,
3-[(4-(3-Methoxypyridin-4-yl)-1,3,5-triazin-2-yl)amino]benzenemethane-sulfonamide,
3-[(4-(2-((Morpholin-4-yl)methyl)phenyl)-1,3,5-triazin-2-yl)amino]benzenemethanesulfonamide,
3-[(4-(2-((Piperidin-1-yl)methyl)phenyl)-1,3,5-triazin-2-yl)amino]benzenemethanesulfonamide,
3-[(4-(2-(Cyclopropylamino-methyl)phenyl)-1,3,5-triazin-2-yl)amino]benzenemethanesulfonamide,
3-[(4-(6-Aminopyridin-3-yl)-1,3,5-triazin-2-yl)amino]benzenemethane-sulfonamide,

3-[(4-(2-(Methoxymethyl)phenyl)-1,3,5-triazin-2-yl)amino]benzene-methanesulfonamide,
3 -[(4-(2-Methoxyphenyl)-1,3,5-triazin-2-yl)amino]benzenesulfonamide,
2-[3-((4-(2-Methoxyphenyl)-1,3,5-triazin-2-yl)amino)phenyl]ethanesulfonamide,
2-[3-((4-(4-Fluoro-2-methoxyphenyl)-1,3,5-triazin-2-yl)amino)phenyl]ethanesulfonamide,
3-[(4-(2-Methoxyphenyl)-1,3,5-triazin-2-yi)amino]benzamide,
6-[(4-(2-Methoxyphenyl)-1,3,5-triazin-2-yl)amino]-2,3-dihydro-1H-indole-1-sulfonamide,
rac-S-[3-((4-(2-Methoxyphenyl)-1,3,5-triazin-2-yl)amino)phenyl]-N-ethoxy-carbonyl-S-methyl-sulfoximide,
4-(2-Methoxyphenyl)-N-(3-nitrophenyl)-1,3,5-triazine-2-amine,
3-[(4-(2-(4-Aminobutoxy)phenyl)-1,3,5-triazin-2-yl)amino]benzenemethane-sulfonamide,
N-(3-Aminophenyl)-4-(2-methoxyphenyl)-1,3,5-triazine-2-amine,
N-[3-((4-(2-Methoxyphenyl)-1,3,5-triazin-2-yl)amino)phenyl]-methanesulfonamide,
N-[3-((4-(2-Methoxyphenyl)-1,3,5-triazin-2-yl)amino)phenyl]-propanesulfonamide,
N-[3-((4-(2-Methoxyphenyl)-1,3,5-triazin-2-yl)amino)phenyl]acetamide,
N-[3-((4-(2-Methoxyphenyl)-1,3,5-triazin-2-yl)amino)phenyl]-N'-phenyl-urea,
3-[(4-(2-Methoxy-5-(methylamino-methyl)phenyl)-1,3,5-triazin-2-yl)amino]-benzenemethanesulfonamide,
4-(2-Methoxyphenyl)-N-phenyl-1,3,5-triazine-2-amine, tert-Butyl-[4-((3-((4-(4-Fluoro-2-methoxyphenyl)-1,3,5-triazin-2-yl)amino)phenyl)-methylsulfonamido) butyl]carbamate,
N-(4-Aminobutyl)-1-[3-((4-(4-fluoro-2-methoxyphenyl)-1,3,5-triazin-2-yl)amino)-phenyl]methanesulfonamide,
4-(2-Methoxyphenyl)-N-(3-(methysulfonyl)phenyl)-1,3,5-triazin-2-amine,
4-[(4-(2-Methoxyphenyl)-1,3,5-triazin-2-yl)amino]benzenemethane-sulfonamide,
1-[3-({4-[4-fluoro-2-(trifluoromethyl)phenyl]-1,3,5-triazin-2-yl}amino)phenyl]-methanesulfonamide,
1-[3-({4-[4-fluoro-2-(propan-2-yloxy)phenyl]-1,3,5-triazin-2-yl}amino)phenyl]-methanesulfonamide,
1-(3-{[4-(2-cyano-4-fluorophenyl)-1,3,5-triazin-2-yl]amino}phenyl)methane-sulfonamide, N-[5-fluoro-2-(4-{[3-(sulfamoylmethyl)phenyl]amino}-1,3,5-triazin-2-yl)phenyl]-acetamide,
1-[3-({4-[2-(cyclopropylmethoxy)-4-fluorophenyl]-1,3,5-triazin-2-yl}amino)phenyl]-methanesulfonamide,
1-(3-{[4-(3,4-difluoro-2-methoxyphenyl)-1,3,5-triazin-2-yl]amino}phenyl)methanesulfonamide,
1-(3-{[4-(4,5-difiuoro-2-methoxyphenyl)-1,3,5-triazin-2-yl]amino}phenyl)methanesulfonamide,
4-(4-fluoro-2-methoxyphenyl)-N-[6-(methylsulfonyl)pyridin-3-yl]-1,3,5-triazin-2-amine, 3-[(4-(2-Methoxyphenyl)-1,3,5-triazin-2-yl)amino]benzenemethanesulfonamide trifluoroacetic acid salt,
1-(3-{[4-(4-fluoro-2-methoxyphenyl)-1,3,5-triazin-2-yl]amino}phenyl)methanesulfonamide hydrochloride,
3-[(4-(4-Fluoro-2-methoxyphenyl)-1,3,5-triazin-2-yl)amino]benzene-methanesulfonamide trifluoroacetic acid salt,
3-[(4-(2-Benzyloxyphenyl)-1,3,5-triazin-2-yl)amino] benzenemethanesulfon-amide trifluoroacetic acid salt,
3-[(4-(2-Methoxyphenyl)-1,3,5-triazin-2-yl)amino]benzenesulfonamide trifluoroacetic acid salt.

**11.** The CDK9 inhibitor for use according to any of claims 1, 3 and 4, or the method according to any one of claims 2 to 4, wherein said inhibitor is selected from the group consisting of

3-[(4-(2-Methoxyphenyl)-1,3,5-triazin-2-yl)amino]benzenemethanesulfonamide (Cpd B1);
3-[(4-(4-Fluoro-2-methoxyphenyl)-1,3,5-triazin-2-yl)amino] benzenemethanesulfonamide (Cpd B2);
3-[(4-(5-Fluoro-2-methoxyphenyl)-1,3,5-triazin-2-yl)amino] benzenemethanesulfonamide (Cpd B3);
3-[(4-(6-Fluoro-2-methoxyphenyl)-1,3,5-triazin-2-yl)amino] benzenemethanesulfonamide (Cpd B4);
3-[(4-(4-Chloro-2-methoxyphenyl)-1,3,5-triazin-2-yl)amino] benzenemethanesulfonamide (Cpd B6);
3-[(4-(5-Chloro-2-methoxyphenyl)-1,3,5-triazin-2-yl)amino] benzenemethanesulfonamide (Cpd B7);
3-[(4-(2-Ethoxyphenyl)-1,3,5-triazin-2-yl)amino]benzenemethanesulfonamide (Cpd B12);
3-[(4-(2-Benzyloxyphenyl)-1,3,5-triazin-2-yl)amino] benzenemethanesulfonamide (Cpd B 13);
1-(3-{[4-(2-phenoxyphenyl)-1,3,5-triazin-2-yl]amino}phenyl)methanesulfonamide (Cpd B 14);
3-[(4-(2-((4-Pyridiyl)methoxy)phenyl)-1,3,5-triazin-2-yl)amino]benzenemethanesulfonamide (Cpd B16);
3-[(4-(2-(4-(tert-Butoxycarbonylamino)butoxy)phenyl)-1,3,5-triazin-2-yl)amino]benzenemethanesulfonamide (Cpd B17);
3-[(4-(3-Methoxypyridin-4-yl)-1,3,5-triazin-2-yl)amino]benzenemethanesulfonamide (Cpd B18);
3-[(4-(6-Aminopyridin-3-yl)-1,3,5-triazin-2-yl)amino]benzenemethanesulfonamide (Cpd B23);
3-[(4-(2-Methoxyphenyl)-1,3,5-triazin-2-yl)amino]benzenesulfonamide (Cpd B24);
2-[3-((4-(2-Methoxyphenyl)-1,3,5-triazin-2-yl)amino)phenyl]ethanesulfonamide (Cpd C1);
N-[3-((4-(2-Methoxyphenyl)-1,5-triazin-yl)amino)phenyl]-methanesulfonamide (Cpd D1);
N-[3-((4-(2-Methoxyphenyl)-1,3,5-triazin-2-yl)amino)phenyl]-propanesulfonamide (Cpd L1);
tert-Butyl [4-((3-((4-(4-Fluoro-2-methoxyphenyl)-1,3,5-triazin-2-yl)amino)phenyl)methylsulfonamido) butyl]carbamate (Cpd Q1);

N-(4-Aminobutyl)-1-3-((4-(4-fluoro-2-methoxyphenyl)-1,3,5-triazin-2-yl)amino)phenyl]methanesulfonamide (Cpd R1);
4-(2-Methoxyphenyl)-N-(3-(methylsulfonyl)phenyl)-1,3,5-triazin-2-amine (Cpd S1); 1-[3-({4-[4-Fluoro-2-(propan-2-yloxy)phenyl]-1,3,5-triazin-2-yl}amino)phenyl]methanesulfonamide (Cpd U2);
1-[3-({4-[2-(Cyclopropylmethoxy)-4-fluorophenyl]-1,3,5-triazin-2-yl}amino)phenyl]methanesulfonamide (Cpd U5);
1-(3- {[4-(4,5-Difluoro-2-methoxyphenyl)-1,3,5-triazin-2-yl]amino}phenyl)methanesulfonamide (Cpd U7);
3-[(4-(2-Methoxyphenyl)pyridin-2-yl)amino]benzenesulfonamide (Cpd 24);
4-(2-Methoxyphenyl)-N-(3-(methylsulfonyl)phenyl)pyridin-2-amine (Cpd 25);
[3-((4-(4-Fluoro-2-methoxyphenyl)pyridin-2-yl)amino)phenyl]methanesulfonamide (Cpd 26);
[3-((4-(2-Methoxyphenyl)pyridin-2-yl)amino)phenyl]methanesulfonamide (Cpd 27);
1-[3-((4-(4-Fluoro-2-methoxyphenyl)pyridin-2-yl)amino)phenyl]-N,N-dimethylmethanesulfonamide (Cpd 28);
2-[3-((4-(2-Methoxyphenyl)pyridin-2-yl)amino)phenyl]ethanesulfonamide (Cpd 29);
N-[3-((4-(4-Fluoro-2-meihoxyphenyl)pyridin-2-yi)amino)phenyl]methanesulfonamide (Cpd 30);
N-[3-((4-(4-Fluoro-2-methoxyphenyl)pyridin-2-yl)amino)phenyl]acetamide (Cpd 31); 1-[3-((4-(4-Fluoro-2-methoxyphenyl)pyridin-2-yl)amino)phenyl]-N-propylmethanesulfonamide (Cpd 32);
(R)-Methyl 1-[4-((3-(Sulfamoylmethyl)phenyl)amino)-1,3,5-triazin-2-yl]piperidine-2-carboxylate (Cpd 33);
(R)-3-[(4-(2-(Methoxymethyl)pyrrolidin-1-yl)-1,3,5-triazin-2-yl)amino]benzenemethanesulfonamide (Cpd 34);
(R)-Methyl 1-[4-((3-(Sulfamoylmethyl)phenyl)amino)-1,3,5-triazin-2-yl]pyrrolidine-2-carboxylate )Cpd 35);
rac-3-[(4-(2-Phenylpyrrolidin-1-yl)-1,3,5-triazin-2-yl)amino]benzenemethanesulfonamide (Cpd 36);
(R)-3-[(4-(2-Phenylpyrrolidin-1-yl)-1,3,5-triazin-2-yl)amino]benzenemethanesulfonamide (Cpd 37);
3-[(4-(7,8-Dihydro-1,6-naphthyridin-6(5H)-yl)-1,3,5-triazin-2-yl)amino]benzenemethanesulfonamide (Cpd 38);
3-[(4-(3,4-Dihydroquinolin-1(2H)-yl)-1,3,5-triazin-2-yl)amino]benzenemethanesulfonamide (Cpd 39);
3-[(4-(6,7-Dihydro-3H-imidazo[4,5-c]pyridin-5(4H)-yl)-1,3,5-triazin-2-yl)amino]benzenemethanesulfonamide (Cpd 40);
3-[(4-(1H-Pyrrolo[3,4-c]pyridin-2(3H)-yl)-1,3,5-triazin-2-yl)amino]benzenemethanesuifonamide (Cpd 41);
3-[(4-(Pyrrolo[3,4-c]pyrazol-5(1H,4H,6H)-yl)-1,3,5-triazin-2-yl)amino]benzenemethanesulfonamide (Cpd 42);
3-[(4-(Indolin-1-yl)-1,3,5-triazin-2-yl)amino]benzenemethanesulfonamide (Cpd 43); and (S)-3-[(4-(2-Methylpyrrolidin-1-yl)-1,3,5-triazin-2-yl)amino]benzenemethanesulfonamide (Cpd 44).

**12.** The CDK9 inhibitor for use according to any of claims 1, 3 and 4, or the method according to any one of claims 2 to 4, wherein said inhibitor is selected from the group consisting of Piperidine-4-carboxylic acid [5-(5-tert-butyl-oxazol-2-ylmethylsulfanyl)-thiazol-2-yl]-amide (SNS-032);

2-(2-Chloro-phenyl)-5,7-dihydroxy-8-(3-hydroxy-1-methyl-piperidin-4-yl)-chromen-4-one, (flavopiridol);
N-(5-((6-(3-aminophenyl)pyrimidin-4-yl)amino)-2-methylphenyl)propane-1-sulfonamide (AX35427);
[4-amino-2-(1-methanesulfonylpiperidin-4-ylamino)pyrimidin-5-yl]-(2,3-difluoro-6-methoxyphenyl)methanone (R-547);
3-[[6-(2-methoxyphenyl)-4-pyrimidinyl]amino]-Benzenemethanesulfonamide (1073485-20-7P);
3-((6-(2-methoxyphenyl)pyrimidin-4-yl)amino)benzenesulfonamide (AX38679);
1,5,6,7-tetrahydro-2-(4-pyridinyl)-4H-pyrrolo[3,2-c]pyridin-4-one, (PHA767491);
5,6-dichloro-1-b-ribofuaranosyl-benzimidazole (DRB);
6-Benzylamino-2[(R)-(1'-ethyl-2'-hydroxyethylamino)]-9-isopropylpurine (Roscovitine);
4-[[4-Amino-5-(2,6-difluorobenzoyl)thiazol-2-yl]amino]-N-((R)-2-dimethylamino-1-methylethyl)benzamide (AG-012986);
4H-1-Benzopyran-4-one, 2-(2-chlorophenyl)-5,7-dihydroxy-8-[(2R,3S)-2-(hydroxymethyl)-1-methyl-3-pyrrolidinyl]-, hydrochloride (1:1) (P276-00);
4-[3-Chloro-5-(4-methylpiperazin-1-yl)benzoylamino]-1H-pyrazole-3-carboxylic acid cyclohexylamide (ZK 304-709);
4-(2,6-Dichlorobenzoylamino)-1H-pyrazole-3-carboxylic acid N-(piperidin-4-yl)amide (AT7519);
N-[2-(dimethylamino)ethyl]-2-fluoro-4-[[5-fluoro-4-[2-methyl-1-(1-methylethyl)-1H-imidazol-5-yl]-2-pyrimidinyl]amino] (Compound 7d);
[4-[[5-fluoro-4-[2-methyl-1-(1-methylethyl)-1H-imidazol-5-yl]-2-pyrimidinyl]amino]phenyl] [(3S)-3-(methylamino)-1-pyrrolidinyl](AZD5597);
N-[1,4-dihydro-3-[4-[[4-(2-methoxyethyl)-1-piperazinyl]methyl]phenyl]-4-oxoindeno[1,2-c]pyrazol-5-yl]-N'-4-morpholinyl-, hydrochloride (1:2) (RGB-286638); and
4-(2,6-dichlorobenzamido)-N-(1-(methylsulfonyl)piperidin-4-yl)-1H-pyrazole-3-carboxamide (LCQ195/AT9311);

**13.** The CDK9 inhibitor for use according to any of claims 3 to 12, or the method according to any of claims 3 to 12, wherein said NUT midline carcinoma (NMC) is **characterized by** the presence of at least one rearrangement in the NUT gene in a tumor or cancer cell in said NUT midline carcinoma (NMC).

**14.** The CDK9 inhibitor for use according to claim 13, or the method according to claim 13, wherein said rearrangement in the NUT gene is a t15;19 translocation as reflected in formation of a Brd4/NUT fusion gene.

**15.** The CDK9 inhibitor for use according to claim 13, or the method according to claim 13, wherein said rearrangement in the NUT gene is a formation of a NUT variant fusion gene.

**16.** The CDK9 inhibitor for use according to claim 13, or the method according to claim 13, wherein said NUT variant fusion gene is a Brd3/NUT fusion gene.

**17.** The CDK9 inhibitor for use according to any of claims 13 to 16, or the method according to according to any of claims 13 to 16, wherein said rearrangement in the NUT gene is reflected in expression of the formed NUT fusion gene and whereby the expression level of the formed NUT fusion gene is detected.

**18.** The CDK9 inhibitor for use according to claim 17, or the method according claim 17, wherein the expression level is detected by an immunohistochemical method, real-time PCR, and/or Northern Blot.

**19.** The CDK9 inhibitor for use according to any of claims 13 to 16, or the method according to according to any of claims 13 to 16, wherein said rearrangement in the NUT gene is detected by an in situ hybridization method.

**20.** The CDK9 inhibitor for use according to claim 19, or the method according to claim 19, wherein the in situ hybridization method is selected from the group consisting of fluorescent in situ hybridization (FISH), chromogenic in situ hybridization (CISH) and silver in situ hybridization (SISH).

**21.** The method of any one of claims 2 to 20, wherein the subject is a human.

**22.** Use of a CDK9 inhibitor as defined in any one of claims 1 to 12 for the preparation of a pharmaceutical composition for the treatment, amelioration and/or prevention of midline carcinoma.

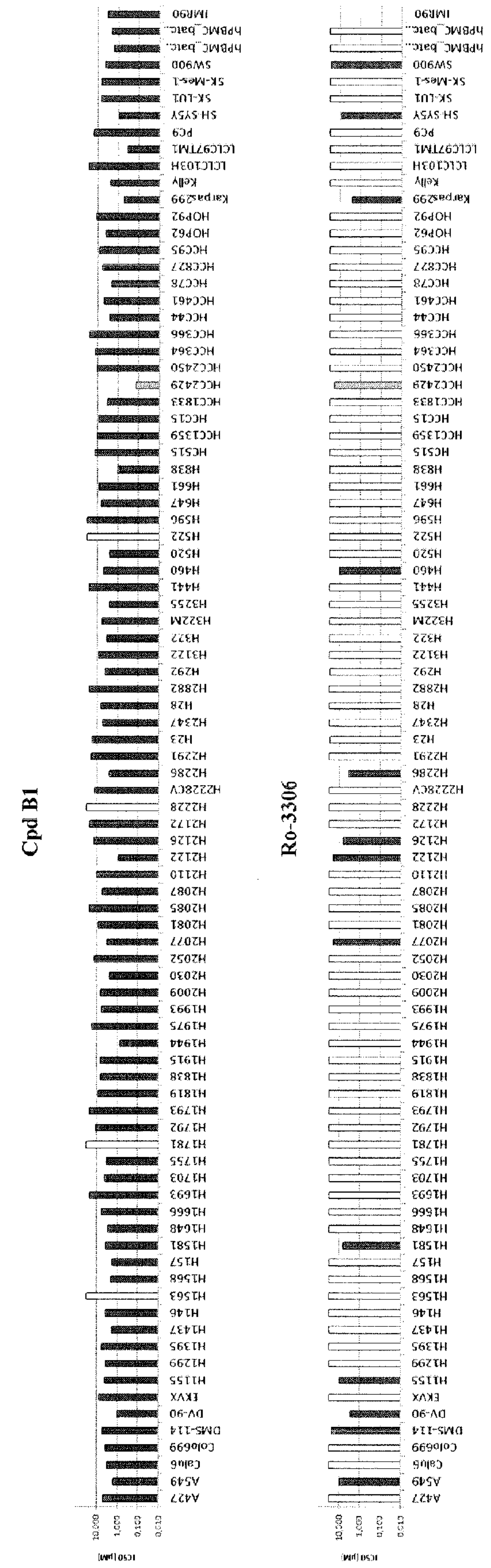
Cpd B1
Ro-3306
IC50 [µM]
10.000
1.000
0.100
0.010
A427
A549
Calu6
Colo699
DMS-114
DV-90
EKVX
H1155
H1299
H1395
H1437
H146
H1563
H1568
H157
H1581
H1648
H1666
H1693
H1703
H1755
H1781
H1792
H1793
H1819
H1838
H1915
H1944
H1975
H1993
H2009
H2030
H2052
H2077
H2081
H2085
H2087
H2110
H2122
H2126
H2172
H2228
H2228CV
H2286
H2291
H23
H2347
H28
H2882
H292
H3122
H322
H322M
H3255
H441
H460
H520
H522
H596
H647
H661
H838
HCS15
HCC1359
HCC15
HCC1833
HCC2429
HCC2450
HCC364
HCC366
HCC44
HCC461
HCC78
HCC827
HCC95
HOP62
HOP92
Karpas299
Kelly
LCLC103H
LCLC97TM1
PC9
SH-SY5Y
SK-LU1
SK-Mes-1
SW900
hPBMC_batc...
hPBMC_batc...
IMR90

Figure 1.

**Figure 2.**

| **trivial name** | **specificity** | **CDK1 LANCE Assay IC50 [µM]** | **CDK2 LANCE Assay IC50 [µM]** | **CDK4 LANCE Assay IC50 [µM]** | **CDK6 LANCE Assay IC50 [µM]** | **CDK7 LANCE Assay IC50 [µM]** | **CDK9 LANCE Assay IC50 [µM]** |
|---|---|---|---|---|---|---|---|
| SNS-032 | CDK | 0,047 | 0,007 | 0,067 | 1,357 | 0,058 | 0,005 |
| flavopiridol | CDK1, CDK2, CDK9 | 0,037 | 0,053 | 0,211 | 0,793 | 0,246 | 0,016 |
| AX35427 | CDK1, CDK2, CDK9 | 0,455 | 0,291 | >1 | >1 | >1 | 0,007 |
| R-547 | CDKs -CDK7 | 0,005 | 0,005 | 0,005 | 0,011 | 0,149 | 0,010 |
| 1073485-20-7P | CDK9 | 5,505 | 2,780 | 5,929 | >10 | >10 | 0,111 |
| Ro-3306 | CDK1 | | | | | | |
| AX38679 | CDK9 | 3,744 | 0,545 | 6,953 | >10 | >10 | 0,030 |
| Cpd 24 | CDK9 | 2,469 | 0,831 | >10 | >10 | >10 | 0,015 |
| Cpd C1 | CDK9 | 1,349 | 0,781 | 1,505 | >10 | >10 | 0,021 |
| Cpd B1 | CDK9 | 12,050 | 3,083 | >10 | >10 | >10 | 0,096 |
| PHA767491 | CDK9, CHK1 | 0,853 | 0,280 | >10 | >10 | >10 | 0,031 |
| Cpd B2 | CDK9 | 0,899 | 0,568 | 3,205 | >10 | >10 | 0,011 |
| PD332991 | CDK4, CDK6 | | | | | | |
| BS-181 | CDK7 | 21,060 | 3,178 | >10 | >10 | 0,057 | 1,944 |

**Figure 3.**

| Compound | 1073485-20-7P | Cpd B1 |
|---|---|---|
| ABL1 wt | 92 | 61 |
| AKT1 | 114 | 104 |
| Aurora-A | 89 | 52 |
| CDK9/CycT | 3 | 1 |
| CK2-alpha1 | 97 | 97 |
| EGF-R wt | 87 | 47 |
| EPHB4 | 101 | 76 |
| FGF-R1 wt | 101 | 74 |
| FLT3 wt | 59 | 63 |
| GSK3-beta | 77 | 64 |
| IGF1-R | 105 | 97 |
| IKK-beta | 104 | 95 |
| JNK1 | 107 | 107 |
| LCK | 97 | 80 |
| MAPKAPK3 | 93 | 86 |
| MEK1 wt | 106 | 89 |
| MET | 103 | 96 |
| PDGFR-beta | 85 | 52 |
| PLK1 | 109 | 109 |
| p38-alpha | 111 | 99 |
| ROCK2 | 105 | 109 |
| TGFB-R1 | 104 | 92 |
| VEGF-R2 | 105 | 63 |

**Figure 4.**

| trivial name | specificity | HCC2429 IC50 [µM] | TY-82 IC50 [µM] | 690100 IC50 [µM] | 143100 IC50 [µM] | Hela IC50 [µM] |
|---|---|---|---|---|---|---|
| SNS-032 | CDK | <0,014 | <0,014 | <0,014 | <0,014 | 0,209 |
| flavopiridol | CDK1, CDK2, CDK9 | 0,021 | 0,035 | 0,058 | 0,020 | 0,364 |
| AX35427 | CDK1, CDK2, CDK9 | 0,046 | 0,167 | 0,420 | 0,078 | 2,725 |
| R-547 | CDKs -CDK7 | 0,020 | 0,236 | 0,210 | 0,024 | 2,528 |
| 1073485-20-7P | CDK9 | 1,299 | 1,385 | 4,438 | 1,691 | 26,060 |
| Ro-3306 | CDK1 | 4,755 | 3,254 | 6,220 | 4,079 | 12,457 |
| AX38679 | CDK9 | 0,146 | 0,447 | 0,711 | 0,197 | 4,166 |
| Cpd 24 | CDK9 | 0,030 | 0,091 | 0,144 | 0,046 | 0,615 |
| Cpd C1 | CDK9 | 0,022 | 0,061 | 0,099 | 0,036 | 0,873 |
| Cpd B1 | CDK9 | 0,151 | 0,321 | 0,580 | 0,202 | 4,193 |
| PHA767491 | CDK9, CHK1 | 0,067 | 0,318 | 0,318 | 0,097 | 1,761 |
| Cpd B2 | CDK9 | 0,026 | 0,072 | 0,114 | 0,044 | 0,805 |
| PD332991 | CDK4, CDK6 | 6,440 | 4,316 | 6,607 | 0,356 | >30 |
| BS-181 | CDK7 | 16,866 | 8,911 | 13,695 | 14,259 | >30 |

**Figure 4 (cont.)**

| trivial name | specificity | hPBMCs IC50 [µM] | HCC366 IC50 [µM] | H460 IC50 [µM] | HCC1143 IC50 [µM] |
|---|---|---|---|---|---|
| SNS-032 | CDK | 0,065 | 0,353 | 0,052 | 0,171 |
| flavopiridol | CDK1, CDK2, CDK9 | 0,307 | 0,745 | 0,075 | 0,551 |
| AX35427 | CDK1, CDK2, CDK9 | 3,578 | 10,202 | 0,817 | 6,748 |
| R-547 | CDKs -CDK7 | 3,230 | 4,975 | 0,019 | 6,699 |
| 1073485-20-7P | CDK9 | 15,861 | >30 | 12,532 | 29,727 |
| Ro-3306 | CDK1 | 23,723 | 25,365 | 2,157 | >30 |
| AX38679 | CDK9 | 3,533 | 15,909 | 2,466 | 8,602 |
| Cpd 24 | CDK9 | 0,549 | 1,807 | 0,209 | 2,511 |
| Cpd C1 | CDK9 | 0,635 | 16,905 | 0,868 | 4,698 |
| Cpd B1 | CDK9 | 3,462 | >30 | 4,508 | 7,431 |
| PHA767491 | CDK9, CHK1 | 1,077 | >30 | 9,508 | 4,577 |
| Cpd B2 | CDK9 | 0,825 | >30 | 2,698 | 2,325 |
| PD332991 | CDK4, CDK6 | >30 | >30 | 0,513 | >30 |
| BS-181 | CDK7 | 28,702 | >30 | 19,102 | >30 |

Figure 5.

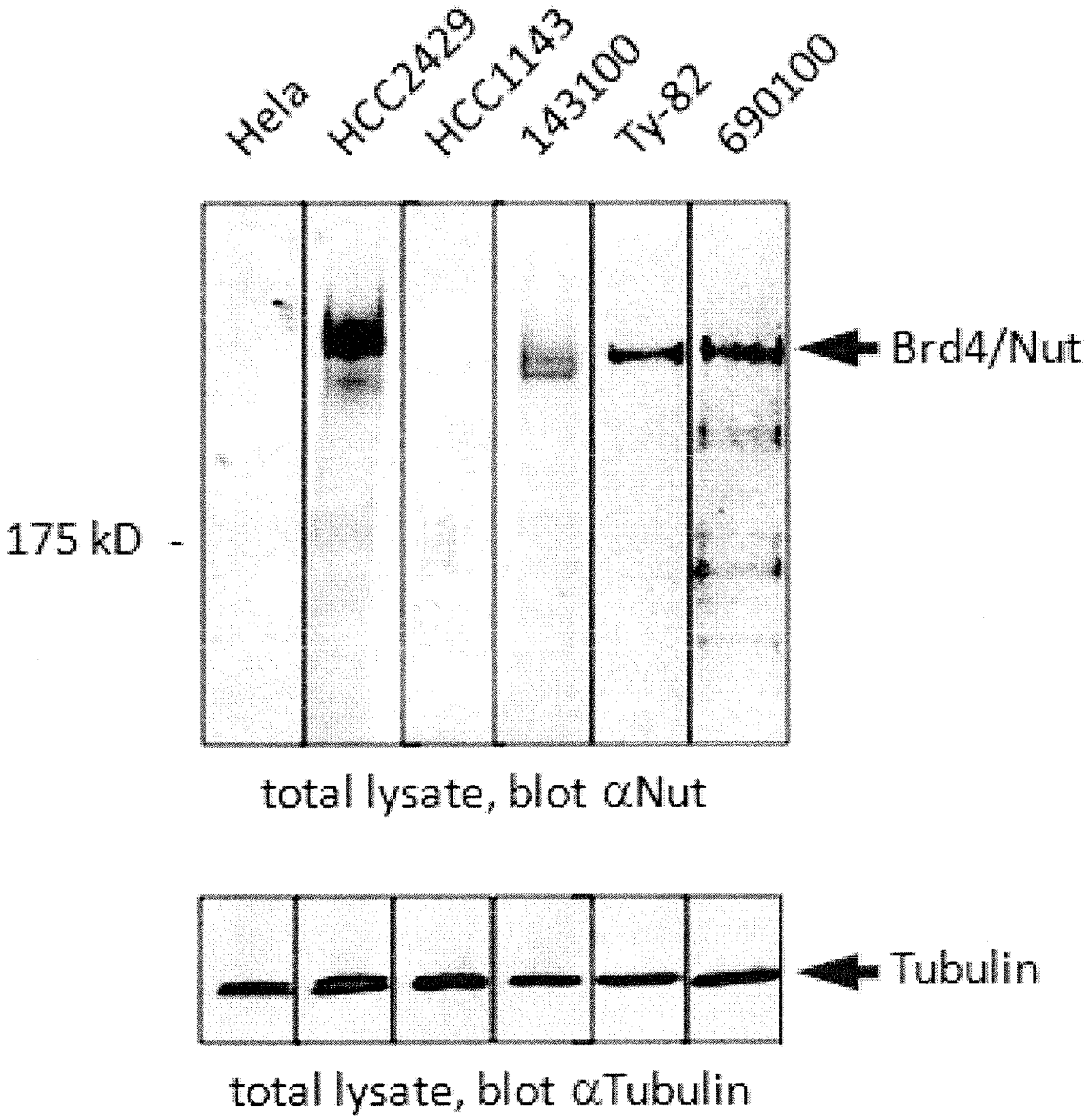
Hela
HCC2429
HCC1143
143100
Ty-82
690100
175 kD -
Brd4/Nut
total lysate, blot αNut
Tubulin
total lysate, blot αTubulin

Europäisches Patentamt
European Patent Office
Office européen des brevets

# EUROPEAN SEARCH REPORT

Application Number
EP 11 17 8328

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | WO 2011/116951 A1 (LEAD DISCOVERY CT GMBH [DE]; BAYER PHARMA AG [DE]; EICKHOFF JAN [DE];) 29 September 2011 (2011-09-29) * claims * ----- | 1-22 | INV. A61K31/00 A61K31/4418 A61K31/53 |
| A | WO 2010/011700 A2 (BRIGHAM & WOMENS HOSPITAL [US]; DANA FARBER CANCER INST INC [US]; FREN) 28 January 2010 (2010-01-28) * claims * ----- | 1-22 | ADD. A61P35/00 |
| A | J. YAN ET AL: "Perturbation of BRD4 Protein Function by BRD4-NUT Protein Abrogates Cellular Differentiation in NUT Midline Carcinoma", JOURNAL OF BIOLOGICAL CHEMISTRY, vol. 286, no. 31, 5 August 2011 (2011-08-05), pages 27663-27675, XP55021164, ISSN: 0021-9258, DOI: 10.1074/jbc.M111.246975 * p. 27669, first of right col.; Fig. 8 * ----- | 1-22 | |
| | | | TECHNICAL FIELDS SEARCHED (IPC) |
| X | WO 2009/047359 A1 (INGENIUM PHARMACEUTICALS GMBH [DE]; ALLGEIER HANS [DE]; AUGUSTIN MARTI) 16 April 2009 (2009-04-16) * p. 1, first ; example 8 * ----- | 22 | A61K |

The present search report has been drawn up for all claims

| Place of search | Date of completion of the search | Examiner |
|---|---|---|
| Munich | 25 April 2012 | Dahse, Thomas |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 17 8328

This annex lists the patent family members relating to the patent documents cited in the above-mentioned European search report.
The members are as contained in the European Patent Office EDP file on
The European Patent Office is in no way liable for these particulars which are merely given for the purpose of information.

25-04-2012

| Patent document cited in search report | Publication date | Patent family member(s) | Publication date |
|---|---|---|---|
| WO 2011116951 A1 | 29-09-2011 | UY 33286 A | 31-10-2011 |
| | | WO 2011116951 A1 | 29-09-2011 |
| WO 2010011700 A2 | 28-01-2010 | US 2011213012 A1 | 01-09-2011 |
| | | WO 2010011700 A2 | 28-01-2010 |
| WO 2009047359 A1 | 16-04-2009 | AT 510827 T | 15-06-2011 |
| | | AU 2008309517 A1 | 16-04-2009 |
| | | CA 2702008 A1 | 16-04-2009 |
| | | CN 101889004 A | 17-11-2010 |
| | | DK 2212297 T3 | 05-09-2011 |
| | | EA 201000554 A1 | 29-04-2011 |
| | | EP 2212297 A1 | 04-08-2010 |
| | | HR 20110470 T1 | 31-07-2011 |
| | | JP 2011500539 A | 06-01-2011 |
| | | KR 20100090772 A | 17-08-2010 |
| | | NZ 584454 A | 30-09-2011 |
| | | SI 2212297 T1 | 30-09-2011 |
| | | US 2010249149 A1 | 30-09-2010 |
| | | WO 2009047359 A1 | 16-04-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010011700 A
- EP 2011001445 W
- EP 10075131 A
- EP 11075037 A
- EP 11075038 A
- WO 2009047359 A
- WO 2010003133 A
- WO 200879933 A
- WO 2011012661 A
- WO 2005026129 A
- WO 547 R
- WO 2008132138 A
- WO 9720842 A1
- WO 2010020618 A
- WO 2010020619 A


### Non-patent literature cited in the description


- **FRENCH.** *J Clin Oncology,* 2004, vol. 22 (20), 4135-4139
- **FRENCH.** *J Clin Pathol,* 2010, vol. 63, 492-496
- **KUZUME.** *Int J Cancer,* 1992, vol. 50, 259-264
- **FRENCH.** *Am J Pathol,* 2001, vol. 159 (6), 1987-1992
- **FRENCH.** *Cancer Genetics and Cytogenetics,* 2010, vol. 203, 16-20
- **ZIAI.** *Head and Neck Pathol,* 2010, vol. 4, 163-168
- **FRENCH.** *Cancer Res,* 2003, vol. 63, 304-307
- **FRENCH.** *Oncogene,* 2008, vol. 27, 2237-2242
- **FRENCH.** *J Clin Pathol,* 2010
- **HAACK.** *Am J Surg Pathol,* 2009, vol. 33 (7), 984-991
- **SCHWARTZ.** *Cancer Res,* 2011, vol. 71 (7), 2686-2696
- **ALSARRAJ.** *Cancer Res,* 2011
- **MOSHINSKY DJ ; RUSLIM L ; BLAKE RA ; TANG F.** *J Biomol Screen,* August 2003, vol. 8 (4), 447-52
- *J. Org. Chem.,* 1995, vol. 60, 8428-8430
- **KRYSTOF.** *Medicinal Research Reviews,* 2009
- **MISRA RN et al.** *J Med Chem.,* 2004, vol. 47 (7), 1719-28
- **LLOYD R KELLAND.** *Expert Opinion on Investigational Drugs,* 2000, vol. 9 (12), 2903-2911
- **DEPINTO W et al.** *Mol Cancer Ther,* 2006, vol. 5, 2644-2658
- **MONTAGNOLI, A.** *Nat Chem Biol,* 2008, vol. 4 (6), 357-365
- **ALI S et al.** *Cancer Res.,* 2009, vol. 69 (15), 6208-15
- **MANCEBO HS ; LEE G ; FLYGARE J ; TOMASSINI J ; LUU P ; ZHU Y ; PENG J ; BLAU C ; HAZUDA D ; PRICE D.** P-TEFb kinase is required for HIV Tat transcriptional activation in vivo and in vitro. *Genes Dev,* 1997, vol. 11, 2633-2644
- **ZHANG C ; LUNDGREN K ; YAN Z ; ARANGO ME ; PRICE S ; HUBER A ; HIGGINS J ; TROCHE G ; SKAPTASON J ; KOUDRIAKOVA T.** Pharmacologic properties of AG-012986, a pan-cyclin-dependent kinase inhibitor with antitumor efficacy. *Mol Cancer Ther,* 2008, vol. 7, 818-828
- **JOSHI, KALPANA S. ; RATHOS, MAGGIE J. ; JOSHI, RAJENDRA D. ; SIVAKUMAR, MEENAKSHI ; MASCARENHAS, MALCOLM ; KAMBLE, SHRIKANT ; LAL, BANSI ; SHARMA, SOMESH.** In vitro antitumor properties of a novel cyclin-dependent kinase inhibitor, P276-00. *Molecular Cancer Therapeutics,* 2007, vol. 6 (3), 918-925
- **G.; LUECKING, U. ; WAGNER, C. ; DETJEN, K. ; MC COY, C. ; BOSSLET, KLAUS.** Molecular and pharmacodynamic characteristics of the novel multi-target tumor growth inhibitor ZK304709. *Biomedicine & Pharmacotherapy,* 2006, vol. 60 (6), 269-272
- **HEUER TS.** Discovery of Selective CDK9 Small Molecule Inhibitors: CDK9 Inhibition in Tumor Cells is Associated with Inhibition of Proliferation and Induction of Apoptosis. *AACR-NCIEORTC International Conference on Molecular Targets and Cancer Therapeutics,* 21 October 2008

- **SQUIRES MS ; FELTELL RE ; LOCK V ; SMITH D ; LEWIS EJ ; HIGGINS J ; YULE M ; THOMPSON NT ; COOKE L ; CROCE DELLA K.** AT7519, a potent CDK inhibitor, is active in leukemia models and primary CLL patient samples. *49th Annual Meeting and Exposition of American Society for Hematology,* 08 December 2007
- **JONES CD ; ANDREWS DM ; BARKER AJ ; BLADES K ; DAUNT P ; EAST S ; GEH C ; GRAHAM MA ; JOHNSON KM ; LODDICK SA.** The discovery of AZD5597, a potent imidazole pyrimidine amide CDK inhibitor suitable for intravenous dosing. *Bioorg Med Chem Lett,* 2008, vol. 18, 6369-6373
- **KRYSTOF V ; CHAMRÁD I ; JORDA R ; KOHOUTEK J.** Pharmacological targeting of CDK9 in cardiac hypertrophy. *Med Res Rev.,* July 2010, vol. 30 (4), 646-66
- **MCMILLIN, DOUGLAS W. ; DELMORE, JAKE ; NEGRI, JOSEPH ; BUON, LEUTZ ; JACOBS, HANNAH M. ; LAUBACH, JACOB ; JAKUBIKOVA, JANA ; OOI, MELISSA ; HAYDEN, PATRICK ; SCHLOSSMAN, ROBERT.** Molecular and cellular effects of multi-targeted cyclin-dependent kinase inhibition in myeloma: biological and clinical implications. *British Journal of Haematology,* 2011, vol. 152 (4), 420-432
- **ELBASHIR.** *Nature,* 2001, vol. 411, 494-498
- **PADDISON.** *Genes Dev.,* 2002, vol. 16, 948-958
- **ELBASHIR.** *Methods,* 2002, vol. 26, 199-213
- **NOVINA.** *Mat. Med.,* 03 June 2002
- **DONZE.** *Nucl. Acids Res.,* 2002, vol. 30, e46
- **PAUL.** *Nat. Biotech,* 2002, vol. 20, 505-508
- **LEE.** *Nat. Biotech.,* 2002, vol. 20, 500-505
- **MIYAGASHI.** *Nat. Biotech.,* 2002, vol. 20, 497-500
- **YU.** *PNAS,* 2002, vol. 99, 6047-6052
- **BRUMMELKAMP.** *Science,* 2002, vol. 296, 550-553
- **THOMAS RK et al.** *Nature Med,* 2007
- **THOMAS RK et al.** *Nat Gen,* 2007
- **MARGUERAT S et al.** *Biochem Soc Trans,* 2008
- The Cell. Garland Publishing, Inc,
- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press Oxford, 1985
- **THOMPSON.** *Nucl. Acids Res.,* 1994, vol. 2, 4673-4680
- **BRUTLAG.** *Comp. App. Biosci.,* 1990, vol. 6, 237-245
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **HENIKOFF.** *PNAS,* 1989, vol. 89, 10915
- **FRENCH CA.** *Cancer Genet Cytogenet.,* November 2010, vol. 203 (1), 16-20
- **FRENCH.** *Cancer Genet Cytogenet,* 2010
- **FRENCH.** *Clin Pathol,* 2010
- *Cancer Research,* 1999, vol. 59, 5169-5175
- *Pathol Int,* 1996, vol. 46, 801-804
- **VOGESER.** *Dtsch Arztebl,* 2007, vol. 104 (31-32), A2194-200
- **LLOYD R KELLAND.** *Expert Opinion on Investigational Drugs,* 2000, vol. 9 (12), 2903-2911